# EUROPEAN PATENT SPECIFICATION

(11) **EP 2 911 684 B1**
(45) Date of publication and mention of the grant of the patent: **19.06.2019**
(21) Application number: 13849581.7
(22) Date of filing: 23.10.2013
(51) Int. Cl.: A61K 38/20, A61P 35/00, A61P 35/04, A61P 37/04

(54) **IL-15R ALPHA FORMS, CELLS EXPRESSING IL-15R ALPHA FORMS, AND THERAPEUTIC USES OF IL-15R ALPHA AND IL-15/IL-15R ALPHA COMPLEXES**
IL-15R-ALPHA-FORMEN, IL-15R-ALPHA-FORMEN EXPRIMIERENDE ZELLEN UND THERAPEUTISCHE VERWENDUNG VON IL-15R-ALPHA UND IL-15/IL-15R-ALPHA-KOMPLEXEN
FORMES D'IL-15R ALPHA, CELLULES EXPRIMANT DES FORMES D'IL-15R ALPHA, ET UTILISATIONS THÉRAPEUTIQUES D'IL-15R ALPHA ET DE COMPLEXES IL-15/IL-15R ALPHA

(30) Priority: 24.10.2012 US 201261718169 P; 06.05.2013 US 201361820035 P
(43) Date of publication of application: 02.09.2015
(73) Proprietor: NOVARTIS AG, 4056 Basel (CH); The United States of America, as represented by The Secretary, Department of Health and Human Services, Bethesda, MD 20892-7660 (US)
(72) Inventor: FELBER, Barbara, K., Rockville, MD 20850 (US); FINKIELSZTTEIN, Sergio, Newton, MA 02459 (US); PAVLAKIS, George, N., Rockville, MD 20850 (US); VOURNAKIS, John, N., Charleston, SC 29403 (US)
(74) Representative: Thompson, Sarah Louise
(86) International application number: PCT/US2013/066424
(87) International publication number: WO 2014/066527

(56) References cited:
- WO-A1-2008/143794
- WO-A1-2011/020047
- US-A1- 2007 160 578
- US-A1- 2009 082 299
- US-A1- 2009 238 791
- US-A1- 2011 081 311
- T. A. WALDMANN ET AL: "Safety (toxicity), pharmacokinetics, immunogenicity, and impact on elements of the normal immune system of recombinant human IL-15 in rhesus macaques", BLOOD, vol. 117, no. 18, 5 May 2011 (2011-05-05), pages 4787-4795, XP055251912, US ISSN: 0006-4971, DOI: 10.1182/blood-2010-10-311456
- C. BERGER ET AL: "Safety and immunologic effects of IL-15 administration in nonhuman primates", BLOOD, vol. 114, no. 12, 17 September 2009 (2009-09-17), pages 2417-2426, XP055252042, US ISSN: 0006-4971, DOI: 10.1182/blood-2008-12-189266
- E. CHERTOVA ET AL: "Characterization and Favorable in Vivo Properties of Heterodimeric Soluble IL-15{middle dot}IL-15R Cytokine Compared to IL-15 Monomer", JOURNAL OF BIOLOGICAL CHEMISTRY, vol. 288, no. 25, 6 May 2013 (2013-05-06), pages 18093-18103, XP055134195, ISSN: 0021-9258, DOI: 10.1074/jbc.M113.461756

### SEQUENCE LISTING

<110> Admune Therapeutics LLC United States Department of Health and Human Services
<120> IL-15R ALPHA FORMS, CELLS EXPRESSING IL-15R ALPHA FORMS, AND THERAPEUTIC USES OF IL-15R ALPHA AND IL-15/IL-15R ALPHA COMPLEXEs
<130> 13346-002-228
<140> to be assigned
   <141>
<150> 61/718,169
   <151> 2012-10-24
<150> 61/820,035
   <151> 2013-05-06
<160> 46
<170> FastSEQ for Windows Version 4.0
<210> 1
   <211> 162
   <212> PRT
   <213> Homo sapiens
<220>
   <221> SIGNAL
   <222> (1)...(48)
<220>
   <223> immature/precursor form of native human IL-15
<400> 1
   Met Arg Ile Ser Lys Pro His Leu Arg Ser Ile Ser Ile Gln Cys Tyr
    1               5                  10                  15
   Leu Cys Leu Leu Leu Asn Ser His Phe Leu Thr Glu Ala Gly Ile His
               20                  25                  30
   Val Phe Ile Leu Gly Cys Phe Ser Ala Gly Leu Pro Lys Thr Glu Ala
           35                  40                  45
   Asn Trp Val Asn Val Ile Ser Asp Leu Lys Lys Ile Glu Asp Leu Ile
       50                  55                  60
   Gln Ser Met His Ile Asp Ala Thr Leu Tyr Thr Glu Ser Asp Val His
   65                  70                  75                  80
   Pro Ser Cys Lys Val Thr Ala Met Lys Cys Phe Leu Leu Glu Leu Gln
                   85                  90                  95
   Val Ile Ser Leu Glu Ser Gly Asp Ala Ser Ile His Asp Thr Val Glu
               100                 105                 110
   Asn Leu Ile Ile Leu Ala Asn Asn Ser Leu Ser Ser Asn Gly Asn Val
           115                 120                 125
   Thr Glu Ser Gly Cys Lys Glu Cys Glu Glu Leu Glu Glu Lys Asn Ile
       130                 135                 140
   Lys Glu Phe Leu Gln Ser Phe Val His Ile Val Gln Met Phe Ile Asn
   145                 150                 155                 160
   Thr Ser
<210> 2
   <211> 489
   <212> DNA
   <213> Homo sapiens
<220>
   <221> sig_peptide
   <222> (1)...(145)
<220>
   <223> coding sequence of immature/precursor form of native human IL-15
<400> 2
   atgagaattt cgaaaccaca tttgagaagt atttccatcc agtgctactt gtgtttactt 60
   ctaaacagtc attttctaac tgaagctggc attcatgtct tcattttggg ctgtttcagt 120
   gcagggcttc ctaaaacaga agccaactgg gtgaatgtaa taagtgattt gaaaaaaatt 180
   gaagatctta ttcaatctat gcatattgat gctactttat atacggaaag tgatgttcac 240
   cccagttgca aagtaacagc aatgaagtgc tttctcttgg agttacaagt tatttcactt 300
   gagtccggag atgcaagtat tcatgataca gtagaaaatc tgatcatcct agcaaacaac 360
   agtttgtctt ctaatgggaa tgtaacagaa tctggatgca aagaatgtga ggaactggag 420
   gaaaaaaata ttaaagaatt tttgcagagt tttgtacata ttgtccaaat gttcatcaac 480
   acttcttga                                                         489
<210> 3
   <211> 267
   <212> PRT
   <213> Homo sapiens
<220>
   <221> SIGNAL
   <222> (1)...(30)
<220>
   <223> immature form of the native full length human IL-15 receptor alpha
<400> 3
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr Val Ala Ile
           195                 200                 205
   Ser Thr Ser Thr Val Leu Leu Cys Gly Leu Ser Ala Val Ser Leu Leu
       210                 215                 220
   Ala Cys Tyr Leu Lys Ser Arg Gln Thr Pro Pro Leu Ala Ser Val Glu
   225                 230                 235                 240
   Met Glu Ala Met Glu Ala Leu Pro Val Thr Trp Gly Thr Ser Ser Arg
                   245                 250                 255
   Asp Glu Asp Leu Glu Asn Cys Ser His His Leu
               260                 265
<210> 4
   <211> 205
   <212> PRT
   <213> Homo sapines
<220>
   <221> SIGNAL
   <222> (1)...(30)
<220>
   <223> immature form of the native soluble human IL-15 receptor alpha
<400> 4
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr
           195                 200                 205
<210> 5
   <211> 804
   <212> DNA
   <213> Homo sapiens
<220>
   <221> sig_peptide
   <222> (1)...(90)
<220>
   <223> coding sequence of immature form of the native full length human IL-15 receptor alpha
<400> 5
   atggccccgc ggcgggcgcg cggctgccgg accctcggtc tcccggcgct gctactgctg 60
   ctgctgctcc ggccgccggc gacgcggggc atcacgtgcc ctccccccat gtccgtggaa 120
   cacgcagaca tctgggtcaa gagctacagc ttgtactcca gggagcggta catttgtaac 180
   tctggtttca agcgtaaagc cggcacgtcc agcctgacgg agtgcgtgtt gaacaaggcc 240
   acgaatgtcg cccactggac aacccccagt ctcaaatgca ttagagaccc tgccctggtt 300
   caccaaaggc cagcgccacc ctccacagta acgacggcag gggtgacccc acagccagag 360
   agcctctccc cttctggaaa agagcccgca gcttcatctc ccagctcaaa caacacagcg 420
   gccacaacag cagctattgt cccgggctcc cagctgatgc cttcaaaatc accttccaca 480
   ggaaccacag agataagcag tcatgagtcc tcccacggca ccccctctca gacaacagcc 540
   aagaactggg aactcacagc atccgcctcc caccagccgc caggtgtgta tccacagggc 600
   cacagcgaca ccactgtggc tatctccacg tccactgtcc tgctgtgtgg gctgagcgct 660
   gtgtctctcc tggcatgcta cctcaagtca aggcaaactc ccccgctggc cagcgttgaa 720
   atggaagcca tggaggctct gccggtgact tgggggacca gcagcagaga tgaagacttg 780
   gaaaactgct ctcaccacct atga                                        804
<210> 6
   <211> 615
   <212> DNA
   <213> Homo sapiens
<220>
   <221> sig_peptide
   <222> (1)...(90)
<220>
   <223> coding sequence of immature form of the native soluble human IL-15 receptor alpha
<400> 6
   atggccccgc ggcgggcgcg cggctgccgg accctcggtc tcccggcgct gctactgctg 60
   ctgctgctcc ggccgccggc gacgcggggc atcacgtgcc ctccccccat gtccgtggaa 120
   cacgcagaca tctgggtcaa gagctacagc ttgtactcca gggagcggta catttgtaac 180
   tctggtttca agcgtaaagc cggcacgtcc agcctgacgg agtgcgtgtt gaacaaggcc 240
   acgaatgtcg cccactggac aacccccagt ctcaaatgca ttagagaccc tgccctggtt 300
   caccaaaggc cagcgccacc ctccacagta acgacggcag gggtgacccc acagccagag 360
   agcctctccc cttctggaaa agagcccgca gcttcatctc ccagctcaaa caacacagcg 420
   gccacaacag cagctattgt cccgggctcc cagctgatgc cttcaaaatc accttccaca 480
   ggaaccacag agataagcag tcatgagtcc tcccacggca ccccctctca gacaacagcc 540
   aagaactggg aactcacagc atccgcctcc caccagccgc caggtgtgta tccacagggc 600
   cacagcgaca ccact                                                 615
<210> 7
   <211> 4
   <212> PRT
   <213> Artificial Sequence
<220>
   <223> heterologous protease cleavage sites recognized by furin protease
<220>
   <221> VARIANT
   <222> 2,3
   <223> Xaa = any amino acid
<400> 7
   Arg Xaa Xaa Arg
   1
<210> 8
   <211> 6
   <212> PRT
   <213> Artificial Sequence
<220>
   <223> heterologous protease cleavage sites recognized by thrombin protease
<220>
   <221> VARIANT
   <222> 1,2
   <223> Xaa = hydrophobic amino acids
<220>
   <221> VARIANT
   <222> 5,6
   <223> Xaa = nonacidic amino acids
<400> 8
   Xaa Xaa Pro Arg Xaa Xaa
   1               5
<210> 9
   <211> 1847
   <212> DNA
   <213> Artificial Sequence
<220>
   <223> huIL15opt - nucleic acid construct encoding optimized human IL-15
<400> 9
   cctggccatt gcatacgttg tatccatatc ataatatgta catttatatt ggctcatgtc 60
   caacattacc gccatgttga cattgattat tgactagtta ttaatagtaa tcaattacgg 120
   ggtcattagt tcatagccca tatatggagt tccgcgttac ataacttacg gtaaatggcc 180
   cgcctggctg accgcccaac gacccccgcc cattgacgtc aataatgacg tatgttccca 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   atggtaaatg gcccgcctgg cattatgccc agtacatgac cttatgggac tttcctactt 420
   ggcagtacat ctacgtatta gtcatcgcta ttaccatggt gatgcggttt tggcagtaca 480
   tcaatgggcg tggatagcgg tttgactcac ggggatttcc aagtctccac cccattgacg 540
   tcaatgggag tttgttttgg caccaaaatc aacgggactt tccaaaatgt cgtaacaact 600
   ccgccccatt gacgcaaatg ggcggtaggc gtgtacggtg ggaggtctat ataagcagag 660
   ctcgtttagt gaaccgtcag atcgcctgga gacgccatcc acgctgtttt gacctccata 720
   gaagacaccg ggaccgatcc agcctccgcg ggcgcgcgtc gacaagaaat gcggatctcg 780
   aagccgcacc tgcggtcgat atcgatccag tgctacctgt gcctgctcct gaactcgcac 840
   ttcctcacgg aggccggtat acacgtcttc atcctgggct gcttctcggc ggggctgccg 900
   aagacggagg cgaactgggt gaacgtgatc tcggacctga agaagatcga ggacctcatc 960
   cagtcgatgc acatcgacgc gacgctgtac acggagtcgg acgtccaccc gtcgtgcaag 1020
   gtcacggcga tgaagtgctt cctcctggag ctccaagtca tctcgctcga gtcgggggac 1080
   gcgtcgatcc acgacacggt ggagaacctg atcatcctgg cgaacaactc gctgtcgtcg 1140
   aacgggaacg tcacggagtc gggctgcaag gagtgcgagg agctggagga gaagaacatc 1200
   aaggagttcc tgcagtcgtt cgtgcacatc gtccagatgt tcatcaacac gtcgtgaggg 1260
   cccggcgcgc cgaattcgcg gatatcggtt aacggatcca gatctgctgt gccttctagt 1320
   tgccagccat ctgttgtttg cccctccccc gtgccttcct tgaccctgga aggtgccact 1380
   cccactgtcc tttcctaata aaatgaggaa attgcatcgc attgtctgag taggtgtcat 1440
   tctattctgg ggggtggggt ggggcaggac agcaaggggg aggattggga agacaatagc 1500
   aggcatgctg gggatgcggt gggctctatg ggtacccagg tgctgaagaa ttgacccggt 1560
   tcctcctggg ccagaaagaa gcaggcacat ccccttctct gtgacacacc ctgtccacgc 1620
   ccctggttct tagttccagc cccactcata ggacactcat agctcaggag ggctccgcct 1680
   tcaatcccac ccgctaaagt acttggagcg gtctctccct ccctcatcag cccaccaaac 1740
   caaacctagc ctccaagagt gggaagaaat taaagcaaga taggctatta agtgcagagg 1800
   gagagaaaat gcctccaaca tgtgaggaag taatgagaga aatcata                1847
<210> 10
   <211> 162
   <212> PRT
   <213> Artificial Sequence
<220>
   <223> huIL15opt - amino acid sequence of optimized human IL-15
<400> 10
   Met Arg Ile Ser Lys Pro His Leu Arg Ser Ile Ser Ile Gln Cys Tyr
    1               5                  10                  15
   Leu Cys Leu Leu Leu Asn Ser His Phe Leu Thr Glu Ala Gly Ile His
               20                  25                  30
   Val Phe Ile Leu Gly Cys Phe Ser Ala Gly Leu Pro Lys Thr Glu Ala
           35                  40                  45
   Asn Trp Val Asn Val Ile Ser Asp Leu Lys Lys Ile Glu Asp Leu Ile
       50                  55                  60
   Gln Ser Met His Ile Asp Ala Thr Leu Tyr Thr Glu Ser Asp Val His
   65                  70                  75                  80
   Pro Ser Cys Lys Val Thr Ala Met Lys Cys Phe Leu Leu Glu Leu Gln
                   85                  90                  95
   Val Ile Ser Leu Glu Ser Gly Asp Ala Ser Ile His Asp Thr Val Glu
               100                 105                 110
   Asn Leu Ile Ile Leu Ala Asn Asn Ser Leu Ser Ser Asn Gly Asn Val
           115                 120                 125
   Thr Glu Ser Gly Cys Lys Glu Cys Glu Glu Leu Glu Glu Lys Asn Ile
       130                 135                 140
   Lys Glu Phe Leu Gln Ser Phe Val His Ile Val Gln Met Phe Ile Asn
   145                 150                 155                 160
   Thr Ser
<210> 11
   <211> 1808
   <212> DNA
   <213> Artificial Sequence
<220>
   <223> CMV huIL15tPA6 - nucleic acid construct encoding optimized human IL-15
<400> 11
   cctggccatt gcatacgttg tatccatatc ataatatgta catttatatt ggctcatgtc 60
   caacattacc gccatgttga cattgattat tgactagtta ttaatagtaa tcaattacgg 120
   ggtcattagt tcatagccca tatatggagt tccgcgttac ataacttacg gtaaatggcc 180
   cgcctggctg accgcccaac gacccccgcc cattgacgtc aataatgacg tatgttccca 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   atggtaaatg gcccgcctgg cattatgccc agtacatgac cttatgggac tttcctactt 420
   ggcagtacat ctacgtatta gtcatcgcta ttaccatggt gatgcggttt tggcagtaca 480
   tcaatgggcg tggatagcgg tttgactcac ggggatttcc aagtctccac cccattgacg 540
   tcaatgggag tttgttttgg caccaaaatc aacgggactt tccaaaatgt cgtaacaact 600
   ccgccccatt gacgcaaatg ggcggtaggc gtgtacggtg ggaggtctat ataagcagag 660
   ctcgtttagt gaaccgtcag atcgcctgga gacgccatcc acgctgtttt gacctccata 720
   gaagacaccg ggaccgatcc agcctccgcg ggcgcgcgtc gacaagaaat ggatgcaatg 780
   aagagagggc tctgctgtgt gctgctgctg tgtggagcag tcttcgtttc gcccagccag 840
   gaaatccatg cccgattcag aagaggagcc agaaactggg tgaacgtgat ctcggacctg 900
   aagaagatcg aggacctcat ccagtcgatg cacatcgacg cgacgctgta cacggagtcg 960
   gacgtccacc cgtcgtgcaa ggtcacggcg atgaagtgct tcctcctgga gctccaagtc 1020
   atctcgctcg agtcggggga cgcgtcgatc cacgacacgg tggagaacct gatcatcctg 1080
   gcgaacaact cgctgtcgtc gaacgggaac gtcacggagt cgggctgcaa ggagtgcgag 1140
   gagctggagg agaagaacat caaggagttc ctgcagtcgt tcgtgcacat cgtccagatg 1200
   ttcatcaaca cgtcgtgagg gcccggcgcg ccgaattcgc ggatatcggt taacggatcc 1260
   agatctgctg tgccttctag ttgccagcca tctgttgttt gcccctcccc cgtgccttcc 1320
   ttgaccctgg aaggtgccac tcccactgtc ctttcctaat aaaatgagga aattgcatcg 1380
   cattgtctga gtaggtgtca ttctattctg gggggtgggg tggggcagga cagcaagggg 1440
   gaggattggg aagacaatag caggcatgct ggggatgcgg tgggctctat gggtacccag 1500
   gtgctgaaga attgacccgg ttcctcctgg gccagaaaga agcaggcaca tccccttctc 1560
   tgtgacacac cctgtccacg cccctggttc ttagttccag ccccactcat aggacactca 1620
   tagctcagga gggctccgcc ttcaatccca cccgctaaag tacttggagc ggtctctccc 1680
   tccctcatca gcccaccaaa ccaaacctag cctccaagag tgggaagaaa ttaaagcaag 1740
   ataggctatt aagtgcagag ggagagaaaa tgcctccaac atgtgaggaa gtaatgagag 1800
   aaatcata                                                          1808
<210> 12
   <211> 149
   <212> PRT
   <213> Artificial Sequence
<220>
   <223> CMV huIL15tPA6 - amino acid sequence of optimized human IL-15
<400> 12
   Met Asp Ala Met Lys Arg Gly Leu Cys Cys Val Leu Leu Leu Cys Gly
   1               5                   10                  15
   Ala Val Phe Val Ser Pro Ser Gln Glu Ile His Ala Arg Phe Arg Arg
               20                  25                  30
   Gly Ala Arg Asn Trp Val Asn Val Ile Ser Asp Leu Lys Lys Ile Glu
           35                  40                  45
   Asp Leu Ile Gln Ser Met His Ile Asp Ala Thr Leu Tyr Thr Glu Ser
       50                  55                  60
   Asp Val His Pro Ser Cys Lys Val Thr Ala Met Lys Cys Phe Leu Leu
   65                  70                  75                  80
   Glu Leu Gln Val Ile Ser Leu Glu Ser Gly Asp Ala Ser Ile His Asp
                   85                  90                  95
   Thr Val Glu Asn Leu Ile Ile Leu Ala Asn Asn Ser Leu Ser Ser Asn
               100                 105                 110
   Gly Asn Val Thr Glu Ser Gly Cys Lys Glu Cys Glu Glu Leu Glu Glu
           115                 120                 125
   Lys Asn Ile Lys Glu Phe Leu Gln Ser Phe Val His Ile Val Gln Met
       130                 135                 140
   Phe Ile Asn Thr Ser
   145
<210> 13
   <211> 2140
   <212> DNA
   <213> Artificial Sequence
<220>
   <223> huIL15Ra - nucleic acid construct encoding optimized human IL-15Ra
<400> 13
   cctggccatt gcatacgttg tatccatatc ataatatgta catttatatt ggctcatgtc 60
   caacattacc gccatgttga cattgattat tgactagtta ttaatagtaa tcaattacgg 120
   ggtcattagt tcatagccca tatatggagt tccgcgttac ataacttacg gtaaatggcc 180
   cgcctggctg accgcccaac gacccccgcc cattgacgtc aataatgacg tatgttccca 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   atggtaaatg gcccgcctgg cattatgccc agtacatgac cttatgggac tttcctactt 420
   ggcagtacat ctacgtatta gtcatcgcta ttaccatggt gatgcggttt tggcagtaca 480
   tcaatgggcg tggatagcgg tttgactcac ggggatttcc aagtctccac cccattgacg 540
   tcaatgggag tttgttttgg caccaaaatc aacgggactt tccaaaatgt cgtaacaact 600
   ccgccccatt gacgcaaatg ggcggtaggc gtgtacggtg ggaggtctat ataagcagag 660
   ctcgtttagt gaaccgtcag atcgcctgga gacgccatcc acgctgtttt gacctccata 720
   gaagacaccg ggaccgatcc agcctccgcg ggcgcgcgtc gacgctagca agaaatggcc 780
   ccgaggcggg cgcgaggctg ccggaccctc ggtctcccgg cgctgctact gctcctgctg 840
   ctccggccgc cggcgacgcg gggcatcacg tgcccgcccc ccatgtccgt ggagcacgca 900
   gacatctggg tcaagagcta cagcttgtac tcccgggagc ggtacatctg caactcgggt 960
   ttcaagcgga aggccggcac gtccagcctg acggagtgcg tgttgaacaa ggccacgaat 1020
   gtcgcccact ggacgacccc ctcgctcaag tgcatccgcg acccggccct ggttcaccag 1080
   cggcccgcgc caccctccac cgtaacgacg gcgggggtga ccccgcagcc ggagagcctc 1140
   tccccgtcgg gaaaggagcc cgccgcgtcg tcgcccagct cgaacaacac ggcggccaca 1200
   actgcagcga tcgtcccggg ctcccagctg atgccgtcga agtcgccgtc cacgggaacc 1260
   acggagatca gcagtcatga gtcctcccac ggcaccccct cgcaaacgac ggccaagaac 1320
   tgggaactca cggcgtccgc ctcccaccag ccgccggggg tgtatccgca aggccacagc 1380
   gacaccacgg tggcgatctc cacgtccacg gtcctgctgt gtgggctgag cgcggtgtcg 1440
   ctcctggcgt gctacctcaa gtcgaggcag actcccccgc tggccagcgt tgagatggag 1500
   gccatggagg ctctgccggt gacgtggggg accagcagca gggatgagga cttggagaac 1560
   tgctcgcacc acctataatg agaattcgat ccagatctgc tgtgccttct agttgccagc 1620
   catctgttgt ttgcccctcc cccgtgcctt ccttgaccct ggaaggtgcc actcccactg 1680
   tcctttccta ataaaatgag gaaattgcat cgcattgtct gagtaggtgt cattctattc 1740
   tggggggtgg ggtggggcag gacagcaagg gggaggattg ggaagacaat agcaggcatg 1800
   ctggggatgc ggtgggctct atgggtaccc aggtgctgaa gaattgaccc ggttcctcct 1860
   gggccagaaa gaagcaggca catccccttc tctgtgacac accctgtcca cgcccctggt 1920
   tcttagttcc agccccactc ataggacact catagctcag gagggctccg ccttcaatcc 1980
   cacccgctaa agtacttgga gcggtctctc cctccctcat cagcccacca aaccaaacct 2040
   agcctccaag agtgggaaga aattaaagca agataggcta ttaagtgcag agggagagaa 2100
   aatgcctcca acatgtgagg aagtaatgag agaaatcata                       2140
<210> 14
   <211> 267
   <212> PRT
   <213> Artificial Sequence
<220>
   <223> huIL15Ra - amino acid sequence of optimized human IL-15Ra
<400> 14
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr Val Ala Ile
           195                 200                 205
   Ser Thr Ser Thr Val Leu Leu Cys Gly Leu Ser Ala Val Ser Leu Leu
       210                 215                 220
   Ala Cys Tyr Leu Lys Ser Arg Gln Thr Pro Pro Leu Ala Ser Val Glu
   225                 230                 235                 240
   Met Glu Ala Met Glu Ala Leu Pro Val Thr Trp Gly Thr Ser Ser Arg
                   245                 250                 255
   Asp Glu Asp Leu Glu Asn Cys Ser His His Leu
               260                 265
<210> 15
   <211> 1971
   <212> DNA
   <213> Artificial Sequence
<220>
   <223> CMV hu sIL15Ra **-** nucleic acid construct encoding optimized human IL-15Ra
<400> 15
   cctggccatt gcatacgttg tatccatatc ataatatgta catttatatt ggctcatgtc 60
   caacattacc gccatgttga cattgattat tgactagtta ttaatagtaa tcaattacgg 120
   ggtcattagt tcatagccca tatatggagt tccgcgttac ataacttacg gtaaatggcc 180
   cgcctggctg accgcccaac gacccccgcc cattgacgtc aataatgacg tatgttccca 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   atggtaaatg gcccgcctgg cattatgccc agtacatgac cttatgggac tttcctactt 420
   ggcagtacat ctacgtatta gtcatcgcta ttaccatggt gatgcggttt tggcagtaca 480
   tcaatgggcg tggatagcgg tttgactcac ggggatttcc aagtctccac cccattgacg 540
   tcaatgggag tttgttttgg caccaaaatc aacgggactt tccaaaatgt cgtaacaact 600
   ccgccccatt gacgcaaatg ggcggtaggc gtgtacggtg ggaggtctat ataagcagag 660
   ctcgtttagt gaaccgtcag atcgcctgga gacgccatcc acgctgtttt gacctccata 720
   gaagacaccg ggaccgatcc agcctccgcg ggcgcgcgtc gacgctagca agaaatggcc 780
   ccgaggcggg cgcgaggctg ccggaccctc ggtctcccgg cgctgctact gctcctgctg 840
   ctccggccgc cggcgacgcg gggcatcacg tgcccgcccc ccatgtccgt ggagcacgca 900
   gacatctggg tcaagagcta cagcttgtac tcccgggagc ggtacatctg caactcgggt 960
   ttcaagcgga aggccggcac gtccagcctg acggagtgcg tgttgaacaa ggccacgaat 1020
   gtcgcccact ggacgacccc ctcgctcaag tgcatccgcg acccggccct ggttcaccag 1080
   cggcccgcgc caccctccac cgtaacgacg gcgggggtga ccccgcagcc ggagagcctc 1140
   tccccgtcgg gaaaggagcc cgccgcgtcg tcgcccagct cgaacaacac ggcggccaca 1200
   actgcagcga tcgtcccggg ctcccagctg atgccgtcga agtcgccgtc cacgggaacc 1260
   acggagatca gcagtcatga gtcctcccac ggcaccccct cgcaaacgac ggccaagaac 1320
   tgggaactca cggcgtccgc ctcccaccag ccgccggggg tgtatccgca aggccacagc 1380
   gacaccacgt aatgagaatt cgcggatatc ggttaacgga tccagatctg ctgtgccttc 1440
   tagttgccag ccatctgttg tttgcccctc ccccgtgcct tccttgaccc tggaaggtgc 1500
   cactcccact gtcctttcct aataaaatga ggaaattgca tcgcattgtc tgagtaggtg 1560
   tcattctatt ctggggggtg gggtggggca ggacagcaag ggggaggatt gggaagacaa 1620
   tagcaggcat gctggggatg cggtgggctc tatgggtacc caggtgctga agaattgacc 1680
   cggttcctcc tgggccagaa agaagcaggc acatcccctt ctctgtgaca caccctgtcc 1740
   acgcccctgg ttcttagttc cagccccact cataggacac tcatagctca ggagggctcc 1800
   gccttcaatc ccacccgcta aagtacttgg agcggtctct ccctccctca tcagcccacc 1860
   aaaccaaacc tagcctccaa gagtgggaag aaattaaagc aagataggct attaagtgca 1920
   gagggagaga aaatgcctcc aacatgtgag gaagtaatga gagaaatcat a          1971
<210> 16
   <211> 205
   <212> PRT
   <213> Artificial Sequence
<220>
   <223> CMV hu sIL15Ra - amino acid sequence of optimized human IL-15Ra
<400> 16
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr
           195                 200                 205
<210> 17
   <211> 1754
   <212> DNA
   <213> Artificial Sequence
<220>
   <223> huIL-15 huGM-CSF - nucleic acid construct encoding optimized human IL-15 with a signal peptide of human GM-CSF
<400> 17
   cctggccatt gcatacgttg tatccatatc ataatatgta catttatatt ggctcatgtc 60
   caacattacc gccatgttga cattgattat tgactagtta ttaatagtaa tcaattacgg 120
   ggtcattagt tcatagccca tatatggagt tccgcgttac ataacttacg gtaaatggcc 180
   cgcctggctg accgcccaac gacccccgcc cattgacgtc aataatgacg tatgttccca 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   atggtaaatg gcccgcctgg cattatgccc agtacatgac cttatgggac tttcctactt 420
   ggcagtacat ctacgtatta gtcatcgcta ttaccatggt gatgcggttt tggcagtaca 480
   tcaatgggcg tggatagcgg tttgactcac ggggatttcc aagtctccac cccattgacg 540
   tcaatgggag tttgttttgg caccaaaatc aacgggactt tccaaaatgt cgtaacaact 600
   ccgccccatt gacgcaaatg ggcggtaggc gtgtacggtg ggaggtctat ataagcagag 660
   ctcgtttagt gaaccgtcag atcgcctgga gacgccatcc acgctgtttt gacctccata 720
   gaagacaccg ggaccgatcc agcctccgcg ggcgcgcgtc gacaagaaat gtggctccag 780
   agcctgctac tcctggggac ggtggcctgc agcatctcga actgggtgaa cgtgatctcg 840
   gacctgaaga agatcgagga cctcatccag tcgatgcaca tcgacgcgac gctgtacacg 900
   gagtcggacg tccacccgtc gtgcaaggtc acggcgatga agtgcttcct cctggagctc 960
   caagtcatct cgctcgagtc gggggacgcg tcgatccacg acacggtgga gaacctgatc 1020
   atcctggcga acaactcgct gtcgtcgaac gggaacgtca cggagtcggg ctgcaaggag 1080
   tgcgaggagc tggaggagaa gaacatcaag gagttcctgc agtcgttcgt gcacatcgtc 1140
   cagatgttca tcaacacgtc gtgagggccc ggcgcgccga attcgcggat atcggttaac 1200
   ggatccagat ctgctgtgcc ttctagttgc cagccatctg ttgtttgccc ctcccccgtg 1260
   ccttccttga ccctggaagg tgccactccc actgtccttt cctaataaaa tgaggaaatt 1320
   gcatcgcatt gtctgagtag gtgtcattct attctggggg gtggggtggg gcaggacagc 1380
   aagggggagg attgggaaga caatagcagg catgctgggg atgcggtggg ctctatgggt 1440
   acccaggtgc tgaagaattg acccggttcc tcctgggcca gaaagaagca ggcacatccc 1500
   cttctctgtg acacaccctg tccacgcccc tggttcttag ttccagcccc actcatagga 1560
   cactcatagc tcaggagggc tccgccttca atcccacccg ctaaagtact tggagcggtc 1620
   tctccctccc tcatcagccc accaaaccaa acctagcctc caagagtggg aagaaattaa 1680
   agcaagatag gctattaagt gcagagggag agaaaatgcc tccaacatgt gaggaagtaa 1740
   tgagagaaat cata                                                   1754
<210> 18
   <211> 131
   <212> PRT
   <213> Artificial Sequence
<220>
   <223> huIL-15 huGM-CSF - amino acid sequence of optimized human IL-15 with a signal peptide of human GM-CSF
<400> 18
   Met Trp Leu Gln Ser Leu Leu Leu Leu Gly Thr Val Ala Cys Ser Ile
    1               5                  10                  15
   Ser Asn Trp Val Asn Val Ile Ser Asp Leu Lys Lys Ile Glu Asp Leu
               20                  25                  30
   Ile Gln Ser Met His Ile Asp Ala Thr Leu Tyr Thr Glu Ser Asp Val
           35                  40                  45
   His Pro Ser Cys Lys Val Thr Ala Met Lys Cys Phe Leu Leu Glu Leu
       50                  55                  60
   Gln Val Ile Ser Leu Glu Ser Gly Asp Ala Ser Ile His Asp Thr Val
   65                  70                  75                  80
   Glu Asn Leu Ile Ile Leu Ala Asn Asn Ser Leu Ser Ser Asn Gly Asn
                   85                  90                  95
   Val Thr Glu Ser Gly Cys Lys Glu Cys Glu Glu Leu Glu Glu Lys Asn
               100                 105                 110
   Ile Lys Glu Phe Leu Gln Ser Phe Val His Ile Val Gln Met Phe Ile
           115                 120                 125
   Asn Thr Ser
       130
<210> 19
   <211> 267
   <212> PRT
   <213> Artificial Sequence
<220>
   <223> synthetic human interleukin-15 (IL-15) receptor alpha (IL15Ra), isoform 1 (OPT)
<400> 19
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr Val Ala Ile
           195                 200                 205
   Ser Thr Ser Thr Val Leu Leu Cys Gly Leu Ser Ala Val Ser Leu Leu
       210                 215                 220
   Ala Cys Tyr Leu Lys Ser Arg Gln Thr Pro Pro Leu Ala Ser Val Glu
   225                 230                 235                 240
   Met Glu Ala Met Glu Ala Leu Pro Val Thr Trp Gly Thr Ser Ser Arg
                   245                 250                 255
   Asp Glu Asp Leu Glu Asn Cys Ser His His Leu
               260                 265
<210> 20
   <211> 205
   <212> PRT
   <213> Artificial Sequence
<220>
   <223> synthetic human soluble interleukin-15 (IL-15) receptor alpha (IL-15sRa) (OPT)
<400> 20
   Met Ala Pro Arg Arg Ala Arg Gly Cys Arg Thr Leu Gly Leu Pro Ala
   1               5                   10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr
           195                 200                 205
<210> 21
   <211> 436
   <212> PRT
   <213> Artificial Sequence
<220>
   <223> synthetic sIL-15Ralpha-Fc fusion protein huIL15sRa205-Fc
<400> 21
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr Thr Pro Lys Ser
           195                 200                 205
   Cys Asp Lys Thr His Thr Cys Pro Pro Cys Pro Ala Pro Glu Leu Leu
       210                 215                 220
   Gly Gly Pro Ser Val Phe Leu Phe Pro Pro Lys Pro Lys Asp Thr Leu
   225                 230                 235                 240
   Met Ile Ser Arg Thr Pro Glu Val Thr Cys Val Val Val Asp Val Ser
                   245                 250                 255
   His Glu Asp Pro Glu Val Lys Phe Asn Trp Tyr Val Asp Gly Val Glu
               260                 265                 270
   Val His Asn Ala Lys Thr Lys Pro Arg Glu Glu Gln Tyr Asn Ser Thr
           275                 280                 285
   Tyr Arg Val Val Ser Val Leu Thr Val Leu His Gln Asp Trp Leu Asn
       290                 295                 300
   Gly Lys Glu Tyr Lys Cys Lys Val Ser Asn Lys Ala Leu Pro Ala Pro
   305                 310                 315                 320
   Ile Glu Lys Thr Ile Ser Lys Ala Lys Gly Gln Pro Arg Glu Pro Gln
                   325                 330                 335
   Val Tyr Thr Leu Pro Pro Ser Arg Asp Glu Leu Thr Lys Asn Gln Val
               340                 345                 350
   Ser Leu Thr Cys Leu Val Lys Gly Phe Tyr Pro Ser Asp Ile Ala Val
           355                 360                 365
   Glu Trp Glu Ser Asn Gly Gln Pro Glu Asn Asn Tyr Lys Thr Thr Pro
       370                 375                 380
   Pro Val Leu Asp Ser Asp Gly Ser Phe Phe Leu Tyr Ser Lys Leu Thr
   385                 390                 395                 400
   Val Asp Lys Ser Arg Trp Gln Gln Gly Asn Val Phe Ser Cys Ser Val
                   405                 410                 415
   Met His Glu Ala Leu His Asn His Tyr Thr Gln Lys Ser Leu Ser Leu
               420                 425                 430
   Ser Pro Gly Lys
           435
<210> 22
   <211> 431
   <212> PRT
   <213> Artificial Sequence
<220>
   <223> synthetic sIL-15Ralpha-Fc fusion protein huIL15sRa200-Fc
<400> 22
   Met Ala Pro Arg Arg Ala Arg Gly Cys Arg Thr Leu Gly Leu Pro Ala
   1 5 10 15
   Leu Leu Leu Leu Leu Leu Leu Arg Pro Pro Ala Thr Arg Gly Ile Thr
   20 25 30
   Cys Pro Pro Pro Met Ser Val Glu His Ala Asp Ile Trp Val Lys Ser
   35 40 45
   Tyr Ser Leu Tyr Ser Arg Glu Arg Tyr Ile Cys Asn Ser Gly Phe Lys
   50 55 60
   Arg Lys Ala Gly Thr Ser Ser Leu Thr Glu Cys Val Leu Asn Lys Ala
   65 70 75 80
   Thr Asn Val Ala His Trp Thr Thr Pro Ser Leu Lys Cys Ile Arg Asp
   85 90 95
   Pro Ala Leu Val His Gln Arg Pro Ala Pro Pro Ser Thr Val Thr Thr
   100 105 110
   Ala Gly Val Thr Pro Gln Pro Glu Ser Leu Ser Pro Ser Gly Lys Glu
   115 120 125
   Pro Ala Ala Ser Ser Pro Ser Ser Asn Asn Thr Ala Ala Thr Thr Ala
   130 135 140
   Ala Ile Val Pro Gly Ser Gln Leu Met Pro Ser Lys Ser Pro Ser Thr
   145 150 155 160
   Gly Thr Thr Glu Ile Ser Ser His Glu Ser Ser His Gly Thr Pro Ser
   165 170 175
   Gln Thr Thr Ala Lys Asn Trp Glu Leu Thr Ala Ser Ala Ser His Gln
   180 185 190
   Pro Pro Gly Val Tyr Pro Gln Gly Pro Lys Ser Cys Asp Lys Thr His
   195 200 205
   Thr Cys Pro Pro Cys Pro Ala Pro Glu Leu Leu Gly Gly Pro Ser Val
   210 215 220
   Phe Leu Phe Pro Pro Lys Pro Lys Asp Thr Leu Met Ile Ser Arg Thr
   225 230 235 240
   Pro Glu Val Thr Cys Val Val Val Asp Val Ser His Glu Asp Pro Glu
   245 250 255
   Val Lys Phe Asn Trp Tyr Val Asp Gly Val Glu Val His Asn Ala Lys
   260 265 270
   Thr Lys Pro Arg Glu Glu Gln Tyr Asn Ser Thr Tyr Arg Val Val Ser
   275 280 285
   Val Leu Thr Val Leu His Gln Asp Trp Leu Asn Gly Lys Glu Tyr Lys
   290 295 300
   Cys Lys Val Ser Asn Lys Ala Leu Pro Ala Pro Ile Glu Lys Thr Ile
   305 310 315 320
   Ser Lys Ala Lys Gly Gln Pro Arg Glu Pro Gln Val Tyr Thr Leu Pro
   325 330 335
   Pro Ser Arg Asp Glu Leu Thr Lys Asn Gln Val Ser Leu Thr Cys Leu
   340 345 350
   Val Lys Gly Phe Tyr Pro Ser Asp Ile Ala Val Glu Trp Glu Ser Asn
   355 360 365
   Gly Gln Pro Glu Asn Asn Tyr Lys Thr Thr Pro Pro Val Leu Asp Ser
   370 375 380
   Asp Gly Ser Phe Phe Leu Tyr Ser Lys Leu Thr Val Asp Lys Ser Arg
   385 390 395 400
   Trp Gln Gln Gly Asn Val Phe Ser Cys Ser Val Met His Glu Ala Leu
   405 410 415
   His Asn His Tyr Thr Gln Lys Ser Leu Ser Leu Ser Pro Gly Lys
   420 425 430
<210> 23
   <211> 396
   <212> DNA
   <213> Homo sapiens
<220>
   <223> human IL-15 with GMCSF signal peptide
<400> 23
   atgtggctcc agagcctgct actcctgggg acggtggcct gcagcatctc gaactgggtg 60
   aacgtgatct cggacctgaa gaagatcgag gacctcatcc agtcgatgca catcgacgcg 120
   acgctgtaca cggagtcgga cgtccacccg tcgtgcaagg tcacggcgat gaagtgcttc 180
   ctcctggagc tccaagtcat ctcgctcgag tcgggggacg cgtcgatcca cgacacggtg 240
   gagaacctga tcatcctggc gaacaactcg ctgtcgtcga acgggaacgt cacggagtcg 300
   ggctgcaagg agtgcgagga gctggaggag aagaacatca aggagttcct gcagtcgttc 360
   gtgcacatcg tccagatgtt catcaacacg tcgtga                          396
<210> 24
   <211> 131
   <212> PRT
   <213> Homo sapiens
<220>
   <223> human IL-15 with GMCSF signal peptide
<400> 24
   Met Trp Leu Gln Ser Leu Leu Leu Leu Gly Thr Val Ala Cys Ser Ile
    1               5                  10                  15
   Ser Asn Trp Val Asn Val Ile Ser Asp Leu Lys Lys Ile Glu Asp Leu
               20                  25                  30
   Ile Gln Ser Met His Ile Asp Ala Thr Leu Tyr Thr Glu Ser Asp Val
           35                  40                  45
   His Pro Ser Cys Lys Val Thr Ala Met Lys Cys Phe Leu Leu Glu Leu
       50                  55                  60
   Gln Val Ile Ser Leu Glu Ser Gly Asp Ala Ser Ile His Asp Thr Val
   65                  70                  75                  80
   Glu Asn Leu Ile Ile Leu Ala Asn Asn Ser Leu Ser Ser Asn Gly Asn
                   85                  90                  95
   Val Thr Glu Ser Gly Cys Lys Glu Cys Glu Glu Leu Glu Glu Lys Asn
               100                 105                 110
   Ile Lys Glu Phe Leu Gln Ser Phe Val His Ile Val Gln Met Phe Ile
           115                 120                 125
   Asn Thr Ser
       130
<210> 25
   <211> 807
   <212> DNA
   <213> Homo sapiens
<220>
   <223> Human interleukin 15 receptor alpha (IL15Ra)
<400> 25
<210> 26
   <211> 8
   <212> PRT
   <213> homo sapiens
<220>
   <223> C-terminal end of the soluble form of human IL-15Ra
<400> 26
   Pro Gln Gly His Ser Asp Thr Thr
   1               5
<210> 27
   <211> 7
   <212> PRT
   <213> Artificial Sequence
<220>
   <223> C-terminal end of the soluble form of human IL-15Ra
<400> 27
   Pro Gln Gly His Ser Asp Thr
   1               5
<210> 28
   <211> 6
   <212> PRT
   <213> Artificial Sequence
<220>
   <223> C-terminal end of the soluble form of human IL-15Ra
<400> 28
   Pro Gln Gly His Ser Asp
   1               5
<210> 29
   <211> 5
   <212> PRT
   <213> Artificial Sequence
<220>
   <223> C-terminal end of the soluble form of human IL-15Ra
<400> 29
   Pro Gln Gly His Ser
   1 5
<210> 30
   <211> 4
   <212> PRT
   <213> Artificial Sequence
<220>
   <223> C-terminal end of the soluble form of human IL-15Ra
<400> 30
   Pro Gln Gly His
   1
<210> 31
   <211> 3
   <212> PRT
   <213> Artificial Sequence
<220>
   <223> C-terminal end of the soluble form of human IL-15Ra
<400> 31
   Pro Gln Gly
   1
<210> 32
   <211> 200
   <212> PRT
   <213> Homo sapiens
<220>
   <223> immature form of the native soluble human IL-15Ra
<400> 32
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly
           195                 200
<210> 33
   <211> 170
   <212> PRT
   <213> Artificial Sequence
<220>
   <223> a soluble form of human IL-15Ra
<400> 33
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Thr Val
   65                  70                  75                  80
   Thr Thr Ala Gly Val Thr Pro Gln Pro Glu Ser Leu Ser Pro Ser Gly
                   85                  90                  95
   Lys Glu Pro Ala Ala Ser Ser Pro Ser Ser Asn Asn Thr Ala Ala Thr
               100                 105                 110
   Thr Ala Ala Ile Val Pro Gly Ser Gln Leu Met Pro Ser Lys Ser Pro
           115                 120                 125
   Ser Thr Gly Thr Thr Glu Ile Ser Ser His Glu Ser Ser His Gly Thr
       130                 135                 140
   Pro Ser Gln Thr Thr Ala Lys Asn Trp Glu Leu Thr Ala Ser Ala Ser
   145                 150                 155                 160
   His Gln Pro Pro Gly Val Tyr Pro Gln Gly
                   165                 170
<210> 34
   <211> 201
   <212> PRT
   <213> Artificial Sequence
<220>
   <223> a soluble form of human IL-15Ra
<400> 34
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His
           195                 200
<210> 35
   <211> 171
   <212> PRT
   <213> Artificial Sequence
<220>
   <223> a soluble form of human IL-15Ra
<400> 35
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Thr Val
   65                  70                  75                  80
   Thr Thr Ala Gly Val Thr Pro Gln Pro Glu Ser Leu Ser Pro Ser Gly
                   85                  90                  95
   Lys Glu Pro Ala Ala Ser Ser Pro Ser Ser Asn Asn Thr Ala Ala Thr
               100                 105                 110
   Thr Ala Ala Ile Val Pro Gly Ser Gln Leu Met Pro Ser Lys Ser Pro
           115                 120                 125
   Ser Thr Gly Thr Thr Glu Ile Ser Ser His Glu Ser Ser His Gly Thr
       130                 135                 140
   Pro Ser Gln Thr Thr Ala Lys Asn Trp Glu Leu Thr Ala Ser Ala Ser
   145                 150                 155                 160
   His Gln Pro Pro Gly Val Tyr Pro Gln Gly His
                   165                 170
<210> 36
   <211> 202
   <212> PRT
   <213> Artificial Sequence
<220>
   <223> a soluble form of human IL-15Ra
<400> 36
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser
           195                 200
<210> 37
   <211> 172
   <212> PRT
   <213> Artificial Sequence
<220>
   <223> a soluble form of human IL-15Ra
<400> 37
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Thr Val
   65                  70                  75                  80
   Thr Thr Ala Gly Val Thr Pro Gln Pro Glu Ser Leu Ser Pro Ser Gly
                   85                  90                  95
   Lys Glu Pro Ala Ala Ser Ser Pro Ser Ser Asn Asn Thr Ala Ala Thr
               100                 105                 110
   Thr Ala Ala Ile Val Pro Gly Ser Gln Leu Met Pro Ser Lys Ser Pro
           115                 120                 125
   Ser Thr Gly Thr Thr Glu Ile Ser Ser His Glu Ser Ser His Gly Thr
       130                 135                 140
   Pro Ser Gln Thr Thr Ala Lys Asn Trp Glu Leu Thr Ala Ser Ala Ser
   145                 150                 155                 160
   His Gln Pro Pro Gly Val Tyr Pro Gln Gly His Ser
                   165                 170
<210> 38
   <211> 203
   <212> PRT
   <213> Artificial Sequence
<220>
   <223> a soluble form of human IL-15Ra
<400> 38
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp
           195                 200
<210> 39
   <211> 173
   <212> PRT
   <213> Artificial Sequence
<220>
   <223> a soluble form of human IL-15Ra
<400> 39
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Thr Val
   65                  70                  75                  80
   Thr Thr Ala Gly Val Thr Pro Gln Pro Glu Ser Leu Ser Pro Ser Gly
                   85                  90                  95
   Lys Glu Pro Ala Ala Ser Ser Pro Ser Ser Asn Asn Thr Ala Ala Thr
               100                 105                 110
   Thr Ala Ala Ile Val Pro Gly Ser Gln Leu Met Pro Ser Lys Ser Pro
           115                 120                 125
   Ser Thr Gly Thr Thr Glu Ile Ser Ser His Glu Ser Ser His Gly Thr
       130                 135                 140
   Pro Ser Gln Thr Thr Ala Lys Asn Trp Glu Leu Thr Ala Ser Ala Ser
   145                 150                 155                 160
   His Gln Pro Pro Gly Val Tyr Pro Gln Gly His Ser Asp
                   165                 170
<210> 40
   <211> 204
   <212> PRT
   <213> Artificial Sequence
<220>
   <223> a soluble form of human IL-15Ra
<400> 40
   Met Ala Pro Arg Arg Ala Arg Gly Cys Arg Thr Leu Gly Leu Pro Ala
    1               5                  10                  15
   Leu Leu Leu Leu Leu Leu Leu Arg Pro Pro Ala Thr Arg Gly Ile Thr
               20                  25                  30
   Cys Pro Pro Pro Met Ser Val Glu His Ala Asp Ile Trp Val Lys Ser
           35                  40                  45
   Tyr Ser Leu Tyr Ser Arg Glu Arg Tyr Ile Cys Asn Ser Gly Phe Lys
       50                  55                  60
   Arg Lys Ala Gly Thr Ser Ser Leu Thr Glu Cys Val Leu Asn Lys Ala
   65                  70                  75                  80
   Thr Asn Val Ala His Trp Thr Thr Pro Ser Leu Lys Cys Ile Arg Asp
                   85                  90                  95
   Pro Ala Leu Val His Gln Arg Pro Ala Pro Pro Ser Thr Val Thr Thr
               100                 105                 110
   Ala Gly Val Thr Pro Gln Pro Glu Ser Leu Ser Pro Ser Gly Lys Glu
           115                 120                 125
   Pro Ala Ala Ser Ser Pro Ser Ser Asn Asn Thr Ala Ala Thr Thr Ala
       130                 135                 140
   Ala Ile Val Pro Gly Ser Gln Leu Met Pro Ser Lys Ser Pro Ser Thr
   145                 150                 155                 160
   Gly Thr Thr Glu Ile Ser Ser His Glu Ser Ser His Gly Thr Pro Ser
                   165                 170                 175
   Gln Thr Thr Ala Lys Asn Trp Glu Leu Thr Ala Ser Ala Ser His Gln
               180                 185                 190
   Pro Pro Gly Val Tyr Pro Gln Gly His Ser Asp Thr
           195                 200
<210> 41
   <211> 174
   <212> PRT
   <213> Artificial Sequence
<220>
   <223> a soluble form of human IL-15Ra
<400> 41
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Thr Val
   65                  70                  75                  80
   Thr Thr Ala Gly Val Thr Pro Gln Pro Glu Ser Leu Ser Pro Ser Gly
                   85                  90                  95
   Lys Glu Pro Ala Ala Ser Ser Pro Ser Ser Asn Asn Thr Ala Ala Thr
               100                 105                 110
   Thr Ala Ala Ile Val Pro Gly Ser Gln Leu Met Pro Ser Lys Ser Pro
           115                 120                 125
   Ser Thr Gly Thr Thr Glu Ile Ser Ser His Glu Ser Ser His Gly Thr
       130                 135                 140
   Pro Ser Gln Thr Thr Ala Lys Asn Trp Glu Leu Thr Ala Ser Ala Ser
   145                 150                 155                 160
   His Gln Pro Pro Gly Val Tyr Pro Gln Gly His Ser Asp Thr
                   165                 170
<210> 42
   <211> 19
   <212> PRT
   <213> Artificial Sequence
<220>
   <223> purified glycosylated soluble form of human IL-15Ra
<400> 42
   Asn Trp Glu Leu Thr Ala Ser Ala Ser His Gln Pro Pro Gly Val Tyr
    1               5                  10                  15
   Pro Gln Gly
<210> 43
   <211> 17
   <212> PRT
   <213> Artificial Sequence
<220>
   <223> purified glycosylated soluble form of human IL-15Ra
<400> 43
   Ile Thr Cys Pro Pro Pro Met Ser Val Glu His Ala Asp Ile Trp Val
    1               5                  10                  15
   Lys
<210> 44
   <211> 31
   <212> PRT
   <213> Artificial Sequence
<220>
   <223> purified glycosylated soluble form of human IL-15Ra
<400> 44
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser
               20                  25                  30
<210> 45
   <211> 175
   <212> PRT
   <213> Artificial Sequence
<220>
   <223> a soluble form of human IL-15Ra
<400> 45
   Ile Thr Cys Pro Pro Pro Met Ser Val Glu His Ala Asp Ile Trp Val
    1               5                  10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Thr Val
   65                  70                  75                  80
   Thr Thr Ala Gly Val Thr Pro Gln Pro Glu Ser Leu Ser Pro Ser Gly
                   85                  90                  95
   Lys Glu Pro Ala Ala Ser Ser Pro Ser Ser Asn Asn Thr Ala Ala Thr
               100                 105                 110
   Thr Ala Ala Ile Val Pro Gly Ser Gln Leu Met Pro Ser Lys Ser Pro
           115                 120                 125
   Ser Thr Gly Thr Thr Glu Ile Ser Ser His Glu Ser Ser His Gly Thr
       130                 135                 140
   Pro Ser Gln Thr Thr Ala Lys Asn Trp Glu Leu Thr Ala Ser Ala Ser
   145                 150                 155                 160
   His Gln Pro Pro Gly Val Tyr Pro Gln Gly His Ser Asp Thr Thr
                   165                 170                 175
<210> 46
   <211> 600
   <212> DNA
   <213> Homo sapiens
<220>
   <223> nucleotide sequence encoding the mature human soluble native IL-15Ra
<400> 46
   atggccccgc ggcgggcgcg cggctgccgg accctcggtc tcccggcgct gctactgctg 60
   ctgctgctcc ggccgccggc gacgcggggc atcacgtgcc ctccccccat gtccgtggaa 120
   cacgcagaca tctgggtcaa gagctacagc ttgtactcca gggagcggta catttgtaac 180
   tctggtttca agcgtaaagc cggcacgtcc agcctgacgg agtgcgtgtt gaacaaggcc 240
   acgaatgtcg cccactggac aacccccagt ctcaaatgca ttagagaccc tgccctggtt 300
   caccaaaggc cagcgccacc ctccacagta acgacggcag gggtgacccc acagccagag 360
   agcctctccc cttctggaaa agagcccgca gcttcatctc ccagctcaaa caacacagcg 420
   gccacaacag cagctattgt cccgggctcc cagctgatgc cttcaaaatc accttccaca 480
   ggaaccacag agataagcag tcatgagtcc tcccacggca ccccctctca gacaacagcc 540
   aagaactggg aactcacagc atccgcctcc caccagccgc caggtgtgta tccacagggc 600

## Description

This invention was created in the performance of a Cooperative Research and Development Agreement with the National Institutes of Health, an Agency of the Department of Health and Human Services. The Government of the United States has certain rights in this invention.

### 1. FIELD

In one aspect, described herein are cyclical administration regimens for the administration of complexes comprising interleukin-15 ("IL-15") covalently or noncovalently bound to IL-15 receptor alpha ("IL-15Ra") to patients in order to enhance IL-15-mediated immune function. In one aspect, these cyclical administration regimens achieve plasma levels of IL-15 above basal levels while minimizing the toxicity associated with IL-15 administration. In a specific aspect, the cyclical administration regimens are useful in the prevention, treatment, and/or management of disorders in which enhancing IL-15-mediated function is beneficial, such as cancer, infectious diseases, immunodeficienices and lymphopenia. Also described herein are purified soluble forms of IL-15Ra, cells that recombinantly express soluble forms of IL-15Ra, and compositions comprising complexes of IL-15 covalently or non-covalently bound to soluble forms of IL-15Ra. Further described herein are host cells that recombinantly express IL-15-Ra derivatives comprising a mutation or deletion in the extracellular domain cleavage site, including IL-15Ra derivatives comprising the extracellular domain of IL-15Ra and a transmembrane domain of a heterologous molecule. In addition, described herein are methods for propagating, activating and/or differentiating IL-15 responsive cells, comprising co-culturing an IL-15 responsive cell(s) with a host cell(s) that recombinantly expresses IL-15Ra, and isolating the IL-15 responsive cell(s) from the host cell(s). The IL-15 responsive cells that are immune cells can be administered to prevent, treat and/or manage various disorders, including cancer, an infectious disease, an immunodeficiency and lymphopenia. Further, described herein are methods of enhancing IL-15-mediated immune function as well as methods for preventing, treating and/or managing disorders in which enhancing IL-15-mediated function is beneficial, such as cancer, in a subject, the methods comprising administering to the subject a host cell that recombinantly expresses an IL-15Ra described herein.

### 2. BACKGROUND

The cytokine, interleukin-15 (IL-15), is a member of the four alpha-helix bundle family of lymphokines produced by many cells in the body. IL-15 plays a pivotal role in modulating the activity of both the innate and adaptive immune system, e.g., maintenance of the memory T-cell response to invading pathogens, inhibition of apoptosis, activation of dendritic cells, and induction of Natural Killer (NK) cell proliferation and cytotoxic activity.

The IL-15 receptor consists of three polypeptides, the type-specific IL-15 receptor alpha ("IL-15Ra"), the IL-2/IL-15 receptor beta (or CD122) ("β"), and the common gamma chain (or CD132) ("γ") that is shared by multiple cytokine receptors. The IL-15Ra is thought to be expressed by a wide variety of cell types, but not necessarily in conjunction with β and γ. IL-15 signaling has been shown to occur through the heterodimeric complex of IL-15Ra, β, and γ; through the heterodimeric complex of β and γ, or through a subunit, IL-15RX, found on mast cells.

IL-15 is a soluble protein, but endogenous IL-15 is not readily detectable in serum or body fluids - instead, it occurs predominantly as a membrane-bound form that is expressed or acquired by several types of accessory cells. For instance, although IL-15 mRNA is detected in cells of both hematopoietic and non-hematopoietic lineage, T cells do not produce IL-15. Instead, IL-15 binds to the IL-15Ra, forming cell-surface complexes on T cells. IL-15 specifically binds to the IL-15Ra with high affinity via the "sushi domain" in exon 2 of the extracellular domain of the receptor. After trans-endosomal recycling and migration back to the cell surface, these IL-15 complexes acquire the property to activate bystander cells expressing the IL-15R βγ low-affinity receptor complex, inducing IL-15-mediated signaling via the Jak/Stat pathway. A naturally occurring soluble form of IL-15Ra ("sIL-15Ra"), which is cleaved at a cleavage site in the extracellular domain immediately distal to the transmembrane domain of the receptor has been observed. Tumor necrosis factor-alpha-converting enzyme (TACE/ADAM17) has been implicated as a protease involved in this process.

An alternative interpretation of the existing data is that IL-15Ra has evolved very high affinity for IL-15 and is always co-expressed with IL-15 in the same cell. The two molecules form heterodimeric complexes in the Endoplasmic Reticulum and are transported to the plasma membrane. *See*, *e*.*g*., Bergamaschi et al., 2012, Blood 120: e1-e8. This heterodimeric complex can bind to the IL-2/IL-15 βγ receptor and activate the cells via the Jak/Stat pathway. Therefore, based upon this interpretation of the data, the IL-15Ra and the soluble form sIL-15Ra are part of the cytokine and not part of the receptor. *Id.*

Based on its multifaceted role in the immune system, various therapies designed to modulate IL-15-mediated function have been explored. For example, the administration of exogenous IL-15 can enhance the immune function of patients infected with human immunodeficiency virus (HIV). In keeping with its immune enhancing activity, increased expression of endogenous IL-15 is observed in patients with autoimmune diseases, e.g., rheumatoid arthritis, multiple sclerosis, ulcerative colitis, and psoriasis. Because some studies reported that the soluble form of the IL-15Ra (sIL-15Ra) is an antagonist of IL-15-mediated signaling, the sIL-15Ra has been explored for treating autoimmune inflammatory diseases. Nevertheless, recent reports suggest that IL-15, when complexed with the sIL-15Ra, or the sushi domain, maintains its immune enhancing function.

Despite the amount of progress made in understanding the function of IL-15, it is unclear how various forms of the IL-15Ra, alone or when complexed to IL-15, can be used to modulate IL-15 function as part of a therapeutic regimen.

### 3. SUMMARY

In an aspect, provided herein are methods for enhancing IL-15-mediated immune function, comprising administering to subjects agents that induce IL-15 signal transduction and enhance IL-15-mediated immune function. More specifically, provided herein are methods for enhancing IL-15-mediated immune function, comprising administering to subjects complexes that bind to the βγ subunits of the IL-15 receptor, induce IL-15 signal transduction and enhance IL-15-mediated immune function, wherein the complexes comprise IL-15 covalently or noncovalently bound to interleukin-15 receptor alpha ("IL-15Ra") ("IL-15/IL-15Ra complexes" or "Therapeutic Agents"). Since enhancing IL-15-mediated immune function is beneficial for the prevention, treatment and/or management of certain disorders, provided herein are methods for the prevention, treatment and/or management of such disorders comprising administering to a subject in need thereof IL-15/IL-15Ra complexes.

The methods described herein are based, in part, on the surprising discovery that subcutaneous administration of IL-15/IL-15Ra complexes avoids the high peak plasma levels and toxicity associated with intravenous administration of the complexes. The subcutaneous administration of IL-15/IL-15Ra complexes maintain plasma levels of IL-15 while minimizing side effects, such as a decrease in blood pressure and an increase in body temperature. The subcutaneous administration of IL-15/IL-15Ra complexes can achieve a systemic effect (not just a local effect) with minimal side effects. In a specific embodiment, the subcutaneous administration of IL-15/IL-15Ra complexes does not alter blood pressure or body temperature. Surprisingly, high doses, such as 50 µg/kg, subcutaneously administered to a subject resulted in minimal toxicity. *See*, *e*.*g*., the examples in Section 6 *infra.*

In one aspect, the methods described herein maintain plasma levels of IL-15 above basal levels for approximately 18 to 24 hours or approximately 24 to 36 hours, or approximately 36 to 38 hours following administration of IL-15/IL-15Ra complexes. Basal plasma levels of IL-15 are approximately 1 pg/ml in humans, approximately 8-10 pg/ml in monkeys (such as macaques), and approximately 12 pg/m in rodents (such as mice). Thus, in some embodiments, the methods described herein maintain plasma levels above approximately 1 pg/ml in humans, above approximately 8-10 pg/ml in monkeys (such macaques) and above 12 pg/ml in rodents (such as mice). Without being bound by any theory, the stability of the IL-15 plasma levels maximizes lymphocyte growth and activation while minimizing any side effects associated with IL-15 administration. In one embodiment, the methods described herein achieve stable plasma levels of IL-15 above basal plasma levels by administering subcutaneously doses of approximately 0.1 µg/kg to approximately 10 µg/kg of an IL-15/IL-15Ra complex to a subject. In another embodiment, the methods described herein achieve high plasma levels of IL-15 by administering subcutaneously doses of approximately 0.1 µg/kg to approximately 20 µg/kg, approximately 10 µg/kg to approximately 20 µg/kg, approximately 20 µg/kg to approximately 40 µg/kg, or approximately 25 µg/kg to 50 µg/kg of an IL-15/IL-15Ra complex to a subject. In other words, described herein are methods for enhancing IL-15-mediated immune function as well as methods for preventing, treating and/or managing disorders in which enhancement of IL-15-mediated immune function is beneficial (e.g., cancer, infectious diseases, immunodeficiencies, lymphopenia and wounds), comprising administering subcutaneously to a subject in need thereof an effective amount of IL-15/IL-15Ra complexes.

In one aspect, provided herein is a method for enhancing IL-15-mediated immune function, comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 or 7 days. In some embodiments, the IL-15/IL-15Ra is administered 1, 2, 3, 4, 5, 6 or 7 days per week. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long. In an embodiment, the dose of the first cycle and each subsequent cycle is 0.1 µg/kg to 1 µg/kg, 1 µg/kg to 5 µg/kg, or 5 µg/kg to 10 µg/kg. In another embodiment, the first dose of the first cycle and each subsequent cycle is 0.1 µg/kg to 0.5 µg/kg, 1 µg/kg to 2 µg/kg, 1 µg/kg to 3 µg/kg, 2 µg/kg to 5 µg/kg, or 2 µg/kg to 4 µg/kg. In another embodiment, the dose of the first cycle and each subsequent cycle is 0.1 µg/kg, 0.25 µg/kg, 0.5 µg/kg, 1 µg/kg, 1.25 µg/kg, 1.5 µg/kg, 1.75 µg/kg, 2 µg/kg, 2.25 µg/kg, 2.5 µg/kg, 2.75 µg/kg, 3 µg/kg, 3.25 µg/kg, 3.5 µg/kg, 4 µg/kg, 4.25 µg/kg, 4.5 µg/kg, 4.75 µg/kg, or 5 µg/kg. In some embodiments, the dose of the first cycle differs from the dose used in one or more subsequent cycles of the cyclical regimen.

In one embodiment, provided herein is a method for enhancing IL-15-mediated immune function, comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject a certain number of times per week for a first period of time; and (b) no administration of IL-15/IL-15Ra complex for a second period of time. In some embodiments, the dose of the IL-15/IL-15Ra administered during the first cycle of the cyclical regimen is sequentially escalated. For example, if an IL-15/IL-15Ra complex is administered to a subject 3 times per week for two weeks, then the dose administered to the subject the second time during the first cycle of the cyclical regimen is increased relative to the dose administered the first time, the dose administered to the subject the third time during the first cycle of the cyclical regimen is increased relative to the dose administered the second time, the dose administered to the subject the fourth time is increased relative to the dose administered the third time, the dose administered to the subject the fifth time is increased relative the dose administered the fourth time, and the dose administered to the subject the sixth time is increased relative to the dose administered the fifth time. In some embodiments, the plasma levels of IL-15 and/or lymphocyte counts are monitored. In some embodiments, the subject is monitored for side effects such as a decrease in blood pressure and/or an increase in body temperature and/or an increase in cytokines in plasma. In some embodiments, the dose of the IL-15/IL-15Ra complex administered during the first cycle of the cyclical regimen is sequentially escalated if the subject does not have any side effects. In some embodiments, the dose of the IL-15/IL-15Ra complex administered during the first cycle of the cyclical regimen is sequentially escalated if the subject does not experience any adverse events, such as grade 3 or 4 lymphopenia, grade 3 granulocytopenia, grade 3 leukocytosis (WBC > 100,000/mm³), or organ dysfunction. In some embodiments, the IL-15/IL-15Ra is administered 1, 2, 3, 4, 5, 6, or 7 days per week. In some embodiments, the cyclical regimen is repeated 2, 3, 4, 5, 6, 7, 8, 9, 10 or more times. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long. In some embodiments, the dose during the first cycle of the cyclical regimen is 0.1 µg/kg, 0.25 µg/kg, 0.75 µg/kg, 1 µg/kg, 1.5 µg/kg, 2 µg/kg, 2.5 µg/kg, 3 µg/kg, 3.5 µg/kg, 4 µg/kg, 4.5 µg/kg, or 5 µg/kg. In other embodiments, the dose during the first cycle of the cylical regimen is 0.1 µg/kg to 1 µg/kg, 1 µg/kg to 2 µg/kg, 2 µg/kg to 4 µg/kg or 3 to 5 µg/kg. In some embodiments, the dose used during the a cycle subsequent to the first cycle of the cyclical regimen is 0.1 µg/kg, 0.25 µg/kg, 0.75 µg/kg, 1 µg/kg, 1.5 µg/kg, 2 µg/kg, 2.5 µg/kg, 3 µg/kg, 3.5 µg/kg, 4 µg/kg, 4.5 µg/kg, 5 µg/kg, 10 µg/kg, 15 µg/kg or 20 µg/kg higher than the dose used for the first cycle of the cyclical regimen. In other embodiments, the dose used during a cycle subsequent to the first cycle of the cyclical regimen is 0.1 µg/kg to 1 µg/kg, 1 to 5 µg/kg, 5 µg/kg to 10 µg/kg, or 10 to 20 µg/kg higher than the dose used for the first cycle of the cylical regimen.

In a particular aspect, provided herein is a method for enhancing IL-15-mediated immune function, comprising subcutaneously administering to a subject an IL-15/IL-15Ra complex in a cyclical regimen, wherein cyclical regimen comprises: (a) subcutaneously administering a first dose of the IL-15/IL-15Ra complex to the subject at a certain frequency for a first period of time; (b) no administration of IL-15/IL-15Ra complex for a second period of time; and (c) subcutaneously administereing a second dose of the IL-15/IL-15Ra complex to the subject at a certain frequency for a third period of time. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 or 7 days. In some embodiments, the IL-15/IL-15Ra complex is administered 3 days per week. In some embodiments, the first, second and third periods of time are the same. In other embodiments, the first, second, and third periods of time are different. In some embodiments, the first and third periods for administration of the IL-15/IL-15Ra complex are 1 week to 4 weeks, 2 to 4 weeks, 2 to 3 weeks, or 1 to 2 weeks long. In other embodiments, the first and third periods for administration of the IL-15/IL-15Ra complex are 1 week, 2 weeks, 3 weeks, or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 2 weeks or 1 week long. In some embodiments, the first and second doses of the IL-15/IL-15Ra complex are the same. In other embodiments, the first and second doses of the IL-15/IL-15Ra complex are different. In an embodiment, the first dose is 0.1 µg/kg, 0.25 µg/kg, 0.5 µg/kg, 0.75 µg/kg, 1 µg/kg, 1.5 µg/kg, 2 µg/kg, 2.5 µg/kg, 3 µg/kg, 3.5 µg/kg, 4 µg/kg or 5 µg/kg. In other embodiments, the first dose is 0.1 µg/kg to 1 µg/kg, 1 µg/kg to 2 µg/kg, 2 µg/kg to 4 µg/kg, or 3 µg/kg to 5 µg/kg. In some embodiments, the second dose is 0.1 µg/kg, 0.25 µg/kg, 0.5 µg/kg, 0.75 µg/kg, 1 µg/kg, 1.25 µg/kg, 1.5 µg/kg, 1.75 µg/kg 2 µg/kg, 2.5 µg/kg, 3 µg/kg, 3.5 µg/kg, 4 µg/kg, 4.5 µg/kg, 5 µg/kg, 5.5 µg/kg, 6 µg/kg, 6.5 µg/kg, 7 µg/kg, 7.5 µg/kg, 8 µg/kg, 8.5 µg/kg, 9 µg/kg, 9.5 µg/kg, or 10 µg/kg higher than the first dose. In other embodiments, the second dose is 0.1 µg/kg to 1 µg/kg, 1 µg/kg to 2 µg/kg, 2 µg/kg to 5 µg/kg, 5 µg/kg to 10 µg/kg, 10 µg/kg to 15 µg/kg, 15 µg/kg to 20 µg/kg, 20 µg/kg to 25 µg/kg higher than the first dose. In some embodiments, the second dose of the IL-15/IL-15Ra complex is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In other embodiments, the second dose of the IL-15/IL-15Ra complex is 4 µg/kg, 5 µg/kg, 6 µg/kg, 7 µg/kg, 8 µg/kg, 9 µg/kg, 10 µg/kg, 15 µg/kg, 20 µg/kg, or 25 µg/kg.

In an aspect, provided herein are methods of enhancing IL-15-mediated immune function, wherein the methods involve a cyclical administration regimen comprising a first period of 1 to 3 weeks of subcutaneous administration of an IL-15/IL-15Ra complex to a subject followed by a second period of 1 week to 2 months in which no IL-15/IL-15Ra complex is administered to the subject followed by a third period of 1 week to 3 weeks of subcutaneous administration of the IL-15/IL-15Ra complex to the subject. In some embodiments, a dose of 0.1 to 10 µg/kg, 0.1 to 8 µg/kg, 0.1 to 5 µg/kg, or 0.1 to 2 µg/kg, or about 0.1 µg/kg, about 0.25 µg/kg, about 0.5 µg/kg, about 0.75 µg/kg, about 1 µg/kg, about 1.5 µg/kg, about 2 µg/kg, about 3 µg/kg, about 4 µg/kg, or about 5 µg/kg of IL-15/IL-15Ra complex is subcutaneously administered to the subject every 1 to 7 days, every 1 to 5 days, or every 1 to 3 days during the first and third periods of the cyclical administration regimen. The cyclical administration regimen may be conducted one time, two times, three times, four times, five times, six times, seven times or more, or 2 to 5 times, 5 to 10 times, 10 to 15 times, 15 to 20 times, 20 to 25 times or more. In some embodiments, the cyclical administration regimen is repeated for at least 2 months, at least 3 months, at least 4 months, at least 5 months, at least 6 months, at least 7 months, at least 8 months, at least 9 months, at least 10 months, at least 11 months, at least 1 year or more. In some embodiments, the cyclical administration regimen is repeated for about 2 months, about 3 months, about 4 months, about 5 months, about 6 months, about 7 months, about 8 months, about 9 months, about 10 months, about 11 months, about 1 year or more. In some embodiments, the cyclical administration regimen is repeated for 3 to 6 months, 6 to 9 months, 6 to 12 months, 1 to 1.5 years, 1 to 2 years, 1.5 to 2 years, or more. In some embodiments, the plasma levels of IL-15 and/or lymphocyte counts in the subject are monitored after each dose, after every other dose, and/or prior to administration of the IL-15/IL-15Ra complex in the third period. In some embodiments, the subject is monitored for side effects such as a decrease in blood pressure and/or an increase in body temperature and/or an increase in cytokines in plasma.

In another aspect, provided herein are methods for enhancing IL-15-mediated immune function that involve a cyclical administration regimen comprising a first two week period of subcutaneous administration of an IL-15/IL-15Ra complex to a subject followed by a second two week period in which no IL-15/IL-15Ra complex is administered to the subject followed by a third two week period of subcutaneous administration of the IL-15/IL-15Ra complex to the subject. In some embodiments, a dose of 0.1 to 10 µg/kg of IL-15/IL-15Ra complex is subcutaneously administered to the subject every 1 to 5 days, every 1 to 3 days, or every 1 to 3 days during the first and third periods of the cyclical administration regimen. This cyclical administration regimen may be conducted one time, two times, three times or more. In some embodiments, the plasma levels of IL-15 and/or lymphocyte counts in the subject are monitored after each dose, after every other dose, or prior to administration of the IL-15/IL-15Ra complex in the third period. In some embodiments, the subject is monitored for side effects such as a decrease in blood pressure and/or an increase in body temperature and/or an increase in cytokines in plasma.

In one embodiment, provided herein is a method for enhancing IL-15-mediated immune function in a subject in need thereof, comprising administering an IL-15/IL-15Ra complex to the subject using a cyclical administration regimen, wherein the cyclical administration regimen comprises: (a) administering subcutaneously to the subject a dose of 0.1 to 10 µg/kg of the IL-15/IL-15Ra complex every 1 to 7 days, every 1 to 5 days, every 1 to 4 days, every 1 to 3 days over a first period of 1 week to 3 weeks; and (b) after a second period of 1 week to 2 months in which no IL-15/IL-15Ra complex is administered to the subject, administering subcutaneously to the subject a dose of 0.1 to 10 µg/kg of the IL-15/IL-15Ra complex every 1 to 7 days, every 1 to 5 days, every 1 to 4 days, every 1 to 3 days over a third period of 1 week to 3 weeks. In another embodiment, provided herein is a method for enhancing IL-15-mediated immune function in a subject in need thereof, comprising administering an IL-15/IL-15Ra complex to the subject using a cyclical administration regimen, wherein the cyclical administration regimen comprises: (a) administering subcutaneously to the subject a dose of 0.1 to 10 µg/kg, 0.1 to 8 µg/kg , 0.1 to 5 µg/kg , 0.1 to 2.5 µg/kg, 0.1 to 2 µg/kg, or 0.1 to 1 µg/kg of the IL-15/IL-15Ra complex every 1, 2, 3, 4, 5, 6 or 7 days over a first period of 1 week to 3 weeks; and (b) after a second period of 1 week to 2 months in which no IL-15/IL-15Ra complex is administered to the subject, administering subcutaneously to the subject a dose of 0.1 to 10 µg/kg, 0.1 to 8 µg/kg, 0.1 to 5 µg/kg, 0.1 to 2.5 µg/kg, 0.1 to 2 µg/kg, or 0.1 to 1 µg/kg of the IL-15/IL-15Ra complex every 1, 2, 3, 4, 5, 6 or 7 days over a third period of 1 week to 3 weeks. The cyclical administration regimen may be conducted one time, two times, three times, four times, five times, six times, seven times, eight times, nine times, ten times, or more, or 2 to 5 times, 5 to 10 times, 10 to 15 times, 15 to 20 times, 20 to 25 times or more. In some embodiments, the cyclical administration regimen is repeated for at least 2 months, at least 3 months, at least 4 months, at least 5 months, at least 6 months, at least 7 months, at least 8 months, at least 9 months, at least 10 months, at least 11 months, at least 1 year or more. In some embodiments, the cyclical administration regimen is repeated for about 2 months, about 3 months, about 4 months, about 5 months, about 6 months, about 7 months, about 8 months, about 9 months, about 10 months, about 11 months, about 1 year or more. In some embodiments, the cyclical administration regimen is repeated for 3 to 6 months, 6 to 9 months, 6 to 12 months, 1 to 1.5 years, 1 to 2 years, 1.5 to 2 years, or more. In some embodiments, the plasma levels of IL-15 and/or lymphocyte counts in the subject are monitored after each dose in step (a) and/or step (b) of the cyclical administration regimen, after every other dose in step (a) and/or (b) of the cyclical administration regimen, and/or prior to administration of the IL-15/IL-15Ra complex in the third period in step (b) of the cyclical administration regimen. In some embodiments, the subject is monitored for side effects, such as a decrease in blood pressure and/or an increase in body temperature and/or an increase in cytokines in plasma, during the cyclical administration regimen and/or following the cessation of the cyclical administration.

In another embodiment, provided herein is a method for enhancing IL-15-mediated immune function, comprising: (a) administering subcutaneously to a subject a dose of approximately 0.1 µg/kg to approximately 10 µg/kg (in some embodiments, approximately 0.1 µg/kg to approximately 5 µg/kg, approximately 0.1 µg/kg to approximately 2 µg/kg, or approximately 0.1 µg/kg to approximately 1 µg/kg) of an IL-15/IL-15Ra complex every 1, 2 or 3 days over a first period of 12 to 14 days; and (b) after a second period of 12 to 14 days in which no IL-15/IL-15Ra complex is administered, administering subcutaneously to the subject a dose of approximately 0.1 µg/kg to approximately 10 µg/kg (in some embodiments, approximately 0.1 µg/kg to approximately 5 µg/kg, approximately 0.1 µg/kg to approximately 2 µg/kg, or approximately 0.1 µg/kg to approximately 1 µg/kg) of an IL-15/IL-15Ra complex every 1, 2 or 3 days over a third period of 12 to 14 days. In some embodiments, steps (a) through (c) are repeated two, three, four, five, six, seven, eight, nine, ten or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In some embodiments, the subject is monitored for side effects, such as a decrease in blood pressure and/or an increase in body temperature and/or an increase in cytokines in plasma.

In another embodiment, provided herein is a method for enhancing IL-15-mediated immune function, comprising: (a) administering subcutaneously to a subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered, administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a third period of 12 to 14 days. In some embodiments, steps (a) through (c) are repeated two, three, four, five, six, seven, eight, nine, ten or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In some embodiments, the subject is monitored for side effects such as a decrease in blood pressure and/or an increase in body temperature and/or an increase in cytokines in plasma.

In another aspect, provided herein is a method for preventing, treating and/or managing a disorder in which enhancing IL-15-mediated immune function is beneficial (e.g., cancer, an infectious disease, an immunodeficiency or lymphopenia), comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 or 7 days. In other embodiments, the IL-15/IL-15Ra complex is administered at a frequency of 1, 2, 3, 4, 5, 6 or 7 days per week. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks,, 2 to 3 weeks, 1 to 2 weeks, 3 weeks, 2 weeks or 1 week long. In an embodiment, the dose of the first cycle and each subsequent cycle is 0.1 µg/kg to 1 µg/kg, 1 µg/kg to 5 µg/kg, or 5 µg/kg to 10 µg/kg. In another embodiment, the dose of the first cycle and each subsequent cycle is 0.1 µg/kg to 0.5 µg/kg, 1 µg/kg to 2 µg/kg, 1 µg/kg to 3 µg/kg, 2 µg/kg to 5 µg/kg, or 2 µg/kg to 4 µg/kg. In another embodiment, the dose of the first cycle and each subsequent cycle is 0.1 µg/kg, 0.25 µg/kg, 0.5 µg/kg, 1 µg/kg, 1.25 µg/kg, 1.5 µg/kg, 1.75 µg/kg, 2 µg/kg, 2.25 µg/kg, 2.5 µg/kg, 2.75 µg/kg, 3 µg/kg, 3.25 µg/kg, 3.5 µg/kg, 4 µg/kg, 4.25 µg/kg, 4.5 µg/kg, 4.75 µg/kg, or 5 µg/kg. In some embodiments, the dose of the first cycle differs from the dose used in one or more subsequent cycles of the cyclical regimen.

In one embodiment, provided herein is a method for preventing, treating and/or managing a disorder in which enhancing IL-15-mediated immune function is beneficial (e.g., cancer, an infectious disease, an immunodeficiency or lymphopenia), comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject a certain number of times per week for a first period of time; and (b) no administration of IL-15/IL-15Ra complex for a second period of time. In some embodiments, the dose of the IL-15/IL-15Ra administered during the first cycle of the cyclical regimen is sequentially escalated. For example, if an IL-15/IL-15Ra complex is administered to a subject 3 times per week for two weeks, then the dose administered to the subject the second time during the first cycle of the cyclical regimen is increased relative to the dose administered the first time, the dose administered to the subject the third time during the first cycle of the cyclical regimen is increased relative to the dose administered the second time, the dose administered to the subject the fourth time is increased relative to the dose administered the third time, the dose administered to the subject the fifth time is increased relative the dose administered the fourth time, and the dose administered to the subject the sixth time is increased relative to the dose administered the fifth time. In some embodiments, the plasma levels of IL-15 and/or lymphocyte counts are monitored. In some embodiments, the subject is monitored for side effects such as a decrease in blood pressure and/or an increase in body temperature and/or an increase in cytokines in plasma. In some embodiments, the dose of the IL-15/IL-15Ra complex administered during the first cycle of the cyclical regimen is sequentially escalated if the subject does not have any side effects. In some embodiments, the dose of the IL-15/IL-15Ra complex administered during the first cycle of the cyclical regimen is sequentially escalated if the subject does not experience any adverse events, such as grade 3 or 4 lymphopenia, grade 3 granulocytopenia, grade 3 leukocytosis (WBC > 100,000/mm³), or organ dysfunction. In some embodiments, the IL-15/IL-15Ra is administered 1, 2, 3, 4, 5, 6 or 7 days per week. In some embodiments, the cyclical regimen is repeated 2, 3, 4, 5, 6, 7, 8, 9, 10 or more times. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long. In some embodiments, the dose during the first cycle of the cyclical regimen is 0.1 µg/kg, 0.25 µg/kg, 0.75 µg/kg, 1 µg/kg, 1.5 µg/kg, 2 µg/kg, 2.5 µg/kg, 3 µg/kg, 3.5 µg/kg, 4 µg/kg, 4.5 µg/kg, or 5 µg/kg. In other embodiments, the dose during the first cycle of the cylical regimen is 0.1 µg/kg to 1 µg/kg, 1 µg/kg to 2 µg/kg, 2 µg/kg to 4 µg/kg or 3 to 5 µg/kg. In some embodiments, the dose used during the a cycle subsequent to the first cycle of the cyclical regimen is 0.1 µg/kg, 0.25 µg/kg, 0.75 µg/kg, 1 µg/kg, 1.5 µg/kg, 2 µg/kg, 2.5 µg/kg, 3 µg/kg, 3.5 µg/kg, 4 µg/kg, 4.5 µg/kg, 5 µg/kg, 10 µg/kg, 15 µg/kg or 20 µg/kg higher than the dose used for the first cycle of the cyclical regimen. In other embodiments, the dose used during a cycle subsequent to the first cycle of the cyclical regimen is 0.1 µg/kg to 1 µg/kg, 1 to 5 µg/kg, 5 µg/kg to 10 µg/kg, or 10 to 20 µg/kg higher than the dose used for the first cycle of the cylical regimen.

In another aspect, provided herein is a method for preventing, treating and/or managing a disorder in which enhancing IL-15-mediated immune function is beneficial (e.g., cancer, an infectious disease, an immunodeficiency or lymphopenia), comprising subcutaneously administering to a subject an IL-15/IL-15Ra complex in a cyclical regimen, wherein cyclical regimen comprises: (a) subcutaneously administering a first dose of the IL-15/IL-15Ra complex to the subject at a certain frequency for a first period of time; (b) no administration of IL-15/IL-15Ra complex for a second period of time; and (c) subcutaneously administering a second dose of the IL-15/IL-15Ra complex to the subject at a certain frequency for a third period of time. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 or 7 days. In some embdodiments, the IL-15/IL-15Ra complex is administered 1, 2, 3, 4, 5, 6 or 7 days per week. In some embodiments, the first, second and third periods of time are the same. In other embodiments, the first, second, and third periods of time are different. In some embodiments, the first and third periods for administration of the IL-15/IL-15Ra complex are 1 week to 4 weeks, 2 to 4 weeks, 2 to 3 weeks or 1 to 2 weeks long. In other embodiments, the first and third periods for administration of the IL-15/IL-15Ra complex are 1 week, 2 weeks, 3 weeks or 4 weeks long. In some embodiments, the second period of administration is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 1 to 3 weeks, 2 weeks, or 1 week long. In some embodiments, the first and second doses of the IL-15/IL-15Ra complex are the same. In other embodiments, the first and second doses of the IL-15/IL-15Ra complex are different. In an embodiment, the first dose is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In some embodiments, the second dose is 0.1 µg/kg, 0.25 µg/kg, 0.5 µg/kg, 0.75 µg/kg, 1 µg/kg, 1.25 µg/kg, 1.5 µg/kg, 1.75 µg/kg 2 µg/kg, 2.5 µg/kg, 3 µg/kg, 3.5 µg/kg, 4 µg/kg, 4.5 µg/kg, 5 µg/kg, 5.5 µg/kg, 6 µg/kg, 6.5 µg/kg, 7 µg/kg, 7.5 µg/kg, 8 µg/kg, 8.5 µg/kg, 9 µg/kg, 9.5 µg/kg, or 10 µg/kg higher than the first dose. In other embodiments, the second dose is 0.1 µg/kg to 1 µg/kg, 1 µg/kg to 2 µg/kg, 2 µg/kg to 5 µg/kg, 5 µg/kg to 10 µg/kg, 10 µg/kg to 15 µg/kg, 15 µg/kg to 20 µg/kg, 20 µg/kg to 25 µg/kg higher than the first dose. In some embodiments, the second dose of the IL-15/IL-15Ra complex is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In other embodiments, the second dose of the IL-15/IL-15Ra complex is 4 µg/kg, 5 µg/kg, 6 µg/kg, 7 µg/kg, 8 µg/kg, 9 µg/kg, 10 µg/kg, 15 µg/kg, 20 µg/kg, or 25 µg/kg.

In an aspect, provided herein are methods for preventing, treating and/or managing a disorder in which enhancing IL-15-mediated immune function is beneficial, wherein the methods involve a cyclical administration regimen comprising a first period of 1 to 3 weeks of subcutaneous administration of an IL-15/IL-15Ra complex to a subject followed by a second period of 1 week to 2 months in which no IL-15/IL-15Ra complex is administered to the subject followed by a third period of 1 week to 3 weeks of subcutaneous administration of the IL-15/IL-15Ra complex to the subject. In some embodiments, a dose of 0.1 to 10 µg/kg, 0.1 to 8 µg/kg, 0.1 to 5 µg/kg, or 0.1 to 2 µg/kg, or about 0.1 µg/kg, about 0.25 µg/kg, about 0.5 µg/kg, about 0.75 µg/kg, about 1 µg/kg, about 1.5 µg/kg, about 2 µg/kg, about 3 µg/kg, about 4 µg/kg, or about 5 µg/kg of IL-15/IL-15Ra complex is subcutaneously administered to the subject every 1 to 5 days, every 1 to 3 days, or every 1 to 3 days during the first and third periods of the cyclical administration regimen. The cyclical administration regimen may be conducted one time, two times, three times, four times, five times, six times, seven times or more, or 2 to 5 times, 5 to 10 times, 10 to 15 times, 15 to 20 times, 20 to 25 times or more. In some embodiments, the cyclical administration regimen is repeated for at least 2 months, at least 3 months, at least 4 months, at least 5 months, at least 6 months, at least 7 months, at least 8 months, at least 9 months, at least 10 months, at least 11 months, at least 1 year or more. In some embodiments, the cyclical administration regimen is repeated for about 2 months, about 3 months, about 4 months, about 5 months, about 6 months, about 7 months, about 8 months, about 9 months, about 10 months, about 11 months, about 1 year or more. In some embodiments, the cyclical administration regimen is repeated for 3 to 6 months, 6 to 9 months, 6 to 12 months, 1 to 1.5 years, 1 to 2 years, 1.5 to 2 years, or more. In some embodiments, the plasma levels of IL-15 and/or lymphocyte counts in the subject are monitored after each dose, after every other dose, and/or prior to administration of the IL-15/IL-15Ra complex in the third period. In some embodiments, the subject is monitored for side effects such as a decrease in blood pressure and/or an increase in body temperature and/or an increase in cytokines in plasma.

In an aspect, provided herein are methods for preventing, treating and/or managing a disorder in which enhancing IL-15-mediated immune function is beneficial, wherein the methods involve a cyclical administration regimen comprising a first two week period of subcutaneous administration of an IL-15/IL-15Ra complex to a subject followed by a second two week period in which no IL-15/IL-15Ra complex is administered to the subject followed by a third two week period of subcutaneous administration of the IL-15/IL-15Ra complex to the subject. In some embodiments, a dose of 0.1 to 10 µg/kg of IL-15/IL-15Ra complex is subcutaneously administered to the subject every 1 to 5 days, every 1 to 3 days, or every 1 to 3 days during the first and third periods of the cyclical administration regimen. This cyclical administration regimen may be conducted one time, two times, three times or more. In some embodiments, the plasma levels of IL-15 and/or lymphocyte counts in the subject are monitored after each dose, after every other dose, or prior to administration of the IL-15/IL-15Ra complex in the third period. In some embodiments, the subject is monitored for side effects such as a decrease in blood pressure and/or an increase in body temperature and/or an increase in cytokines in plasma.

Non-limiting examples of disorders in which it is beneficial to enhance IL-15-mediated immune function include cancer, lymphopenia, immunodeficiencies, infectious diseases, and wounds. In a specific embodiment, the disorder in which it is beneficial to enhance IL-15-mediated immune function is cancer, including metastatic cancer. In another specific embodiment, the disorder in which it is beneficial to enhance IL-15-mediated immune function is melanoma, renal cell carcinoma, non-small cell lung cancer or colon cancer.

In one embodiment, provided herein is a method for preventing, treating and/or managing a disorder in which enhancing IL-15-mediated function is beneficial (*e*.*g*., cancer, an infectious disease, an immunodeficiency or lymphopenia) in a subject in need thereof comprising administering an IL-15/IL-15Ra complex to the subject using a cyclical administration regimen, wherein the cyclical administration regimen comprises: (a) administering subcutaneously to the subject a dose of 0.1 to 10 µg/kg of the IL-15/IL-15Ra complex every 1 to 7 days, every 1 to 5 days, every 1 to 4 days, every 1 to 3 days over a first period of 1 week to 3 weeks; and (b) after a second period of 1 week to 2 months in which no IL-15/IL-15Ra complex is administered to the subject, administering subcutaneously to the subject a dose of 0.1 to 10 µg/kg of the IL-15/IL-15Ra complex every 1 to 7 days, every 1 to 5 days, every 1 to 4 days, every 1 to 3 days over a third period of 1 week to 3 weeks. In another embodiment, provided herein is a method for preventing, treating and/or managing a disorder in which enhancing IL-15-mediated function is beneficial (*e*.*g*., cancer, an infectious disease, an immunodeficiency or lymphopenia) in a subject in need thereof comprising administering an IL-15/IL-15Ra complex to the subject using a cyclical administration regimen, wherein the cyclical administration regimen comprises: (a) administering subcutaneously to the subject a dose of 0.1 to 10 µg/kg of the IL-15/IL-15Ra complex every 1, 2, 3, 4, 5, 6 or 7 days over a first period of 1 week to 3 weeks; and (b) after a second period of 1 week to 2 months in which no IL-15/IL-15Ra complex is administered to the subject, administering subcutaneously to the subject a dose of 0.1 to 10 µg/kg of the IL-15/IL-15Ra complex every 1, 2, 3, 4, 5, 6 or 7 days over a third period of 1 week to 3 weeks. The cyclical administration regimen may be conducted one time, two times, three times, four times, five times, six times, seven times, eight times, nine times, ten times, or more, or 2 to 5 times, 5 to 10 times, 10 to 15 times, 15 to 20 times, 20 to 25 times or more. In some embodiments, the cyclical administration regimen is repeated for at least 2 months, at least 3 months, at least 4 months, at least 5 months, at least 6 months, at least 7 months, at least 8 months, at least 9 months, at least 10 months, at least 11 months, at least 1 year or more. In some embodiments, the cyclical administration regimen is repeated for about 2 months, about 3 months, about 4 months, about 5 months, about 6 months, about 7 months, about 8 months, about 9 months, about 10 months, about 11 months, about 1 year or more. In some embodiments, the cyclical administration regimen is repeated for 3 to 6 months, 6 to 9 months, 6 to 12 months, 1 to 1.5 years, 1 to 2 years, 1.5 to 2 years, or more. In some embodiments, the plasma levels of IL-15 and/or lymphocyte counts in the subject are monitored after each dose in step (a) and/or step (b) of the cyclical administration regimen, after every other dose in step (a) and/or (b) of the cyclical administration regimen, and/or prior to administration of the IL-15/IL-15Ra complex in step (b) of the cyclical administration regimen. In some embodiments, the subject is monitored for side effects, such as a decrease in blood pressure and/or an increase in body temperature and/or an increase in cytokines in plasma, during the cyclical administration regimen and/or following the cessation of the cyclical administration.

In another embodiment, provided herein is a method for preventing, treating and/or managing a disorder in which enhancing IL-15-mediated immune function is beneficial, wherein the method comprises: (a) administering subcutaneously to a subject a dose of approximately 0.1 µg/kg to approximately 10 µg/kg (in some embodiments, approximately 0.1 µg/kg to approximately 5 µg/kg, approximately 0.1 µg/kg to approximately 2 µg/kg, or approximately 0.1 µg/kg to approximately 1 µg/kg) of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg to approximately 10 µg/kg (in some embodiments, approximately 0.1 µg/kg to approximately 5 µg/kg, approximately 0.1 µg/kg to approximately 2 µg/kg, or approximately 0.1 µg/kg to approximately 1 µg/kg) of an IL-15/IL-15Ra complex every 1, 2 or 3 days over a third period of 12 to 14 days. In some embodiments, steps (a) through (c) are repeated two, three, four, five, six, seven, eight, nine, ten or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In some embodiments, the subject is monitored for side effects such as a decrease in blood pressure and/or an increase in body temperature and/or an increase in cytokines in plasma.

In another embodiment, provided herein is a method for preventing, treating and/or managing a disorder in which enhancing IL-15-mediated immune function is beneficial, wherein the method comprises: (a) administering subcutaneously to a subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a third period of 12 to 14 days. In some embodiments, steps (a) through (c) are repeated two, three, four, five, six, seven, eight, nine or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In some embodiments, the subject is monitored for side effects such as a decrease in blood pressure and/or an increase in body temperature and/or an increase in cytokines in plasma.

In another embodiment, provided herein is a method for treating or managing cancer in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In a specific embodiment, the cancer is melanoma, renal cell carcinoma, lung cancer (e.g., non-small cell lung cancer) or colon cancer. In some embodiments, the cancer is metastatic. In a specific embodiment, the cancer is metastatic melanoma, metastatic renal cell carcinoma, metastatic lung cancer (e.g., metastatic non-small cell lung cancer) or metastatic colon cancer.

In an embodiment, provided herein is a method for preventing, treating and/or managing an infectious disease in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 0.25 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In some embodiments, the infectious disease is caused by a viral, bacterial, fungal or parasite infection.

In an embodiment, provided herein is a method for preventing, treating and/or managing an immunodeficiency in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 0.25 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In some embodiments, the immunodeficiency is caused by AIDS or a disorder (*e*.*g*., a genetic disorder).

In an embodiment, provided herein is a method for preventing, treating and/or managing lymphopenia in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In some embodiments, the lymphopenia is caused by a therapy (e.g., chemotherapy, an antiviral agent, or an immunosuppressive agent), or a disease that causes depletion of peripheral circulating lymphocytes.

In another aspect, provided herein are methods for enhancing IL-15-mediated immune function, comprising: (a) administering subcutaneously to a subject a dose of approximately 0.1 µg/kg to approximately 10 µg/kg (in some embodiments, approximately 0.1 µg/kg to approximately 5 µg/kg, approximately 0.1 µg/kg to approximately 2 µg/kg, or approximately 0.1 µg/kg to approximately 1 µg/kg) of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma.

In another aspect, provided herein are methods for preventing, treating and/or managing a disorder in which enhancing IL-15-mediated immune function is beneficial, wherein the methods comprise: (a) administering subcutaneously to a subject a dose of approximately 0.1 µg/kg to approximately 10 µg/kg (in some embodiments, approximately 0.1 µg/kg to approximately 5 µg/kg, approximately 0.1 µg/kg to approximately 2 µg/kg, or approximately 0.1 µg/kg to approximately 1 µg/kg) of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose of the IL-15/IL-15Ra complex recited in step (a). In other embodiments, the dose of the IL-15/IL-15Ra recited in step (c) is the same as the dose of IL-15/IL-15Ra complex administered in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma.

In another embodiment, provided herein is a method for treating or managing cancer in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose of the IL-15/IL-15Ra complex recited in step (a). In other embodiments, the dose of the IL-15/IL-15Ra recited in step (c) is the same as the dose of IL-15/IL-15Ra complex administered in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In a specific embodiment, the cancer is melanoma, renal cell carcinoma, lung cancer (*e*.*g*., non-small cell lung cancer) or colon cancer. In some embodiments, the cancer is metastatic. In a specific embodiment, the cancer is metastatic melanoma, metastatic renal cell carcinoma, metastatic lung cancer (*e*.*g*., metastatic non-small cell lung cancer) or metastatic colon cancer.

In an embodiment, provided herein is a method for preventing, treating and/or managing an infectious disease in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose of the IL-15/IL-15Ra complex recited in step (a). In other embodiments, the dose of the IL-15/IL-15Ra recited in step (c) is the same as the dose of IL-15/IL-15Ra complex administered in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In some embodiments, the infectious disease is caused by a viral, bacterial, fungal or parasite infection.

In an embodiment, provided herein is a method for preventing, treating and/or managing an immunodeficiency in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose of the IL-15/IL-15Ra complex recited in step (a). In other embodiments, the dose of the IL-15/IL-15Ra recited in step (c) is the same as the dose of IL-15/IL-15Ra complex administered in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In some embodiments, the immunodeficiency is caused by AIDS or a disorder (*e*.*g*., a genetic disorder).

In an embodiment, provided herein is a method for preventing, treating and/or managing lymphopenia in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose of the IL-15/IL-15Ra complex recited in step (a). In other embodiments, the dose of the IL-15/IL-15Ra recited in step (c) is the same as the dose of IL-15/IL-15Ra complex administered in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In some embodiments, the lymphopenia is caused by a therapy (e.g., chemotherapy, an antiviral agent, or an immunosuppressive agent), or a disease that causes depletion of peripheral circulating lymphocytes.

In another aspect, provided herein is a method for enhancing IL-15-mediated immune function, comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated a certain number of times and the dose is sequentially escalated as the regimen is repeated. In some embodiments, the plasma levels of IL-15 and/or lymphocyte counts in a subject are monitored at a certain frequency, *e*.*g*., after the each dose and/or after every other dose. In some embodiments, the subject is monitored for side effects such as a decrease in blood pressure and/or an increase in body temperature and/or an increase in cytokines in plasma. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks, or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In an embodiment, the dose of the first cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In another embodiment, the dose of the second cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg higher than the dose used in the first cycle of the cyclical regimen. In another embodiment, the dose of the first cycle of the cylical regimen is 0.1 µg/kg, 0.25 µg/kg, 0.5 µg/kg, 0.75 µg/kg, or 1 µg/kg, and the dose of each subsequent cycle is 0.1 µg/kg, 0.25 µg/kg, 0.5 µg/kg, 0.75 µg/kg, 1 µg/kg, 1.5 µg/kg, 2 µg/kg, 2.5 µg/kg, 3 µg/kg, 4 µg/kg, 5 µg/kg, 6 µg/kg, 7 µg/kg, 8 µg/kg, 9 µg/kg or 10 µg/kg higher than the dose used in the previous cycle of the cyclical regimen. In some embodiments, the dose of the first cycle of the cyclical regimen is 0.1 µg/kg, the dose of the second cycle of the cylical regimen is 0.25 µg/kg, the dose of the third cycle of the cylical regimen is 0.5 µg/kg, the dose of the fourth cycle of the cylical regimen is 1 µg/kg, and the dose of the fifth cycle of the cylical regimen is 2 µg/kg. In other embodiments, the dose of the first cycle of the cyclical regimen is 0.1 µg/kg to 0.25 µg/kg, the dose of the second cycle of the cylical regimen is 0.5 µg/kg to 1 µg/kg, the dose of the third cycle of the cylical regimen is 1 µg/kg to 3 µg/kg, the dose of the fourth cycle of the cylical regimen is 4 µg/kg to 6 µg/kg, and the dose of the fifth cycle of the cylical regimen is 7 µg/kg to 10 µg/kg.

In one embodiment, provided herein is a method for enhancing IL-15-mediated immune function, comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated at least 5 times, and wherein the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 10 µg/kg, the dose during the fourth cycle of the cylical regimen is 10 µg/kg to 20 µg/kg, and the dose during the fifth cycle of the cylical regimen is 20 µg/kg to 30 µg/kg. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In some embodiments, the plasma levels of IL-15 and/or lymphocyte counts in a subject are monitored at a certain frequency, *e*.*g*., after the each dose and/or after every other dose. In some embodiments, the subject is monitored for side effects such as a decrease in blood pressure and/or an increase in body temperature and/or an increase in cytokines in plasma.

In another aspect, provided herein is a method for preventing, treating and/or managing a disorder in which enhancing IL-15-mediated immune function is beneficial (*e*.*g*., cancer, an infectious disease, an immunodeficiency or lymphopenia), comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated a certain number of times and the dose is sequentially escalated as the regimen is repeated. In some embodiments, the plasma levels of IL-15 and/or lymphocyte counts in a subject are monitored at a certain frequency, *e*.*g*., after the each dose and/or after every other dose. In some embodiments, the subject is monitored for side effects such as a decrease in blood pressure and/or an increase in body temperature and/or an increase in cytokines in plasma. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks, or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In an embodiment, the dose of the first cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In another embodiment, the dose of the second cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg higher than the dose used in the first cycle of the cyclical regimen. In another embodiment, the dose of the first cycle of the cylical regimen is 0.1 µg/kg, 0.25 µg/kg, 0.5 µg/kg, 0.75 µg/kg, or 1 µg/kg, and the dose of each subsequent cycle is 0.1 µg/kg, 0.25 µg/kg, 0.5 µg/kg, 0.75 µg/kg, 1 µg/kg, 1.5 µg/kg, 2 µg/kg, 2.5 µg/kg, 3 µg/kg, 4 µg/kg, 5 µg/kg, 6 µg/kg, 7 µg/kg, 8 µg/kg, 9 µg/kg or 10 µg/kg higher than the dose used in the previous cycle of the cyclical regimen. In some embodiments, the dose of the first cycle of the cyclical regimen is 0.1 µg/kg, the dose of the second cycle of the cylical regimen is 0.25 µg/kg, the dose of the third cycle of the cylical regimen is 0.5 µg/kg, the dose of the fourth cycle of the cylical regimen is 1 µg/kg, and the dose of the fifth cycle of the cylical regimen is 2 µg/kg. In other embodiments, the dose of the first cycle of the cyclical regimen is 0.1 µg/kg to 0.25 µg/kg, the dose of the second cycle of the cylical regimen is 0.5 µg/kg to 1 µg/kg, the dose of the third cycle of the cylical regimen is 1 µg/kg to 3 µg/kg, the dose of the fourth cycle of the cylical regimen is 4 µg/kg to 6 µg/kg, and the dose of the fifth cycle of the cylical regimen is 7 µg/kg to 10 µg/kg.

In one embodiment, provided herein is a a method for preventing, treating and/or managing a disorder in which enhancing IL-15-mediated immune function is beneficial (e.g., cancer, an infectious disease, an immunodeficiency or lymphopenia), comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated at least 5 times, and wherein the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 10 µg/kg, the dose during the fourth cycle of the cylical regimen is 10 µg/kg to 20 µg/kg, and the dose during the fifth cycle of the cylical regimen is 20 µg/kg to 30 µg/kg. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks, or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In some embodiments, the plasma levels of IL-15 and/or lymphocyte counts in a subject are monitored at a certain frequency, *e*.*g*., after the each dose and/or after every other dose. In some embodiments, the subject is monitored for side effects such as a decrease in blood pressure and/or an increase in body temperature and/or an increase in cytokines in plasma.

In another embodiment, provided herein is a method for preventing, treating and/or managing a disorder in which enhancing IL-15-mediated immune function is beneficial, comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated, and wherein the dose during each cycle is sequentially escalated until the maximum tolerated dose is achieved or until the subject exhibits one or more adverse events. In some embodiments, the plasma levels of IL-15 and/or lymphocyte counts in a subject are monitored at a certain frequency, *e*.*g*., after the each dose and/or after every other dose. In some embodiments, the subject is monitored for side effects such as a decrease in blood pressure and/or an increase in body temperature and/or an increase in cytokines in plasma, and adverse events, such as grade 3 or 4 lymphopenia, grade 3 granulocytopenia, grade 3 leukocytosis (WBC > 100,000/mm3), or organ dysfunction. In some embodiments, the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 10 µg/kg, the dose during the fourth cycle of the cylical regimen is 10 µg/kg to 20 µg/kg, and the dose during the fifth cycle of the cylical regimen is 20 µg/kg to 30 µg/kg. In other embodiments, the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 15 µg/kg, the dose during the fourth cycle of the cylical regimen is 15 µg/kg to 30 µg/kg, and the dose during the fifth cycle of the cylical regimen is 30 µg/kg to 50 µg/kg. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In some embodiments, the disorder is an infectious disease.

The IL-15/IL-15Ra complex administered to a subject in accordance with the methods described herein may comprise native IL-15 or an IL-15 derivative covalently or noncovalently bound to native IL-15Ra or an IL-15Ra derivative. In one embodiment, the IL-15/IL-15Ra complex comprises native IL-15 and native IL-15Ra. In another embodiment, the IL-15/IL-15Ra complex comprises an IL-15 derivative and native IL-15Ra. In another embodiment, the IL-15/IL-15Ra complex is in the native heterodimeric form. In another embodiment, the IL-15 is human IL-15 and IL-15Ra is human IL-15Ra. In an embodiment, the human IL-15 comprises the amino acid sequence of SEQ ID NO: 1 or amino acid residues 49 to 162 of SEQ ID NO:1 and the human IL-15Ra comprises the amino acid sequence of SEQ ID NO: 3 or a fragment thereof. In another embodiment the IL-15 comprises the amino acid sequence of SEQ ID NO:1 or amino acid residues 49 to 162 of SEQ ID NO:1 and the IL-15Ra comprises the amino acid sequence of SEQ ID NO:4, 32, 33, 34, 35, 36, 37, 38, 39, 40, 41 or 45. In other embodiments, the IL-15Ra is glycosylated such that glycosylation accounts for at least or more than 20%, 30%, 40% or 50% of the mass of the IL-15Ra.

In another embodiment, the IL-15/IL-15Ra complex comprises native IL-15 and an IL-15Ra derivative. In another embodiment, the IL-15/IL-15Ra complex comprises an IL-15 derivative and an IL-15Ra derivative. In one embodiment, the IL-15Ra derivative is a soluble form of the native IL-15Ra. In another embodiment, the IL-15Ra derivative comprises mutation that inhibits cleavage by an endogenous protease. In an embodiment, the extracellular domain cleavage site of IL-15Ra is replaced with a cleavage site that is specifically recognized by a heterologous protease. In one embodiment, the extracellular domain cleavage site of IL-15Ra is replaced with a heterologous extracellular domain cleavage site (*e*.*g*., heterologous transmembrane domain that is recognized and cleaved by another enzyme unrelated to the endogenous processing enzyme that cleaves the IL-15Ra).

In some embodiments, the human IL-15Ra is modified either simultaneously or alternatively as follows: O-glycosylated on Thr5 of amino acid sequence NWELTASASHQPPGVYPQG (SEQ ID NO: 42) in the IL-15Ra; O-glycosylated on Ser7 of amino acid sequence NWELTASASHQPPGVYPQG (SEQ ID NO: 42) in the IL-15Ra; N-glycosylated on Ser 8 of amino acid sequence ITCPPPMSVEHADIWVK (SEQ ID NO: 43) in the IL-15Ra; N-glycosylated on Ser 8 of amino acid sequence ITCPPPMSVEHADIWVKSYSLYSRERYICNS (SEQ ID NO: 44) in the IL-15Ra; N-glycosylated on Ser 18 of amino acid sequence ITCPPPMSVEHADIWVKSYSLYSRERYICNS (SEQ ID NO: 44) in the IL-15Ra; N-glycosylated on Ser 20 of amino acid sequence ITCPPPMSVEHADIWVKSYSLYSRERYICNS (SEQ ID NO: 44) in the IL-15Ra; N-glycosylated on Ser 23 of amino acid sequence ITCPPPMSVEHADIWVKSYSLYSRERYICNS (SEQ ID NO: 44) in the IL-15Ra; and/or N-glycosylated on Ser 31 of amino acid sequence ITCPPPMSVEHADIWVKSYSLYSRERYICNS (SEQ ID NO: 44) in the IL-15Ra.

In some embodiments, the IL-15Ra is a soluble form of IL-15Ra. In an s embodiment, the soluble form of IL-15Ra is a soluble form of human IL-15Ra. In a particular embodiment, the human IL-15Ra comprises SEQ ID NO: 3. In one embodiment, the last amino acids at the C-terminal end of the soluble form of human IL-15Ra consist of amino acid residues PQGHSDTT (SEQ ID NO: 26), wherein T is at the C-terminal end of the amino acid sequence. In one embodiment, the last amino acids at the C-terminal end of the soluble form of human IL-15Ra consist of amino acid residues PQGHSDT (SEQ ID NO: 27), wherein T is at the C-terminal end of the amino acid sequence. In one embodiment, the last amino acids at the C-terminal end of the soluble form of human IL-15Ra consist of amino acid residues PQGHSD (SEQ ID NO: 28), wherein D is at the C-terminal end of the amino acid sequence. In one embodiment, the last amino acids at the C-terminal end of the soluble form of IL-15Ra consist of amino acid residues PQGHS (SEQ ID NO: 29), wherein S is at the C-terminal end of the amino acid sequence. In one embodiment, the last amino acids at the C-terminal end of the soluble form of human IL-15Ra consist of amino acid residues PQGH (SEQ ID NO: 30), wherein H is at the C-terminal end of the amino acid sequence. In one embodiment, the last amino acids at the C-terminal end of the soluble form of human IL-15Ra consist of amino acid residues PQG (SEQ ID NO: 31), wherein G is at the C-terminal end of the amino acid sequence. In some embodiments, the IL-15 is human IL-15. In some embodiments of the methods provided herein, the human IL-15 comprises SEQ ID NO:1 or amino acid residues 49 to 162 of SEQ ID NO:1. In a particular embodiment, the subject treated in accordance with the methods provided herein is human.

In some embodiments, an IL-15/IL-15Ra complex is associated with a cell. In an embodiment, the extracellular domain cleavage site of IL-15Ra that is cleaved by an endogenous processing enzyme is replaced with a heterologous domain (*e*.*g*., heterologous transmembrane domain) or a synthetic amino acid sequence that does not allow cleavage and generation of soluble IL-15Ra. In some embodiments, the extracellular domain cleavage site of IL-15Ra that is cleaved by an endogenous processing enzyme is mutated to inhibit cleavage and generation of soluble IL-15Ra.

In addition to IL-15 and IL-15Ra, the IL-15/IL-15Ra complexes may comprise a heterologous molecule. The heterologous molecule may be conjugated to IL-15 and/or IL-15Ra. The heterologous molecule is conjugated to IL-15 or IL-15Ra in a manner that does not interfere or prevent IL-15 and IL-15Ra from binding to one another and does not interfere or prevent the interaction between the IL-15/IL-15Ra complex and the βγ subunits of the IL-15 receptor. In some embodiments, the heterologous molecule is an antigen associated with a disease that one intends to prevent, treat and/or manage. Non-limiting examples of such antigens include viral antigens, bacterial antigens, parasitic antigens, and tumor antigens. In other embodiments, the heterologous molecule is an antibody that specifically binds to an antigen associated with a disease that one intends to prevent, treat and/or manage. In some embodiments, the antibody specifically binds to a cellular antigen (*e*.*g*., a receptor) expressed by a cell that one desires to target. In some embodiments, the heterologous molecule increases protein stability. In some embodiments, the heterologous molecule is an Fc domain of an immunoglobulin or a fragment thereof. In an embodiment, IL-15Ra is conjugated/fused to the Fc domain of an immunoglobulin (*e*.*g*., an IgG1). In other embodiments, the heterologous molecule is not an Fc domain of an immunoglobulin molecule or a fragment thereof.

In a specific aspect, the present invention provides an IL-15/IL-15Ra complex for use in treating cancer in a subject in need thereof, comprising administering the IL-15/IL-15Ra complex to the subject using cyclical administration regimen, wherein the cyclical administration regimen comprises: (a) administering subcutaneously to the subject a dose of 0.1 to 10 µg/kg of the IL-15/IL-15Ra complex every 1, 2 or 3 days over a first period of 1 week to 3 weeks; and (b) after a second period of 1 week to 2 months in which no IL-15/IL-15Ra complex is administered to the subject, administering subcutaneously to the subject a dose of 0.1 to 10 µg/kg of the IL-15/IL-15Ra complex every 1, 2 or 3 days over a third period of 1 week to 3 weeks. The cancer may be melanoma, renal cell carcinoma, non-small cell lung cancer or colon cancer and/or is a metastatic cancer. In a specific embodiment, the dose of the IL-15/IL-15Ra complex administered over the first period and over the third period may be 0.1 µg/kg, 0.25 µg/kg, 0.5 µg/kg, 1 µg/kg, 2 µg/kg or 5 µg/kg and the first period, the second period, and/or the third period may be 12 to 14 days. Furthermore, the cyclical administration regimen may be repeated at least 5 or at least 10 times and for at least 6 months or at least 1 year. In a specific embodiment, the IL-15/IL-15Ra complex is a heterodimeric complex of native IL-15 and native soluble IL-15Ra, wherein the IL-15 is human IL-15, and wherein IL-15Ra is a soluble form of human IL-15Ra. In a specific embodiment, the IL-15 comprises amino acid residues 49 to 162 of the amino acid sequence of SEQ ID NO: 1 and IL-15Ra comprises the amino acid sequence of SEQ ID NO: 33, 35, 37, 39, 41 or 45. Alternatively, the IL-15 comprises amino acid residues 49 to 162 of the amino acid sequence of SEQ ID NO: 1 and IL-15Ra comprises the amino acid sequence of SEQ ID NO: 33 or the IL-15Ra is glycosylated such that glycosylation accounts for at least or more than 20%, 30%, 40% or 50% of the mass of the IL-15Ra. In addition, the IL-15/IL-15Ra complex for use in treating cancer further comprises administering another therapy, wherein the other therapy is an antibody that immunospecifically binds to PD-1 or an antibody that immunospecifically binds to PD-L1.

### 3.1 TERMINOLOGY

As used herein, the terms "about" and "approximately," when used to a modify numeric value or numeric range, indicate that the numeric value or range as well as reasonable deviations from the value or range, typically 5% or 10% above and 5% or 10% below the value or range, are within the intended meaning of the recited value or range.

As used herein, the terms "disease" and "disorder" are used interchangeably to refer to a condition, in particular, a pathological condition. In certain embodiments, the terms "disease" and "disorder" are used interchangeably to refer to a disease affected by IL-15 signal transduction. In other embodiments, the terms "disease" and "disorder" are used interchangeably to refer to a disease in which the administration of immune cells is beneficial.

As used herein, the terms "specifically binds," "specifically recognizes" and analogous terms in the context of a receptor (*e*.*g*., native IL-15Ra or IL-15 receptor βγ) and a ligand (*e*.*g*., native IL-15) interaction refer to the specific binding or association between the ligand and receptor. Preferably, the ligand has higher affinity for the receptor than for other molecules. In a specific embodiment, the ligand is native IL-15 and the native receptor is IL-15Ra. In another specific embodiment, the ligand is the native IL-15/IL-15Ra complex and the native receptor is the βγ receptor complex. In a further embodiment, the IL-15/IL-15Ra complex binds to the βγ receptor complex and activates IL-15 mediated signal transduction. Ligands that specifically bind a receptor can be identified, for example, by immunoassays, BIAcore, or other techniques known to those of skill in the art.

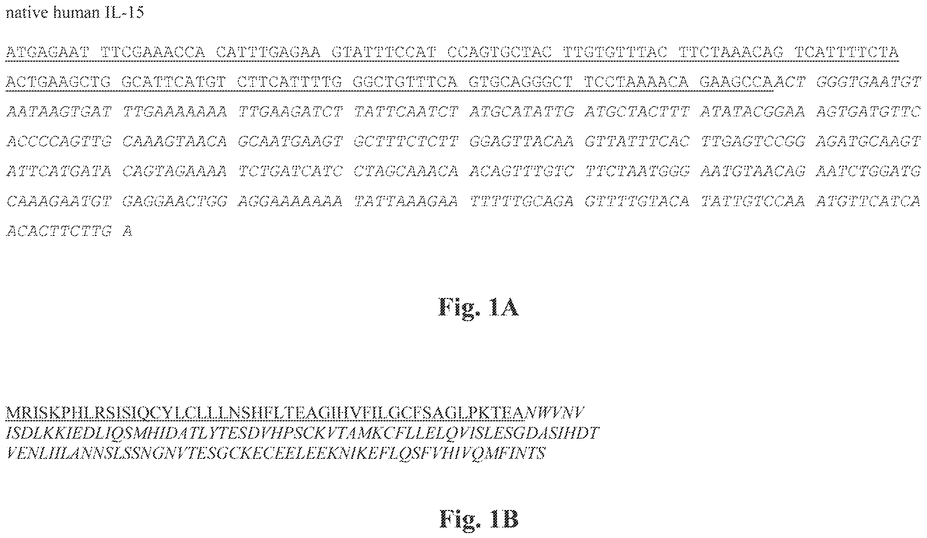

As used herein, the terms "native IL-15" and "native interleukin-15" in the context of proteins or polypeptides refer to any naturally occurring mammalian interleukin-15 amino acid sequences, including immature or precursor and mature forms. Non-limiting examples of GeneBank Accession Nos. for the amino acid sequence of various species of native mammalian interleukin-15 include NP_000576 (human, immature form), CAA62616 (human, immature form), NP_001009207 (Felis catus, immature form), AAB94536 (rattus, immature form), AAB41697 (rattus, immature form), NP_032383 (Mus musculus, immature form), AAR19080 (canine), AAB60398 (macaca mulatta, immature form), AAI00964 (human, immature form), AAH23698 (mus musculus, immature form), and AAH18149 (human). The amino acid sequence of the immature/precursor form of native human IL-15, which comprises the long signal peptide (underlined) and the mature human native IL-15 (italicized), is provided: MRISKPHLRSISIQCYLCLLLNSHFLTEAGIHVFILGCFSAGLPKTEA*NWVNVISDLKKIE DLIQSMHIDATLYTESDVHPSCKVTAMKCFLLELQVISLESGDASIHDTVENLIILANNSLSS NGNVTESGCKECEELEEKNIKEFLQSFVHIVQMFINTS* (SEQ ID NO: 1; FIG. 1B). In some embodiments, native IL-15 is the immature or precursor form of a naturally occurring mammalian IL-15. In other embodiments, native IL-15 is the mature form of a naturally occurring mammalian IL-15. In an embodiment, native IL-15 is the precursor form of naturally occurring human IL-15. In another embodiment, native IL-15 is the mature form of naturally occurring human IL-15. In one embodiment, the native IL-15 protein/polypeptide is isolated or purified.

As used herein, the terms "native IL-15" and "native "interleukin-15" in the context of nucleic acids refer to any naturally occurring nucleic acid sequences encoding mammalian interleukin-15, including the immature or precursor and mature forms. Non-limiting examples of GeneBank Accession Nos. for the nucleotide sequence of various species of native mammalian IL-15 include NM_000585 (human), NM_008357 (Mus musculus), and RNU69272 (rattus norvegicus). The nucleotide sequence encoding the immature/precursor form of native human IL-15, which comprises the nucleotide sequence encoding the long signal peptide (underlined) and the nucleotide sequence encoding the mature human native IL-15 (italicized), is provided: atgagaat ttcgaaacca catttgagaa gtatttccat ccagtgctac ttgtgtttac ttctaaacag tcattttcta actgaagctg gcattcatgt cttcattttg ggctgtttca gtgcagggct tcctaaaaca gaagcca*act gggtgaatgt aataagtgat ttgaaaaaaa ttgaagatct tattcaatct atgcatattg atgctacttt atatacggaa agtgatgttc accccagttg caaagtaaca gcaatgaagt gctttctctt ggagttacaa gttatttcac ttgagtccgg agatgcaagt attcatgata cagtagaaaa tctgatcatc ctagcaaaca acagtttgtc ttctaatggg aatgtaacag aatctggatg caaagaatgt gaggaactgg aggaaaaaaa tattaaagaa tttttgcaga gttttgtaca tattgtccaa atgttcatca acacttcttg a* (SEQ ID NO: 2; FIG. 1A). In an embodiment, the nucleic acid is an isolated or purified nucleic acid. In some embodiments, nucleic acids encode the immature or precursor form of a naturally occurring mammalian IL-15. In other embodiments, nucleic acids encode the mature form of a naturally occurring mammalian IL-15. In an embodiment, nucleic acids encoding native IL-15 encode the precursor form of naturally occurring human IL-15. In another embodiment, nucleic acids encoding native IL-15 encode the mature form of naturally occurring human IL-15.

As used herein, the terms "IL-15 derivative" and "interleukin-15 derivative" in the context of proteins or polypeptides refer to: (a) a polypeptide that is at least 40%, 45%, 50%, 55%, 60%, 65%, 70%, 75%, 80%, 85%, 90%, 95%, 98% or 99% identical to a native mammalian IL-15 polypeptide; (b) a polypeptide encoded by a nucleic acid sequence that is at least 40%, 45%, 50%, 55%, 60%, 65%, 70%, 75%, 80%, 85%, 90%, 95%, 98% or 99% identical a nucleic acid sequence encoding a native mammalian IL-15 polypeptide; (c) a polypeptide that contains 1, 2, 3, 4, 5, 6, 7, 8, 9, 10, 11, 12, 13, 14, 15, 16, 17, 18, 19, 20 or more amino acid mutations (*i.e*., additions, deletions and/or substitutions) relative to a native mammalian IL-15 polypeptide; (d) a polypeptide encoded by nucleic acids can hybridize under high, moderate or typical stringency hybridization conditions to nucleic acids encoding a native mammalian IL-15 polypeptide; (e) a polypeptide encoded by a nucleic acid sequence that can hybridize under high, moderate or typical stringency hybridization conditions to a nucleic acid sequence encoding a fragment of a native mammalian IL-15 polypeptide of at least 20 contiguous amino acids, at least 30 contiguous amino acids, at least 40 contiguous amino acids, at least 50 contiguous amino acids, at least 100 contiguous amino acids, or at least 150 contiguous amino acids; and/or (f) a fragment of a native mammalian IL-15 polypeptide. IL-15 derivatives also include a polypeptide that comprises the amino acid sequence of a naturally occurring mature form of a mammalian IL-15 polypeptide and a heterologous signal peptide amino acid sequence. In an embodiment, an IL-15 derivative is a derivative of a native human IL-15 polypeptide. In another embodiment, an IL-15 derivative is a derivative of an immature or precursor form of naturally occurring human IL-15 polypeptide. In another embodiment, an IL-15 derivative is a derivative of a mature form of naturally occurring human IL-15 polypeptide. In another embodiment, an IL-15 derivative is the IL-15N72D described in, *e.g.,* Zhu et al., 2009, J. Immunol. 183: 3598 or U.S. Patent No. 8,163,879. In another embodiment, an IL-15 derivative is one of the IL-15 variants described in U.S. Patent No. 8,163,879. In one embodiment, an IL-15 derivative is isolated or purified.

In a preferred embodiment, IL-15 derivatives retain at least 50%, 55%, 60%, 65%, 70%, 75%, 80%, 85%, 90%, 95%, 98% or 99% of the function of native mammalian IL-15 polypeptide to bind IL-15Ra polypeptide, as measured by assays well known in the art, *e*.*g*., ELISA, Biacore, co-immunoprecipitation. In another preferred embodiment, IL-15 derivatives retain at least 50%, 55%, 60%, 65%, 70%, 75%, 80%, 85%, 90%, 95%, 98% or 99% of the function of native mammalian IL-15 polypeptide to induce IL-15-mediated signal transduction, as measured by assays well-known in the art, *e*.*g*., electromobility shift assays, ELISAs and other immunoassays. In an embodiment, IL-15 derivatives bind to IL-15Ra and/or IL-15Rβγ as assessed by, *e*.*g*., ligand/receptor binding assays well-known in the art.

Percent identity can be determined using any method known to one of skill in the art. In an embodiment, the percent identity is determined using the "Best Fit" or "Gap" program of the Sequence Analysis Software Package (Version 10; Genetics Computer Group, Inc., University of Wisconsin Biotechnology Center, Madison, Wisconsin). Information regarding hybridization conditions (e.g., high, moderate, and typical stringency conditions) have been described, see, *e.g.,* U.S. Patent Application Publication No. US 2005/0048549 (e.g., paragraphs 72-73).

As used herein, the terms "IL-15 derivative" and "interleukin-15 derivative" in the context of nucleic acids refer to: (a) a nucleic acid sequence that is at least 40%, 45%, 50%, 55%, 60%, 65%, 70%, 75%, 80%, 85%, 90%, 95%, 98% or 99% identical to the naturally occurring nucleic acid sequence encoding a mammalian IL-15 polypeptide; (b) a nucleic acid sequence encoding a polypeptide that is at least 40%, 45%, 50%, 55%, 60%, 65%, 70%, 75%, 80%, 85%, 90%, 95%, 98% or 99% identical the amino acid sequence of a native mammalian IL-15 polypeptide; (c) a nucleic acid sequence that contains 1, 2, 3, 4, 5, 6, 7, 8, 9, 10, 11, 12, 13, 14, 15, 16, 17, 18, 19, 20 or more nucleic acid base mutations (*i*.*e*., additions, deletions and/or substitutions) relative to the naturally occurring nucleic acid sequence encoding a mammalian IL-15 polypeptide; (d) a nucleic acid sequence that hybridizes under high, moderate or typical stringency hybridization conditions to a naturally occurring nucleic acid sequence encoding a mammalian IL-15 polypeptide; (e) a nucleic acid sequence that hybridizes under high, moderate or typical stringency hybridization conditions to a fragment of a naturally occurring nucleic acid sequence encoding a mammalian IL-15 polypeptide; and/or (f) a nucleic acid sequence encoding a fragment of a naturally occurring nucleic acid sequence encoding a mammalian IL-15 polypeptide. In an embodiment, an IL-15 derivative in the context of nucleic acids is a derivative of a naturally occurring nucleic acid sequence encoding a human IL-15 polypeptide. In another embodiment, an IL-15 derivative in the context of nucleic acids is a derivative of a naturally occurring nucleic acid sequence encoding an immature or precursor form of a human IL-15 polypeptide. In another embodiment, an IL-15 derivative in the context of nucleic acids is a derivative of a naturally occurring nucleic acid sequence encoding a mature form of a human IL-15 polypeptide. In another embodiment, an IL-15 derivative in the context of nucleic acids is the nucleic acid sequence encoding the IL-15N72D described in, *e.g.,* Zhu et al., 2009, J. Immunol. 183: 3598 or U.S. Patent No. 8,163,879. In another embodiment, an IL-15 derivative in the context of nucleic acids is the nucleic acid sequence encoding one of the IL-15 variants described in U.S. Patent No. 8,163,879.

IL-15 derivative nucleic acid sequences include codon-optimized nucleic acid sequences that encode native mammalian IL-15 polypeptide, including mature and immature forms of IL-15 polypeptide. In other embodiments, IL-15 derivative nucleic acids include nucleic acids that encode mammalian IL-15 RNA transcripts containing mutations that eliminate potential splice sites and instability elements (*e*.*g*., A/T or A/U rich elements) without affecting the amino acid sequence to increase the stability of the mammalian IL-15 RNA transcripts. In some embodiments, the IL-15 derivative nucleic acid sequence is the codon-optimized sequence in SEQ ID NO: 9 (the amino acid sequence encoded by such a nucleic acid sequence is provided in SEQ ID NO: 10).

In a preferred embodiment, IL-15 derivative nucleic acid sequences encode proteins or polypeptides that retain at least 50%, 55%, 60%, 65%, 70%, 75%, 80%, 85%, 90%, 95%, 98% or 99% of the function of a native mammalian IL-15 polypeptide to bind IL-15Ra, as measured by assays well known in the art, *e.g.,* ELISA, Biacore, co-immunoprecipitation. In another preferred embodiment, IL-15 derivative nucleic acid sequences encode proteins or polypeptides that retain at least 50%, 55%, 60%, 65%, 70%, 75%, 80%, 85%, 90%, 95%, 98% or 99% of the function of a native mammalian IL-15 polypeptide to induce IL-15-mediated signal transduction, as measured by assays well-known in the art, e.g., electromobility shift assays, ELISAs and other immunoassays. In an embodiment, IL-15 derivative nucleic acid sequences encode proteins or polypeptides that bind to IL-15Ra and/or IL-15Rβγ as assessed by, *e*.*g*., ligand/receptor assays well-known in the art.

As used herein, the terms "IL-15" and "interleukin-15" refer to a native IL-15, an IL-15 derivative, or a native IL-15 and an IL-15 derivative.

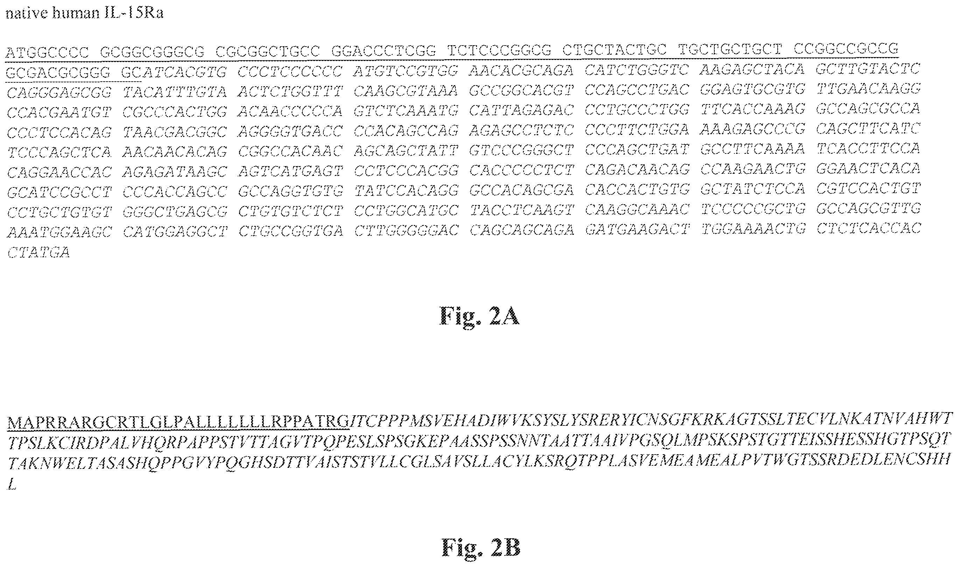

As used herein, the terms "native IL-15Ra" and "native interleukin-15 receptor alpha" in the context of proteins or polypeptides refer to any naturally occurring mammalian interleukin-15 receptor alpha ("IL-15Ra") amino acid sequence, including immature or precursor and mature forms and naturally occurring isoforms. Non-limiting examples of GeneBank Accession Nos. for the amino acid sequence of various native mammalian IL-15Ra include NP_002180 (human), ABK41438 (Macaca mulatta), NP_032384 (Mus musculus), Q60819 (Mus musculus), CAI41082 (human). The amino acid sequence of the immature form of the native full length human IL-15Ra, which comprises the signal peptide (underlined) and the mature human native IL-15Ra (italicized), is provided: MAPRRARGCR TLGLPALLLL LLLRPPATRG *ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQG HSDTTVAIST STVLLCGLSA VSLLACYLKS RQTPPLASVE MEAMEALPVT WGTSSRDEDL ENCSHHL* (SEQ ID NO: 3; FIG. 2B). The amino acid sequence of the immature form of the native soluble human IL-15Ra, which comprises the signal peptide (underlined) and the mature human native soluble IL-15Ra (italicized), is provided: MAPRRARGCR TLGLPALLLL LLLRPPATRG *ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQG* (SEQ ID NO:32). *See* Sections 5.1 and 6 (in particular, Section 6.4), *infra*, for further discussion regarding the immature and mature forms of human native soluble IL-15Ra. n some embodiments, native IL-15Ra is the immature form of a naturally occurring mammalian IL-15Ra polypeptide. In other embodiments, native IL-15Ra is the mature form of a naturally occurring mammalian IL-15Ra polypeptide. In certain embodiments, native IL-15Ra is the naturally occurring soluble form of mammalian IL-15Ra polypeptide. In other embodiments, native IL-15Ra is the full-length form of a naturally occurring mammalian IL-15Ra polypeptide. In an embodiment, native IL-15Ra is the immature form of a naturally occurring human IL-15Ra polypeptide. In another embodiment, native IL-15Ra is the mature form of a naturally occurring human IL-15Ra polypeptide. In certain embodiments, native IL-15Ra is the naturally occurring soluble form of human IL-15Ra polypeptide. In other embodiments, native IL-15Ra is the full-length form of a naturally occurring human IL-15Ra polypeptide. In one embodiment, a native IL-15Ra protein or polypeptide is isolated or purified.

As used herein, the terms "native IL-15Ra" and "native interleukin-15 receptor alpha" in the context of nucleic acids refer to any naturally occurring nucleic acid sequences encoding mammalian interleukin-15 receptor alpha, including the immature or precursor and mature forms. Non-limiting examples of GeneBank Accession Nos. for the nucleotide sequence of various species of native mammalian IL-15Ra include NM_002189 (human), EF033114 (Macaca mulatta), and NM_008358 (Mus musculus). The nucleotide sequence encoding the immature form of native human IL-15Ra, which comprises the nucleotide sequence encoding the signal peptide (underlined) and the nucleotide sequence encoding the mature human native IL-15Ra (italicized), is provided: atggcccc gcggcgggcg cgcggctgcc ggaccctcgg tctcccggcg ctgctactgc tgctgctgct ccggccgccg gcgacgcggg gc*atcacgtg ccctcccccc atgtccgtgg aacacgcaga catctgggtc aagagctaca gcttgtactc cagggagcgg tacatttgta actctggttt caagcgtaaa gccggcacgt ccagcctgac ggagtgcgtg ttgaacaagg ccacgaatgt cgcccactgg acaaccccca gtctcaaatg cattagagac cctgccctgg ttcaccaaag gccagcgcca ccctccacag taacgacggc aggggtgacc ccacagccag agagcctctc cccttctgga aaagagcccg cagcttcatc tcccagctca aacaacacag cggccacaac agcagctatt gtcccgggct cccagctgat gccttcaaaa tcaccttcca caggaaccac agagataagc agtcatgagt cctcccacgg caccccctct cagacaacag ccaagaactg ggaactcaca gcatccgcct cccaccagcc gccaggtgtg tatccacagg gccacagcga caccactgtg gctatctcca cgtccactgt cctgctgtgt gggctgagcg ctgtgtctct cctggcatgc tacctcaagt caaggcaaac tcccccgctg gccagcgttg aaatggaagc catggaggct ctgccggtga cttgggggac cagcagcaga gatgaagact tggaaaactg ctctcaccac ctatga* (SEQ ID NO: 5; FIG. 2A). The nucleotide sequence encoding the immature form of native soluble human IL-15Ra protein or polypeptide, which comprises the nucleotide sequence encoding the signal peptide (underlined) and the nucleotide sequence encoding the mature human soluble native IL-15Ra (italicized), is provided: atggcccc gcggcgggcg cgcggctgcc ggaccctcgg tctcccggcg ctgctactgc tgctgctgct ccggccgccg gcgacgcggg gc*atcacgtg ccctcccccc atgtccgtgg aacacgcaga catctgggtc aagagctaca gcttgtactc cagggagcgg tacatttgta actctggttt caagcgtaaa gccggcacgt ccagcctgac ggagtgcgtg ttgaacaagg ccacgaatgt cgcccactgg acaaccccca gtctcaaatg cattagagac cctgccctgg ttcaccaaag gccagcgcca ccctccacag taacgacggc aggggtgacc ccacagccag agagcctctc cccttctgga aaagagcccg cagcttcatc tcccagctca aacaacacag cggccacaac agcagctatt gtcccgggct cccagctgat gccttcaaaa tcaccttcca caggaaccac agagataagc agtcatgagt cctcccacgg caccccctct cagacaacag ccaagaactg ggaactcaca gcatccgcct cccaccagcc gccaggtgtg tatccacagg gc* (SEQ ID NO: 46). In an embodiment, the nucleic acid is an isolated or purified nucleic acid. In some embodiments, naturally occurring nucleic acids encode the immature form of a naturally occurring mammalian IL-15Ra polypeptide. In other embodiments, naturally occurring nucleic acids encode the mature form of a naturally occurring mammalian IL-15Ra polypeptide. In some embodiments, naturally occurring nucleic acids encode the soluble form of a naturally occurring mammalian IL-15Ra polypeptide. In other embodiments, naturally occurring nucleic acids encode the full-length form of a naturally occurring mammalian IL-15Ra polypeptide. In an embodiment, naturally occurring nucleic acids encode the precursor form of naturally occurring human IL-15 polypeptide. In another embodiment, naturally occurring nucleic acids encode the mature of naturally occurring human IL-15 polypeptide. In some embodiments, naturally occurring nucleic acids encode the soluble form of a naturally occurring human IL-15Ra polypeptide. In other embodiments, naturally occurring nucleic acids encode the full-length form of a naturally occurring human IL-15Ra polypeptide.

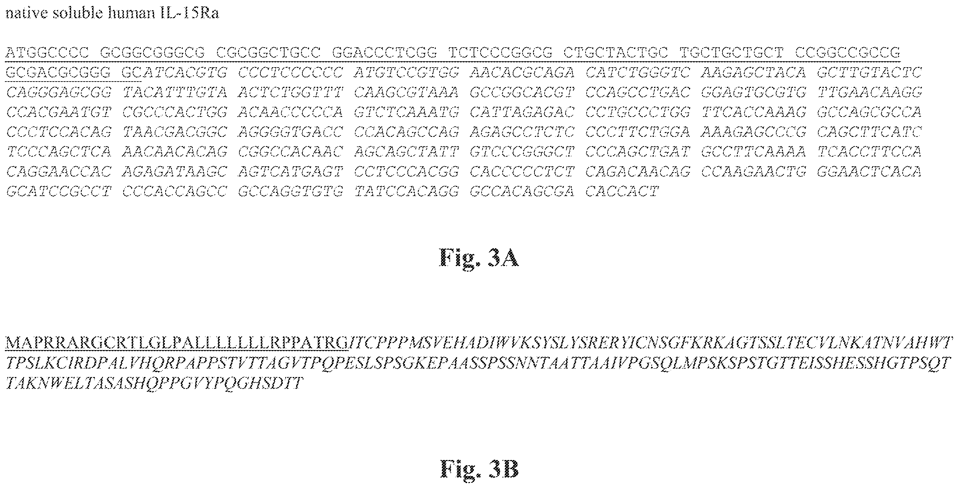

As used herein, the terms "IL-15Ra derivative" and "interleukin-15 receptor alpha derivative" in the context of a protein or polypeptide refer to: (a) a polypeptide that is at least 40%, 45%, 50%, 55%, 60%, 65%, 70%, 75%, 80%, 85%, 90%, 95%, 98% or 99% identical to a native mammalian IL-15 polypeptide; (b) a polypeptide encoded by a nucleic acid sequence that is at least 40%, 45%, 50%, 55%, 60%, 65%, 70%, 75%, 80%, 85%, 90%, 95%, 98% or 99% identical a nucleic acid sequence encoding a native mammalian IL-15Ra polypeptide; (c) a polypeptide that contains 1, 2, 3, 4, 5, 6, 7, 8, 9, 10, 11, 12, 13, 14, 15, 16, 17, 18, 19, 20 or more amino acid mutations (*i.e*., additions, deletions and/or substitutions) relative to a native mammalian IL-15Ra polypeptide; (d) a polypeptide encoded by a nucleic acid sequence that can hybridize under high, moderate or typical stringency hybridization conditions to a nucleic acid sequence encoding a native mammalian IL-15Ra polypeptide; (e) a polypeptide encoded by a nucleic acid sequence that can hybridize under high, moderate or typical stringency hybridization conditions to nucleic acid sequences encoding a fragment of a native mammalian IL-15 polypeptide of at least 20 contiguous amino acids, at least 30 contiguous amino acids, at least 40 contiguous amino acids, at least 50 contiguous amino acids, at least 100 contiguous amino acids, or at least 150 contiguous amino acids; (f) a fragment of a native mammalian IL-15Ra polypeptide; and/or (g) a specific IL-15Ra derivative described herein. IL-15Ra derivatives also include a polypeptide that comprises the amino acid sequence of a naturally occurring mature form of mammalian IL-15Ra polypeptide and a heterologous signal peptide amino acid sequence. In an c embodiment, an IL-15Ra derivative is a derivative of a native human IL-15Ra polypeptide. In another embodiment, an IL-15Ra derivative is a derivative of an immature form of naturally occurring human IL-15 polypeptide. In another embodiment, an IL-15Ra derivative is a derivative of a mature form of naturally occurring human IL-15 polypeptide. In one embodiment, an IL-15Ra derivative is a soluble form of a native mammalian IL-15Ra polypeptide. In other words, in some embodiments, an IL-15Ra derivative includes soluble forms of native mammalian IL-15Ra, wherein those soluble forms are not naturally occurring. An example of an amino acid sequence of a truncated, soluble form of an immature form of the native human IL-15Ra comprises the following signal peptide (underlined) and the following truncated form of human native IL-15Ra (italicized): MAPRRARGCR TLGLPALLLL LLLRPPATRG *ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQG HSDTT* (SEQ ID NO: 4; FIG. 3B). Other examples of IL-15Ra derivatives include the truncated, soluble forms of native human IL-15Ra described in Section 5.1, *infra.* In an embodiment, an IL-15Ra derivative is purified or isolated.

In a preferred embodiment, IL-15Ra derivatives retain at least 50%, 55%, 60%, 65%, 70%, 75%, 80%, 85%, 90%, 95%, 98% or 99% of the function of a native mammalian IL-15Ra polypeptide to bind an IL-15 polypeptide, as measured by assays well known in the art, *e.g.,* ELISA, Biacore, co-immunoprecipitation. In another preferred embodiment, IL-15Ra derivatives retain at least 50%, 55%, 60%, 65%, 70%, 75%, 80%, 85%, 90%, 95%, 98% or 99% of the function of a native mammalian IL-15Ra polypeptide to induce IL-15-mediated signal transduction, as measured by assays well-known in the art, *e*.*g*., electromobility shift assays, ELISAs and other immunoassays. In an embodiment, IL-15Ra derivatives bind to IL-15 as assessed by methods well-known in the art, such as, *e*.*g*., ELISAs.

As used herein, the terms "IL-15Ra derivative" and "interleukin-15 receptor alpha derivative" in the context of nucleic acids refer to: (a) a nucleic acid sequence that is at least 40%, 45%, 50%, 55%, 60%, 65%, 70%, 75%, 80%, 85%, 90%, 95%, 98% or 99% identical to the naturally occurring nucleic acid sequence encoding a mammalian IL-15Ra polypeptide; (b) a nucleic acid sequence encoding a polypeptide that is at least 40%, 45%, 50%, 55%, 60%, 65%, 70%, 75%, 80%, 85%, 90%, 95%, 98% or 99% identical the amino acid sequence of a native mammalian IL-15Ra polypeptide; (c) a nucleic acid sequence that contains 1, 2, 3, 4, 5, 6, 7, 8, 9, 10, 11, 12, 13, 14, 15, 16, 17, 18, 19, 20 or more nucleic acid mutations (*i.e*., additions, deletions and/or substitutions) relative to the naturally occurring nucleic acid sequence encoding a mammalian IL-15Ra polypeptide; (d) a nucleic acid sequence that hybridizes under high, moderate or typical stringency hybridization conditions to a naturally occurring nucleic acid sequence encoding a mammalian IL-15Ra polypeptide; (e) a nucleic acid sequence that hybridizes under high, moderate or typical stringency hybridization conditions to a fragment of a naturally occurring nucleic acid sequence encoding a mammalian IL-15Ra polypeptide; (f) a nucleic acid sequence encoding a fragment of a naturally occurring nucleic acid sequence encoding a mammalian IL-15Ra polypeptide; and/or (g) a nucleic acid sequence encoding a specific IL-15Ra derivative described herein. In an embodiment, an IL-15Ra derivative in the context of nucleic acids is a derivative of a naturally occurring nucleic acid sequence encoding a human IL-15Ra polypeptide. In another embodiment, an IL-15Ra derivative in the context of nucleic acids is a derivative of a naturally occurring nucleic acid sequence encoding an immature form of a human IL-15Ra polypeptide. In another embodiment, an IL-15Ra derivative in the context of nucleic acids is a derivative of a naturally occurring nucleic acid sequence encoding a mature form of a human IL-15Ra polypeptide. In one embodiment, an IL-15Ra derivative in the context of nucleic acids refers to a nucleic acid sequence encoding a derivative of mammalian IL-15Ra polypeptide that is soluble. In some embodiments, an IL-15Ra derivative in context of nucleic acids refers to a nucleic acid sequence encoding a soluble form of native mammalian IL-15Ra, wherein the soluble form is not naturally occurring. In some embodiments, an IL-15Ra derivative in the context of nucleic acids refers to a nucleic acid sequence encoding a derivative of human IL-15Ra, wherein the derivative of the human IL-15Ra is a soluble form of IL-15Ra that is not naturally occurring. An example of an IL-15Ra derivative nucleic acid sequence is the nucleotide sequence encoding the truncated, soluble, immature form of a native human IL-15Ra protein or polypeptide that comprises the following nucleotide sequence encoding the signal peptide (underlined) and the following nucleotide sequence encoding a truncated form of the mature human native IL-15Ra (italicized): atggcccc gcggcgggcg cgcggctgcc ggaccctcgg tctcccggcg ctgctactgc tgctgctgct ccggccgccg gcgacgcggg gc*atcacgtg ccctcccccc atgtccgtgg aacacgcaga catctgggtc aagagctaca gcttgtactc cagggagcgg tacatttgta actctggttt caagcgtaaa gccggcacgt ccagcctgac ggagtgcgtg ttgaacaagg ccacgaatgt cgcccactgg acaaccccca gtctcaaatg cattagagac cctgccctgg ttcaccaaag gccagcgcca ccctccacag taacgacggc aggggtgacc ccacagccag agagcctctc cccttctgga aaagagcccg cagcttcatc tcccagctca aacaacacag cggccacaac agcagctatt gtcccgggct cccagctgat gccttcaaaa tcaccttcca caggaaccac agagataagc agtcatgagt cctcccacgg caccccctct cagacaacag ccaagaactg ggaactcaca gcatccgcct cccaccagcc gccaggtgtg tatccacagg gccacagcga caccact* (SEQ ID NO:6; FIG. 3A). In some embodiments, an IL-15Ra derivative nucleic acid sequence is isolated or purified.

IL-15Ra derivative nucleic acid sequences include codon-optimized nucleic acid sequences that encode native IL-15Ra polypeptide, including mature and immature forms of IL-15Ra polypeptide. In other embodiments, IL-15Ra derivative nucleic acids include nucleic acids that encode IL-15Ra RNA transcripts containing mutations that eliminate potential splice sites and instability elements (*e*.*g*., A/T or A/U rich elements) without affecting the amino acid sequence to increase the stability of the IL-15Ra RNA transcripts. In some embodiments, the IL-15Ra derivative nucleic acid sequence is the codon-optimized sequence in SEQ ID NO: 11, 13, 15 or 17 (the amino acid sequences encoded by such a nucleic acid sequences are provided in SEQ ID NO: 12, 14, 16 and 19, respectively).

In a preferred embodiment, IL-15Ra derivative nucleic acid sequences encode proteins or polypeptides that retain at least 50%, 55%, 60%, 65%, 70%, 75%, 80%, 85%, 90%, 95%, 98% or 99% of the function of a native mammalian IL-15Ra polypeptide to bind IL-15, as measured by assays well known in the art, *e.g*., ELISA, Biacore, co-immunoprecipitation. In another preferred embodiment, IL-15Ra derivative nucleic acid sequnces encode proteins or polypeptides that retain at least 50%, 55%, 60%, 65%, 70%, 75%, 80%, 85%, 90%, 95%, 98% or 99% of the function of a native mammalian IL-15Ra to induce IL-15-mediated signal transduction, as measured by assays well-known in the art, *e.g*., electromobility shift assays, ELISAs and other immunoassays. In an embodiment, IL-15Ra derivative nucleic acid sequences encode proteins or polypeptides that bind to IL-15 as assessed by methods well-known in the art, such as, *e.g*., ELISAs.

As used herein, the terms "IL-15Ra" and "interleukin-15 receptor alpha" refer to a native IL-15Ra, an IL-15Ra derivative, or a native IL-15Ra and an IL-15Ra derivative.

As used herein, the term "IL-15/IL-15Ra complex" refers to a complex comprising IL-15 and IL-15Ra covalently or noncovalently bound to each other. In a preferred embodiment, the IL-15Ra has a relatively high affinity for IL-15, *e.g*., K_{d} of 10 to 50 pM as measured by a technique known in the art, *e*.*g*., KinEx A assay, plasma surface resonance (*e*.*g*., BIAcore assay). In another preferred embodiment, the IL-15/IL-15Ra complex induces IL-15-mediated signal transduction, as measured by assays well-known in the art, *e*.*g*., electromobility shift assays, ELISAs and other immunoassays. In some embodiments, the IL-15/IL-15Ra complex retains the ability to specifically bind to the βγ chain. In an embodiment, the IL-15/IL-15Ra complex is isolated from a cell.

As used herein, the terms "subject" and "patient" are used interchangeably and refer to a mammal such as a non-primate (*e*.*g*., cows, pigs, horses, cats, dogs, rats etc.) and a primate (*e*.*g*., monkey and human), most preferably a human.

As used herein, the terms "purified" and "isolated" in the context of a compound or agent (including, *e*.*g*., proteinaceous agents) that is chemically synthesized refers to a compound or agent that is substantially free of chemical precursors or other chemicals when chemically synthesized. In an embodiment, the compound or agent is 60%, 65%, 75%, 80%, 85%, 90%, 95%, or 99% free (by dry weight) of other, different compounds or agents.

As used herein, the terms "purified " and "isolated" when used in the context of a compound or agent (including proteinaceous agents such as polypeptides) that can be obtained from a natural source, *e.g*., cells, refers to a compound or agent which is substantially free of contaminating materials from the natural source, *e.g*., soil particles, minerals, chemicals from the environment, and/or cellular materials from the natural source, such as but not limited to cell debris, cell wall materials, membranes, organelles, the bulk of the nucleic acids, carbohydrates, proteins, and/or lipids present in cells. The phrase "substantially free of natural source materials" refers to preparations of a compound or agent that has been separated from the material (*e*.*g*., cellular components of the cells) from which it is isolated. Thus, a compound or agent that is isolated includes preparations of a compound or agent having less than about 30%, 20%, 10%, 5%, 2%, or 1% (by dry weight) of cellular materials and/or contaminating materials.

An "isolated" nucleic acid sequence or nucleotide sequence is one which is separated from other nucleic acid molecules which are present in a natural source of the nucleic acid sequence or nucleotide sequence. Moreover, an "isolated", nucleic acid sequence or nucleotide sequence, such as a cDNA molecule, can be substantially free of other cellular material or culture medium when produced by recombinant techniques, or substantially free of chemical precursors when chemically synthesized. In certain embodiments, an "isolated" nucleic acid sequence or nucleotide sequence is a nucleic acid sequence or nucleotide sequence that is recombinantly expressed in a heterologous cell.

In some embodiments, the terms "nucleic acid", "nucleotide" and "polynucleotide" refer to deoxyribonucleotides, deoxyribonucleic acids, ribonucleotides, and ribonucleic acids, and polymeric forms thereof, and include either single- or double-stranded forms. In certain embodiments, such terms include known analogues of natural nucleotides, for example, peptide nucleic acids ("PNA"s), that have similar binding properties as the reference nucleic acid. In some embodiments, such terms refer to deoxyribonucleic acids (*e.g*., cDNA or DNA). In other embodiments, such terms refer to ribonucleic acid (*e.g*., mRNA or RNA).

As used herein, the terms "therapies" and "therapy" can refer to any protocol(s), method(s), compositions, formulations, and/or agent(s) that can be used in the prevention, treatment, management, or amelioration of a disease, *e*.*g*., cancer, infectious disease, lymphopenia, and immunodeficiencies, or a symptom associated therewith. In certain embodiments, the terms "therapies" and "therapy" refer to biological therapy, supportive therapy, and/or other therapies useful in treatment, management, prevention, or amelioration of a disease or a symptom associated therewith known to one of skill in the art. In one embodiment, a therapy includes a Therapeutic Agent. In another embodiment, a therapy is not a Therapeutic Agent.

As used herein, the terms "protein(s)" and "polypeptide(s)" interchangeably to refer to a chain of amino acids linked together by peptide bonds. In some embodiments, the terms "protein(s)" and "polypeptide(s)" refer to a macromolecule which comprises amino acids that are linked together by peptide bonds.

As used herein, the term "fragment" in the context of a nucleotide sequence refers to a nucleotide sequence comprising an nucleic acid sequence of at least 5 contiguous nucleic acid bases, at least 10 contiguous nucleic acid bases, at least 15 contiguous nucleic acid bases, at least 20 contiguous nucleic acid bases, at least 25 contiguous nucleic acid bases, at least 40 contiguous nucleic acid bases, at least 50 contiguous nucleic acid bases, at least 60 contiguous nucleic acid bases, at least 70 contiguous nucleic acid bases, at least 80 contiguous nucleic acid bases, at least 90 contiguous nucleic acid bases, at least 100 contiguous nucleic acid bases, at least 125 contiguous nucleic acid bases, at least 150 contiguous nucleic acid bases, at least 175 contiguous nucleic acid bases, at least 200 contiguous nucleic acid bases, or at least 250 contiguous nucleic acid bases of the nucleotide sequence of the gene of interest, *e.g*., *IL-15, IL-15Ra.* The nucleic acid may be RNA, DNA, or a chemically modified variant thereof. In an embodiment, the fragment is a fragment of *IL-15* or *IL-15Ra.*

As used herein, the term "fragment" is the context of a fragment of a proteinaceous agent (*e.g*., a protein or polypeptide) refers to a fragment that is composed of 8 or more contiguous amino acids, 10 or more contiguous amino acids, 15 or more contiguous amino acids, 20 or more contiguous amino acids, 25 or more contiguous amino acids, 50 or more contiguous amino acids, 75 or more contiguous amino acids, 100 or more contiguous amino acids, 150 or more contiguous amino acids, 200 or more contiguous amino acids, 10 to 150 contiguous amino acids, 10 to 200 contiguous amino acids, 10 to 250 contiguous amino acids, 10 to 300 contiguous amino acids, 50 to 100 contiguous amino acids, 50 to 150 contiguous amino acids, 50 to 200 contiguous amino acids, 50 to 250 contiguous amino acids or 50 to 300 contiguous amino acids of a proteinaceous agent, *e.g*., IL-15 and IL-15Ra polypeptides.

As used herein, the term "in combination" refers to the use of more than one therapies (*e*.*g*., one or more prophylactic and/or therapeutic agents). The use of the term "in combination" does not restrict the order in which therapies are administered to a subject with a disease or disorder. A first therapy (*e.g*., a prophylactic or therapeutic agent) can be administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g*., a prophylactic or therapeutic agent) to a subject with a disease or disorder or a symptom thereof.

As used herein, the term "host cell" refers to any type of cell, *e.g*., a primary cell or a cell from a cell line. In some embodiments, the term "host cell" refers a cell transfected with a nucleic acid molecule and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

As used herein, the term "Engineered Cell(s)" refers to a host cell that recombinantly expresses an IL-15Ra polypeptide described herein (*e.g*., Section 3.1, *supra,* Section 5.1 and/or Section 5.2, *infra*)*,* or a host cell that recombinantly expresses an IL-15Ra polypeptide described herein (*e.g*., Section 3.1, *supra*, Section 5.1 and/or Section 5.2, *infra*) and an IL-15 polypeptide. An Engineered Cell(s) may be produced as described in Section 5.3.2, *infra*, and an Engineered Cell(s) may be used as described in Sections 5.3.2, 5.6 to 5.9, and 5.11, *infra.*

As used herein, the term "premature human infant" refers to a human infant born at less than 37 weeks of gestational age.

As used herein, the term "human infant" refers to a newborn to 1 year old year human.

As used herein, the term "human child" refers to a human that is 1 year to 18 years old.

As used herein, the term "human adult" refers to a human that is 18 years or older.

As used herein, the term "elderly human" refers to a human 65 years or older.

As used herein, the terms "treat", "treating" and "treatment" in the context of the administration of a therapy to a subject refer to the beneficial effects that a subject derives from a therapy. Non-limiting examples of such benefits include the reduction or inhibition of the progression, spread and/or duration of a disease or disorder, the reduction or amelioration of the severity of a disease or disorder, amelioration of one or more symptoms of a disease or disorder, and/or the reduction in the duration of one or more symptom of a disease or disorder resulting from the administration of one or more therapies.

As used herein, the terms "prevent," " preventing" and "prevention" in the context of the administration of a therapy to a subject refer to the inhibition of the onset or recurrence of a disease or disorder in a subject.

As used herein, the terms "manage," "managing," and "management," in the context of the administration of a therapy to a subject, refer to the beneficial effects that a subject derives from a therapy, which does not result in a cure of a disease or disorder. In certain embodiments, a subject is administered one or more therapies to "manage" a disease or disorder so as to prevent the progression or worsening of symptoms associated with a disease or disorder.

As used herein, the term "immunospecifically binds" and "specifically binds" in the context of antibodies refer to molecules that specifically bind to an antigen (*e*.*g*., an epitope or an immune complex) and do not specifically bind to another molecule. A molecule that specifically binds to an antigen may bind to other antigens with a lower affinity as determined by, *e*.*g*., immunoassays, BIAcore or other assays known in the art. In a specific embodiment, molecules that bind to an antigen do not cross-react with other antigens.

When a dose of an IL-15/IL-15Ra complex is referenced herein, the dose is according to the mass of the single-chain IL-15. The single-chain IL-15 equivalent is calculated from (i) the mass of an IL-15/IL-15Ra complex by amino acid analysis and (ii) the ratio of IL-15 to IL-15Ra (*e.g*., soluble IL-15Ra) in the specific preparation as determined experimentally by RP-HPLC or by amino acid analysis.

### 4. BRIEF DESCRIPTION OF THE DRAWINGS

**Figures. 1A****-B:** Nucleic acid and amino acid sequences of native human IL-15. The nucleic acid sequence (FIG. 1A) (SEQ ID NO: 2) and amino acid sequence (FIG. 1B) (SEQ ID NO: 1) are shown. The amino acid sequence and nucleic acid sequence of the long signal peptide (underlined) and mature form (italicized) are indicated.
**Figures 2A****-B:** Nucleic acid and amino acid sequences of full length native human IL-15Ra. The nucleic acid sequence (FIG. 2A) (SEQ ID NO: 5) and amino acid sequence (FIG. 2B) (SEQ ID NO: 3) are shown. The amino acid sequence and nucleic acid sequence of the signal peptide (underlined) and mature form (italicized) are indicated.
**Figures 3A****-B:** Nucleic acid and amino acid sequences of a soluble form of human IL-15. The nucleic acid sequence (FIG. 3A) (SEQ ID NO: 6) and amino acid sequence (FIG. 3B) (SEQ ID NO: 4) are shown. The amino acid sequence and nucleic acid sequence of the signal peptide (underlined) and mature form (italicized) are indicated.

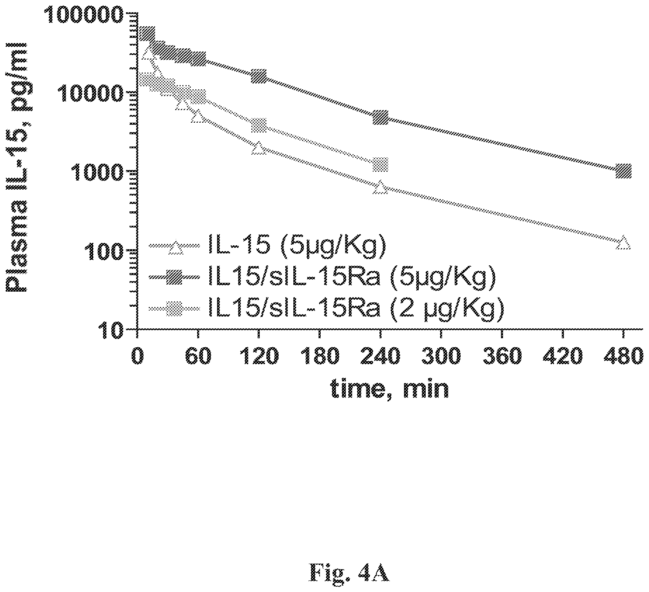

**Figure 4A-D**. Decay in plasma concentration of IL-15 overtime and AUC for plasma IL-15 levels after i.v. and s.c. injections. (A) Decay in plasma concentration of IL-15 overtime after i.v. injection. Three macaques were i.v. injected with *E*.*coli*-derived monomeric IL-15 (line with triangles) or with heterodimeric IL-15/IL-15Ra (lines with squares) at the indicated dose. Blood was collected at the indicated time point and plasma IL-15 levels were determined by ELISA. (B) Decay in plasma concentration of IL-15 overtime after s.c. injection. Two macaques were injected s.c. with heterodimeric IL-15/sIL-15Ra at a dose of 5 µg/Kg. Blood was collected at the indicated time points and plasma IL-15 levels were determined by ELISA. (C) AUC for plasma IL-15 levels upon i.v. and s.c. injection. The AUC were determined for four macaques injected with the indicated IL-15 formulations at the dose of 5 µg/Kg. First three Bars refer to macaques that received IL-15/IL-15Ra heterodimer either s.c. or i.v. The last bar refers to macaque that received monomeric IL-15 i.v. Blood was collected over a period of 72 hours after injection and plasma IL-15 levels were determined by ELISA. AUC was calculated using Prism Software Package. (D) i.v. versus s.c. delivery in macaques (5 µg/Kg). Pharmacokinetics of s.c. administration of IL-15 heterodimer compared to the same amount of i.v. administered IL-15 heterodimer. Administration of same concentration of *E*. *coli*-produced IL-15 is also shown (line with triangles).

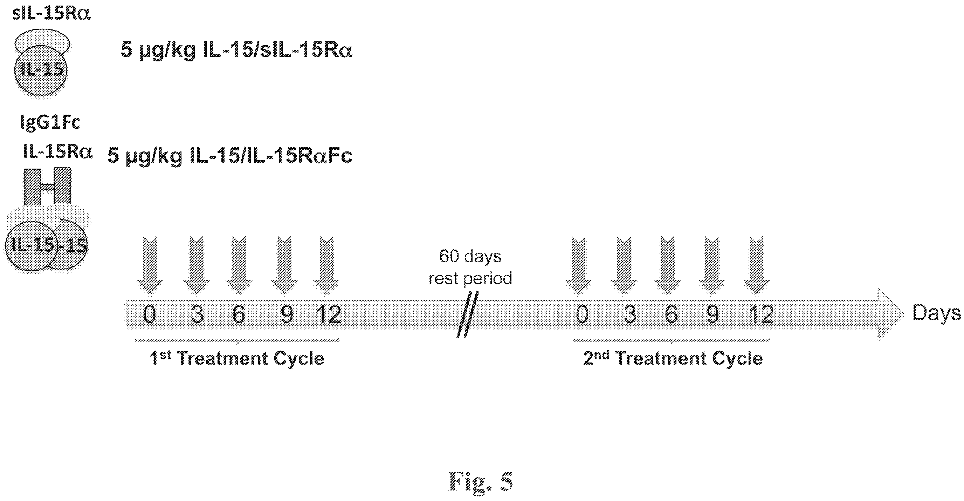

**Figure 5**. Study design showing two cycle treatments of 5 s.c. doses of IL-15/sIL-15Ra or IL-15/IL-15RaFc in Macaques.

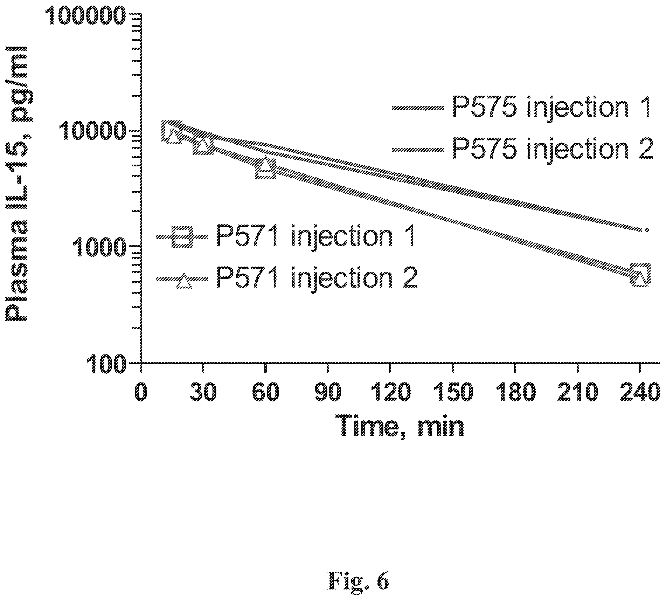

**Figure 6**. Plasma IL-15 levels after repeated i.v. injection of IL-15/IL-15Ra. Two macaques (P571 and M575) received 12 daily i.v. injections of IL-15/IL-15Ra at the dose of 2 µg/Kg. Blood was drawn at different time points after the first and the second injection and plasma IL-15 levels were determined by ELISA.

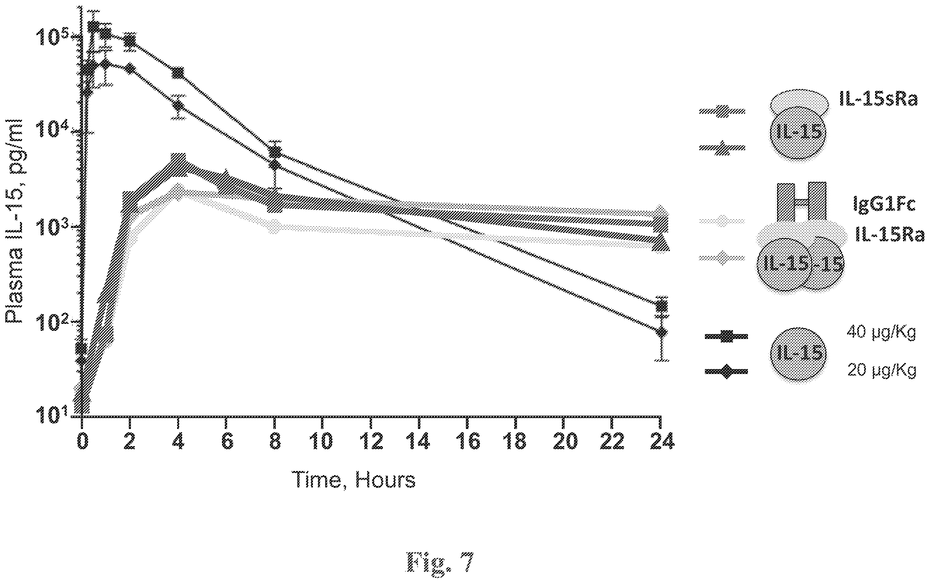

**Figure 7**. Plasma IL-15 levels after s.c. injection of IL-15/IL-15Ra forms. Pharmacokinetics of heterodimeric IL-15 forms in macaques when administered s.c. at 5 µg/Kg.

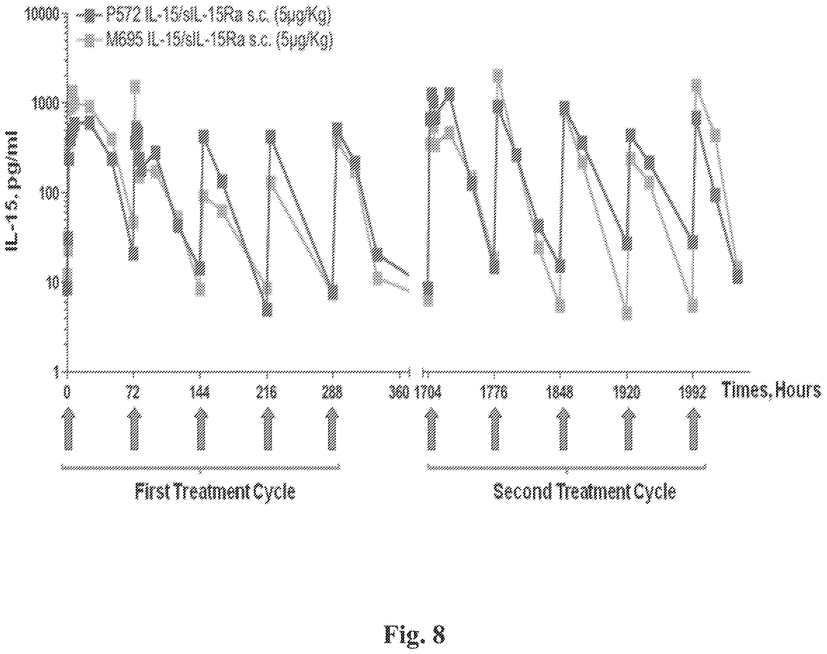

**Figure 8****.** Plasma IL-15 levels after repeated s.c. injection of IL-15/IL-15Ra. Two macaques (P572 and M695) received 5 s.c. injections of IL-15/IL-15Ra at the dose of 5 µg/Kg every 3 days. After a rest period of two months, a second cycle treatment identical to the first was conducted. Arrows indicate the time of IL-15/IL-15Ra injections. Blood was drawn at different time points after each injection and plasma IL-15 levels were determined by ELISA and plotted as function of time after initiation of the first treatment cycle.

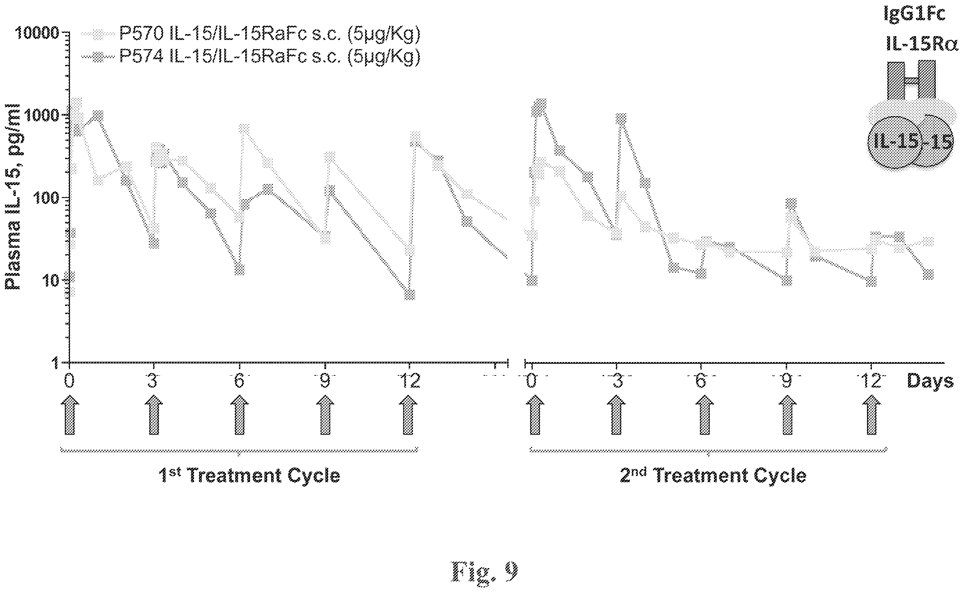

**Figure 9**. Plasma IL-15 levels upon repeated s.c. injections of IL-15/IL-15RaFc. Plasma levels of IL-15 as measured by ELISA after s.c. injections of two macaques. Two macaques (P570 and P574) received 5 µg/kg body weight of IL-15 heterodimers every 3^{rd} day (5 s.c. injections). Two 2-week treatment cycles were completed. Arrows indicate the time of IL-15/IL-15RaFc injections.

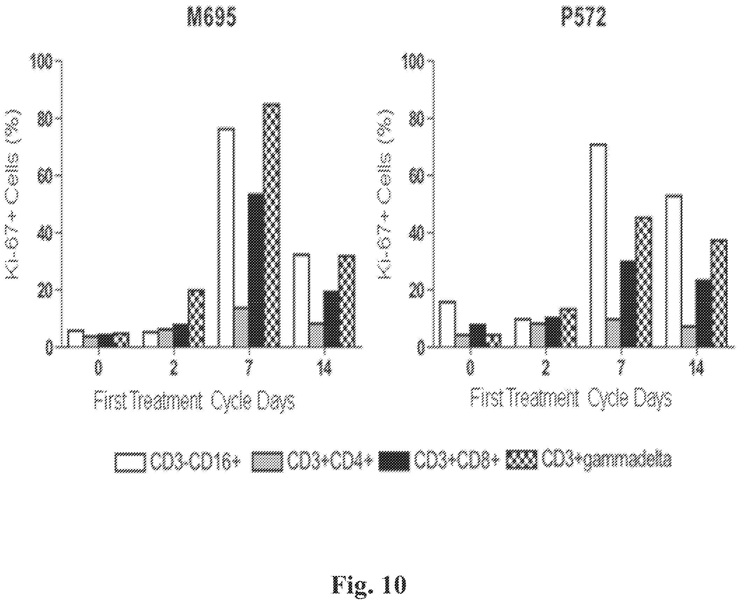

**Figure 10****.** Expression of Ki-67 on NK, CD4+T, CD8+T and gammadelta T cells after repeated s.c. injections of IL-15/IL-15Ra at 5 µg/Kg. PBMC from macaques M695 and P572 were obtained at the indicated time point after the initiation of the first treatment cycle and were examined for the intracellular expression of the proliferative marker Ki-67 within the NK cell (CD3-CD16+), CD4+T cell (CD3+CD4+), CD8+ T cell (CD3+CD8+) and gammadelta T cell (CD3+gammadelta+) subsets. The percentage (%) of Ki-67+ cells in each subset at the indicated time points is shown.

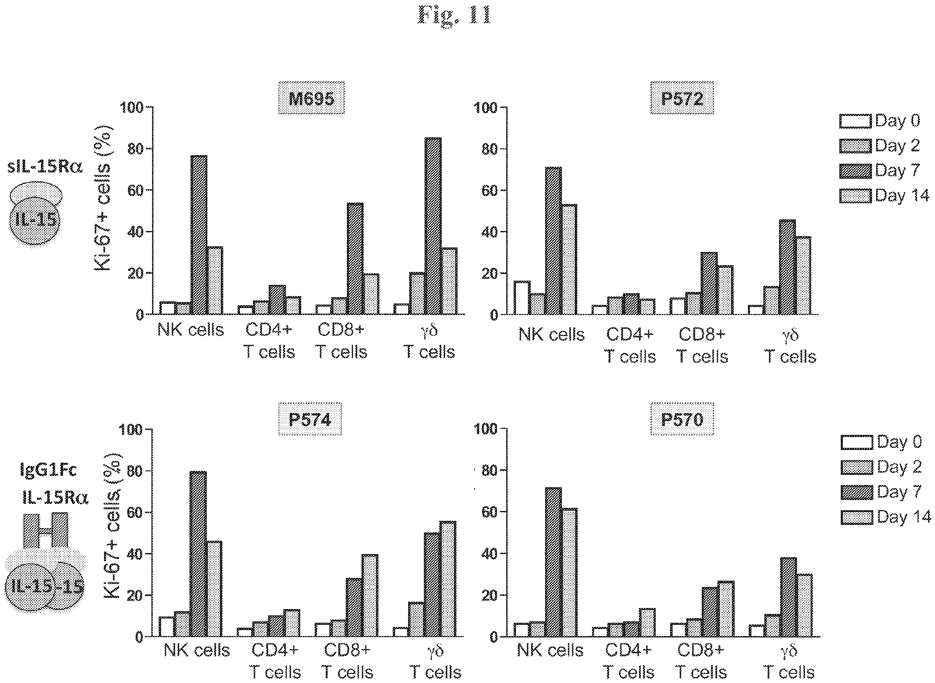

**Figure 11****.** IL-15/sIL-15Ra and IL-15/IL-15RaFc induce similar proliferation of NK and T cells. Increased proliferation of lymphocyte subsets after a single two week cycle of SC IL-15 injections (every third day as indicated in Fig. 1). Two animals were injected by the native purified IL-15 heterodimer and two animals with the IgG1Fc fusion of heterodimer via the IL-15Ra (M695, P572, P574 and P570 macaques used). Lymphocyte subsets were analyzed by multiparameter flow cytometry at days 0, 2, 7 and 14 as indicated. The percentage (%) of Ki-67+ cells in each subset at the indicated time points is shown.

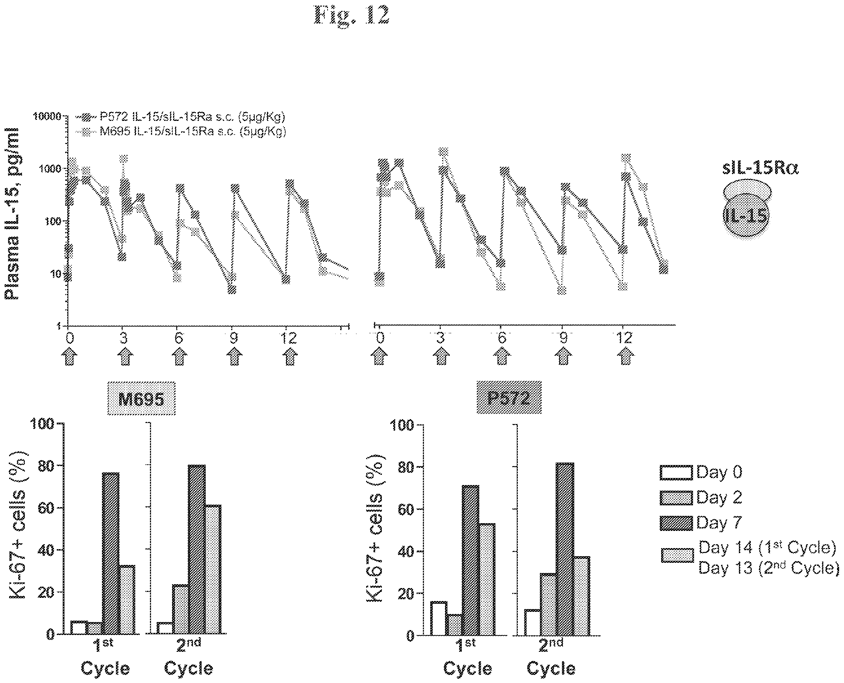

**Figure 12**. Repeated Treatment Cycles with IL-15/sIL-15Ra result in similar peak expansion of NK cells. Biological activity of IL-15 heterodimer after two cycles of s.c. injections. During both cycles the levels of cycling NK cells were increased at day 7 and 13 or 14. Cycling of NK cells was induced earlier during the second IL-15 regimen (day 2). The percentage (%) of Ki-67+ cells in each subset at the indicated time points is shown.

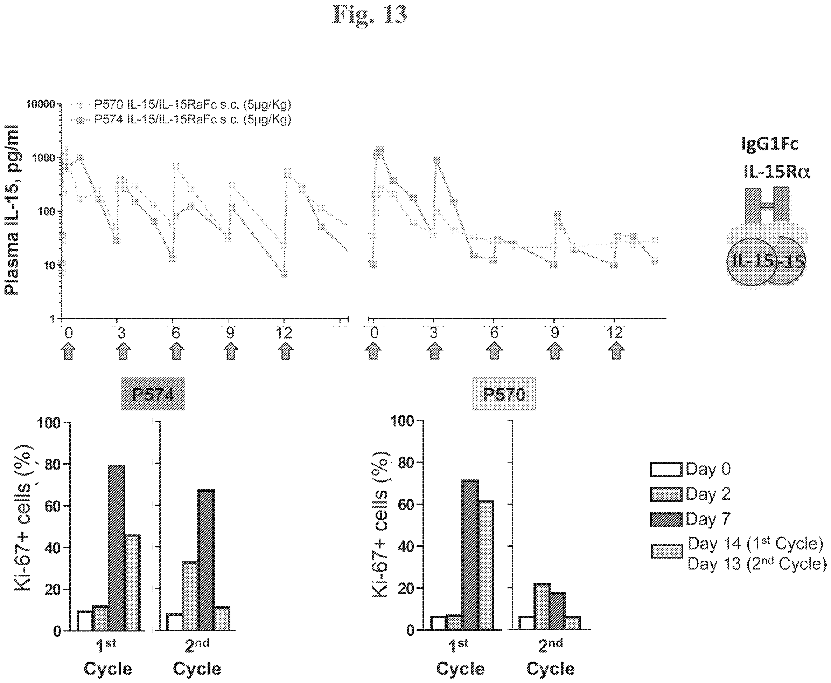

**Figure 13**. Proliferation of NK cells after the 1^{st} and 2^{nd} cycle of IL-15/IL-15RaFc reflects plasma IL-15 levels. The percentage (%) of Ki-67+ cells in each subset at the indicated time points is shown.

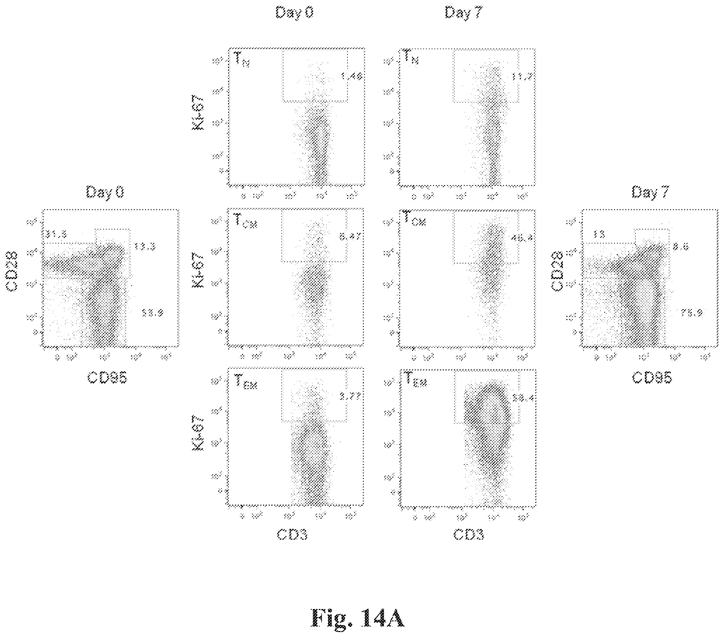

**Figure 14A-C****.** Expression of Ki-67 on CD8+ and CD4+T naive (T_{N}), T central memory (T_{CM}) and T effector memory (T_{EM}) cells after repeated s.c. injections of IL-15/IL-15Ra at 5 µg/Kg. PBMC from macaques M695 (squares) and P572 (triangles) were obtained at the indicated time points after the initiation of the first treatment cycle and were examined for the intracellular expression of the proliferative marker Ki-67 within the CD4+and CD8+ T_{N} (CD95-CD28+), T_{CM} (CD95+CD28+) and T_{EM} (CD95+CD28-). (A) Representative data from CD8+ T cells in macaque M695 receiving 5 s.c. injections of IL-15/IL-15Ra heterodimer at 5 µg/Kg every 3 days. The left panel shows the pre-treatment distribution of CD8+ TN, TCM and TEM subsets. The right panel shows the same distribution at day 7 after the first IL-15/sIL-15Ra injection. The panels in the middle show the pre-treatment and day 7 frequency of Ki-67+ cells in each gated population. (B) The percentage (%) of Ki-67+ cells in each subset at the indicated time points is shown. (C) IL-15 heterodimers target both central and effector memory CD8 T cells and mainly effector memory CD4 T cells. Data from M695, P572, P570 and P574 macaques is presented. The percentage (%) of Ki-67+ cells in each subset at the indicated time points is shown.

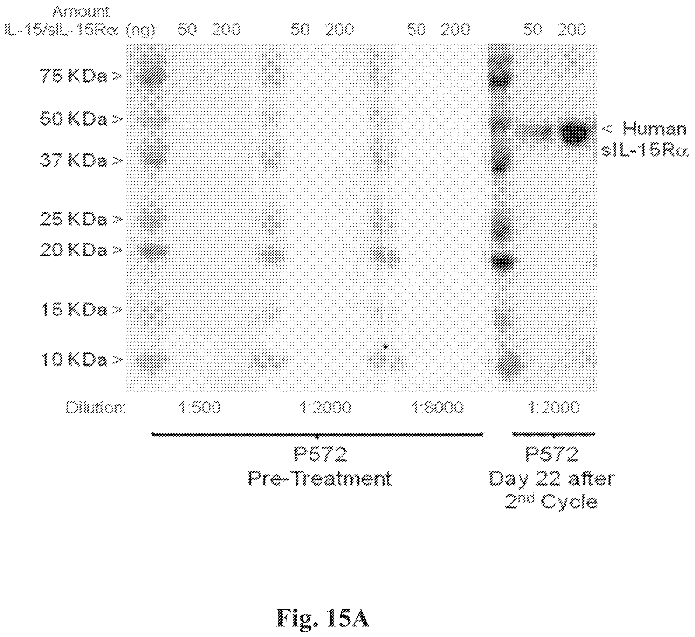

**Figure 15A-B****.** Development of anti-human IL-15Ra antibodies after repeated s.c. injections with IL-15/IL-15Ra (see A and B). The indicated amounts of IL-15/IL-15Ra were loaded in a 12% polyacrylamide gel and denatured proteins were transferred onto a nitrocellulose membranes. The membrane was probed with monkey plasma at the indicated dilutions using a pre-treatment sample and a sample corresponding to day 22 after the second IL-15/IL-15Ra treatment cycle.

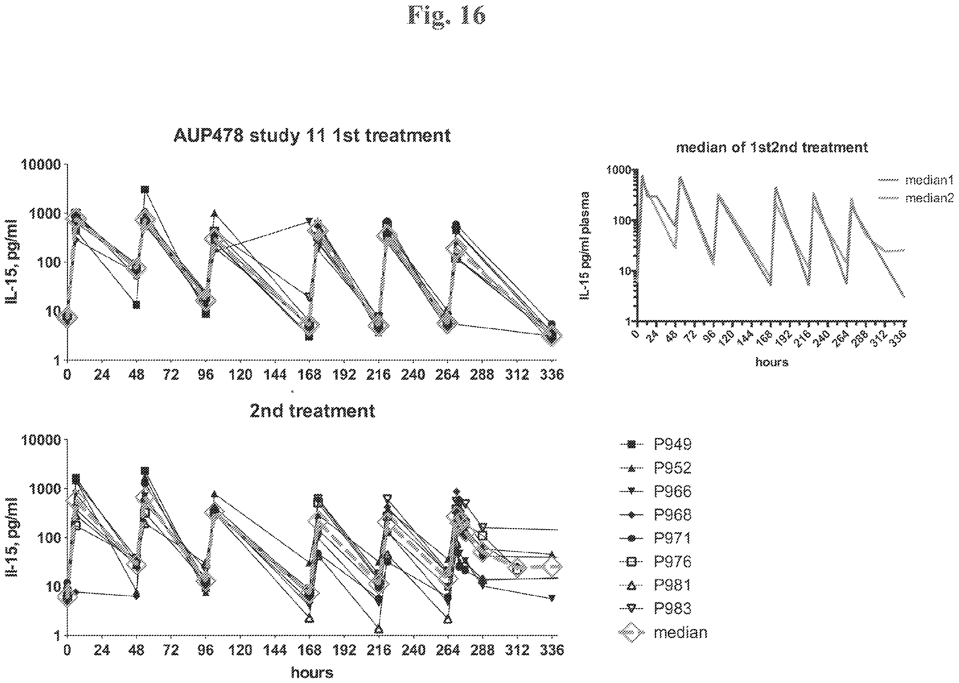

**Figure 16****.** Plasma IL-15 levels after repeated s.c. injection of IL-15/IL-15Ra. Eight macaques received 6 s.c. injections of IL-15/IL-15Ra at the dose of 5 µg/Kg on day 0, 2, 4, 7, 9 and 12. After a rest period of one month, a second cycle treatment identical to the first was conducted. Blood was drawn at different time points after each injection and plasma IL-15 levels were determined by ELISA and plotted as function of time after initiation of the treatment. Lines with diamond shapes represent median.

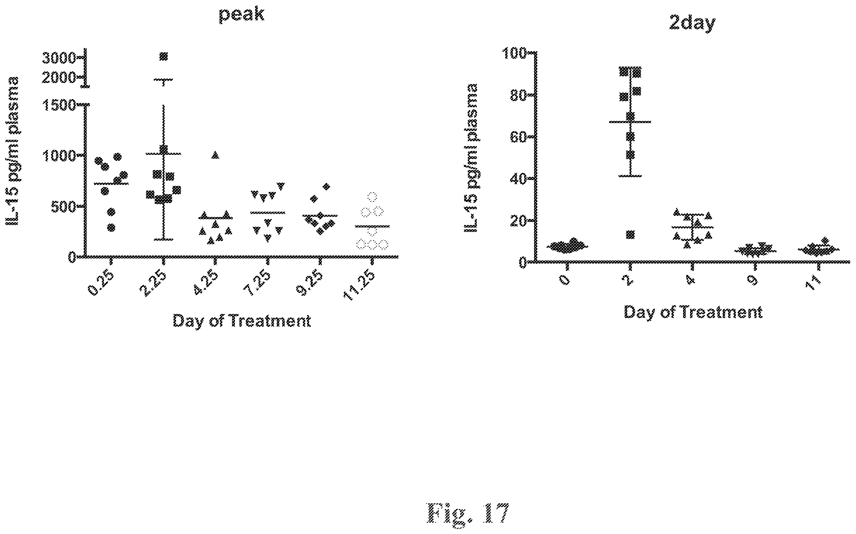

**Figure 17****.** Peak and trough levels of plasma IL-15 heterodimers. Plasma levels in 8 macaques inoculated with 5 µg/kg body weight of IL-15 heterodimer. Peak levels are 6 hours post injection. 0 indicates levels prior to the first injection. The trough levels two days after inoculation decreased over time.

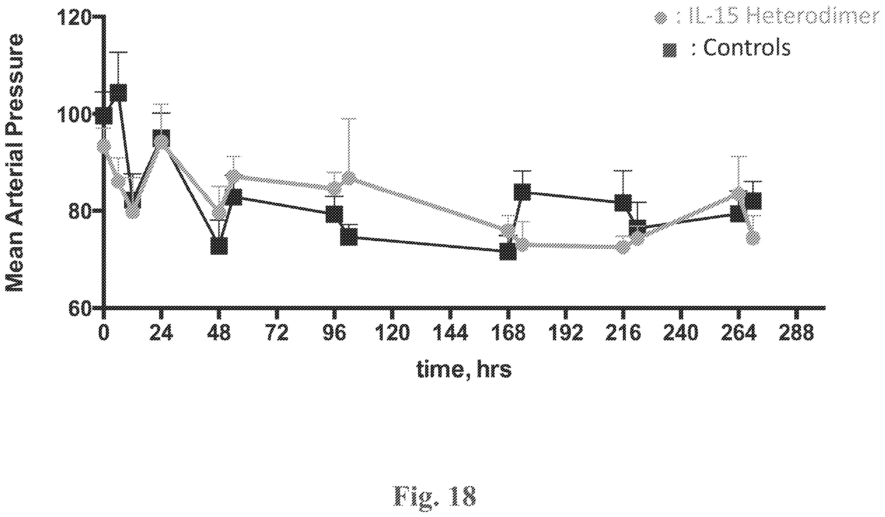

**Figure 18****.** Mean arterial pressure in 8 macaques injected every 2 days with 5 µg/kg body weight of IL-15 heterodimer. Line with circles=IL-15 heterodimer. Line with squares=controls.

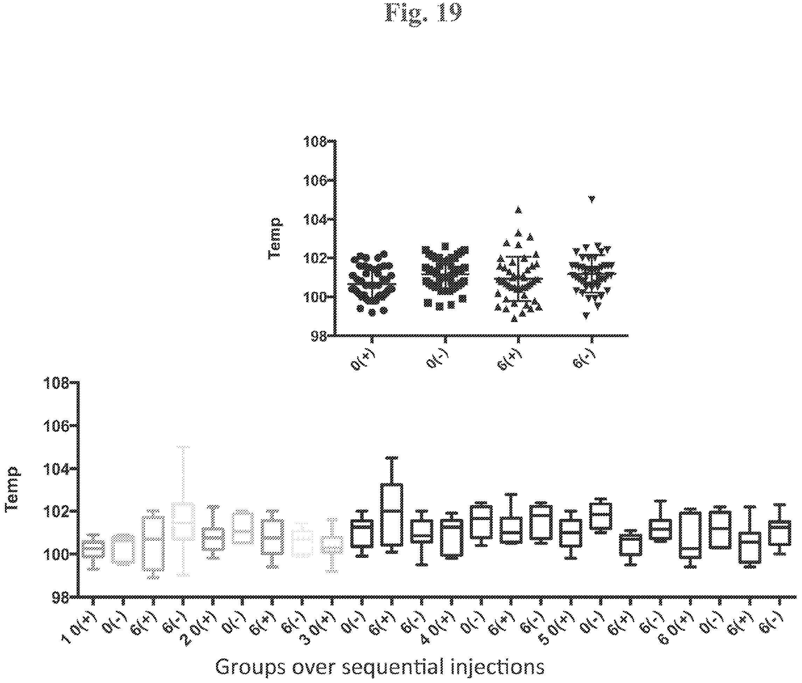

**Figure 19**. Macaque body temperature after IL-15 heterodimer injection (5 µg s.c.). Body Temperature of 8 macaques injected every 2 days with 5 µg/kg SC IL-15 heterodimer. The group of 8 macaques was compared to 8 controls injected with saline. All measurements of the IL-15 (+) and the controls (-) at the time of injection (0) and 6 hours later (6) were grouped. At the bottom, sequential injections are shown for the IL-15 and control groups. No consistent differences were detected.

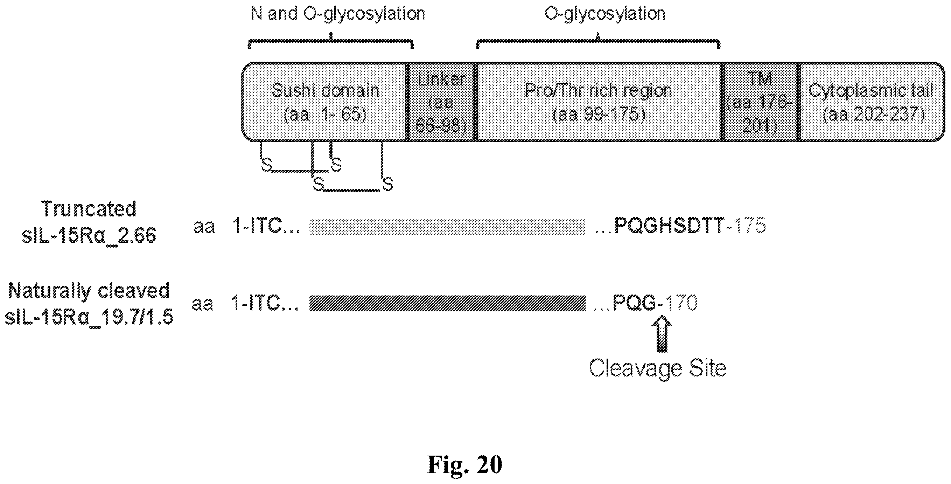

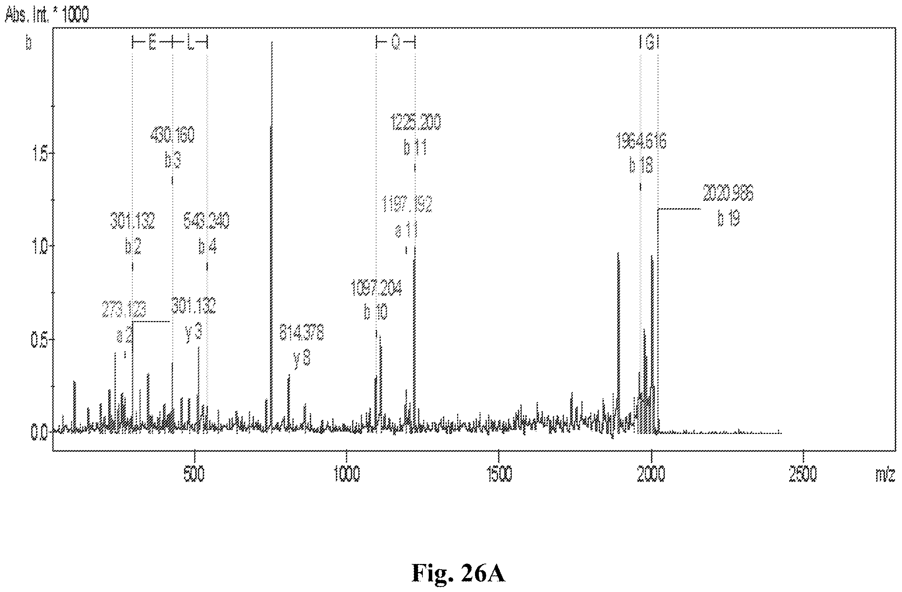

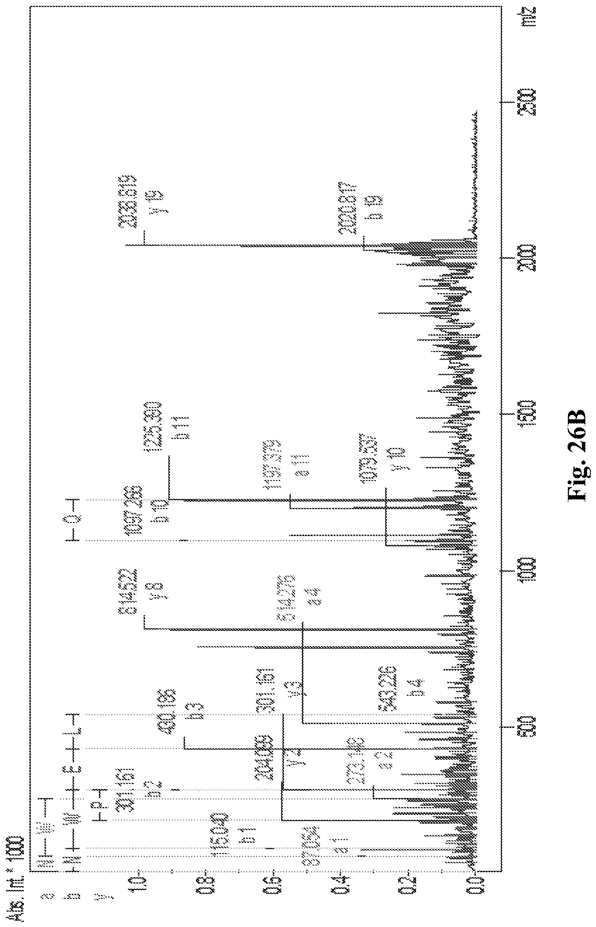

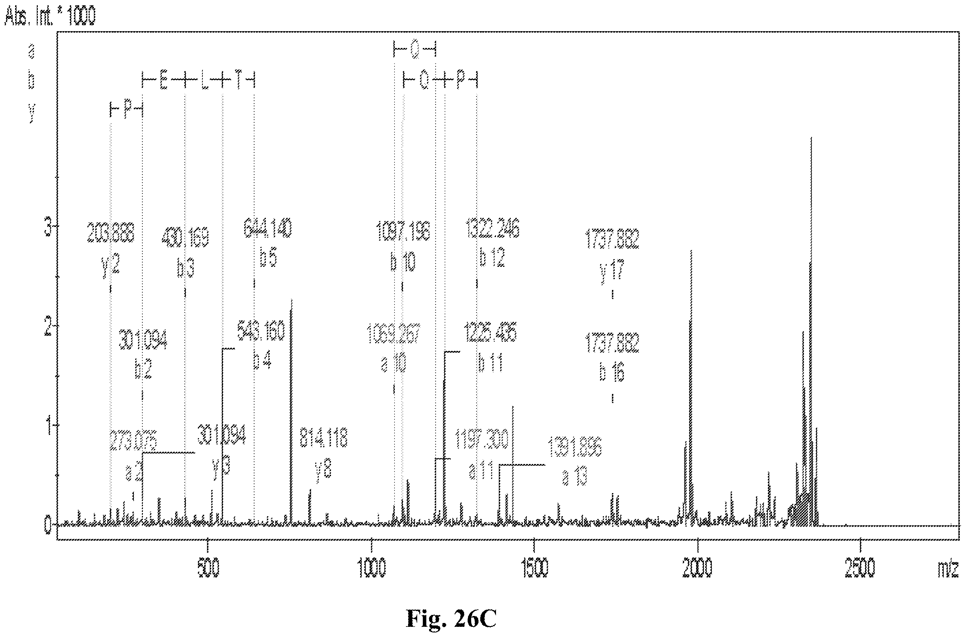

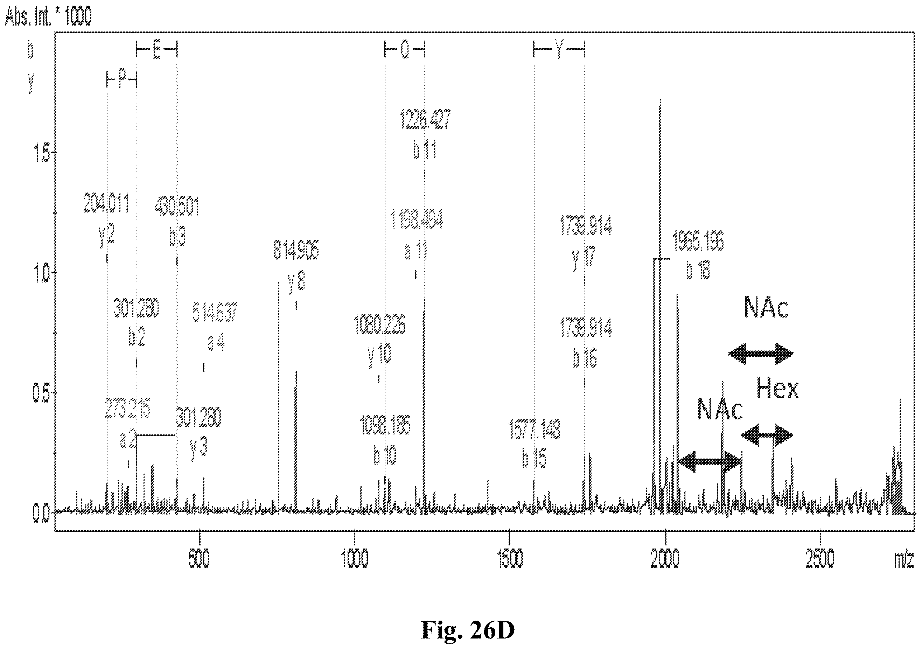

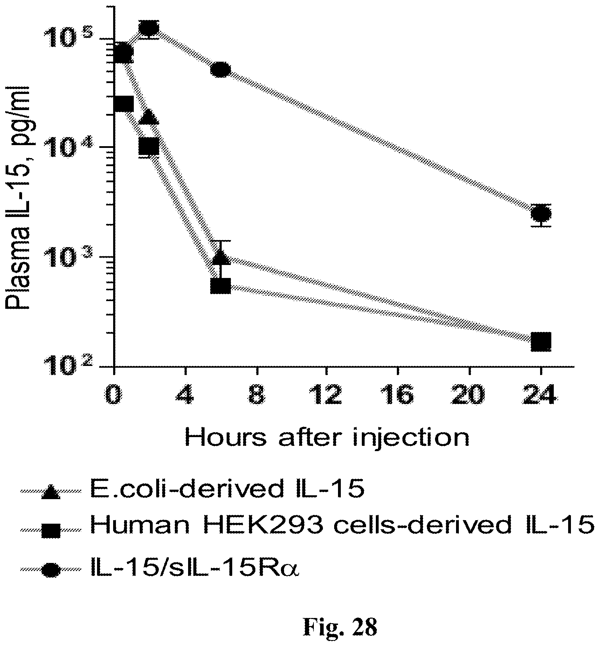

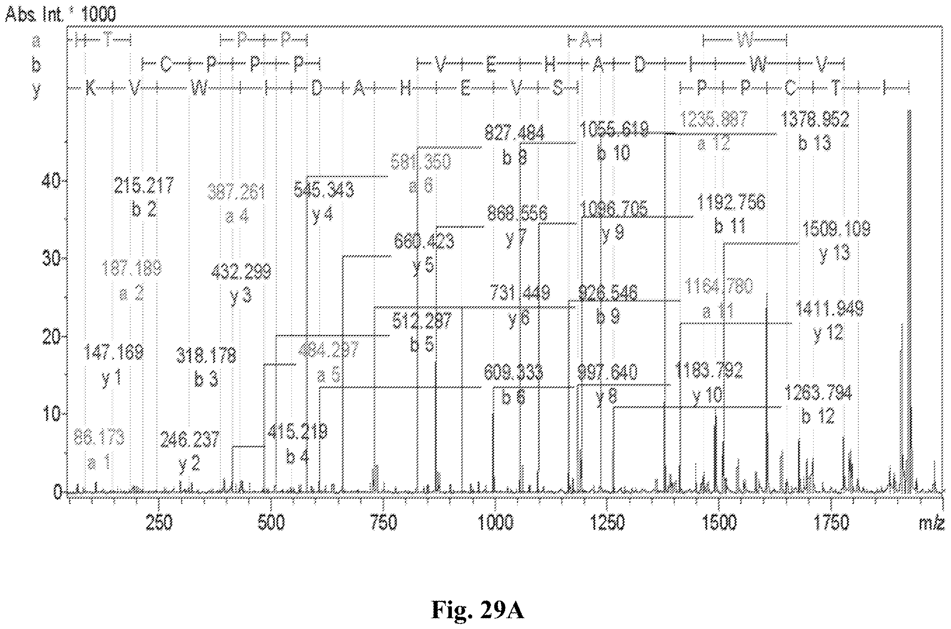

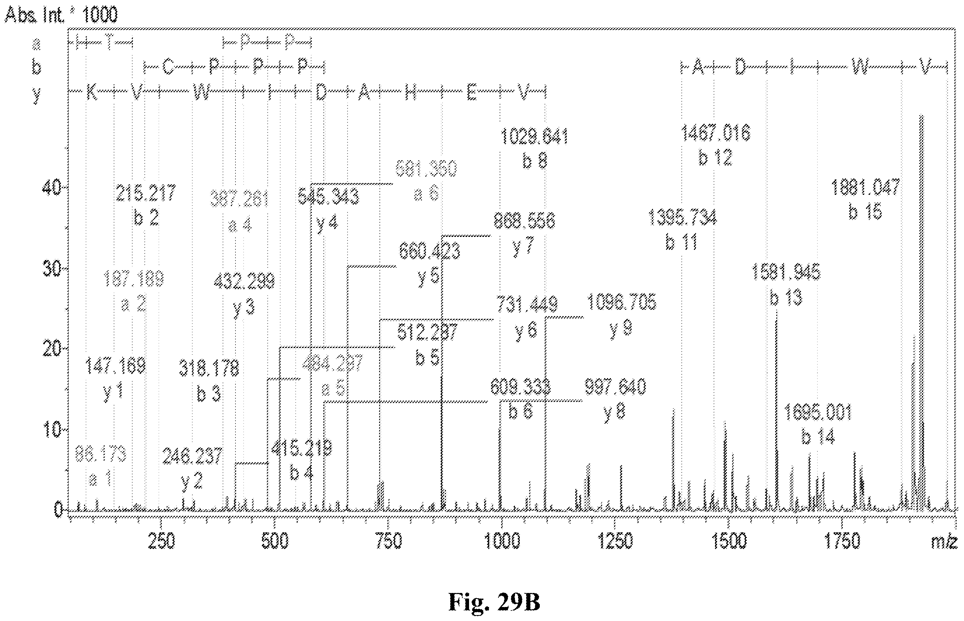

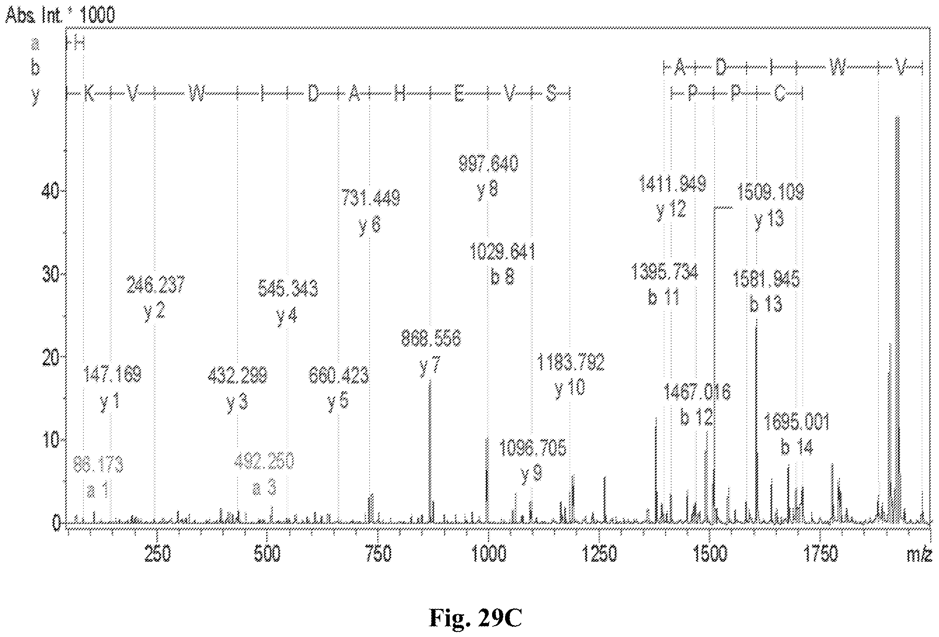

**Figure 20****.** Schematic representation of mature IL-15Rα. The different domains of mature IL-15Rα are shown (signal peptide is not included). The sushi domain forms 2 disulfide bonds and is characterized by several N- and O-glycosylation sites (HexNAc on Ser 8, 18, 20, 23 and 31 as reported in Fig. 26 and 29). The Pro/Thr rich domain contains O-glycosylation at Thr156 or Ser158 (as reported in Fig. 28). Truncated sIL-15Rα comprises 175 aa. Naturally cleaved sIL-15Rα from both clones 19.7 and 1.5 comprises 170 aa; the arrow indicates the cleavage site upon expression of IL-15Rα on the cell membrane.

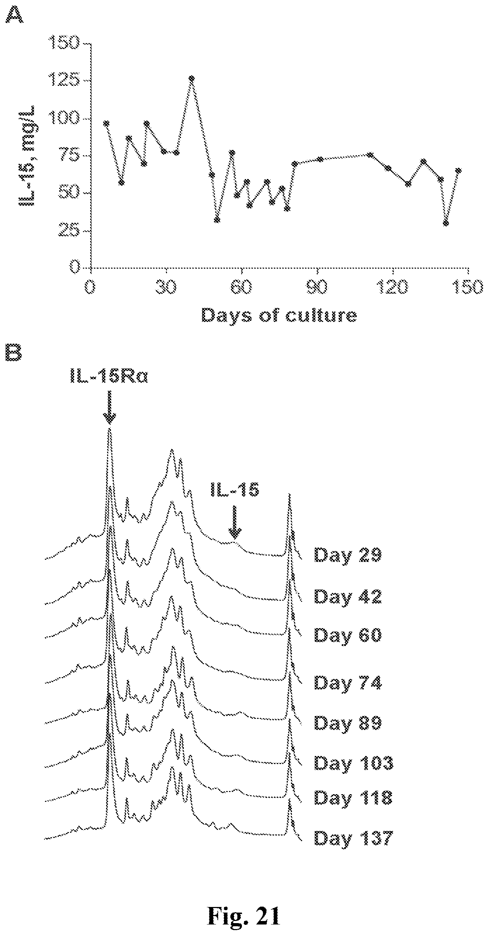

**Figure 21****.** Stable production of IL-15/sIL-15Rα heterodimers from clone 19.7. A. Daily harvests from clone 19.7 were evaluated for IL-15 amounts by ELISA. ELISA results show stable IL-15/sIL-15Rα production over 150 days in culture. B. RP-HPLC analysis of clone 19.7 supernatants over time. Arrows indicate peaks corresponding to sIL-15Rα and IL-15.

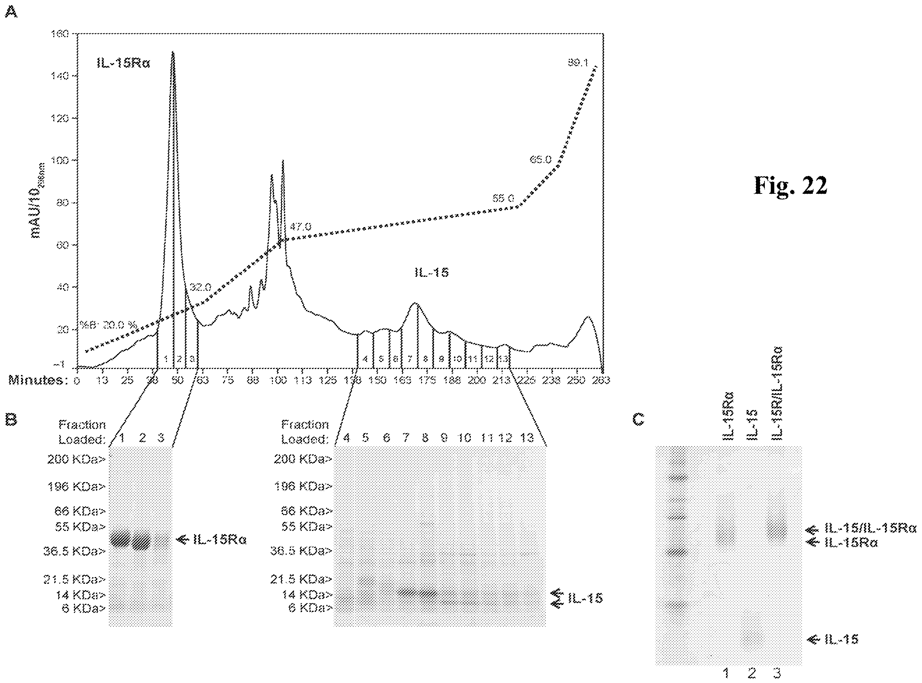

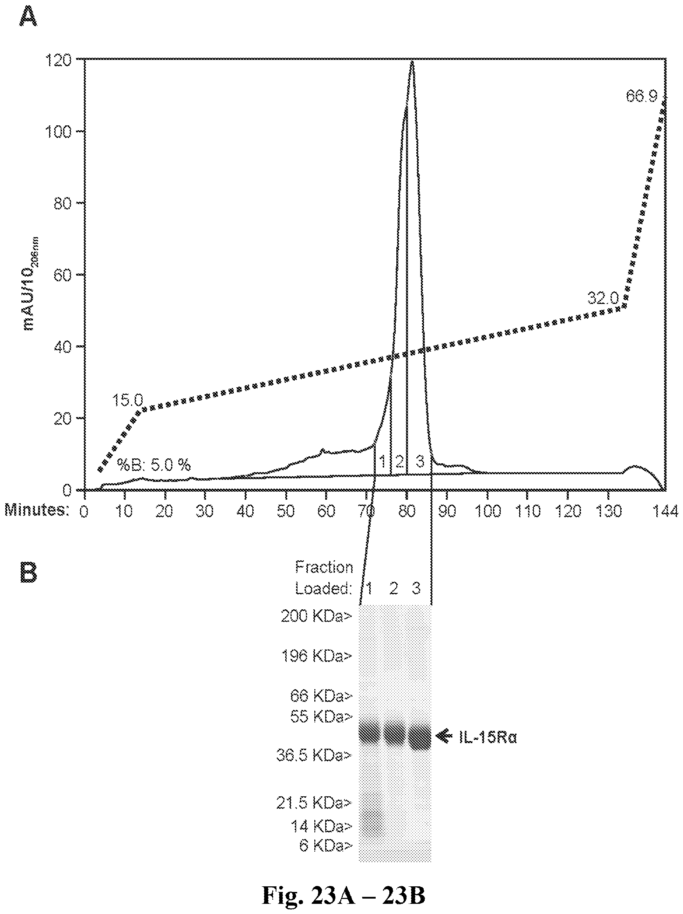

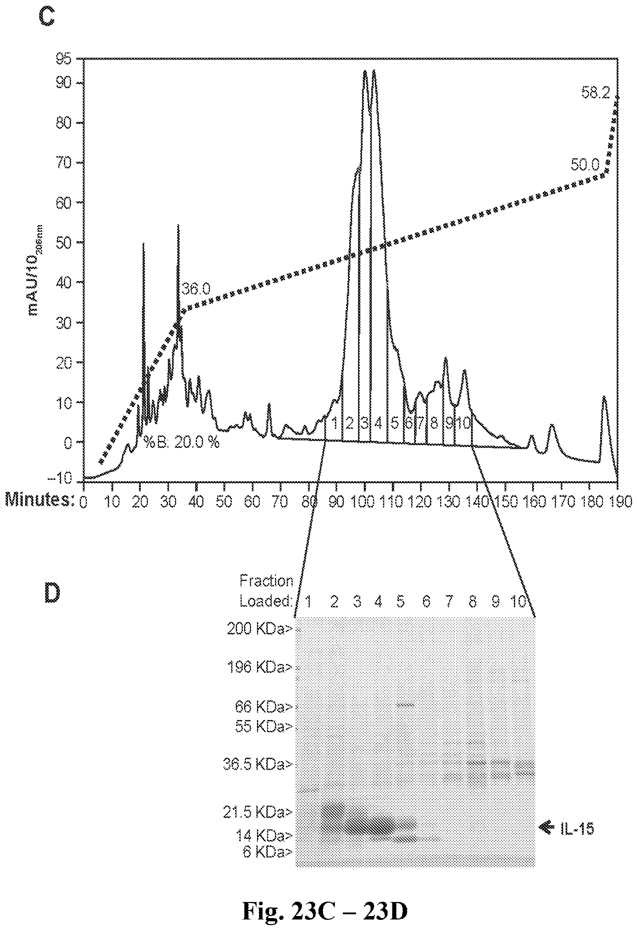

**Figure 22****.** Production and purification of human IL-15/sIL-15Ra. A. HPLC purification of IL-15/sIL-15Rα heterodimers from HEK293-derived human cell line 19.7. B. Proteins were eluted from the column and were analyzed by SDS-PAGE. C. After final purification (see Figure 23A, B, C and D) IL-15, sIL-15Rα and the re-constituted IL-15/sIL-15Rα heterodimer (at a 1:1 molar ratio) were visualized on native PAGE.
**Figure 23**. Additional purification of sIL-15Rα and IL-15 from clone 19.7. HPLC purification of sIL-15Rα Fx1-3 (Figure 22) and IL-15 Fx4-13 (Figure 22) was performed on 16 x 100 mm POROS R2/10 column (A and C, respectively) and eluted proteins were analyzed by SDS-PAGE (B and D, respectively).

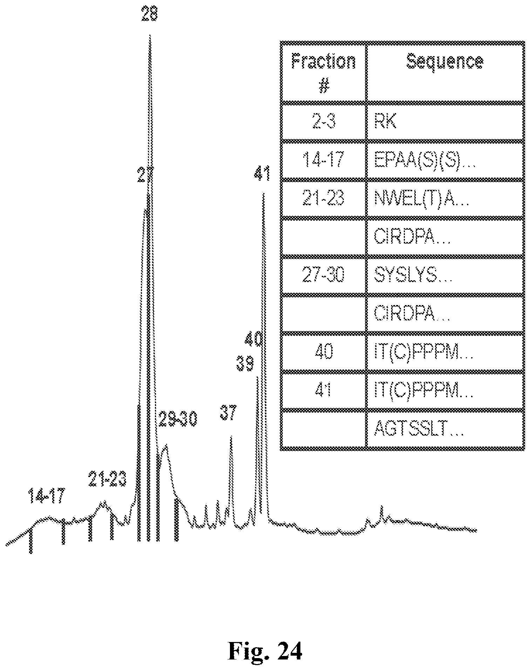

**Figure 24**. HPLC separation of peptides after LysC digestion of purified naturally cleaved sIL-15Rα under non-reducing conditions. sIL-15Rα from cell clone 19.7 was digested by Lys-C protease and peptides were separated by HPLC. Fractions were analyzed by Applied Biosystems Inc. 477 A protein sequencer. The identified sequences shown in the insert were also confirmed by MALDI-TOF MS.

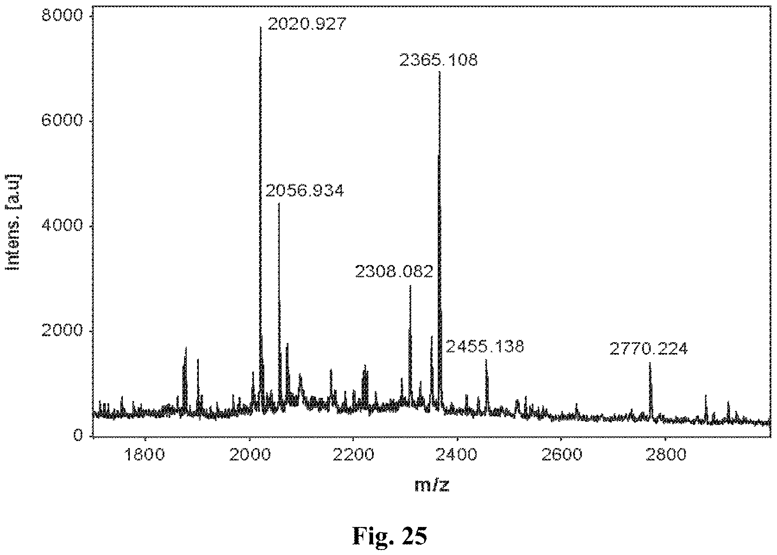

**Figure 25****.** MS analysis of HPLC fraction containing the C-terminal peptide of sIL15Rα. MALDI-TOF MS revealed the presence of several peptides with m/z close (2020.927) or bigger (2056.934, 2308.082, 2365.108, 2455.138, 2770.224) than expected for the peptide with sequence NWELTASASHQPPGVYPQG (theoretical [M+H]+ 2038.962) which was determined by protein sequence analysis of this fraction.
**Figure 26****.** MALDI-TOF MS/MS analysis of peptides detected in the fraction containing C-terminal peptide. Fragment spectra of m/z 2020.927 (A), 2056.934 (B), 2365.108 (C), 2770.224 (D) (as well as 2308.082 and 2455.138, not shown) contain common *b* and y-ion-fragments suggesting that they correspond to the same peptide with different post-translational modifications, most likely O-glycosylation of Thr5(156) and Ser7(158). Since all these peptides were modified, Mascot MS/MS Ion Search of protein database (SwissProt) was performed using theoretical mass of the expected unmodified peptide NWELTASASHQPPGVYPQG (2038.962) as a "parent" ion instead of an experimental m/z. Enzyme specificity was set as "semi-trypsin". Panel D shows ion fragments with mass differences equal to masses of N-acetylhexosamine (NAc) and hexose (Hex). The presence of these ion fragments suggests that the peptide is indeed O-glycosylated.

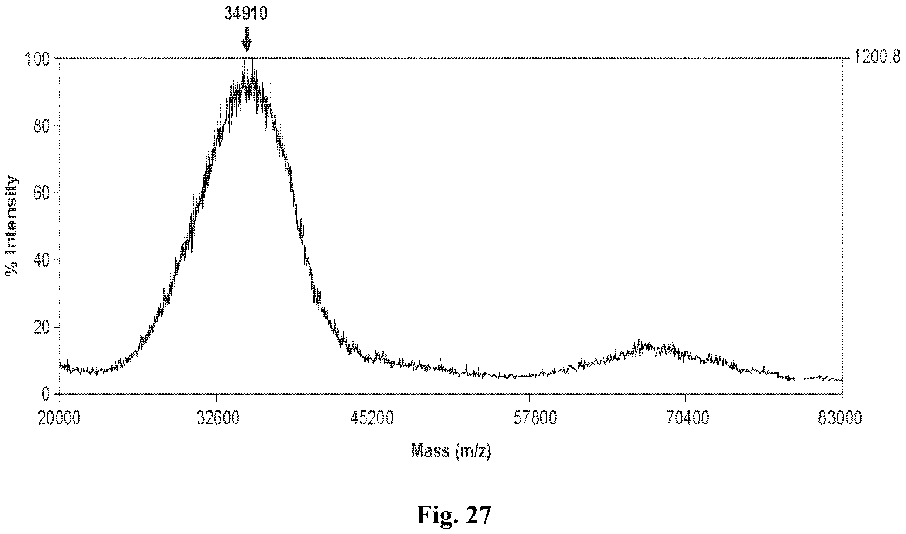

**Figure 27****.** Determination of molecular mass of HPLC purified naturally cleaved sIL15Rα. MALDI-TOF MS spectrum of purified sIL-15Rα on Voyager De-Pro equipped with CovalX HM-1 high mass detector using sinapic acid as matrix.
**Figure 28****.** Association with sIL-15Ra increases the in vivo half-life of human IL-15 in mice. Five mice per group were injected with equimolar quantities of E. coli single-chain IL-15, human HEK293 cell-derived single-chain IL-15 or IL-15/sIL-15Ra heterodimer (3 µg IL-15 equivalent/mice, i.p.). The mice were bled over time (0.5, 2, 6 and 24 h after treatment), plasma IL-15 levels were evaluated by ELISA and reported as mean ±SD.
**Figure 29****.** Identification of HexNAc modification in the N-terminal peptide of naturally cleaved sIL15-Ra (ITCPPPMSVEHADIWVK). MALDI-TOF MS/MS spectrum of m/z 2126.150 from fraction 39. A. Mascot MS/MS ion search with parent ion set as 1922.950 identified N-terminal and C-terminal fragments corresponding to peptide ITCPPPMSVEHADIWVK. B. Analysis of the spectrum in BioTools with HexNAc modification on Ser8. C. Analysis of the spectrum with HexNAc modification on Thr2.

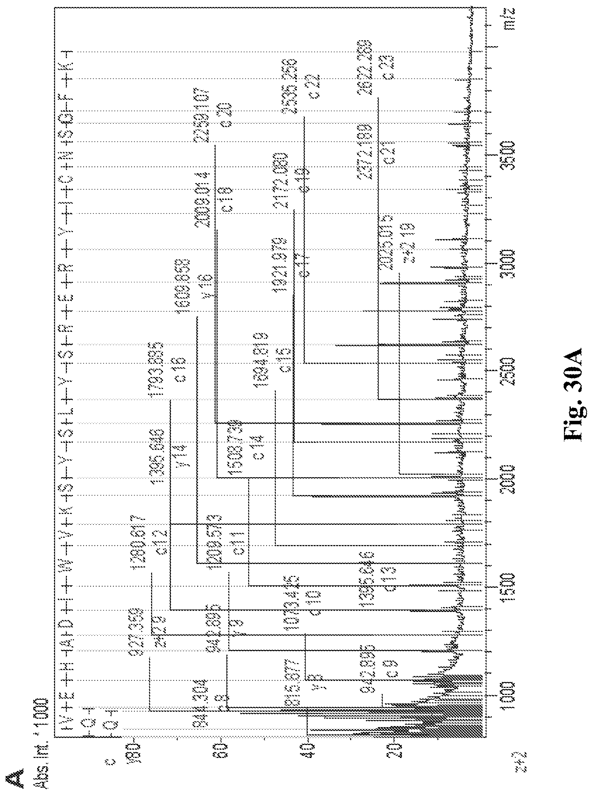

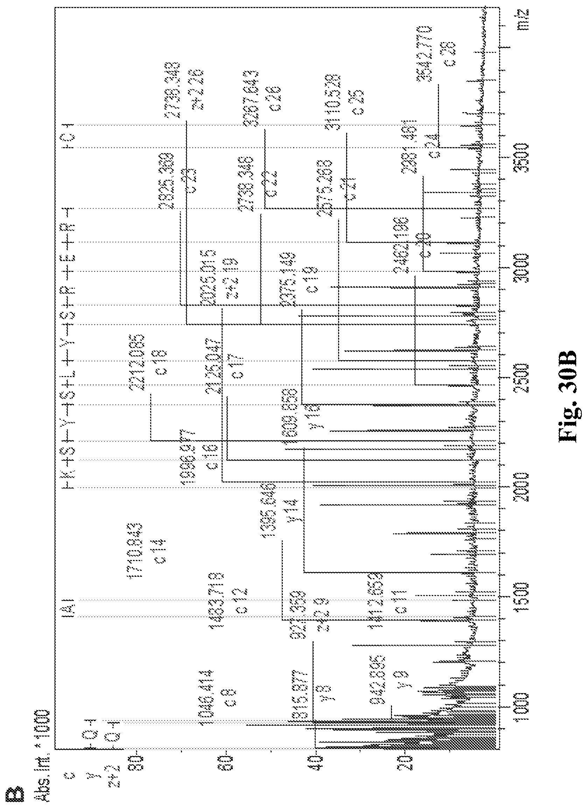

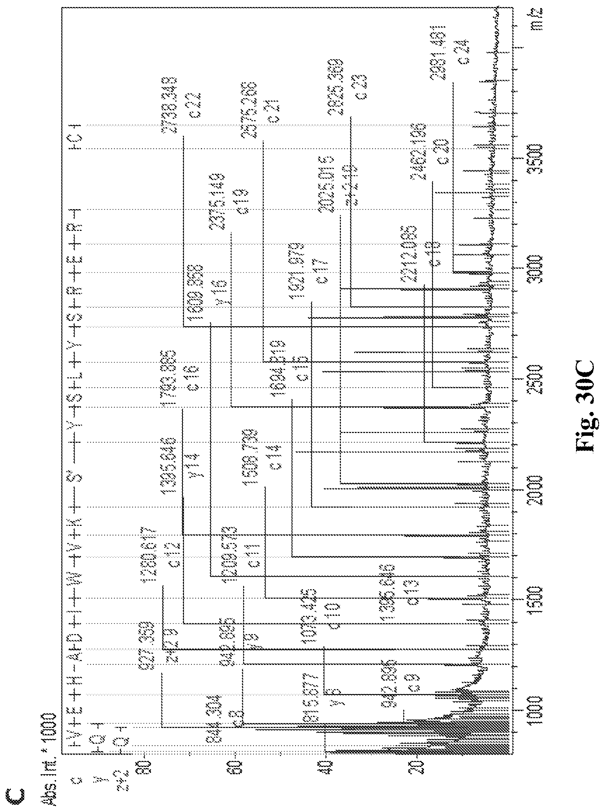

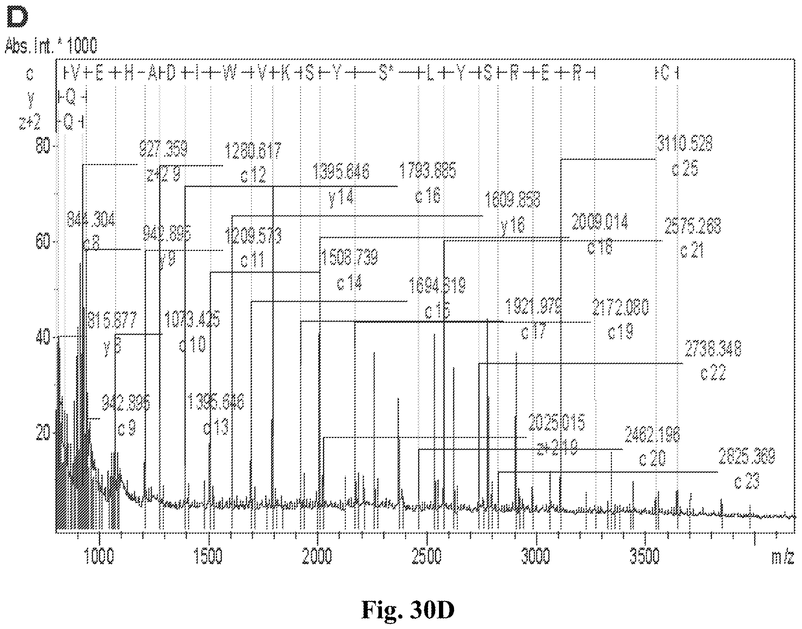

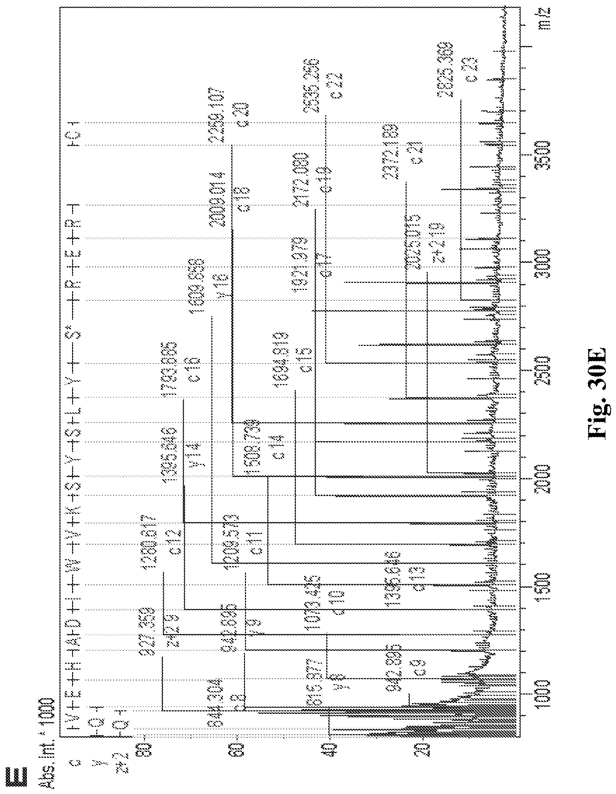

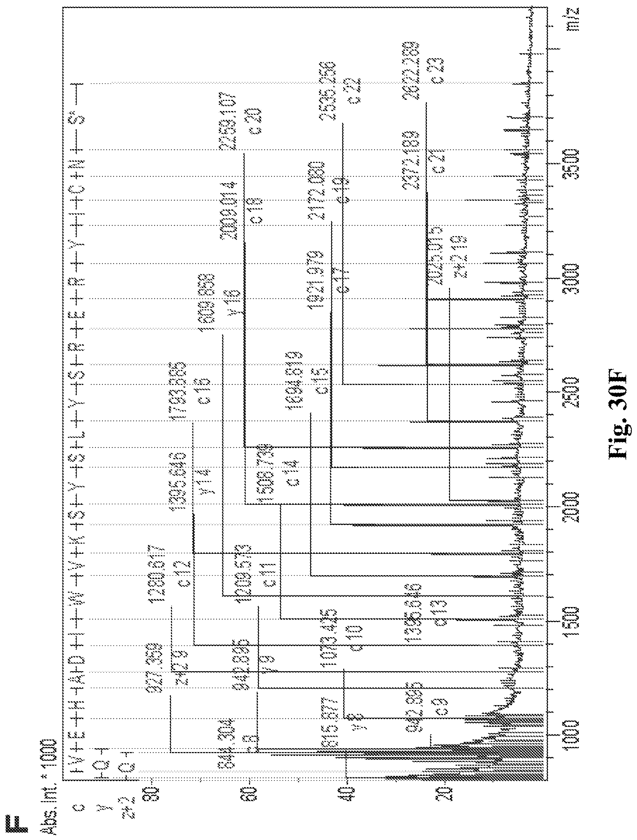

**Figure 30**. Identification of Ser residues in the N-terminal part of naturally cleaved sIL-15Rα modified by HexNAc. Analysis of ISD MALDI-TOF MS spectrum of HPLC purified naturally cleaved sIL15-Rα. A. No modifications; HexNAc modifications on: B. Ser8; C. Ser18; D. Ser20; E. Ser23; F. Ser31.

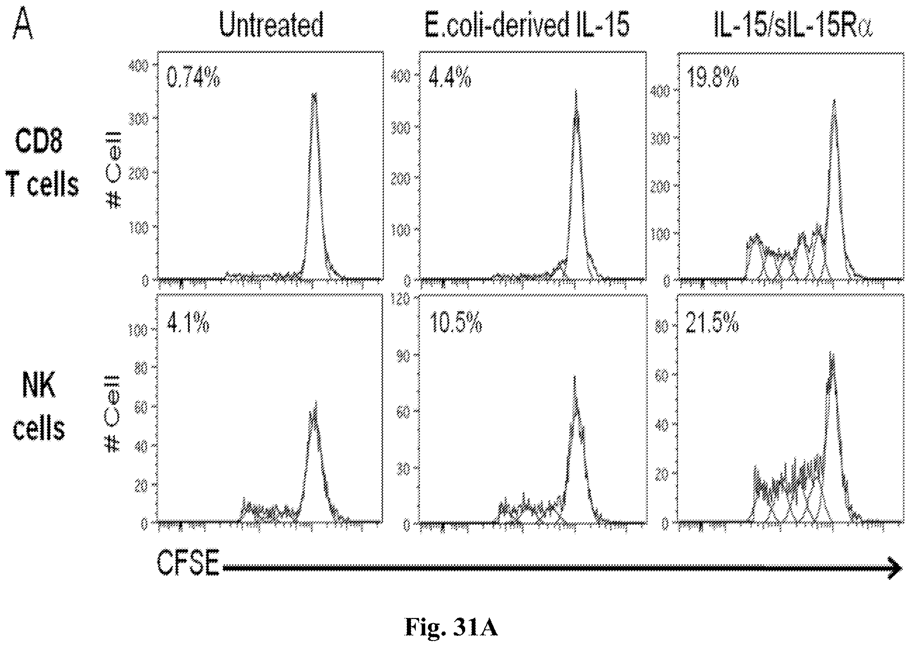

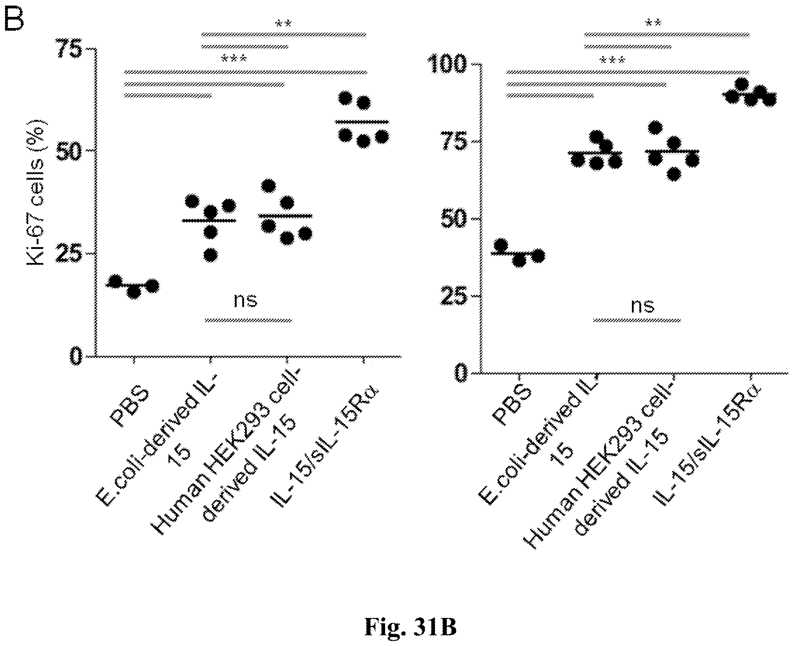

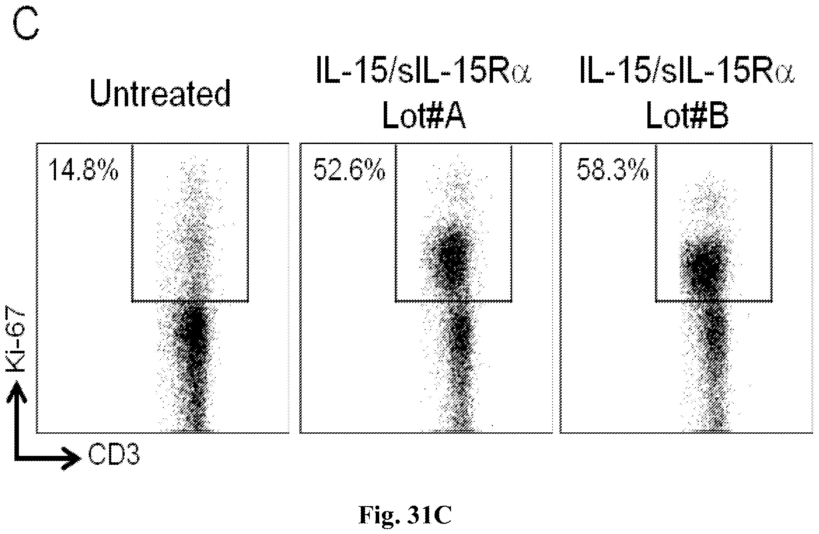

**Figure 31****.** IL-15/sIL-15Ra heterodimer is bioactive in vivo. A. IL-15/sIL-15Rα heterodimer is more potent than single chain IL-15: In vivo lymphocyte proliferation. 12 hours after transfer of CFSE-labeled splenocytes (20x10⁶), mice were treated i.p. with PBS (3 mice); with 3 µg of IL-15 (3 mice); or with equimolar quantity of IL-15/sIL-15Rα (3 mice, 3 µg in single-chain IL-15). CD8+ T cells (top panels) and NK cells (bottom panels) were analyzed on day 4 by flow cytometry for CFSE fluorescence. One representative mouse per group is shown. B. IL-15Rα contributes to the superior activity of IL-15 heterodimers. Mice were treated i.p. with PBS (3 mice), with 3 µg of E.coli-derived single-chain IL-15 (5 mice), with 3 µg of human HEK293 cell-derived single-chain IL-15 (5 mice), or with equimolar quantity of IL-15/sIL-15Rα (5 mice, 3 µg in single-chain IL-15). The frequency of CD8+ T cells (left panels) and NK cells (right panel) expressing the proliferative marker Ki-67 is shown. Individual animals and average are shown for each group. **, p<0.01; ***, p<0.001; ns, non significant. C. Different lots of purified IL-15/sIL-15Rα heterodimer from clone 1.5 induced similar levels of proliferation in splenic CD8+ T cells. Mice were treated i.p. with PBS or with 3 µg of IL-15/sIL-15Rα (clone 1.5 lotA) or 3 µg of IL-15/sIL-15Rα (clone 1.5 lotB). Isolated splenocytes were evaluated on day 3 by flow cytometry for the presence of the proliferation marker Ki67. Percentage of Ki67+ cells within the CD8+ T cells population is shown. One representative mouse per group (3 mice/group) is shown.

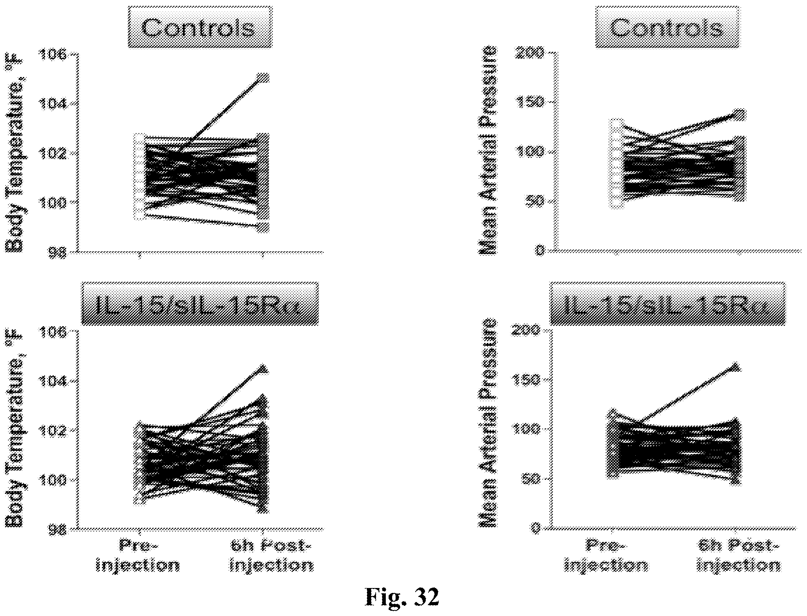

**Figure 32****.** Macaque body temperature and mean arterial pressure in 8 macaques before and after repeated s.c. injections with 5 µg/kg body weight of IL-15 heterodimer vs. 8 macaques as controls before and after repeated s.c. injections with saline. The 16 macaques received 6 s.c. injections of IL-15/IL-15Ra at the dose of 5 µg/Kg or saline on day 0, 2, 4, 7, 9 and 11. After a rest period, a second cycle treatment identical to the first was conducted. Blood pressure and temperature before and after repeated were measured and plotted.

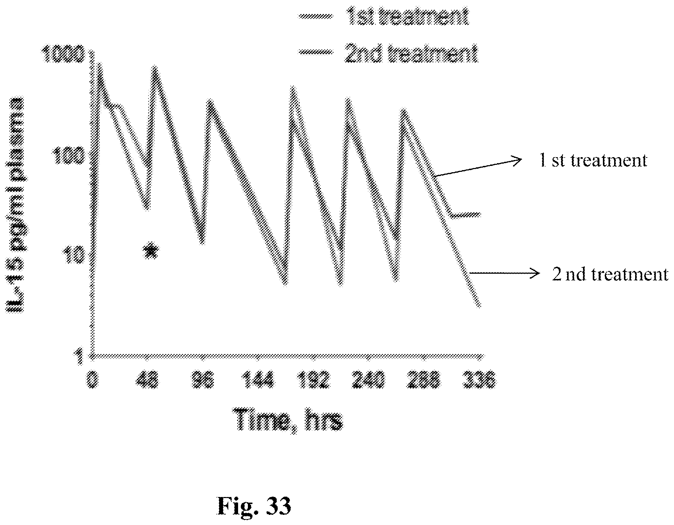

**Figure 33****.** Plasma IL-15 levels during the two treatments in 8 macaques injected with IL-15 heterodimer. Blood was drawn at different time points after each injection and plasma IL-15 levels were determined by ELISA and plotted as function of time after initiation of the treatment.

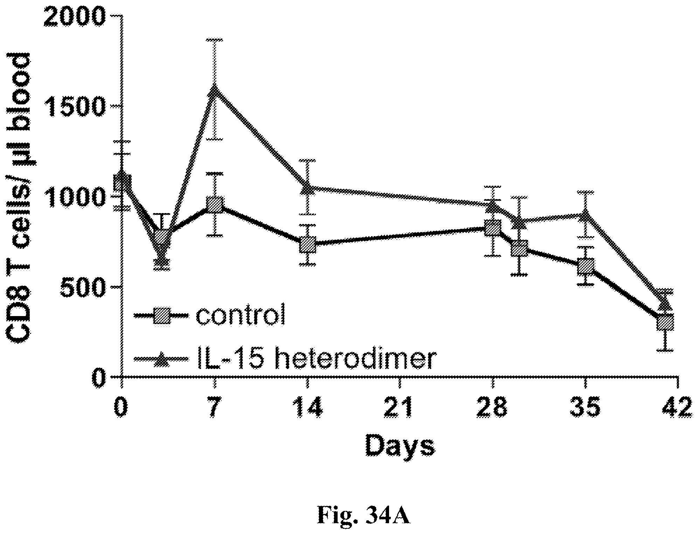

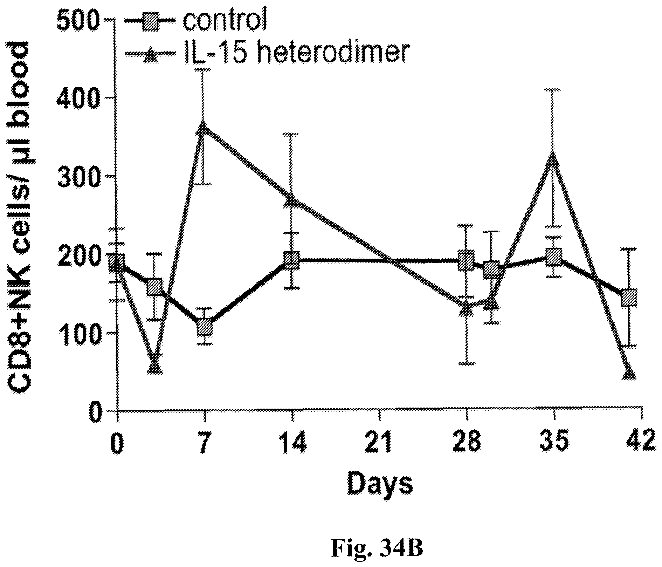

**Figure 34**. NK and CD8 T cells counts after repeated administration of IL-15 heterodimers. Samples of PBMC were taken from the macaques prior, during and after IL-15 administration and were stained with antibodies binding to CD3, CD4, CD8 and CD16, and examined by flow cytometry. The data for CD3+CD8+ T cells (Figure 34A) and CD3-CD16+CD8+ NK cells (Figure 34B) are shown as the absolute cell number of each subset per µl of blood at the indicated time points. Triangles: IL-15 heterodime; Squares: control.

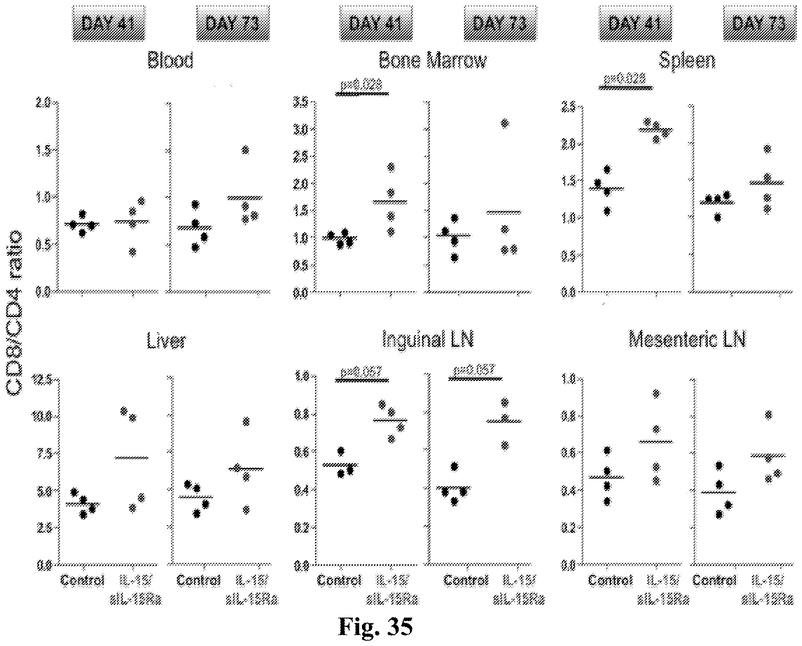

**Figure 35****.** The ratio of CD8/CD4 T cells in blood as well as in different tissues, including bone marrow, spleen, liver, inguinal LC, mesenteric LN, at the days of necropsies (day 41/42 and 73/74).

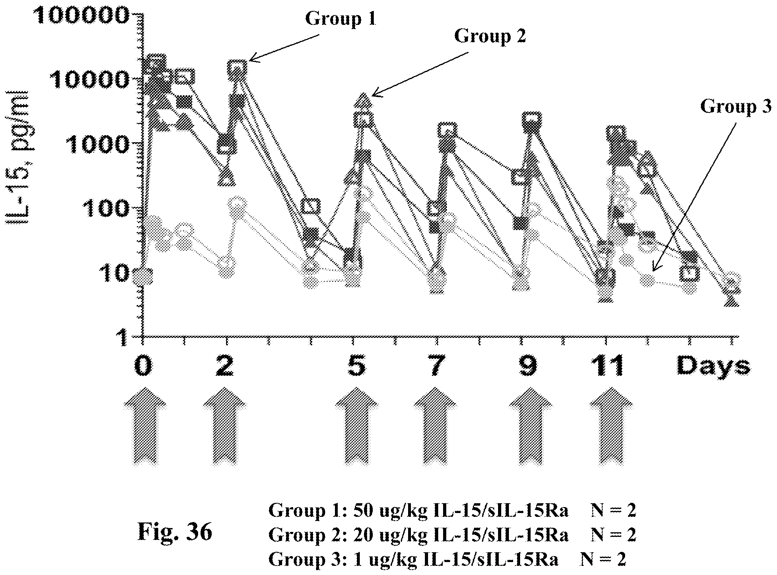

**Figure 36****.** Plasma IL-15 levels in 6 macaques injected with IL-15 heterodimer at escalated doses of 1 µg/kg, 20 µg/kg, and 50 µg/kg. The 6 macaques received 6 s.c. injections of IL-15/IL-15Ra at the dose of 1 µg/Kg, 20 µg/Kg, or 50 µg/Kg (on day 0, 2, 4, 7, 9 and 11. Group 1: 50 µg/kg IL-15/sIL-15Ra; Group 2: 20 µg/kg IL-15/sIL-15Ra; Group 3: 1µg/kg IL-15/sIL-15Ra. Squares: 50 µg/kg; triangles: 20 µg/kg; circles: 1 µg/kg.

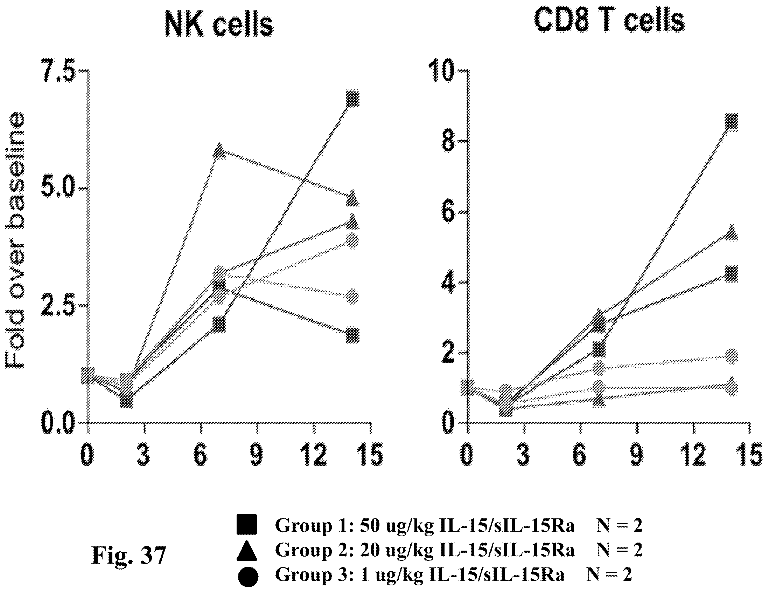

**Figure 37****.** The fold over baseline increase of NK cells and CD8 T cells in peripheral blood in 6 macaques injected with IL-15 heterodimer at escalated doses of 1 µg/kg, 20 µg/kg, and 50 µg/kg. Group 1: 50 µg/kg IL-15/sIL-15Ra; Group 2: 20 µg/kg IL-15/sIL-15Ra; Group 3: 1µg/kg IL-15/sIL-15Ra. Squares: 50 µg/kg; triangles: 20 µg/kg; circles: 1µg/kg.

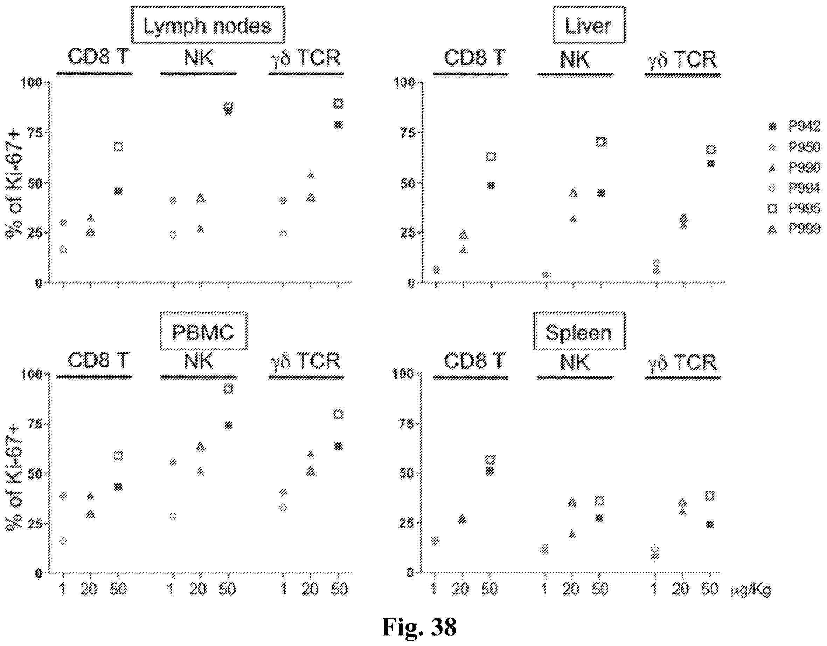

**Figure 38****.** Dose-dependent proliferation of lymphocytes in different tissues, including lymph nodes, liver, PBMC, spleen, upon IL-15 heterodimer s.c. administration.. Squares: 50 µg/kg; triangles: 20 µg/kg; circles: 1 µg/kg.

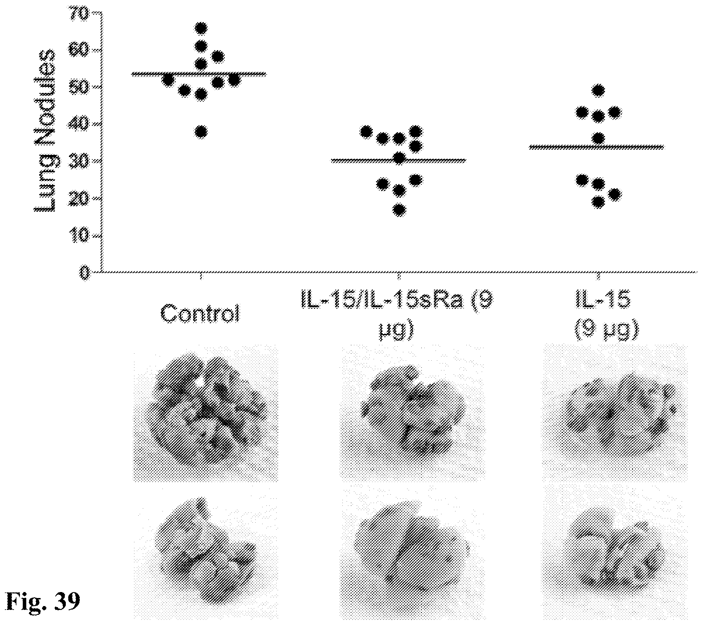

**Figure 39****.** IL-15 reduced lung tumor engraftment of B16 melanoma cells in mice. 9 µg of IL-15 or IL-15/soluble IL-15Ra complex was administered to the mice on days 1,6, and 11 by ip injections.

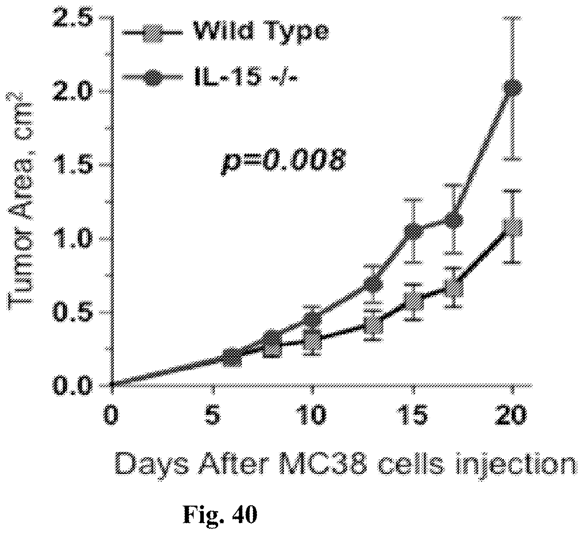

**Figure 40****.** Wild-type and IL-15 ⁻/⁻ C57BL/6 mice were injected with 3 x 10⁵ MC38 colon carcinoma cells subcutaneously and tumor growth was followed overtime.

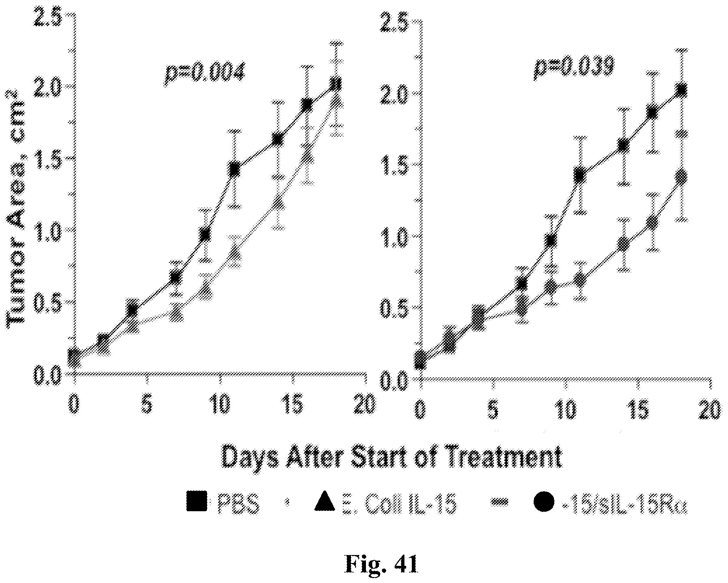

**Figure 41****.** Wild type C57BL/6 mice were injected with 3 x 10⁵ MC38 colon carcinoma cells SC and one week later were treated with the two different IL-15 formulations. Tumor growth was followed overtime. Triangle: *E. coli* derived IL-15; Squares: PBS; Circles: IL-15/soluble IL-15Ra.

### 5. DETAILED DESCRIPTION

### 5.1 Forms of IL-15Ra

Described herein is the naturally occurring soluble form of human IL-15Ra. Also described herein are specific IL-15Ra derivatives that are truncated, soluble forms of human IL-15Ra. These specific IL-15Ra derivatives and the naturally occurring soluble form of human IL-15Ra are based, in part, on the identification of the proteolytic cleavage site of human IL-15Ra. Further described herein are soluble forms of IL-15Ra that are characterized based upon glycosylation of the IL-15Ra.

As shown in Example 4, *infra*, the inventors have discovered that the proteolytic cleavage of membrane-bound human IL-15Ra takes place between Gly170 and His171 in human IL-15Ra. Thus, the proteolytic cleavage of human IL-15Ra takes place between the residues (*i.e*., Gly170 and His171) which are in shown in bold and underlined in the provided amino acid sequence of the immature form of the native full length human IL-15Ra: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQ**G H**SDTTVAIST STVLLCGLSA VSLLACYLKS RQTPPLASVE MEAMEALPVT WGTSSRDEDL ENCSHHL (SEQ ID NO: 3; FIG. 2B).

Accordingly, in one aspect, provided herein is a soluble form of human IL-15Ra (e.g., a purified soluble form of human IL-15Ra), wherein the amino acid sequence of the soluble form of human IL-15Ra terminates at the site of the proteolytic cleavage of the native membrane-bound human IL-15Ra. In particular, provided herein is a soluble form of human IL-15Ra (e.g., a purified soluble form of human IL-15Ra), wherein the amino acid sequence of the soluble form of human IL-15Ra terminates with PQG (SEQ ID NO: 31), wherein G is Gly170. In particular embodiments, provided herein is a soluble form of human IL-15Ra (e.g., a purified soluble form of human IL-15Ra) which has the following amino acid sequence: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQG (SEQ ID NO: 32). In some embodiments, provided herein is an IL-15Ra derivative (e.g., a purified and/or soluble form of IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 32; and (ii) terminates with the amino acid sequence PQG (SEQ ID NO: 31). In other particular embodiments, provided herein is a soluble form of human IL-15Ra (e.g., a purified soluble form of human IL-15Ra) which has the following amino acid sequence: ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQG (SEQ ID NO: 33). In some embodiments, provided herein is an IL-15Ra derivative (e.g., a purified and/or soluble form of an IL-15Ra derivative), which is a polypeptide that is at least 40%, 45%, 50%, 55%, 60%, 65%, 70%, 75%, 80%, 85%, 90%, 95%, 98% or 99% identical to SEQ ID NO: 33, and, optionally, wherein the amino acid sequence of the soluble form of the IL-15Ra derivative terminates with PQG (SEQ ID NO: 31).

In another aspect, provided herein are IL-15Ra derivatives that are truncated, soluble forms of naturally occurring human IL-15Ra. In some embodiments, provided herein is a soluble form of human IL-15Ra (e.g., a purified soluble form of human IL-15Ra), wherein the amino acid sequence of the soluble form of human IL-15Ra terminates with PQGH (SEQ ID NO: 30), wherein H is His171 of SEQ ID NO:45. In particular embodiments, provided herein is a soluble form of human IL-15Ra (e.g., a purified soluble form of human IL-15Ra) which has the following amino acid sequence: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGH (SEQ ID NO: 34). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 34; and (ii) terminates with the amino acid sequence PQGH (SEQ ID NO: 30). In other particular embodiments, provided herein is a soluble form of human IL-15Ra (e.g., a purified soluble form of human IL-15Ra) which has the following amino acid sequence: ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGH (SEQ ID NO: 35). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 35; and (ii) has the amino acid sequence of the soluble form of the IL-15Ra derivative terminates with PQGH (SEQ ID NO: 30).

In some embodiments, provided herein is a soluble form of human IL-15Ra (e.g., a purified soluble form of human IL-15Ra), wherein the amino acid sequence of the soluble form of human IL-15Ra terminates with PQGHS (SEQ ID NO: 29), wherein S is Ser172 of SEQ ID NO:45. In particular embodiments, provided herein is a soluble form of human IL-15Ra (e.g., a purified soluble form of human IL-15Ra) which has the following amino acid sequence: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHS (SEQ ID NO: 36). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 36; and (ii) terminates with the amino acid sequence PQGHS (SEQ ID NO: 29). In other particular embodiments, provided herein is a soluble form of human IL-15Ra (e.g., a purified soluble form of human IL-15Ra) which has the following amino acid sequence: ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHS (SEQ ID NO: 37). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 37; and (ii) terminates with the amino acid sequence PQGHS (SEQ ID NO: 29).

In some embodiments, provided herein is a soluble form of human IL-15Ra (e.g., a purified soluble form of human IL-15Ra), wherein the amino acid sequence of the soluble form of human IL-15Ra terminates with PQGHSD (SEQ ID NO: 28), wherein D is Asp173 of SEQ ID NO:45. In particular embodiments, provided herein is a soluble form of human IL-15Ra (e.g., a purified soluble form of human IL-15Ra) which has the following amino acid sequence: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHSD (SEQ ID NO: 38). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 38; and (ii) terminates with the amino acid sequence PQGHSD (SEQ ID NO: 28). In other particular embodiments, provided herein is a soluble form of human IL-15Ra (e.g., a purified soluble form of human IL-15Ra) which has the following amino acid sequence: ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHSD (SEQ ID NO: 39). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 39; and(ii) terminates with the amino acid sequence PQGHSD (SEQ ID NO: 28).

In some embodiments, provided herein is a soluble form of human IL-15Ra (e.g., a purified soluble form of human IL-15Ra), wherein the amino acid sequence of the soluble form of human IL-15Ra terminates with PQGHSDT (SEQ ID NO: 27), wherein T is Thr174 of SEQ ID NO:45. In particular embodiments, provided herein is a soluble form of human IL-15Ra (e.g., a purified soluble form of human IL-15Ra) which has the following amino acid sequence: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHSDT (SEQ ID NO: 40). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 40; and (ii) terminates with the amino acid sequence PQGHSDT (SEQ ID NO: 27). In other particular embodiments, provided herein is a soluble form of human IL-15Ra (e.g., a purified soluble form of human IL-15Ra) which has the following amino acid sequence: ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHSDT (SEQ ID NO: 41). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 41; and (ii) terminates with the amino acid sequence PQGHSDT (SEQ ID NO: 27).

In some embodiments, provided herein is a soluble form of human IL-15Ra (e.g., a purified soluble form of human IL-15Ra), wherein the amino acid sequence of the soluble form of human IL-15Ra terminates with PQGHSDTT (SEQ ID NO: 26), wherein T is Thr175 of SEQ ID NO:45. In particular embodiments, provided herein is a soluble form of human IL-15Ra (e.g., a purified soluble form of human IL-15Ra) which has the following amino acid sequence: MAPRRARGCR TLGLPALLLL LLLRPPATRG ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHSDTT (SEQ ID NO: 4). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 4; and (ii) terminates with the amino acid sequence PQGHSDTT (SEQ ID NO: 26). In other particular embodiments, provided herein is a soluble form of human IL-15Ra (e.g., a purified soluble form of human IL-15Ra) which has the following amino acid sequence: ITCPPPMSVE HADIWVKSYS LYSRERYICN SGFKRKAGTS SLTECVLNKA TNVAHWTTPS LKCIRDPALV HQRPAPPSTV TTAGVTPQPE SLSPSGKEPA ASSPSSNNTA ATTAAIVPGS QLMPSKSPST GTTEISSHES SHGTPSQTTA KNWELTASAS HQPPGVYPQGHSDTT (SEQ ID NO: 45). In some embodiments, provided herein is an IL-15Ra derivative (e.g., a purified and/or soluble form of an IL-15Ra derivative), which is a polypeptide that: (i) is at least 40%, 45%, 50%, 55%, 60%, 65%, 70%, 75%, 80%, 85%, 90%, 95%, 98% or 99% identical to SEQ ID NO: 45; and (ii) terminates with the amino acid sequence PQGHSDTT (SEQ ID NO: 26).

In some embodiments, provided herein is an IL-15Ra derivative of naturally occurring human IL-15Ra, wherein the IL-15Ra derivative is soluble and: (a) the last amino acids at the C-terminal end of the IL-15Ra derivative consist of amino acid residues PQGHSDTT (SEQ ID NO: 26), wherein T is at the C-terminal end of the amino acid sequence; (b) the last amino acids at the C-terminal end of the IL-15Ra derivative consist of amino acid residues PQGHSDT (SEQ ID NO: 27), wherein T is at the C-terminal end of the amino acid sequence; (c) the last amino acids at the C-terminal end of the IL-15Ra derivative consist of amino acid residues PQGHSD (SEQ ID NO: 28), wherein D is at the C-terminal end of the amino acid sequence; (d) the last amino acids at the C-terminal end of the IL-15Ra derivative consist of amino acid residues PQGHS (SEQ ID NO: 29), wherein S is at the C-terminal end of the amino acid sequence; or (e) the last amino acids at the C-terminal end of the IL-15Ra derivative consist of amino acid residues PQGH (SEQ ID NO: 30), wherein H is at the C-terminal end of the amino acid sequence. In some embodiments, the amino acid sequences of these IL-15Ra derivatives are at least 75%, at least 85%, at least 90%, at least 95%, at least 96%, at least 97%, at least 98%, or at least 99% identical to the amino acid sequence of SEQ ID NO: 45. In some embodiments, provided herein is an IL-15Ra derivative of a naturally occurring human IL-15Ra, wherein the IL-15Ra derivative: (i) is soluble; (ii) comprises an amino acid sequence that is at least 75%, at least 80%, at least 90%, at least 95%, at least 96%, at least 97%, or at least 98% identical to the amino acid sequence of SEQ ID NO:45; and (iii) terminates with the amino acid sequence PQG (SEQ ID NO: 31), wherein G is at the C-terminal end of the amino acid sequence of the IL-15Ra derivative. In some embodiments, these IL-15Ra derivativies are purified.

In another aspect, provided herein are IL-15Ra derivatives in which the cleavage site for an endogenous protease that cleaves native IL-15Ra has been mutated. In one embodiment, provided herein are IL-15Ra derivatives comprising one, two, three, four, five, six, seven or eight mutations (e.g., additions, deletions or substitutions; such as deletions or substitutions of one, two, three, four, five, six, seven or eight amino acid residues) in the extracellular domain cleavage site of IL-15Ra such that cleavage of the IL-15Ra by an endogenous protease that cleaves native IL-15Ra is inhibited. As discussed above, the proteolytic cleavage of membrane-bound human IL-15Ra takes place between Gly170 and His171 in human IL-15Ra. In one embodiment, these amino acid residues or surrounding amino acid residues are mutated such that cleavage of IL-15Ra by an endogenous protease that cleaves native IL-15Ra is inhibited. In some embodiments, the amino acid sequence PQGHSDTT (SEQ ID NO:26) is mutated such that cleavage by an endogenous protease that cleaves native human IL-15Ra is inhibited. In some embodiments, one, two, three, four, five, six, seven, or eight amino acid substitutions and/or deletions (such as substitutions and/or deletions of one, two, three, four, five, six, seven or eight amino acid residues) are introduced into the amino acid sequence PQGHSDTT (SEQ ID NO: 26) of human IL-15Ra such that cleavage by an endogenous proteases that cleaves native human IL-15Ra is inhibited. In some embodiments, the amino acid sequence PQGHSDTT (SEQ ID NO:26) is replaced with a cleavage site that is recognized and cleaved by a heterologous protease. Non-limiting examples of such heterologous protease cleavage sites include Arg-X-X-Arg (SEQ ID NO:7), which is recognized and cleaved by furin protease; and A-B-Pro-Arg-X-Y (SEQ ID NO:8) (A and B are hydrophobic amino acids and X and Y are nonacidic amino acids) and Gly-Arg-Gly, which are recognized and cleaved by the thrombin protease.

In another aspect, provided herein are IL-15Ra derivatives, wherein the IL-15Ra derivatives: (i) comprises a mutated extracellular cleavage site that inhibits cleavage by an endogenous protease that cleaves native IL-15Ra, and (ii) lack all or a fragment of the transmembrane domain of native IL-15Ra. In some embodiments, provided herein are IL-15Ra derivatives, wherein the IL-15Ra derivatives comprise: (i) one, two, three, four, five, six, seven or eight mutations (e.g., substitutions and/or deletions) in the extracellular cleavage site of IL-15Ra such that cleavage of IL-15Ra by an endogenous protease that cleaves native IL-15Ra is inhibited, and (ii) all or a fragment of a transmembrane domain of a heterologous molecule in place of all or a fragment of the transmembrane domain of native IL-15Ra. In some embodiments, provided herein are IL-15Ra derivatives, wherein the IL-15Ra derivatives comprise: (i) one, two, three, four, five, six, seven or eight mutations (e.g., substitutions and/or deletions) in the amino acid sequence PQGHSDTT (SEQ ID NO:26) such that cleavage of IL-15Ra by an endogenous protease that cleaves native IL-15Ra is inhibited, and (ii) all or a fragment of a transmembrane domain of a heterologous molecule in place of all or a fragment of the transmembrane domain of native IL-15Ra. In accordance with these embodiments, the IL-15Ra derivatives may or may not comprise all or a fragment of the cytoplasmic tail of native IL-15Ra. In some embodiments, the heterologous molecule is CD4, CD8, or major histocompatability complex (MHC).

In another aspect, provided herein are glycosylated forms of IL-15Ra (e.g., purified glycosylated forms of IL-15Ra), wherein the glycosylation of the IL-15Ra accounts for at least 20%, at least 25%, at least 30%, at least 35%, at least 40%, at least 45%, at least 50%, or 20% to 25%, 20% to 30%, 25% to 30%, 25% to 35%, 30% to 35%, 30% to 40%, 35% to 40%, 35% to 45%, 40% to 50%, 45% to 50%, 20% to 40%, or 25% to 50% of the mass (molecular weight) of the IL-15Ra as assessed by techniques known to one of skill in the art. The percentage of the mass (molecular weight) of IL-15Ra (e.g., purified IL-15Ra) that glycosylation of IL-15Ra accounts for can be determined using, for example and without limitation, gel electrophoresis and quantitative densitometry of the gels, and comparison of the average mass (molecular weight) of a glycosylated form of IL-15Ra (e.g., a purified glycosylated form of IL-15Ra) to the non-glycosylated form of IL-15Ra (e.g., a purified non-glycosylated form of IL-15Ra). In one embodiment, the average mass (molecular weight) of IL-15Ra (e.g., purified IL-15Ra) can be determined using MALDI-TOF MS spectrum on Voyager De-Pro equipped with CovalX HM-1 high mass detector using sinapic acid as matrix, and the mass of a glycosylated form of IL-15Ra (e.g., purified glycosylated form of IL-15Ra) can be compared to the mass of the non-glycosylated form of IL-15Ra (e.g., purified non-glycosylated form of IL-15Ra) to determine the percentage of the mass that glycosylation accounts for (see, e.g., Figure 27).

In some embodiments, provided herein is a glycosylated IL-15Ra (*e*.*g*., human IL-15Ra), wherein the glycosylation accounts for at least 20%, 25%, 30%, 35%, 40%, 45% or 50% of the mass (molecular weight) of the IL-15Ra. In some embodiments, provided herein is a glycosylated IL-15Ra (*e*.*g*., human IL-15Ra), wherein the glycosylation accounts for 20% to 25%, 20% to 30%, 25% to 30%, 25% to 35%, 30% to 35%, 30% to 40%, 35% to 40%, 35% to 45%, 40% to 50%, 45% to 50%, 20% to 40%, or 25% to 50% of the mass (molecular weight) of the IL-15Ra. In some embodiments, provided herein is a glycosylated IL-15Ra (e.g., human IL-15Ra), wherein the glycosylation accounts for about 20%, about 25%, about 30%, about 35%, about 40%, about 45% or about 50% of the mass (molecular weight) of the IL-15Ra. In some embodiments, the glycosylated IL-15Ra is a native IL-15Ra (*e.g*., a native human IL-15Ra). In other embodiments, the glycosylated IL-15Ra is an IL-15Ra derivative (*e.g*., an IL-15Ra derivative of naturally occurring human IL-15Ra). In some embodiments, the glycosylated IL-15Ra is a native soluble human IL-15Ra, such as SEQ ID NO:32 or 33. In other embodiments, the glycosylated IL-15Ra is an IL-15Ra derivative that is a soluble form of human IL-15Ra. In some embodiments, the glycosylated IL-15Ra has the amino acid sequence of SEQ ID NO: 4, 34, 35, 36, 37, 38, 39, 40, 41, or 45. In particular embodiments, the glycosylated IL-15Ra has an amino acid sequence that is at least 40%, 45%, 50%, 55%, 60%, 65%, 70%, 75%, 80%, 85%, 90%, 95%, 98% or 99% identical to SEQ ID NO: 3, 4,: 32, 33, 34, 35, 36, 37, 38, 39, 40, 41, or 45. In some embodiments, the glycosylated IL-15Ra is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra. In certain embodiments, the glycosylated IL-15Ra is purified or isolated.

In some embodiments, provided herein is a composition comprising IL-15 and glycosylated IL-15Ra (*e*.*g*., human IL-15Ra), wherein the glycosylation of the IL-15Ra accounts for at least 20%, at least 25%, at least 30%, at least 35%, at least 40%, at least 45%, or at least 50% of the mass (molecular weight) of the IL-15Ra as assessed by techniques known to one of skill in the art. In some embodiments, provided herein is provided herein is a composition comprising IL-15 and glycosylated IL-15Ra (*e*.*g*., human IL-15Ra), wherein the glycosylation of the IL-15Ra accounts for 20% to 25%, 20% to 30%, 25% to 30%, 25% to 35%, 30% to 35%, 30% to 40%, 35% to 40%, 35% to 45%, 40% to 50%, 45% to 50%, 20% to 40%, or 25% to 50% of the mass (molecular weight) of the IL-15Ra as assessed by techniques known to one of skill in the art. In other embodiments, provided herein is a composition comprising IL-15 and glycosylated IL-15Ra (*e*.*g*., human IL-15Ra), wherein the glycosylation of the IL-15Ra accounts for about 20%, about 25%, about 30%, about 35%, about 40%, about 45%, or about 50% of the mass (molecular weight) of the IL-15Ra as assessed by techniques known to one of skill in the art. In some embodiments, the IL-15 is glycosylated. In some embodiments, the glycosylated IL-15Ra is a native IL-15Ra (*e.g*., a native human IL-15Ra). In other embodiments, the glycosylated IL-15Ra is an IL-15Ra derivative (e.g., an IL-15Ra derivative of naturally occurring human IL-15Ra). In some embodiments, the glycosylated IL-15Ra is a native soluble human IL-15Ra, such as SEQ ID NO:32 or 33. In other embodiments, the glycosylated IL-15Ra is an IL-15Ra derivative that is a soluble form of human IL-15Ra. In some embodiments, the glycosylated IL-15Ra has the amino acid sequence of SEQ ID NO: 4, 34, 35, 36, 37, 38, 39, 40, 41, or 45. In particular embodiments, the glycosylated IL-15Ra has an amino acid sequence that is at least 40%, 45%, 50%, 55%, 60%, 65%, 70%, 75%, 80%, 85%, 90%, 95%, 98% or 99% identical to SEQ ID NO: 3, 4,: 32, 33, 34, 35, 36, 37, 38, 39, 40, 41, or 45. In some embodiments, the glycosylated IL-15Ra is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra.

In some embodiments, provided herein is an IL-15/IL-15Ra complex comprising glycosylated IL-15Ra (*e*.*g*., human IL-15Ra),wherein the glycosylation of the IL-15Ra accounts for at least 20%, at least 25%, at least 30%, at least 35%, at least 40%, at least 45%, or at least 50% of the mass (molecular weight) of the IL-15Ra as assessed by techniques known to one of skill in the art. In some embodiments, provided herein is an IL-15/IL-15Ra complex comprising glycosylated IL-15Ra (*e*.*g*., human IL-15Ra),wherein the glycosylation of the IL-15Ra accounts for 20% to 25%, 20% to 30%, 25% to 30%, 25% to 35%, 30% to 35%, 30% to 40%, 35% to 40%, 35% to 45%, 40% to 50%, 45% to 50%, 20% to 40%, or 25% to 50% of the mass (molecular weight) of the IL-15Ra as assessed by techniques known to one of skill in the art. In other embodiments, provided herein is an IL-15/IL-15Ra complex comprising glycosylated IL-15Ra (*e*.*g*., human IL-15Ra),wherein the glycosylation of the IL-15Ra accounts for about 20%, about 25%, about 30%, about 35%, about 40%, about 45%, or about 50% of the mass (molecular weight) of the IL-15Ra as assessed by techniques known to one of skill in the art. In some embodiments, the glycosylated IL-15Ra is a native IL-15Ra (*e.g*., a native human IL-15Ra). In other embodiments, the glycosylated IL-15Ra is an IL-15Ra derivative (*e*.*g*., an IL-15Ra derivative of naturally occurring human IL-15Ra). In some embodiments, the glycosylated IL-15Ra is a native soluble human IL-15Ra, such as SEQ ID NO:32 or 33. In other embodiments, the glycosylated IL-15Ra is an IL-15Ra derivative that is a soluble form of human IL-15Ra. In some embodiments, the glycosylated IL-15Ra has the amino acid sequence of SEQ ID NO: 4, 34, 35, 36, 37, 38, 39, 40, 41, or 45. In particular embodiments, the glycosylated IL-15Ra has an amino acid sequence that is at least 40%, 45%, 50%, 55%, 60%, 65%, 70%, 75%, 80%, 85%, 90%, 95%, 98% or 99% identical to SEQ ID NO: 3, 4, 32, 33, 34, 35, 36, 37, 38, 39, 40, 41, or 45. In some embodiments, the glycosylated IL-15Ra is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra. In certain embodiments, the IL-15/IL-15Ra complex is purified or isolated.

In another aspect, provided herein are glycosylated forms of IL-15Ra, wherein the IL-15Ra is glycosylated (N- or O-glycosylated) at certain amino acid residues. In some embodiments, provided herein is a human IL-15Ra which is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra. In some embodiments, the glycosylated IL-15Ra is a native human IL-15Ra. In other embodiments, the glycosylated IL-15Ra is an IL-15Ra derivative of naturally occurring human IL-15Ra. In some embodiments, the glycosylated IL-15Ra is a native soluble human IL-15Ra, such as SEQ ID NO:32 or 33. In other embodiments, the glycosylated IL-15Ra is an IL-15Ra derivative that is a soluble form of human IL-15Ra. In some embodiments, the glycosylated IL-15Ra has the amino acid sequence of SEQ ID NO: 4, 34, 35, 36, 37, 38, 39, 40, 41, or 45. In particular embodiments, the glycosylated IL-15Ra has an amino acid sequence that is at least 40%, 45%, 50%, 55%, 60%, 65%, 70%, 75%, 80%, 85%, 90%, 95%, 98% or 99% identical to SEQ ID NO: 3, 4, 32, 33, 34, 35, 36, 37, 38, 39, 40, 41, or 45. In certain embodiments, the glycosylated IL-15Ra is purified or isolated.

In some embodiments, provided herein is a composition comprising IL-15 and human IL-15Ra, wherein the human IL-15Ra is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra. In some embodiments, the glycosylated IL-15Ra is a native human IL-15Ra. In other embodiments, the glycosylated IL-15Ra is an IL-15Ra derivative of naturally occurring human IL-15Ra. In some embodiments, the glycosylated IL-15Ra is a native soluble human IL-15Ra, such as SEQ ID NO:32 or 33. In other embodiments, the glycosylated IL-15Ra is an IL-15Ra derivative that is a soluble form of human IL-15Ra. In some embodiments, the glycosylated IL-15Ra has the amino acid sequence of SEQ ID NO: 4, 34, 35, 36, 37, 38, 39, 40, 41, or 45. In particular embodiments, the glycosylated IL-15Ra has an amino acid sequence that is at least 40%, 45%, 50%, 55%, 60%, 65%, 70%, 75%, 80%, 85%, 90%, 95%, 98% or 99% identical to SEQ ID NO: 3, 4, 32, 33, 34, 35, 36, 37, 38, 39, 40, 41, or 45. In certain embodiments, the glycosylated IL-15Ra is purified or isolated.

In some embodiments, provided herein is an IL-15/IL-15Ra complex comprising human IL-15Ra which is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra. In some embodiments, the glycosylated IL-15Ra is a native human IL-15Ra. In other embodiments, the glycosylated IL-15Ra is an IL-15Ra derivative of naturally occurring human IL-15Ra. In some embodiments, the glycosylated IL-15Ra is a native soluble human IL-15Ra, such as SEQ ID NO:32 or 33. In other embodiments, the glycosylated IL-15Ra is an IL-15Ra derivative that is a soluble form of human IL-15Ra. In some embodiments, the glycosylated IL-15Ra has the amino acid sequence of SEQ ID NO: 4, 34, 35, 36, 37, 38, 39, 40, 41, or 45. In particular embodiments, the glycosylated IL-15Ra has an amino acid sequence that is at least 40%, 45%, 50%, 55%, 60%, 65%, 70%, 75%, 80%, 85%, 90%, 95%, 98% or 99% identical to SEQ ID NO: 3, 4, 32, 33, 34, 35, 36, 37, 38, 39, 40, 41, or 45. In certain embodiments, the IL-15/IL-15Ra complex is purified or isolated.

In some embodiments, provided herein is a glycosylated form of IL-15Ra (*e.g*., human IL-15Ra), wherein the glycosylation accounts for at least 20%, at least 25%, at least 30%, at least 35%, at least 40%, at least 45%, at least 50%, or 20% to 25%, 20% to 30%, 25% to 30%, 25% to 35%, 30% to 35%, 30% to 40%, 35% to 40%, 35% to 45%, 40% to 50%, 45% to 50%, 20% to 40%, or 25% to 50% of the mass (molecular weight) of the IL-15Ra, and which is glycosylated on at least one, at least two, at least three, at least four, at least five, at least six, or at least seven of the following sites: (i) Thr5 of amino acid sequence NWELTASASHQPPGVYPQG (SEQ ID NO: 42) in the IL-15Ra (*e*.*g*., O-glycosylated); (ii) Ser7 of amino acid sequence NWELTASASHQPPGVYPQG (SEQ ID NO: 42) in the IL-15Ra (*e*.*g*., O-glycosylated); (iii) Ser 8 of amino acid sequence ITCPPPMSVEHADIWVK (SEQ ID NO: 43) or amino acid sequence ITCPPPMSVEHADIWVKSYSLYSRERYICNS (SEQ ID NO: 44) in the IL-15Ra (*e*.*g*., N-glycosylated); (iv) Ser 18 of amino acid sequence ITCPPPMSVEHADIWVKSYSLYSRERYICNS (SEQ ID NO: 44) in the IL-15Ra (*e.g*., N-glycosylated); (v) Ser 20 of amino acid sequence ITCPPPMSVEHADIWVKSYSLYSRERYICNS (SEQ ID NO: 44) in the IL-15Ra (*e.g*., N-glycosylated); (vi) Ser 23 of amino acid sequence ITCPPPMSVEHADIWVKSYSLYSRERYICNS (SEQ ID NO: 44) in the IL-15Ra (*e.g*., N-glycosylated); (vii) Ser 31 of amino acid sequence ITCPPPMSVEHADIWVKSYSLYSRERYICNS (SEQ ID NO: 44) in the IL-15Ra (*e.g*., N-glycosylated). In a particular embodiment, the glycosylated human IL-15Ra comprises amino acid sequence of SEQ ID NO: 3, 4, 32, 33, 34, 35, 36, 37, 38, 39, 40, 41 or 45. In another embodiment, the glycosylated human IL-15Ra is: (i)soluble; and (ii) (a) the last amino acids at the C-terminal end of the soluble form of human IL-15Ra consist of amino acid residues PQGHSDTT (SEQ ID NO: 26), wherein T is at the C-terminal end of the amino acid sequence; (b) the last amino acids at the C-terminal end of the soluble form of human IL-15Ra consist of amino acid residues PQGHSDT (SEQ ID NO: 27), wherein T is at the C-terminal end of the amino acid sequence; (c) the last amino acids at the C-terminal end of the soluble form of human IL-15Ra consist of amino acid residues PQGHSD (SEQ ID NO: 28), wherein D is at the C-terminal end of the amino acid sequence; (d) the last amino acids at the C-terminal end of the soluble form of IL-15Ra consist of amino acid residues PQGHS (SEQ ID NO: 29), wherein S is at the C-terminal end of the amino acid sequence; (e) the last amino acids at the C-terminal end of the soluble form of human IL-15Ra consist of amino acid residues PQGH (SEQ ID NO: 30), wherein H is at the C-terminal end of the amino acid sequence; or (f) the last amino acids at the C-terminal end of the soluble form of human IL-15Ra consist of amino acid residues PQG (SEQ ID NO: 31), wherein G is at the C-terminal end of the amino acid sequence. In some embodiments, the glycosylated IL-15Ra is purified or isolated. In other embodiments, the glycosylated IL-15Ra is part of a composition comprising IL-15. In yet other embodiments, the glycosylated IL-15Ra is part of an IL-15/IL-15Ra complex.

### 5.2 Therapeutic Agents

Provided herein are complexes that bind to the βγ subunits of the IL-15 receptor, induce IL-15 signal transduction (*e*.*g*., Jak/Stat signal transduction) and enhance IL-15-mediated immune function, wherein the complexes comprise IL-15 covalently or noncovalently bound to interleukin-15 receptor alpha ("IL-15Ra") ("IL-15/IL-15Ra complexes" or "Therapeutic Agents"). The IL-15/IL-15Ra complex is able to bind to the βγ receptor complex.

The IL-15/IL-15Ra complexes may be composed of native IL-15 or an IL-15 derivative and native IL-15Ra or an IL-15Ra derivative. In some embodiments, an IL-15/IL-15Ra complex comprises native IL-15 or an IL-15 derivative and an IL-15Ra described in Section 5.1, *supra.* In an embodiment, an IL-15/IL-15Ra complex comprises native IL-15 or an IL-15 derivative and IL-15Ra with the amino acid sequence of SEQ ID NO: 33, 35, 37, 39, 41 or 45. In another embodiment, an IL-15/IL-15Ra complex comprises native IL-15 or an IL-15 derivative and a glycosylated form of IL-15Ra described in Section 5.1, *supra.* Examples of IL-15/IL-15Ra complexes are also described in Section 5.1, *supra.*

In a specific embodiment, an IL-15/IL-15Ra complex comprises native IL-15 or an IL-15Ra derivative and native soluble IL-15Ra (*e*.*g*., native soluble human IL-15Ra). In another embodiment, an IL-15/IL-15Ra complex is composed of an IL-15 derivative and an IL-15Ra derivative. In another embodiment, an IL-15/IL-15Ra complex is composed of native IL-15 and an IL-15Ra derivative. In one embodiment, the IL-15Ra derivative is a soluble form of IL-15Ra. Examples of soluble forms of IL-15Ra are described in Section 5.1, *supra.* In an embodiment, the soluble form of IL-15Ra lacks the transmembrane domain of native IL-15Ra, and optionally, the intracellular domain of native IL-15Ra. In another embodiment, the IL-15Ra derivative is the extracellular domain of native IL-15Ra or a fragment thereof. In some embodiments, the IL-15Ra derivative is a fragment of the extracellular domain comprising the sushi domain or exon 2 of native IL-15Ra. In some embodiments, the IL-15Ra derivative comprises a fragment of the extracellular domain comprising the sushi domain or exon 2 of native IL-15Ra and at least one amino acid that is encoded by exon 3. In some embodiments, the IL-15Ra derivative comprises a fragment of the extracellular domain comprising the sushi domain or exon 2 of native IL-15Ra and an IL-15Ra hinge region or a fragment thereof. In some embodiments, the IL-15Ra comprises the amino acid sequence of SEQ ID NO:19 or 20. In some embodiments, the IL-15Ra comprises the amino acid sequence of SEQ ID NO: 33, 35, 37, 39, 41 or 45. In certain embodiments, the IL-15Ra is the native soluble human IL-15Ra.

In another embodiment, the IL-15Ra derivative comprises a mutation in the extracellular domain cleavage site that inhibits cleavage by an endogenous protease that cleaves native IL-15Ra. As discussed in Section 5.1, *supra*, the extracellular cleavage site of native IL-15Ra has been identified by the inventors. In an embodiment, the extracellular domain cleavage site of IL-15Ra is replaced with a cleavage site that is recognized and cleaved by a heterologous known protease. Non-limiting examples of such heterologous protease cleavage sites include Arg-X-X-Arg (SEQ ID NO: 7), which is recognized and cleaved by furin protease; and A-B-Pro-Arg-X-Y (SEQ ID NO: 8) (A and B are hydrophobic amino acids and X and Y are nonacidic amino acids) and Gly-Arg-Gly, which are recognized and cleaved by thrombin protease.

In a specific embodiment, the IL-15Ra is encoded by a nucleic acid sequence optimized to enhance expression of IL-15Ra, *e*.*g*., using methods as described in U.S. Provisional Application No. 60/812,566, filed on June 9, 2006; International Patent Application Publication Nos. WO 2007/084342 and WO 2010/020047; and U.S. Patent Nos. 5,965,726; 6,174,666; 6,291,664; 6,414,132; and 6,794,498, which are incorporated by reference herein in their entireties. In another embodiment, the IL-15 is encoded by a nucleic acid sequence optimized to enhance expression of IL-15, *e.g*., using methods as described in U.S. Provisional Application Nos. 60/812,566, filed on June 9, 2006 and 60/758,819, filed on January 13, 2006, and International Patent Application Publication Nos. WO 2007/084342 and WO 2010/020047; and U.S. Patent Nos. 5,965,726; 6,174,666; 6,291,664; 6,414,132; and 6,794,498, which are incorporated by reference herein in their entireties.

In addition to IL-15 and IL-15Ra, the IL-15/IL-15Ra complexes may comprise a heterologous molecule. In some embodiments, the heterologous molecule is an antigen associated with a disease that one intends to prevent, treat and/or manage (*e*.*g*., a viral antigen, bacterial antigen, parasitic antigen, or cancer antigen). Non-limiting examples of such antigens include antigens of the flavivirus, West Nile Virus (WNV) including structural proteins, *e.g*., C, M, and E, and non-structural proteins, *e.g*., NS1, NS2A, NS2B, NS3, NS4A, NS4B and NS5; human immunodeficiency virus (HIV) antigens gp41, gp120, gp160, Nef, Gag, and Rev, Tat, Vif, Vpu, Vpr, or vpx; influenza virus hemagglutinin; human respiratory syncytial virus G glycoprotein; core protein, matrix protein or other protein of Dengue virus; measles virus hemagglutinin; herpes simplex virus type 2 glycoprotein gB; poliovirus I VP1 (Emini et al., 1983, Nature 304:699); an envelope glycoprotein of HIV I; hepatitis B surface antigen; diptheria toxin; streptococcus 24M epitope; gonococcal pilin; pseudorabies virus g50 (gpD); pseudorabies virus II (gpB); pseudorabies virus gIII (gpC); pseudorabies virus glycoprotein H; pseudorabies virus glycoprotein E; transmissible gastroenteritis glycoprotein 195; transmissible gastroenteritis matrix protein; swine rotavirus glycoprotein 38; swine parvovirus capsid protein; Serpulina hydodysenteriae protective antigen; bovine viral diarrhea glycoprotein 55; Newcastle disease virus hemagglutinin-neuraminidase; swine flu hemagglutinin; swine flu neuraminidase; antigens of foot and mouth disease virus; antigens of hog cholera virus; antigens of swine influenza virus; antigens of African swine fever virus; Mycoplasma hyopneumoniae; antigens of infectious bovine rhinotracheitis virus (*e*.*g*., infectious bovine rhinotracheitis virus glycoprotein E or glycoprotein G); antigens of infectious laryngotracheitis virus (*e*.*g*., infectious laryngotracheitis virus glycoprotein G or glycoprotein I); a glycoprotein of La Crosse virus; antigens of neonatal calf diarrhea virus; Venezuelan equine encephalomyelitis virus; punta toro virus; murine leukemia virus; mouse mammary tumor virus; hepatitis B virus core protein and/or hepatitis B virus surface antigen or a fragment or derivative thereof (see, *e.g*., U.K. Patent Publication No. GB 2034323A published June 4, 1980; Ganem and Varmus, 1987, Ann. Rev. Biochem. 56:651-693; Tiollais et al., 1985, Nature 317:489-495); antigen of equine influenza virus or equine herpesvirus (*e*.*g*., equine influenza virus type A/Alaska 91 neuraminidase, equine influenza virus type A/Miami 63 neuraminidase; equine influenza virus type A/Kentucky 81 neuraminidase; equine herpes virus type 1 glycoprotein B; equine herpes virus type 1 glycoprotein D); antigen of bovine respiratory syncytial virus or bovine parainfluenza virus (*e*.*g*., bovine respiratory syncytial virus attachment protein (BRSV G); bovine respiratory syncytial virus fusion protein (BRSV F); bovine respiratory syncytial virus nucleocapsid protein (BRSV N); bovine parainfluenza virus type 3 fusion protein; the bovine parainfluenza virus type 3 hemagglutinin neuraminidase); bovine viral diarrhea virus glycoprotein 48 or glycoprotein 53.

Other non-limiting examples of antigens include KS 1/4 pan-carcinoma antigen, ovarian carcinoma antigen (CA125), prostatic acid phosphate, prostate specific antigen, melanoma-associated antigen p97, melanoma antigen gp75, high molecular weight melanoma antigen (HMW-MAA), prostate specific membrane antigen, carcinoembryonic antigen (CEA), polymorphic epithelial mucin antigen, human milk fat globule antigen, Colorectal tumor-associated antigens such as: CEA, TAG-72, CO17-1A; GICA 19-9, CTA-1 and LEA, Burkitt's lymphoma antigen-38.13, CD19, human B-lymphoma antigen-CD20, CD33, melanoma specific antigens such as ganglioside GD2, ganglioside GD3, ganglioside GM2, ganglioside GM3, tumor-specific transplantation type of cell-surface antigen (TSTA) such as virally-induced tumor antigens including T-antigen DNA tumor viruses and envelope antigens of RNA tumor viruses, oncofetal antigen-alpha-fetoprotein such as CEA of colon, bladder tumor oncofetal antigen, differentiation antigen such as human lung carcinoma antigen L6, L20, antigens of fibrosarcoma, human leukemia T cell antigen-Gp37, neoglycoprotein, sphingolipids, breast cancer antigen such as EGFR (Epidermal growth factor receptor), HER2 antigen (p185HER2), EphA2 receptor, polymorphic epithelial mucin (PEM), malignant human lymphocyte antigen-APO-1, differentiation antigen such as I antigen found in fetal erthrocytes and primary endoderm, I(Ma) found in gastric adenocarcinomas, M18 and M39 found in breast epithelium, SSEA-1 found in myeloid cells, VEP8, VEP9, Myl, VIM-D5, and D156-22 found in colorectal cancer, TRA-1-85 (blood group H), C14 found in colonic adenocarcinoma, F3 found in lung adenocarcinoma, AH6 found in gastric cancer, Y hapten, Ley found in embryonal carcinoma cells, TL5 (blood group A), EGF receptor, E1 series (blood group B) found in pancreatic cancer, FC10.2 found in embryonal carcinoma cells, gastric adenocarcinoma, CO-514 (blood group Lea) found in adenocarcinoma, NS-10 found in adenocarcinomas, CO-43 (blood group Leb), G49, 19.9 found in colon cancer, gastric cancer mucins, T5A7 found in myeloid cells, R24 found in melanoma, 4.2, GD3, D1.1, OFA-1, GM2, OFA-2, GD2, M1:22:25:8 found in embryonal carcinoma cells and SSEA-3, SSEA-4 found in 4-8-cell stage embryos.

In other embodiments, the heterologous molecule is an antibody that specifically binds to an antigen associated with a disease that one intends to prevent, treat and/or manage (*e.g*., an antibody that specifically binds to a viral antigen, bacterial antigen, parasitic antigen, or cancer antigen). Non-limiting examples of such antibodies include anti-CD34 antibody, anti-CD56 antibody, anti-CD8 antibody, anti-CD22 antibody, anti-CD20 antibody, anti-CD19 antibody, anti-CD3 antibody, anti-EGFR antibody, anti-HER2 antibody, anti-CD34 antibody, anti-ckit antibody, anti-flt3 antibody, anti-hemagglutinin antibody, anti-gp41 antibody, anti-gp120 antibody, and anti-HSV-II glycoprotein gB antibody. In other embodiments, the antibody immunospecifically binds to one of the antigens listed above. In some embodiments, the antibody specifically binds to a cellular antigen (*e*.*g*., a receptor or cell surface antigen) expressed by a cell that one desires to target. For example, the IL-15/IL-15Ra complex can be targeted to CD34+ progenitor cells with an anti-CD34 antibody to induce development of such cells into CD56⁺ NK cells. The IL-15/IL-15Ra complex can be targeted to CD56+ NK cells with an anti-CD56 antibody to induce proliferation of such cells.

In some embodiments, the heterologous molecule increases protein stability. Non-limiting examples of such molecules include polyethylene glycol (PEG), Fc domain of an IgG immunoglobulin or a fragment thereof, or albumin that increase the half-life of IL-15 or IL-15Ra *in vivo.* In some embodiments, IL-15Ra is conjugated/fused to the Fc domain of an immunoglobulin (*e*.*g*., an IgG1) or a fragment thereof. In an embodiment, the IL-15RaFc fusion protein comprises the amino acid sequence of SEQ ID NO: 21 or 22. In another embodiment, the IL-15RaFc fusion protein is the IL-15Ra/Fc fusion protein described in Han et al., 20011, Cytokine 56: 804-810, U.S. Patent No. 8,507,222 or U.S. Patent No. 8,124,084. In some embodiments, the heterologous molecules is not an Fc domain of an immunoglobulin molecule or a fragment thereof.

In those IL-15/IL-15Ra complexes comprising a heterologous molecule, the heterologous molecule may be conjugated to IL-15 and/or IL-15Ra. In one embodiment, the heterologous molecule is conjugated to IL-15Ra. In another embodiment, the heterologous molecule is conjugated to IL-15.

The components of an IL-15/IL-15Ra complex may be directly fused, using either non-covalent bonds or covalent bonds (*e.g*., by combining amino acid sequences via peptide bonds), and/or may be combined using one or more linkers. In an embodiment, IL-15 and IL-15Ra are directly fused to each other using either non-covalent bonds or covalent bonds (*e.g*., by combining amino acid sequences via peptide bonds), and/or may be combined using one or more linkers. In some embodiments, a polypeptide comprising IL-15 and IL-15Ra directly fused to each other using either non-covalent bonds or covalent bonds is functional (e.g., capable of specifically binding to the IL-15R βγ complex and inducing IL-15-mediated signal transduction and/or IL-15-mediated immune function). Linkers suitable for preparing the IL-15/IL-15Ra complexes comprise peptides, alkyl groups, chemically substituted alkyl groups, polymers, or any other covalently-bonded or non-covalently bonded chemical substance capable of binding together two or more components. Polymer linkers comprise any polymers known in the art, including polyethylene glycol ("PEG"). In some embodiments, the linker is a peptide that is 1, 2, 3, 4, 5,6, 7, 8, 9, 10, 11, 12, 13, 14, 15, 16, 17, 18, 19, 20 or more amino acids long. In an embodiment, the linker is long enough to preserve the ability of IL-15 to bind to the IL-15Ra. In other embodiments, the linker is long enough to preserve the ability of the IL-15/IL-15Ra complex to bind to the βγ receptor complex and to act as an agonist to mediate IL-15 signal transduction.

In particular embodiments, IL-15/IL-15Ra complexes are pre-coupled prior to use in the methods described herein (*e*.*g*., prior to contacting cells with the IL-15/IL-15Ra complexes or prior to administering the IL-15/IL-15Ra complexes to a subject). In other embodiments, the IL-15/IL-15Ra complexes are not pre-coupled prior to use in the methods described herein. In some embodiments, the IL-15/IL-15Ra complex is administered in combination with a vaccine composition to enhance the immune response elicited by the administration of the vaccine composition to a subject. In an embodiment, a Therapeutic Agent comprising IL-15 and IL-15Ra directly fused to each other is administered in combination with a vaccine composition to enhance an immune response elicited by administration of the vaccine composition to a subject.

In an embodiment, a Therapeutic Agent enhances or induces immune function in a subject by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the immune function in a subject not administered the Therapeutic Agent using assays well known in the art, *e.g*., ELISPOT, ELISA, and cell proliferation assays. In an embodiment, the immune function is cytokine release (*e*.*g*., interferon-gamma, IL-2, IL-5, IL-10, IL-12, or transforming growth factor (TGF) -beta). In one embodiment, the IL-15 mediated immune function is NK cell proliferation, which can be assayed, *e*.*g*., by flow cytometry to detect the number of cells expressing markers of NK cells (*e.g*., CD56). In another embodiment, the IL-15 mediated immune function is antibody production, which can be assayed, *e*.*g*., by ELISA. In some embodiments, the IL-15 mediated immune function is effector function, which can be assayed, *e*.*g*., by a cytotoxicity assay or other assays well known in the art.

In some embodiments, examples of immune function enhanced by the Therapeutic Agent include the proliferation/expansion of lymphocytes (*e*.*g*., increase in the number of lymphocytes), inhibition of apoptosis of lymphocytes, activation of dendritic cells (or antigen presenting cells), and antigen presentation. In particular embodiments, an immune function enhanced by the Therapeutic Agent is proliferation/expansion in the number of or activation of CD4⁺ T cells (*e.g*., Th1 and Th2 helper T cells), CD8+ T cells (*e.g*., cytotoxic T lymphocytes, alpha/beta T cells, and gamma/delta T cells), B cells (*e*.*g*., plasma cells), memory T cells, memory B cells, dendritic cells (immature or mature), antigen presenting cells, macrophages, mast cells, natural killer T cells (NKT cells), tumor-resident T cells, CD122⁺ T cells, or natural killer cells (NK cells). In one embodiment, the Therapeutic Agent enhances the proliferation/expansion or number of lymphocyte progenitors. In some embodiments, a Therapeutic Agent increases the number of CD4⁺ T cells (*e.g*., Th1 and Th2 helper T cells), CD8+ T cells (*e*.*g*., cytotoxic T lymphocytes, alpha/beta T cells, and gamma/delta T cells), B cells (*e*.*g*., plasma cells), memory T cells, memory B cells, dendritic cells (immature or mature), antigen presenting cells, macrophages, mast cells, natural killer T cells (NKT cells), tumor-resident T cells, CD122⁺ T cells, or natural killer cells (NK cells) by approximately 1 fold, 2 fold, 3 fold, 4 fold, 5 fold, 6 fold, 7 fold, 8 fold, 9 fold, 10 fold, 20 fold, or more relative a negative control (*e*.*g*., number of the respective cells not treated, cultured, or contacted with a Therapeutic Agent).

### 5.3 Expression of IL-15 and IL-15Ra

### 5.3.1 Nucleic Acids

Provided herein are nucleic acids that encode IL-15 and IL-15Ra. The nucleic acids encode IL-15 and IL-15Ra that are capable of covalently or noncovalently binding to each other to form the IL-15/IL-15Ra complexes described in Section 5.2, *supra.* Such IL-15/IL-15Ra complexes can bind to the βγ receptor complex, and induce IL-15-mediated signal transduction.

Nucleic acid sequences encoding native IL-15 are well known in the art and have been described, for a review, *see*, Fehniger and Caligiuri, Blood, 2001, 97:14-32, which is incorporated by reference herein in its entirety. For example, the nucleic acid sequences encoding native IL-15 can be readily found in publicly available publications and databases, *e*.*g*., National Center for Biotechnology Information website at ncbi.nlm.nih.gov. Nucleic acid sequences encoding native IL-15Ra have been described, *e*.*g*., see International Publication No. WO 95/30695, and can also be readily found in publicly available publications and databases, *e*.*g*., National Center for Biotechnology Information website at ncbi.nlm.nih.gov. Cloning techniques well known in the art can be used to generate nucleic acids encoding IL-15 and IL-15Ra. See, *e.g*., Ausubel et al., Current Protocols in Molecular Biology, John Wiley and Sons, Inc. (1995); Sambrook et al., Molecular Cloning, A Laboratory Manual (2d ed.), Cold Spring Harbor Press, Cold Spring Harbor, N.Y. (1989); Birren et al., Genome Analysis: A Laboratory Manual, volumes 1 through 4, Cold Spring Harbor Press, Cold Spring Harbor, N.Y. (1997-1999).

In a specific embodiment, provided herein are nucleic acids that encode the IL-15 and IL-15Ra polypeptides described herein. In a particular embodiment, provided herein are nucleic acids that encode an IL-15Ra polypeptide described in Section 5.1 or 5.2, *supra.* In another embodiment, provided herein are nucleic acids that encode an IL-15Ra polypeptide comprising the amino acid sequence of SEQ ID NO:3, 4, 32, 33, 34, 35, 36, 37, 38, 39, 40, 41 or 45. In another embodiment, provided herein is a nucleic acid sequence that encodes an IL-15Ra polypeptide, wherein the nucleic acid sequence comprises SEQ ID NO: 5 or 6. In another embodiment, provided herein is a nucleic acid sequence that encodes an IL-15 polypeptide comprising the amino acid sequence of SEQ ID NO: 1 or amino acid residues 49 to 162 of SEQ ID NO: 1. In another embodiment, provided herein is a nucleic acid sequence that encodes an IL-15 polypeptide, wherein the nucleic acid sequence comprises SEQ ID NO:2.

In another specific embodiment, the nucleic acids that encode IL-15 and/or IL-15Ra that are optimized, *e*.*g*., by codon/RNA optimization, replacement with heterologous signal sequences, and elimination of mRNA instability elements. Methods to generate optimized nucleic acids encoding IL-15 and IL-15Ra for expression by introducing codon changes and/or eliminating inhibitory regions in the mRNA can be carried out by adapting the optimization methods described in, *e.g*., U.S. Patent Nos. 5,965,726; 6,174,666; 6,291,664; 6,414,132; and 6,794,498, for IL-15 and IL-15Ra. The contents of each of these references are incorporated by reference herein in its entirety. *See* also U.S. Provisional Application Nos. 60/812,566, filed on June 9, 2006, and 60/758,819, filed on January 13, 2007, and International Patent Application Publication Nos. WO 2007/084342 and WO 2010/020047, which are also incorporated by reference herein in their entireties. For example, potential splice sites and instability elements (*e*.*g*., A/T or A/U rich elements) within the RNA of IL-15 and IL-15Ra can be mutated without altering the amino acids encoded by the nucleic acid sequences to increase stability of the RNA for expression. The alterations utilize the degeneracy of the genetic code, *e*.*g*., using an alternative codon for an identical amino acid. In some embodiments, it may be desirable to alter one or more codons to encode a conservative mutation, *e*.*g*., a similar amino acid with similar chemical structure and properties and/or function as the original amino acid. Such methods can increase expression of IL-15 and/or IL-15Ra proteins by at least 1 fold, 2 fold, 3 fold, 4 fold, 5 fold, 10 fold, 20 fold, 30 fold, 40 fold, 50 fold, 60 fold, 70 fold, 80 fold, 90 fold, or 100 fold or more relative to the expression of IL-15 and/or IL-15Ra proteins encoded by native nucleic acid sequences.

Further, the native signal peptide sequence of IL-15 and/or IL-15Ra can be replaced with a heterologous signal peptide, *e.g*., a signal peptide of human GM-CSF (see FIGs. 8A-D), tissue plasminogen activator (tPA) (see FIGs. 5A-D), preprolactin, growth hormone or an immunoglobulin protein (*e.g*., IgE). In an embodiment, the signal peptide of IL-15 is replaced with the signal sequence of tPA. In other specific embodiments, the signal peptide of IL-15 is replaced with the signal peptide of human GM-CSF. Such alternations can increase expression of IL-15 and/or IL-15Ra proteins/polypeptides by at least 1 fold, 2 fold, 3 fold, 4 fold, 5 fold, 10 fold, 20 fold, 30 fold, 40 fold, 50 fold, 60 fold, 70 fold, 80 fold, 90 fold, or 100 fold or more relative to the expression of IL-15 and/or IL-15Ra proteins with the respective native signal peptide, as measured/detected by a technique known to one of skill in the art, e.g., ELISA.

In some embodiments, an optimized nucleotide sequence encoding IL-15 or IL-15Ra hybridizes to the nucleotide sequence encoding native IL-15 or IL-15Ra, respectively. In some embodiments, an optimized nucleotide sequence encoding IL-15 or IL-15Ra hybridizes under high stringency conditions to a nucleotide sequence encoding native IL-15 or IL-15Ra, respectively, or a fragment thereof. In an embodiment, an optimized nucleotide sequence encoding IL-15 or IL-15Ra hybridizes under high stringency, intermediate or lower stringency hybridization conditions to a nucleotide sequence encoding native IL-15 or IL-15Ra, respectively, or a fragment thereof. Information regarding hybridization conditions have been described, *see*, *e.g*., U.S. Patent Application Publication No. US 2005/0048549 (*e*.*g*., paragraphs 72-73).

Also provided herein are nucleic acids encoding IL-15, IL-15Ra, and a heterologous molecule in a form that allows IL-15 to covalently or noncovalently bind to the IL-15Ra to form IL-15/IL-15Ra complexes. In some embodiments, the heterologous molecule is an antigen associated with a disease that one intends to prevent, treat and/or manage. Non-limiting examples of such antigens include those listed above in Section 5.2. In other embodiments, the heterologous molecule is an antibody that specifically binds to an antigen associated with a disease that one intends to prevent, treat and/or manage. Non-limiting examples of such antibodies include those listed above in Section 5.2 and those known in the art. In some embodiments, the antibody specifically binds to a cellular surface antigen (*e*.*g*., a receptor) expressed by a cell that one desires to target. In some embodiments, the heterologous molecule increases protein stability. Non-limiting examples of such molecules include polyethylene glycol (PEG), Fc domain of an IgG immunoglobulin or a fragment thereof, or albumin that increase the half-life of IL-15 or IL-15Ra *in vivo.* In some embodiments, the heterologous molecules is not an Fc domain of an immunoglobulin molecule or a fragment thereof.

In those IL-15/IL-15Ra complexes comprising a heterologous molecule, the heterologous molecule may be conjugated to IL-15 and/or IL-15Ra. In one embodiment, the heterologous molecule is conjugated to IL-15Ra. In another embodiment, the heterologous molecule is conjugated to IL-15.

In some embodiments, IL-15 and IL-15Ra are encoded by one nucleic acid construct (e.*g*., bicistronic construct). In some embodiments, IL-15 and IL-15Ra are encoded by one nucleic acid construct comprising a single open reading frame (ORF) of IL-15 and IL-15Ra. In some embodiments, IL-15 or IL-15Ra encoded by a nucleic acid construct may be conjugated to a nucleic acid encoding a heterologous molecule, such as an antigen or an antibody of interest. In other embodiments, IL-15 and IL-15Ra are encoded by two nucleic acid constructs, wherein a first nucleic acid construct encodes IL-15 and a second nucleic acid construct encodes IL-15Ra. The IL-15 encoded by the first nucleic acid construct may be conjugated to a nucleic acid encoding a heterologous molecule, such as an antigen or an antibody of interest. Alternatively, or in addition, the IL-15Ra encoded by the second nucleic acid construct may be conjugated to a nucleic acid encoding a heterologous molecule, such as an antigen or an antibody of interest.

The nucleic acids described herein may be administered to a subject (preferably, a human subject) as part of a gene therapy protocol. In some embodiments, nucleic acids encoding an IL-15Ra polypeptide described herein (*see*, *e.g*., Section 3.1, Section 5.1 and/or Section 5.2) may be administered to a subject (preferably, a human subject) as part of a gene therapy protocol. In some embodiments, nucleic acids encoding an IL-15Ra polypeptide described herein (*see*, *e.g*., Section 3.1, Section 5.1 and/or Section 5.2) and nucleic acids encoding an IL-15 polypeptide may be administered to a subject (preferably, a human subject) as part of a gene therapy protocol. In some embodiments, the nucleic acids encode an IL-15Ra described in Section 5.1, *supra.* The nucleic acids encoding an IL-15Ra polypeptide and IL-15 polypeptide that are administered to a subject may be part of a vector, plasmid or other construct such as described in Section 5.3.2, *infra.* The nucleic acids encoding an IL-15Ra polypeptide and IL-15 polypeptide may be administered to a subject to enhance IL-15 mediated immune function and/or to prevent, treat and/or manage a disorder in which enhancing IL-15-mediated immune function is beneficial, such as, *e.g.,* cancer, an infectious disease, an immunodeficiency, and/or lymphopenia.

### 5.3.2 Constructs & Cells

The nucleic acids encoding IL-15 and/or IL-15Ra can be inserted into nucleic acid constructs for expression in mammalian cells, bacteria, yeast, and viruses. IL-15 and IL-15Ra can be recombinantly expressed from the same nucleic acid construct (*e*.*g*., using a bicistronic nucleic acid construct) or from different nucleic acid constructs (*e*.*g*., using monocistronic nucleic acid constructs). In one embodiment, IL-15 and IL-15Ra can be recombinantly expressed from a single nucleic acid construct comprising a single open reading frame (ORF) of IL-15 and IL-15Ra.

The nucleic acid constructs may comprise one or more transcriptional regulatory element(s) operably linked to the coding sequence of IL-15 and/or IL-15Ra. The transcriptional regulatory elements are typically 5' to the coding sequence and direct the transcription of the nucleic acids encoding IL-15 and/or IL-15Ra. In some embodiments, one or more of the transcriptional regulatory elements that are found in nature to regulate the transcription of the native IL-15 and/or native IL-15Ra gene are used to control transcription. In other embodiments, one or more transcriptional regulatory elements that are heterologous to the native IL-15 and/or native IL-15Ra gene are used to control transcription. Any transcriptional regulatory element(s) known to one of skill in the art may be used. Non-limiting examples of the types of transcriptional regulatory element(s) include a constitutive promoter, a tissue-specific promoter, and an inducible promoter. In an embodiment, transcription is controlled, at least in part, by a mammalian (in some embodiments, human) transcriptional regulatory element(s). In an embodiment, transcription is controlled, at least in part, by a strong promoter, *e*.*g*., CMV.

Some examples of promoters which may be used to control transcription include, but are not limited to, the SV40 early promoter region (Bernoist & Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); adenovirus (ADV), cytomegalovirus (CMV), bovine papilloma virus (BPV), parovirus B19p6 promoter, prokaryotic expression vectors such as the .beta.-lactamase promoter (Villa-Kamaroff et al., 1978, Proc. Natl. Acad. Sci. U.S.A. 75:3727-3731), or the tac promoter (DeBoer et al., 1983, Proc. Natl. Acad. Sci. U.S.A. 80:21-25); see also "Useful proteins from recombinant bacteria" in Scientific American, 1980, 242:74-94; plant expression vectors comprising the nopaline synthetase promoter region (Herrera-Estrella et al., Nature 303:209-213) or the cauliflower mosaic virus 35S RNA promoter (Gardner, et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378). In other aspects, an inducible promoter can be used.

The nucleic acid constructs also may comprise one or more post-transcriptional regulatory element(s) operably linked to the coding sequence of IL-15 and/or IL-15Ra. The post-transcriptional regulatory elements can be 5' and/or 3' to the coding sequence and direct the post-transcriptional regulation of the translation of RNA transcripts encoding IL-15 and/or IL-15Ra.

In another aspect, the nucleic acid construct can be a gene targeting vector that replaces a gene's existing regulatory region with a regulatory sequence isolated from a different gene or a novel regulatory sequence as described, *e*.*g*., in International Publication Nos. WO 94/12650 and WO 01/68882, which are incorporated by reference herein in their entireties. In some embodiments, a host cell can be engineered to increase production of endogenous IL-15 and/or IL-15Ra by, *e*.*g*., altering the regulatory region of the endogenous IL-15 and/or IL-15Ra genes.

The nucleic acid construct chosen will depend upon a variety of factors, including, without limitation, the strength of the transcriptional regulatory elements and the host cell to be used to express IL-15 and/or IL-15Ra. The nucleic acid constructs can be a plasmid, phagemid, cosmid, viral vector, phage, artificial chromosome, and the like. In one aspect, the vectors can be episomal or non-homologously integrating vectors, which can be introduced into the appropriate host cells by any suitable means (transformation, transfection, conjugation, protoplast fusion, electroporation, calcium phosphate-precipitation, direct microinjection, etc.) to transform them.

The nucleic acid constructs can be a plasmid or a stable integration vector for transient or stable expression of IL-15 and/or IL-15Ra in host cells. For stable expression, the vector can mediate chromosomal integration at a target site or a random chromosomal site. Non-limiting examples of host cell-vector systems that may be used to express IL-15 and/or IL-15Ra include mammalian cell systems infected with virus (*e*.*g*., vaccinia virus, adenovirus, retroviruses, lentiviruses, etc.); insect cell systems infected with virus (*e*.*g*., baculovirus); microorganisms such as yeast containing yeast vectors, or bacteria transformed with bacteriophage, DNA, plasmid DNA, or cosmid DNA; and stable cell lines generated by transformation using a selectable marker. In some embodiments, the nucleic acid constructs include a selectable marker gene including, but not limited to, neo, gpt, dhfr, ada, pac, hyg, CAD and hisD.

The nucleic acid constructs can be monocistronic or multicistronic. A multicistronic nucleic acid construct may encode 2, 3, 4, 5, 6, 7, 8, 9, 10 or more, or in the range of 2-5, 5-10 or 10-20 genes/nucleotide sequences. For example, a bicistronic nucleic acid construct may comprise in the following order a promoter, a first gene (*e.g*., IL-15), and a second gene and (*e.g*., IL-15Ra). In such a nucleic acid construct, the transcription of both genes is driven by the promoter, whereas the translation of the mRNA from the first gene is by a cap-dependent scanning mechanism and the translation of the mRNA from the second gene is by a cap-independent mechanism, *e*.*g*., by an IRES.

Techniques for practicing these aspects will employ, unless otherwise indicated, conventional techniques of molecular biology, microbiology, and recombinant DNA manipulation and production, which are routinely practiced by one of skill in the art. See, *e*.*g*., Sambrook, 1989, Molecular Cloning, A Laboratory Manual, Second Edition; DNA Cloning, Volumes I and II (Glover, Ed. 1985); Oligonucleotide Synthesis (Gait, Ed. 1984); Nucleic Acid Hybridization (Hames & Higgins, Eds. 1984); Transcription and Translation (Hames & Higgins, Eds. 1984); Animal Cell Culture (Freshney, Ed. 1986); Immobilized Cells and Enzymes (IRL Press, 1986); Perbal, A Practical Guide to Molecular Cloning (1984); Gene Transfer Vectors for Mammalian Cells (Miller & Calos, Eds. 1987, Cold Spring Harbor Laboratory); Methods in Enzymology, Volumes 154 and 155 (Wu & Grossman, and Wu, Eds., respectively), (Mayer & Walker, Eds., 1987); Immunochemical Methods in Cell and Molecular Biology (Academic Press, London, Scopes, 1987), Expression of Proteins in Mammalian Cells Using Vaccinia Viral Vectors in Current Protocols in Molecular Biology, Volume 2 (Ausubel et al., Eds., 1991).

The nucleic acid construct(s) comprising nucleic acids encoding IL-15 and/or IL-15Ra can be administered *in vivo* to a mammal or transfected into primary or immortalized cells in culture. Such a nucleic acid construct(s) can be used to enhance IL-15-mediated function and/or to prevent, treat and/or manage a disease in which enhancement of IL-15-mediated function is beneficial, such as the diseases described in Sections 5.7 to 5.9, *infra.* The nucleic acid constructs comprising nucleic acids encoding IL-15 and/or IL-15Ra can be used to generate cells that express IL-15 and/or IL-15Ra. In some embodiments, the cells are primary cells (*e*.*g*., tumor cells isolated from a patient). In other embodiments, the cells are mammalian cell lines.

The host cells chosen for expression of nucleic acids will depend upon the intended use of the cells. Factors such as whether a cell glycosylates similar to cells that endogenously express, *e.g*., IL-15 and/or IL-15Ra, may be considered in selecting the host cells.

Non-limiting examples of hosts cells that can be used to express the protein(s) encoded by the nucleic acid constructs herein include mammalian cells, bacterial cells, yeast cells, primary cells, immortalized cells, plant cells and insect cells. In a specific embodiment, the host cells are a mammalian cell line. Examples of mammalian cell lines include, but are not limited to, COS, CHO, HeLa, NIH3T3, HepG2, MCF7, HEK 293, HEK 293T, RD, PC12, hybridomas, pre-B cells, 293, 293H, K562, SkBr3, BT474, A204, M07Sb, TFβ1, Raji, Jurkat, MOLT-4, CTLL-2, MC-IXC, SK-N-MC, SK-N-MC, SK-N-DZ, SH-SY5Y, C127, N0, and BE(2)-C cells. Other mammalian cell lines available as hosts for expression are known in the art and include many immortalized cell lines available from the American Type Culture Collection (ATCC). In another embodiment, the host cells are immortalized cell lines derived from a subject. In another embodiment, the host cells are primary or secondary cells from a subject. In a particular embodiment, the host cells are cancer cells. In another embodiment, the host cells are irradiated cells. In a particular embodiment, the host cells are irradiated mammalian cell lines or primary cells from a subject. In another embodiment, the host cells are epithelial cells or endothelial cells. In another embodiment, the host cells are fetal/embryonic cells. In some embodiments, the host cells are progenitor cells. In some embodiments, the host cells are lymphocytes (*e*.*g*., T cells and B cells). In another embodiment, the host cells are stem cells. In yet another embodiment, the host cells engineered to express the nucleic acid constructs described herein are from an adult.

In some embodiments, isolated cells are utilized herein. In an embodiment, the isolated cells are at least 80%, 90%, 95% or 98% free of a different cell type as measured by a technique known to one of skill in the art, such as flow cytometry. In other words, at least 80%, 90%, 95% or 98% of the isolated cells are of the same cell type.

In an embodiment, the nucleic acid constructs encoding IL-15 or IL-15Ra can be co-transfected or transfected into the same host cells or different host cells. Optionally, a nucleic acid construct comprising nucleic acids encoding a selectable marker gene can also be transfected into the same cells to select for transfected cells. If the nucleic acid constructs comprising nucleic acids encoding IL-15 and IL-15Ra are transfected into different cells, IL-15 and IL-15Ra expressed by the different cells can be isolated and contacted with each other under conditions suitable to form IL-15/IL-15Ra complexes described in Section 5.2, *supra.* Any techniques known to one of skill in the art can be used to transfect or transducer host cells with nucleic acids including, e.g., transformation, transfection, conjugation, protoplast fusion, electroporation, calcium phosphate-precipitation, direct microinjection, and infection with viruses, including but not limited to adenoviruses, lentiviruses, and retroviruses.

For long-term, high-yield production of a recombinant of IL-15 and IL-15Ra polypeptides, stable cell lines can be generated. For example, cell lines can be transformed using the nucleic acid constructs described herein which may contain a selectable marker gene on the same or on a separate nucleic acid construct. The selectable marker gene can be introduced into the same cell by co-transfection. Following the introduction of the vector, cells are allowed to grow for 1-2 days in an enriched media before they are switched to selective media to allow growth and recovery of cells that successfully express the introduced nucleic acids. Resistant clones of stably transformed cells may be proliferated using tissue culture techniques well known in the art that are appropriate to the cell type. In a particular embodiment, the cell line has been adapted to grow in serum-free medium. In one embodiment, the cell line has been adapted to grow in serum-free medium in shaker flasks. In one embodiment, the cell line has been adapted to grow in stir or rotating flasks. In some embodiments, the cell line is cultured in suspension. In particular embodiments, the cell line is not adherent or has been adapted to grow as nonadherent cells. In some embodiments, the cell line has been adapted to grow in low calcium conditions. In some embodiments, the cell line is cultured or adapted to grow in low serum medium.

In an embodiment, a particularly preferred method of high-yield production of a recombinant polypeptide of the present invention is through the use of dihydro folate reductase (DHFR) amplification in DHFR-deficient CHO cells, by the use of successively increasing levels of methotrexate as described in U.S. Patent No. 4,889,803, which is incorporated by reference herein in its entirety. The polypeptide obtained from such cells may be in a glycosylated form.

In a specific embodiment, a host cell recombinantly expressing IL-15 and IL-15Ra is produced utilizing the techniques described in Section 6.4, *infra.* In another specific embodiment, the host cell is the HEK293 cell described in Section 6.4, *infra.*

In one embodiment, a host cell recombinantly expresses an IL-15Ra polypeptide that is glycosylated (N- or O-glycosylated) at certain amino acid residues. In some embodiments, a host cell recombinantly expresses an IL-15Ra polypeptide(e.g., a human IL-15Ra polypeptide) that is glycosylated, wherein the glycosylation of the IL-15Ra polypeptide accounts for at least 20%, at least 25%, at least 30%, at least 35%, at least 40%, at least 45%, at least 50%, or 20% to 25%, 20% to 30%, 25% to 30%, 25% to 35%, 30% to 35%, 30% to 40%, 35% to 40%, 35% to 45%, 40% to 50%, 45% to 50%, 20% to 40%, or 25% to 50% of the mass (molecular weight) of the IL-15Ra polypeptide. In some embodiments, a host cell recombinantly expresses a human IL-15Ra polypeptide which is glycosylated at one, two, three, four, five, six, seven, or all, of the following glycosylation sites: (i) O-glycosylation on Thr5 of amino acid sequence NWELTASASHQPPGVYPQG (SEQ ID NO: 42) in the IL-15Ra; (ii) O-glycosylation on Ser7 of amino acid sequence NWELTASASHQPPGVYPQG (SEQ ID NO: 42) in the IL-15Ra; (iii) N-glycosylation on Ser 8 of amino acid sequence ITCPPPMSVEHADIWVK (SEQ ID NO: 43) in the IL-15Ra, or Ser 8 of amino acid sequence ITCPPPMSVEHADIWVKSYSLYSRERYICNS (SEQ ID NO: 44) in the IL-15Ra; (iv) N-glycosylation on Ser 18 of amino acid sequence ITCPPPMSVEHADIWVKSYSLYSRERYICNS (SEQ ID NO: 44) in the IL-15Ra; (v) N-glycosylation on Ser 20 of amino acid sequence ITCPPPMSVEHADIWVKSYSLYSRERYICNS (SEQ ID NO: 44) in the IL-15Ra; (vi) N-glycosylation on Ser 23 of amino acid sequence ITCPPPMSVEHADIWVKSYSLYSRERYICNS (SEQ ID NO: 44) in the IL-15Ra; and/or (vii) N-glycosylated on Ser 31 of amino acid sequence ITCPPPMSVEHADIWVKSYSLYSRERYICNS (SEQ ID NO: 44) in the IL-15Ra.

In one embodiment, cell lines are engineered to express both IL-15 and soluble IL-15Ra, and the purified stable heterodimer of the IL-15 and soluble IL-15Ra, which can be used *in vitro* or *in vivo*, *e.g*., can be administered to a human. In some embodiments, cell lines are engineered to express both native human IL-15 and native human IL-15Ra, and the stable heterodimer of native human IL-15 and native soluble human IL-15Ra which is formed can be purified, and this purified heterodimer can be used be administered to a human. In one embodiment, the stability of IL-15 is increased when produced from cell lines recombinantly expressing both IL-15 and IL-15Ra.

In an embodiment, the host cell recombinantly expresses IL-15 and the full length IL-15Ra. In another specific embodiment, the host cell recombinantly expresses IL-15 and the soluble form of IL-15Ra. In another embodiment, the host cell recombinantly expresses IL-15 and a membrane-bound form of IL-15Ra which is not cleaved from the surface of the cell and remains cell associated. In some embodiments, the host cell recombinantly expressing IL-15 and/or IL-15Ra (full-length or soluble form) also recombinantly expresses another polypeptide (*e.g*., a cytokine or fragment thereof).

In some embodiments, a host cell recombinantly expresses an IL-15Ra polypeptide described herein (*see*, *e.g*., Section 3.1, Section 5.1 and/or Section 5.2, *supra*)*.* In an embodiment, a host cell recombinantly expresses an IL-15Ra polypeptide comprising the amino acid sequence of SEQ ID NO: 3, 4, 32, 33, 34, 35, 36, 37, 38, 39, 40, 41 or 45. In another embodiment, a host cell recombinantly expresses a glycosylated IL-15Ra polypeptide described herein (*see*, *e.g*., Section 5.1, *supra*)*.* In some embodiments, such a host cell recombinantly expresses an IL-15 polypeptide in addition to an IL-15Ra polypeptide.

In some embodiments, a host cell recombinantly expresses an IL-15Ra polypeptide described herein described herein (*see*, *e.g*., Section 3.1, Section 5.1 and/or Section 5.2, *supra*), and IL-15 (*e.g*., the IL-15 described in Section 3.1, *supra*)*.* In an embodiment, a host cell recombinantly expresses an IL-15Ra polypeptide comprising the amino acid sequence of SEQ ID NO: 3, 4, 32, 33, 34, 35, 36, 37, 38, 39, 40, 41 or 45, and IL-15 (*e.g*., the IL-15 described in Section 3.1, *supra*)*.* In another embodiment, a host cell recombinantly expresses a glycosylated IL-15Ra polypeptide described herein (*see*, *e.g*., Section 5.1, *supra*), and IL-15 (*e.g*., the IL-15 described in Section 3.1, *supra*)*.*

In some embodiments, a host cell recombinantly expresses an IL-15Ra polypeptide in which the cleavage site for an endogenous protease that cleaves native IL-15Ra has been mutated. In one embodiment, a host cell recombinantly expresses an IL-15Ra derivative comprising one, two, three, four, five, six, seven or eight mutations in the extracellular domain cleavage site of IL-15Ra such that cleavage of the IL-15Ra by an endogenous protease that cleaves native IL-15Ra is inhibited. In some embodiments, a host cell recombinantly expresses an IL-15Ra derivative in which the amino acid sequence PQGHSDTT (SEQ ID NO:26) is mutated such that cleavage by an endogenous protease that cleaves native human IL-15Ra is inhibited. In some embodiments, one, two, three, four, five, six, seven, or eight amino acid substitutions and/or deletions are introduced into the amino acid sequence PQGHSDTT (SEQ ID NO: 26) of human IL-15Ra such that cleavage by an endogenous proteases that cleaves native human IL-15Ra is inhibited. In some embodiments, a host cell recombinantly expresses an IL-15Ra derivative in which the amino acid sequence PQGHSDTT (SEQ ID NO:26) is replaced with a cleavage site that is recognized and cleaved by a heterologous protease. Non-limiting examples of such heterologous protease cleavage sites include Arg-X-X-Arg (SEQ ID NO:7), which is recognized and cleaved by furin protease; and A-B-Pro-Arg-X-Y (SEQ ID NO:8) (A and B are hydrophobic amino acids and X and Y are nonacidic amino acids) and Gly-Arg-Gly, which are recognized and cleaved by the thrombin protease.

In another embodiment, a host cell recombinantly expresses an IL-15Ra derivative, wherein the IL-15Ra derivative: (i) comprises a mutated extracellular cleavage site that inhibits cleavage by an endogenous protease that cleaves native IL-15Ra, and (ii) lacks all or a fragment of the transmembrane domain of native IL-15Ra. In some embodiments, a host cell recombinantly expresses an IL-15Ra derivative, wherein the IL-15Ra derivative comprises: (i) one, two, three, four, five, six, seven or eight mutations (*e*.*g*., substitutions and/or deletions) in the extracellular cleavage site of IL-15Ra such that cleavage of IL-15Ra by an endogenous protease that cleaves native IL-15Ra is inhibited, and (ii) all or a fragment of a transmembrane domain of a heterologous molecule in place of all or a fragment of the transmembrane domain of native IL-15Ra. In some embodiments, a host cell recombinantly expresses an IL-15Ra derivative, wherein the IL-15Ra derivative comprises: (i) one, two, three, four, five, six, seven or eight mutations (*e*.*g*., substitutions and/or deletions) in the amino acid sequence PQGHSDTT (SEQ ID NO:26) such that cleavage of IL-15Ra by an endogenous protease that cleaves native IL-15Ra is inhibited, and (ii) all or a fragment of a transmembrane domain of a heterologous molecule in place of all or a fragment of the transmembrane domain of native IL-15Ra. In accordance with these embodiments, the IL-15Ra derivatives may or may not comprise all or a fragment of the cytoplasmic tail of native IL-15Ra. In some embodiments, the heterologous molecule is CD4, CD8, or MHC.

The nucleic acids encoding IL-15 and/or IL-15Ra can be used to generate mammalian cells that recombinantly express IL-15 and IL-15Ra in high amounts for the isolation and purification of IL-15 and IL-15Ra, preferably the IL-15 and the IL-15Ra are associated as complexes. In one embodiment, high amounts of IL-15/IL-15Ra complexes refer to amounts of IL-15/IL-15Ra complexes expressed by cells that are at least 1 fold, 2 fold, 3 fold, 4 fold, 5 fold, 6 fold, 7 fold, 8 fold, 9 fold, 10 fold, 20 fold, or more than 20 fold higher than amounts of IL-15/IL-15Ra complexes expressed endogenously by control cells (*e.g*., cells that have not been genetically engineered to recombinantly express IL-15, IL-15Ra, or both IL-15 and IL-15Ra, or cells comprising an empty vector). In some embodiments, a host cell described herein expresses approximately 0.1 pg to 25 pg, 0.1 pg to 20 pg, 0.1 pg to 15 pg, 0.1 pg to 10 pg, 0.1 pg to 5 pg, 0.1 pg to 2 pg, 2 pg to 10 pg, or 5 to 20 pg of IL-15 as measured by a technique known to one of skill in the art (*e.g*., an ELISA). In some embodiments, a host cell described herein expresses approximately 0.1 to 0.25 pg per day, 0.25 to 0.5 pg per day, 0.5 to 1 pg per day, 1 to 2 pg per day, 2 to 5 pg per day, or 5 to 10 pg per day of IL-15 as measured by a technique known to one of skill in the art (*e.g*., an ELISA) In some embodiments, a population of host cells that recombinantly expresses IL-15 and IL-15Ra, expresses between 200 ng/million cells per day to 20,000 ng/million cells per day, 200 ng/million cells per day to 15,000 ng/million cells per day, 200 ng/ million cells per day to 10,000 ng/million cells per day, 200 ng/million cells per day to 5,000 ng/million cells per day, 200 ng/million cells per day to 2,000 ng/million cells per day, 200 ng/million cells per day to 1,000 ng/million cells per day, 200 ng/million cells per day to 600 ng/million cells per day, 200 ng/million cells per day to 500 ng/million cells per day, 300 ng/million cells per day to 600 ng/million cells per day of IL-15. In some embodiments, a population of host cells that recombinantly expresses IL-15 and IL-15Ra, expresses about 200 ng/million cells per day, about 300 ng/million cells per day, about 400 ng/million cells per day, about 500 ng/million cells per day, about 600 ng/million cells per day, about 700 ng/million cells per day, about 800 ng/million cells per day, about 900 ng/million cells per day, about 1,000 ng/million cells per day, about 1,500 ng/million cells per day, about 2,000 ng/million cells per day, about 5,000 ng/million cells per day, about 10,000 ng/million cells per day, about 15,000 ng/million cells per day, or about 20,000 ng/million cells per day of IL-15. In a specific embodiment, the IL-15Ra is the soluble form of IL-15Ra. In a specific embodiment, the IL-15Ra is the soluble form of IL-15Ra associated with IL-15 in a stable heterodimer, which increases yields and simplifies production and purification of bioactive heterodimer IL-15/soluble IL-15Ra cytokine.

Recombinant IL-15 and IL-15Ra can be purified using methods of recombinant protein production and purification are well known in the art, *e.g*., see International Publication No. WO 07/070488, which is incorporated by reference herein in its entirety. Briefly, the polypeptide can be produced intracellularly, in the periplasmic space, or directly secreted into the medium. Cell lysate or supernatant comprising the polypeptide can be purified using, for example, hydroxylapatite chromatography, gel electrophoresis, dialysis, and affinity chromatography. Other techniques for protein purification such as fractionation on an ion-exchange column, ethanol precipitation, Reverse Phase HPLC, chromatography on silica, chromatography on heparin SEPHAROSE™ (gel filtration substance; Pharmacia Inc., Piscataway, New Jersey) chromatography on an anion or cation exchange resin (such as a polyaspartic acid column), chromatofocusing, SDS-PAGE, and ammonium sulfate precipitation are also available.

In some embodiments, IL-15 and IL-15Ra are synthesized or recombinantly expressed by different cells and subsequently isolated and combined to form an IL-15/IL-15Ra complex, *in vitro*, prior to administration to a subject. In other embodiments, IL-15 and IL-15Ra are synthesized or recombinantly expressed by different cells and subsequently isolated and simultaneously administered to a subject an IL-15/IL-15Ra complex *in situ* or *in vivo.* In yet other embodiments, IL-15 and IL-15Ra are synthesized or expressed together by the same cell, and the IL-15/IL-15Ra complex formed is isolated. *See* Section 6.4, *infra*, for purification techniques.

In some aspects, host cells that recombinantly express IL-15 and IL-15Ra are administered to a subject (preferably, a human subject) as part of a gene therapy protocol. *See* Section 5.6, *infra.*

### 5.4 Compositions

Provided herein are compositions comprising an IL-15Ra described herein, *e.g*., a soluble IL-15Ra, such as described in Section 5.1, *supra.* Also provided herein are compositions comprising the Therapeutic Agents. The compositions include bulk drug compositions useful in the manufacture of pharmaceutical compositions (*e*.*g*., impure or non-sterile compositions) and pharmaceutical compositions (*i*.*e*., compositions that are suitable for administration to a subject or patient) which can be used in the preparation of unit dosage forms. The compositions (*e*.*g*., pharmaceutical compositions) comprise an effective amount of a Therapeutic Agent or a combination of Therapeutic Agents and a pharmaceutically acceptable carrier. In some embodiments, the compositions (*e*.*g*., pharmaceutical compositions) comprise an effective amount of one or more Therapeutic Agents and a pharmaceutically acceptable carrier. In some embodiments, the composition further comprises an additional therapeutic, *e*.*g*., anti-cancer agent, anti-viral agent, antiinflammatory agent, adjuvant. Non-limiting examples of such therapeutics are provided *infra*.

In an embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e*.*g*., Freund's adjuvant (complete and incomplete) or, more preferably, MF59C.1 adjuvant available from Chiron, Emeryville, CA), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. In one embodiment, water is a carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

Pharmaceutical compositions may be formulated in any conventional manner using one or more pharmaceutically acceptable carriers or excipients. In a specific embodiment, a Therapeutic Agent administered to a subject in accordance with the methods described herein is administered as a pharmaceutical composition.

Generally, the components of the pharmaceutical compositions comprising Therapeutic Agents are supplied either separately or mixed together in unit dosage form, for example, as a dry lyophilized powder or water free concentrate in a hermetically sealed container such as an ampoule or sachette indicating the quantity of active agent. Where the Therapeutic Agent is to be administered by infusion, it can be dispensed with an infusion bottle containing sterile pharmaceutical grade water or saline (*e*.*g*., PBS). Where the Therapeutic Agent is administered by injection, an ampoule of sterile water for injection or saline can be provided so that the ingredients may be mixed prior to administration.

In some embodiments, Therapeutic Agents may be formulated for administration by any method known to one of skill in the art, including but not limited to, parenteral (*e*.*g*., subcutaneous, intravenous, or intramuscular) administration. In one embodiment, the Therapeutic Agents are formulated for local or systemic parenteral administration. In a specific embodiment, the Therapeutic Agents are formulated for subcutaneous or intravenous administration. In one embodiment, the Therapeutic Agents are formulated in a pharmaceutically compatible solution.

The Therapeutic Agents can be formulated for parenteral administration by injection, *e*.*g*., by bolus injection or continuous infusion. Formulations for injection may be presented in unit dosage form, *e.g*., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient (*i.e*., Therapeutic Agent) may be in powder form for constitution with a suitable vehicle, *e*.*g*., sterile pyrogen-free water, before use.

### 5.5 Prophylactic and Therapeutic Methods Involving Cyclical Administration of IL-15/IL-15Ra Complexes

In one aspect, provided herein are methods for enhancing IL-15-mediated immune function, comprising administering to subjects complexes that bind to the βγ subunits of the IL-15 receptor, induce IL-15 signal transduction and enhance IL-15-mediated immune function, wherein the complexes comprise IL-15 covalently or noncovalently bound to interleukin-15 receptor alpha ("IL-15Ra") ("IL-15/IL-15Ra complexes" or "Therapeutic Agents"). Since enhancing IL-15-mediated immune function is beneficial for the prevention, treatment and/or management of certain disorders, provided herein are methods for the prevention, treatment and/or management of such disorders comprising administering to a subject in need thereof IL-15/IL-15Ra complexes. Non-limiting examples of disorders in which it is beneficial to enhance IL-15-mediated immune function include cancer, lymphopenia, immunodeficiencies, infectious diseases, and wounds.

In one aspect, the methods described herein maintain plasma levels of IL-15 above basal levels for approximately 18 to 24 hours or approximately 24 to 36 hours, or approximately 36 to 38 hours following administration of IL-15/IL-15Ra complexes. Basal plasma levels of IL-15 are approximately 1 pg/ml in humans, approximately 8-10 pg/ml in monkeys (such as macaques), and approximately 12 pg/m in rodents (such as mice). Thus, in some embodiments, the methods described herein maintain plasma levels above approximately 1 pg/ml in humans, above approximately 8-10 pg/ml in monkeys (such macaques) and above 12 pg/ml in rodents (such as mice). Without being bound by any theory, the stability of the IL-15 plasma levels maximizes lymphocyte growth and activation while minimizing any side effects associated with IL-15 administration. In one embodiment, the methods described herein achieve stable plasma levels of IL-15 above basal plasma levels by administering subcutaneously doses of approximately 0.1 µg/kg to approximately 10 µg/kg of an IL-15/IL-15Ra complex to a subject. In another embodiment, the methods described herein achieve high plasma levels of IL-15 by administering subcutaneously doses of approximately 0.1 µg/kg to approximately 20 µg/kg, approximately 10 µg/kg to approximately 20 µg/kg, approximately 20 µg/kg to approximately 40 µg/kg, or approximately 25 µg/kg to 50 µg/kg of an IL-15/IL-15Ra complex to a subject.

In one embodiment, the methods described herein maintain plasma levels of IL-15 above basal levels for at least 18 hours, at least 20 hours, at least 22 hours, at least 24 hours, at least 28 hours, at least 30 hours, at least 32 hours, at least 34 hours, at least 36 hours, at least 38 hours, at least 40 hours, at least 42 hours, at least 44 hours, at least 46 hours, or at least 48 hours. In another embodiment, the methods described herein maintain plasma levels above basal levels for approximately 18 hours, approximately 20 hours, approximately 22 hours, approximately 24 hours, approximately 26 hours, approximately 28 hours, approximately 30 hours, approximately 32 hours, approximately 34 hours, approximately 36 hours, approximately 38 hours, approximately 40 hours, approximately 42 hours, approximately 44 hours, approximately 46 hours, or approximately 48 hours. In an embodiment, the methods described herein maintain plasma levels above basal levels for approximately 18 to 24 hours, approximately 22 to 24 hours, approximately 24 to 28 hours, approximately 24 to 30 hours, approximately 26 to 32 hours, approximately 28 to 32 hours, approximately 30 to 36 hours, approximately 32 to 38 hours, approximately 34 to 38 hours, approximately 36 to 42 hours, approximately 38 to 45 hours, approximately 38 hours, or approximately 40 to 48 hours. Techniques known to one skilled in the art can be utilized to measure IL-15 plasma levels, such as an ELISA.

In an embodiment, the methods described herein maintain plasma levels of 1 pg/ml to 10,000 mg/ml (in some embodiments, 5,000 pg/ml to 10,000 pg/ml, 1,000 pg/ml to 5,000 pg/ml, 1,000 pg/ml to 2,500 pg/ml, or 1,000 to 2,000 pg/ml, or 100 pg/ml to 1,000 pg/ml, 1 pg/ml to 1,000 pg/ml, or 100 pg/ml to 1,000 pg/ml) of IL-15 for at least 18 hours, at least 20 hours, at least 22 hours, at least 24 hours, at least 28 hours, at least 30 hours, at least 32 hours, at least 34 hours, at least 36 hours, at least 38 hours, at least 40 hours, at least 42 hours, at least 44 hours, at least 46 hours, or at least 48 hours. In another embodiment, the methods described herein maintain plasma levels of 10 pg/ml to 1,000 mg/ml 1 pg/ml to 10,000 mg/ml (in some embodiments, 5,000 pg/ml to 10,000 pg/ml, 1,000 pg/ml to 5,000 pg/ml, 1,000 pg/ml to 2,500 pg/ml, or 1,000 to 2,000 pg/ml, or 100 pg/ml to 1,000 pg/ml, 1 pg/ml to 1,000 pg/ml, or 100 pg/ml to 1,000 pg/ml) of IL-15 for approximately 18 hours, approximately 20 hours, approximately 22 hours, approximately 24 hours, approximately 26 hours, approximately 28 hours, approximately 30 hours, approximately 32 hours, approximately 34 hours, approximately 36 hours, approximately 38 hours, approximately 40 hours, approximately 42 hours, approximately 44 hours, approximately 46 hours, or approximately 48 hours. In another embodiment, the methods described herein maintain plasma levels 1 pg/ml to 10,000 mg/ml (in some embodiments, 5,000 pg/ml to 10,000 pg/ml, 1,000 pg/ml to 5,000 pg/ml, 1,000 pg/ml to 2,500 pg/ml, or 1,000 to 2,000 pg/ml, or 100 pg/ml to 1,000 pg/ml, 1 pg/ml to 1,000 pg/ml, or 100 pg/ml to 1,000 pg/ml) of IL-15 for approximately 18 to 24 hours, approximately 22 to 24 hours, approximately 24 to 28 hours, approximately 24 to 30 hours, approximately 26 to 32 hours, approximately 28 to 32 hours, approximately 30 to 36 hours, approximately 32 to 38 hours, approximately 34 to 38 hours, approximately 36 to 42 hours, approximately 38 to 45 hours, approximately 38 hours, or approximately 40 to 48 hours. Techniques known to one skilled in the art can be utilized to measure IL-15 plasma levels, such as an ELISA.

In another embodiment, the methods described herein maintain plasma levels of at least 10 pg/ml, at least 20 pg/ml, at least 30 pg/ml, at least 40 pg/ml, at least 50 pg/ml, at least 60 pg/ml, at least 70 pg/ml, at least 80 pg/ml, at least 90 pg/ml, at least 100 pg/ml, at least 200 pg/ml, at least 300 pg/ml, at least 400 pg/ml, or at least 500 pg/ml of IL-15 for at least 18 hours, at least 20 hours, at least 22 hours, at least 24 hours, at least 28 hours, at least 30 hours, at least 32 hours, at least 34 hours, at least 36 hours, at least 38 hours, at least 40 hours, at least 42 hours, at least 44 hours, at least 46 hours, or at least 48 hours. In another embodiment, the methods described herein maintain plasma levels of at least 600 pg/ml, at least 700 pg/ml, at least 800 pg/ml, at least 1,000 pg/ml, at least 1,200 pg/ml, at least 1,500 pg/ml, at least 1,750 pg/ml, at least 2,000 pg/ml, at least 5,000 pg/ml, at least 7,500 pg/ml, or at least 10,000 mg/ml of IL-15 for at least 18 hours, at least 20 hours, at least 22 hours, at least 24 hours, at least 28 hours, at least 30 hours, at least 32 hours, at least 34 hours, at least 36 hours, at least 38 hours, at least 40 hours, at least 42 hours, at least 44 hours, at least 46 hours, or at least 48 hours. In another embodiment, the methods described herein maintain plasma levels of at least 10 pg/ml, at least 20 pg/ml, at least 30 pg/ml, at least 40 pg/ml, at least 50 pg/ml, at least 60 pg/ml, at least 70 pg/ml, at least 80 pg/ml, at least 90 pg/ml, at least 100 pg/ml, at least 200 pg/ml, at least 300 pg/ml, at least 400 pg/ml, or at least 500 pg/ml of IL-15 for approximately 18 hours, approximately 20 hours, approximately 22 hours, approximately 24 hours, approximately 26 hours, approximately 28 hours, approximately 30 hours, approximately 32 hours, approximately 34 hours, approximately 36 hours, approximately 38 hours, approximately 40 hours, approximately 42 hours, approximately 44 hours, approximately 46 hours, or approximately 48 hours. In another embodiment, the methods described herein maintain plasma levels of at least 600 pg/ml, at least 700 pg/ml, at least 800 pg/ml, at least 1,000 pg/ml, at least 1,200 pg/ml, at least 1,500 pg/ml, at least 1,750 pg/ml, at least 2,000 pg/ml, at least 5,000 pg/ml, at least 7,500 pg/ml, or at least 10,000 mg/ml of IL-15 for approximately 18 hours, approximately 20 hours, approximately 22 hours, approximately 24 hours, approximately 26 hours, approximately 28 hours, approximately 30 hours, approximately 32 hours, approximately 34 hours, approximately 36 hours, approximately 38 hours, approximately 40 hours, approximately 42 hours, approximately 44 hours, approximately 46 hours, or approximately 48 hours. Techniques known to one skilled in the art can be utilized to measure IL-15 plasma levels, such as an ELISA.

In some embodiments, the Area Under the Curve for plasma IL-15 administered as a Therapeutic Agent in accordance with the methods described herein is 1 pg/ml to 10,000 mg/ml (in some embodiments, 5,000 pg/ml to 10,000 pg/ml, 1,000 pg/ml to 5,000 pg/ml, 1,000 pg/ml to 2,500 pg/ml, or 1,000 to 2,000 pg/ml, or 100 pg/ml to 1,000 pg/ml, 1 pg/ml to 1,000 pg/ml, or 100 pg/ml to 1,000 pg/ml).

In another aspect, provided herein is a method for enhancing IL-15-mediated immune function, comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 or 7 days. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long. In an embodiment, the dose of the first cycle and each subsequent cycle is 0.1 µg/kg to 1 µg/kg, 1 µg/kg to 5 µg/kg, or 5 µg/kg to 10 µg/kg. In another embodiment, the dose of the first cycle and each subsequent cycle is 0.1 µg/kg to 0.5 µg/kg, 1 µg/kg to 2 µg/kg, 1 µg/kg to 3 µg/kg, 2 µg/kg to 5 µg/kg, or 2 µg/kg to 4 µg/kg. In another embodiment, the dose of the first cycle and each subsequent cycle is 0.1 µg/kg, 0.25 µg/kg, 0.5 µg/kg, 1 µg/kg, 1.25 µg/kg, 1.5 µg/kg, 1.75 µg/kg, 2 µg/kg, 2.25 µg/kg, 2.5 µg/kg, 2.75 µg/kg, 3 µg/kg, 3.25 µg/kg, 3.5 µg/kg, 4 µg/kg, 4.25 µg/kg, 4.5 µg/kg, 4.75 µg/kg, or 5 µg/kg. In some embodiments, the dose used during the first cycle of the cyclical regimen differs from a dose used during a subsequent cycle of the cylical regimen. In some embodiments, the dose used within a cycle of the regimen varies. For example, the dose used within a cycle or in different cycles of the cyclical regimen may vary depending, e.g., upon the condition of the patient.

In one embodiment, provided herein is a method for enhancing IL-15-mediated immune function, comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject a certain number of times per week for a first period of time; and (b) no administration of IL-15/IL-15Ra complex for a second period of time. In some embodiments, the dose of the IL-15/IL-15Ra administered during the first cycle of the cyclical regimen is sequentially escalated. For example, if an IL-15/IL-15Ra complex is administered to a subject 3 times per week for two weeks, then the dose administered to the subject the second time during the first cycle of the cyclical regimen is increased relative to the dose administered the first time, the dose administered to the subject the third time during the first cycle of the cyclical regimen is increased relative to the dose administered the second time, the dose administered to the subject the fourth time is increased relative to the dose administered the third time, the dose administered to the subject the fifth time is increased relative the dose administered the fourth time, and the dose administered to the subject the sixth time is increased relative to the dose administered the fifth time. In some embodiments, the plasma levels of IL-15 and/or lymphocyte counts are monitored. In some embodiments, the subject is monitored for side effects such as a decrease in blood pressure and/or an increase in body temperature and/or an increase in cytokines in plasma. In some embodiments, the dose of the IL-15/IL-15Ra complex administered during the first cycle of the cyclical regimen is sequentially escalated if the subject does not have any side effects. In some embodiments, the dose of the IL-15/IL-15Ra complex administered during the first cycle of the cyclical regimen is sequentially escalated if the subject does not experience any adverse events, such as grade 3 or 4 lymphopenia, grade 3 granulocytopenia, grade 3 leukocytosis (WBC > 100,000/mm3), or organ dysfunction. In some embodiments, the IL-15/IL-15Ra is administered 1, 2, 3, 4, 5, 6 or 7 days per week. In some embodiments, the cyclical regimen is repeated 2, 3, 4, 5, 6, 7, 8, 9, 10 or more times. In some embodiments, the dose of IL-15/IL-15Ra administered to the subject during the second cycle and/or other subsequent cycles remains the same as the last dose administered to the subject during the first cycle. In other embodiments, the dose administered to the subject during the second cycle and/or other subsequent cycles is increased or decreased relative to the last dose administered to the subject during the first cycle. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long.

In another embodiment, provided herein is a method for enhancing IL-15-mediated immune function, comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject 3 times per week for a first period of time 2 weeks or more; and (b) no administration of IL-15/IL-15Ra complex for a second period of time, wherein the dose of the IL-15/IL-15Ra complex is sequentially increased each time the subject receives the complex during the first period. In some embodiments, the dose of the IL-15/IL-15Ra administered the dose administered to the subject during the first cycle of the cyclical regimen is 0.1 µg/kg to 5 µg/kg, the dose administered to the subject the second time during the first cycle of the cyclical regimen is 5 µg/kg to 15 µg/kg, the dose administered to the subject the third time during the first cycle of the cyclical regimen is 15 µg/kg to 25 µg/kg, the dose administered to the subject the fourth time during the first cycle of the cylical regimen is 25 µg/kg to 35 µg/kg, the dose administered to the subject the fifth time during the first cycle of the cyclical regimen is 35 µg/kg to 45 µg/kg, the dose administered to the subject the sixth time is 50 µg/kg or greater. In some embodiments, the plasma levels of IL-15 and/or lymphocyte counts are monitored. In some embodiments, the subject is monitored for side effects such as a decrease in blood pressure and/or an increase in body temperature and/or an increase in cytokines in plasma. In some embodiments, the dose of the IL-15/IL-15Ra complex administered during the first cycle of the cyclical regimen is sequentially escalated if the subject does not have any side effects. In some embodiments, the dose of the IL-15/IL-15Ra complex administered during the first cycle of the cyclical regimen is sequentially escalated if the subject does not experience any adverse events, such as grade 3 or 4 lymphopenia, grade 3 granulocytopenia, grade 3 leukocytosis (WBC > 100,000/mm3), or organ dysfunction. In some embodiments, the IL-15/IL-15Ra is administered 1, 2, 3, 4, 5, 6 or 7 days per week. In some embodiments, the cyclical regimen is repeated 2, 3, 4, 5, 6, 7, 8, 9, 10 or more times. In some embodiments, the dose of IL-15/IL-15Ra administered to the subject during the second cycle and/or other subsequent cycles remains the same as the last dose administered to the subject during the first cycle. In other embodiments, the dose administered to the subject during the second cycle and/or other subsequent cycle is increased or decreased relative to the last dose administered to the subject during the first cycle. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long.

In another aspect, provided herein are methods for enhancing IL-15-mediated immune function that involve a cyclical administration regimen comprising a first period of subcutaneous administration of an IL-15/IL-15Ra complex to a subject followed by a second period in which no IL-15/IL-15Ra complex is administered to the subject followed by a third period of subcutaneous administration of the IL-15/IL-15Ra complex to the subject. Whether this cyclical administration regimen is repeated and how many times it is repeated may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three times, four times, five times, six times, seven times, eight times, nine times, ten times or more. In some embodiments, this cyclical administration regimen is repeated 2 to 5 times, 5 to 8 times, 5 to 10 times, 8 to 10 times, 10 to 15 times, 10 to 20 times, 15 to 20 times, 20 to 30 times, or 25 to 30 times. In some embodiments, this cyclical administration regimen is repeated at least 2 times, at least 3 times, at least 4 times, at least 5 times, at least 6 times, at least 7 times, at least 8 times, at least 9 times, at least 10 times or more. In some embodiments, this cyclical administration regimen is repeated for a duration of time of at least one month, at least two months, at least three months, at least four months, at least five months, at least six months, at least seven months, at least eight months, at least nine months, at least ten months, at least eleven months, at least twelve months, at least 1.5 years, at least 2 years, at least 3 years, at least 4 years, at least 5 years, at least 6 years, at least 7 years, at least 8 years, at least 9 years, at least 10 years or more. In other some embodiments, this cyclical administration regimen is repeated for a duration of time of about one month, about two months, about three months, about four months, about five months, about six months, about seven months, about eight months, about nine months, about ten months, about eleven months, about twelve months, about 1.5 years, about 2 years, about 3 years, about 4 years, about 5 years, about 6 years, about 7 years, about 8 years, about 9 years, about 10 years or more. In some embodiments, this cyclical administration regimen is repeated for a duration of time of 1 to 3 months, 1 to 5 months, 2 to 5 months, 2 to 6 months, 3 to 6 months, 5 to 10 months, 6 to 10 months 6 to 12 months, 10 to 12 months, 1 year to 1.5 years, 1 to 2 years, 1 to 3 years, 2 to 4 years, 2 to 5 years, or 5 to 10 years. During the periods of administration of the IL-15/IL-15Ra complex to the subject, the complex can be administered every 1, 2, 3, 4, 5, 6, or 7 days. In some embodiments, the amount of the IL-15/IL-15Ra complex administered per dose during the first period and/or the third period is in the range between 0.1 to 10 µg/kg, 0.1 to 5 µg/kg, 0.1 to 2.5 µg/kg, 0.1 to 2 µg/kg, 0.1 to 1 µg/kg, or 0.1 to 0.5 µg/kg. In some embodiments, the amount of the IL-15/IL-15Ra complex administered per dose during the first period and/or the third period is in the range approximately 0.1 µg/kg, approximately 0.25 µg/kg, approximately 0.5 µg/kg, approximately 0.75 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg, approximately 9 µg/kg, or approximately 10 µg/kg. In some embodiments, the plasma levels of IL-15 and/or lymphocyte counts in the subject are monitored after each dose, after every other dose, or prior to administration of the IL-15/IL-15Ra complex in the third period.

In some embodiments, provided herein are methods for enhancing IL-15-mediated immune function that involve a cyclical administration regimen, wherein the cyclical administration comprises comprises a first period of 1 to 3 weeks (in some embodiments, 1 to 2 weeks, 2 to 3 weeks, 1 week, 12 days, 2 weeks, or 3 weeks)of subcutaneous administration of an IL-15/IL-15Ra complex to a subject followed by a second period of 1 week to 2 months (in some embodiments, 1 to 2 weeks, 2 to 3 weeks, 2 to 4 weeks, 3 to 4 weeks, 4 to 6 weeks, 4 to 8 weeks, 6 to 8 weeks, 1 week, 12 days, 2 weeks, 3 weeks, 4 weeks, 5 weeks, 6 weeks, 7 weeks or 8 weeks) in which no IL-15/IL-15Ra complex is administered to the subject followed by a third period of 1 week to 3 weeks (in some embodiments, 1 to 2 weeks, 2 to 3 weeks, 1 week, 12 days, 2 weeks, or 3 weeks) of subcutaneous administration of the IL-15/IL-15Ra complex to the subject. Whether this cyclical administration regimen is repeated and how many times it is repeated may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three times, four times, five times, six times, seven times, eight times, nine times, ten times or more. In some embodiments, this cyclical administration regimen is repeated 2 to 5 times, 5 to 8 times, 5 to 10 times, 8 to 10 times, 10 to 15 times, 10 to 20 times, 15 to 20 times, 20 to 30 times, or 25 to 30 times. In some embodiments, this cyclical administration regimen is repeated at least 2 times, at least 3 times, at least 4 times, at least 5 times, at least 6 times, at least 7 times, at least 8 times, at least 9 times, at least 10 times or more. In some embodiments, this cyclical administration regimen is repeated for a duration of time of at least one month, at least two months, at least three months, at least four months, at least five months, at least six months, at least seven months, at least eight months, at least nine months, at least ten months, at least eleven months, at least twelve months, at least 1.5 years, at least 2 years, at least 3 years, at least 4 years, at least 5 years, at least 6 years, at least 7 years, at least 8 years, at least 9 years, at least 10 years or more. In other embodiments, this cyclical administration regimen is repeated for a duration of time of about one month, about two months, about three months, about four months, about five months, about six months, about seven months, about eight months, about nine months, about ten months, about eleven months, about twelve months, about 1.5 years, about 2 years, about 3 years, about 4 years, about 5 years, about 6 years, about 7 years, about 8 years, about 9 years, about 10 years or more. In some embodiments, this cyclical administration regimen is repeated for a duration of time of 1 to 3 months, 1 to 5 months, 2 to 5 months, 2 to 6 months, 3 to 6 months, 5 to 10 months, 6 to 10 months 6 to 12 months, 10 to 12 months, 1 year to 1.5 years, 1 to 2 years, 1 to 3 years, 2 to 4 years, 2 to 5 years, or 5 to 10 years. In some embodiments, the plasma levels of IL-15 and/or lymphocyte counts in the subject are monitored after each dose, after every other dose, or prior to administration of the IL-15/IL-15Ra complex in the third period. During the periods of administration of the IL-15/IL-15Ra complex to the subject, the complex can be administered every 1, 2, 3, 4, 5, 6, or 7 days. In some embodiments, the amount of the IL-15/IL-15Ra complex administered per dose during the first period and/or the third period is in the range between 0.1 to 10 µg/kg, 0.1 to 5 µg/kg, 0.1 to 2.5 µg/kg , 0.1 to 2 µg/kg, 0.1 to 1 µg/kg, or 0.1 to 0.5 µg/kg. In some embodiments, the amount of the IL-15/IL-15Ra complex administered per dose during the first period and/or the third period is in the range approximately 0.1 µg/kg, approximately 0.25 µg/kg, approximately 0.5 µg/kg, approximately 0.75 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg, approximately 9 µg/kg, or approximately 10 µg/kg. In some embodiments, the plasma levels of IL-15 and/or lymphocyte counts in the subject are monitored after each dose, after every other dose, or prior to administration of the IL-15/IL-15Ra complex in the third period.

In some aspects, provided herein are methods for enhancing IL-15-mediated immune function that involve a cyclical administration regimen, wherein the cyclical administration regimen comprises a first two week period of subcutaneous administration of an IL-15/IL-15Ra complex to a subject followed by a second two week period in which no IL-15/IL-15Ra complex is administered to the subject followed by a third two week period of subcutaneous administration of the IL-15/IL-15Ra complex to the subject. Whether this cyclical administration regimen is repeated and how many times it is repeat may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three times or more. In some embodiments, this cyclical administration regimen is repeated one time, two times, three times, four times, five times, six times, seven times, eight times, nine times, ten times or more. In some embodiments, this cyclical administration regimen is repeated 2 to 5 times, 5 to 8 times, 5 to 10 times, 8 to 10 times, 10 to 15 times, 10 to 20 times, 15 to 20 times, 20 to 30 times, or 25 to 30 times. In some embodiments, this cyclical administration regimen is repeated at least 2 times, at least 3 times, at least 4 times, at least 5 times, at least 6 times, at least 7 times, at least 8 times, at least 9 times, at least 10 times or more. In some embodiments, this cyclical administration regimen is repeated for a duration of time of at least one month, at least two months, at least three months, at least four months, at least five months, at least six months, at least seven months, at least eight months, at least nine months, at least ten months, at least eleven months, at least twelve months, at least 1.5 years, at least 2 years, at least 3 years, at least 4 years, at least 5 years, at least 6 years, at least 7 years, at least 8 years, at least 9 years, at least 10 years or more. In other embodiments, this cyclical administration regimen is repeated for a duration of time of about one month, about two months, about three months, about four months, about five months, about six months, about seven months, about eight months, about nine months, about ten months, about eleven months, about twelve months, about 1.5 years, about 2 years, about 3 years, about 4 years, about 5 years, about 6 years, about 7 years, about 8 years, about 9 years, about 10 years or more. In some embodiments, this cyclical administration regimen is repeated for a duration of time of 1 to 3 months, 1 to 5 months, 2 to 5 months, 2 to 6 months, 3 to 6 months, 5 to 10 months, 6 to 10 months 6 to 12 months, 10 to 12 months, 1 year to 1.5 years, 1 to 2 years, 1 to 3 years, 2 to 4 years, 2 to 5 years, or 5 to 10 years. In some embodiments, the plasma levels of IL-15 and/or lymphocyte counts in the subject are monitored after each dose, after every other dose, or prior to administration of the IL-15/IL-15Ra complex in the third period. During the periods of administration of the IL-15/IL-15Ra complex to the subject, the complex can be administered every 1, 2, 3, 4, 5, 6, or 7 days. In some embodiments, the amount of the IL-15/IL-15Ra complex administered per dose during the first period and/or the third period is in the range between 0.1 to 10 µg/kg, 0.1 to 5 µg/kg, 0.1 to 2.5 µg/kg, 0.1 to 2 µg/kg, 0.1 to 1 µg/kg, or 0.1 to 0.5 µg/kg. In some embodiments, the amount of the IL-15/IL-15Ra complex administered per dose during the first period and/or the third period is in the range approximately 0.1 µg/kg, approximately 0.25 µg/kg, approximately 0.5 µg/kg, approximately 0.75 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg, approximately 9 µg/kg, or approximately 10 µg/kg. In some embodiments, the plasma levels of IL-15 and/or lymphocyte counts in the subject are monitored after each dose, after every other dose, or prior to administration of the IL-15/IL-15Ra complex in the third period.

In some embodiments, provided herein is a method for enhancing IL-15-mediated immune function, comprising: (a) administering subcutaneously to a subject a dose of approximately 0.1 µg/kg to approximately 10 µg/kg (in some embodiments, approximately 0.1 µg/kg to approximately 5 µg/kg, *e.g*., approximately 0.1 µg/kg, approximately 0.25 µg/kg, approximately 0.5 µg/kg, approximately 1 µg/kg, approximately 2 µg/kg, or approximately 5 µg/kg) of an IL-15/IL-15Ra complex every 1, 2 or 3 days over a first period of 1 week to 3 weeks; and (b) after a second period of 1 week to 2 months (or 8 weeks) in which no IL-15/IL-15Ra complex is administered, administering subcutaneously to the subject a dose of approximately 0.1 µg/kg to approximately 10 µg/kg (in some embodiments, approximately 0.1 µg/kg to approximately 5 µg/kg, *e.g*., approximately 0.1 µg/kg, approximately 0.25 µg/kg, approximately 0.5 µg/kg, approximately 1 µg/kg, approximately 2 µg/kg, or approximately 5 µg/kg) of an IL-15/IL-15Ra complex every 1, 2 or 3 days over a third period of 1 week to 3 weeks. In some embodiments, steps (a) through (b) are repeated two, three, four, five, six, seven, eight, nine, ten or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In a specific embodiment, the subject is a human.

In one embodiment, provided herein is a method for enhancing IL-15-mediated immune function, comprising: (a) administering subcutaneously to a subject a dose of approximately 0.1 µg/kg to approximately 10 µg/kg (in some embodiments, approximately 0.1 µg/kg to approximately 5 µg/kg, *e.g*., approximately 0.1 µg/kg, approximately 0.25 µg/kg, approximately 0.5 µg/kg, approximately 1 µg/kg, approximately 2 µg/kg, or approximately 5 µg/kg) of an IL-15/IL-15Ra complex every 1, 2 or 3 days over a first period of 12 to 14 days; and (b) after a second period of 12 to 14 days in which no IL-15/IL-15Ra complex is administered, administering subcutaneously to the subject a dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a third period of 12 to 14 days. In some embodiments, steps (a) through (b) are repeated two, three, four or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In a specific embodiment, the subject is a human.

In another particular embodiment, provided herein is a method for enhancing IL-15-mediated immune function, comprising: (a) administering subcutaneously to a subject a dose of approximately 0.1 µg/kg, approximately 0.5 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg or approximately 10 µg/kg of an IL-15/IL-15Ra complex every 1, 2 or 3 days over a first period of 12 to 14 days; and (b) after a second period of 12 to 14 days in which no IL-15/IL-15Ra complex is administered, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg or approximately 10 µg/kg of an IL-15/IL-15Ra complex every 1, 2 or 3 days over a third period of 12 to 14 days. In some embodiments, steps (a) through (c) are repeated two, three, four or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In a specific embodiment, the subject is a human.

In another aspect, provided herein is a method for preventing, treating and/or managing a disorder in which enhancing IL-15-mediated immune function is beneficial (e.g., cancer, an infectious disease, an immunodeficiency or lymphopenia), comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 or 7 days. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long. In an embodiment, the dose of the first cycle and each subsequent cycle is 0.1 µg/kg to 1 µg/kg, 1 µg/kg to 5 µg/kg, or 5 µg/kg to 10 µg/kg. In another embodiment, the dose of the first cycle and each subsequent cycle is 0.1 µg/kg to 0.5 µg/kg, 1 µg/kg to 2 µg/kg, 1 µg/kg to 3 µg/kg, 2 µg/kg to 5 µg/kg, or 2 µg/kg to 4 µg/kg. In another embodiment, the dose of the first cycle and each subsequent cycle is 0.1 µg/kg, 0.25 µg/kg, 0.5 µg/kg, 1 µg/kg, 1.25 µg/kg, 1.5 µg/kg, 1.75 µg/kg, 2 µg/kg, 2.25 µg/kg, 2.5 µg/kg, 2.75 µg/kg, 3 µg/kg, 3.25 µg/kg, 3.5 µg/kg, 4 µg/kg, 4.25 µg/kg, 4.5 µg/kg, 4.75 µg/kg, or 5 µg/kg. In some embodiments, the dose used during the first cycle of the cyclical regimen differs from a dose used during a subsequent cycle of the cylical regimen. In some embodiments, the dose used within a cycle of the regimen varies. For example, the dose used within a cycle or in different cycles of the cyclical regimen may vary depending, *e.g*., upon the condition of the patient

In one embodiment, provided herein is a method for preventing, treating and/or managing a disorder in which enhancing IL-15-mediated immune function is beneficial (e.g., cancer, an infectious disease, an immunodeficiency or lymphopenia), comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject a certain number of times per week for a first period of time; and (b) no administration of IL-15/IL-15Ra complex for a second period of time. In some embodiments, the dose of the IL-15/IL-15Ra administered during the first cycle of the cyclical regimen is sequentially escalated. For example, if an IL-15/IL-15Ra complex is administered to a subject 3 times per week for two weeks, then the dose administered to the subject the second time during the first cycle of the cyclical regimen is increased relative to the dose administered the first time, the dose administered to the subject the third time during the first cycle of the cyclical regimen is increased relative to the dose administered the second time, the dose administered to the subject the fourth time is increased relative to the dose administered the third time, the dose administered to the subject the fifth time is increased relative the dose administered the fourth time, and the dose administered to the subject the sixth time is increased relative to the dose administered the fifth time. In some embodiments, the plasma levels of IL-15 and/or lymphocyte counts are monitored. In some embodiments, the subject is monitored for side effects such as a decrease in blood pressure and/or an increase in body temperature and/or an increase in cytokines in plasma. In some embodiments, the dose of the IL-15/IL-15Ra complex administered during the first cycle of the cyclical regimen is sequentially escalated if the subject does not have any side effects. In some embodiments, the dose of the IL-15/IL-15Ra complex administered during the first cycle of the cyclical regimen is sequentially escalated if the subject does not experience any adverse events, such as grade 3 or 4 lymphopenia, grade 3 granulocytopenia, grade 3 leukocytosis (WBC > 100,000/mm3), or organ dysfunction. In some embodiments, the IL-15/IL-15Ra is administered 1, 2, 3, 4, 5, 6 or 7 days per week. In some embodiments, the cyclical regimen is repeated 2, 3, 4, 5, 6, 7, 8, 9, 10 or more times. In some embodiments, the dose of IL-15/IL-15Ra administered to the subject during the second cycle and/or other subsequent cycles remains the same as the last dose administered to the subject during the first cycle. In other embodiments, the dose administered to the subject during the second cycle and/or other subsequent cycles is increased or decreased relative to the last dose administered to the subject during the first cycle. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long.

In another embodiment, provided herein is a method for preventing, treating and/or managing a disorder in which enhancing IL-15-mediated immune function is beneficial (e.g., cancer, an infectious disease, an immunodeficiency or lymphopenia), comprising subcutaneously administering to a subject an IL-15/IL-15Ra complex in a cyclical regimen, wherein each cycle of the cyclical regimen comprises: (a) subcutaneously administering a dose of the IL-15/IL-15Ra complex to the subject 3 times per week for a first period of time 2 weeks or more; and (b) no administration of IL-15/IL-15Ra complex for a second period of time, wherein the dose of the IL-15/IL-15Ra complex is sequentially increased each time the subject receives the complex during the first period. In some embodiments, the dose of the IL-15/IL-15Ra administered the dose administered to the subject during the first cycle of the cyclical regimen is 0.1 µg/kg to 5 µg/kg, the dose administered to the subject the second time during the first cycle of the cyclical regimen is 5 µg/kg to 15 µg/kg, the dose administered to the subject the third time during the first cycle of the cyclical regimen is 15 µg/kg to 25 µg/kg, the dose administered to the subject the fourth time during the first cycle of the cylical regimen is 25 µg/kg to 35 µg/kg, the dose administered to the subject the fifth time during the first cycle of the cyclical regimen is 35 µg/kg to 45 µg/kg, the dose administered to the subject the sixth time is 50 µg/kg or greater. In some embodiments, the plasma levels of IL-15 and/or lymphocyte counts are monitored. In some embodiments, the subject is monitored for side effects such as a decrease in blood pressure and/or an increase in body temperature and/or an increase in cytokines in plasma. In some embodiments, the dose of the IL-15/IL-15Ra complex administered during the first cycle of the cyclical regimen is sequentially escalated if the subject does not have any side effects. In some embodiments, the dose of the IL-15/IL-15Ra complex administered during the first cycle of the cyclical regimen is sequentially escalated if the subject does not experience any adverse events, such as grade 3 or 4 lymphopenia, grade 3 granulocytopenia, grade 3 leukocytosis (WBC > 100,000/mm3), or organ dysfunction. In some embodiments, the IL-15/IL-15Ra is administered 1, 2, 3, 4, 5, 6 or 7 days per week. In some embodiments, the cyclical regimen is repeated 2, 3, 4, 5, 6, 7, 8, 9, 10 or more times. In some embodiments, the dose of IL-15/IL-15Ra administered to the subject during the second cycle and/or other subsequent cycles remains the same as the last dose administered to the subject during the first cycle. In other embodiments, the dose administered to the subject during the second cycle and/or other subsequent cycles is increased or decreased relative to the last dose administered to the subject during the first cycle. In some embodiments, the first and second periods of time are the same. In other embodiments, the first and second periods of time are different. In some embodiments, the first period for administration of the IL-15/IL-15Ra complex is 1 week to 4 weeks long, 2 to 4 weeks, 2 to 3 weeks, or 1 to 2 weeks. In other embodiments, the first period for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks, 1 to 2 weeks, 3 weeks, 2 weeks or 1 week long. In one embodiments, the disorder is an infectious disease. In certain embodiments, the disorder is an infectious disease caused by HIV.

In another aspect, provided herein are methods for preventing, treating and/or managing a disorder in which enhancing IL-15-mediated immune function is beneficial (e.g., cancer, lymphopenia, immunodeficiencies, infectious diseases, or wounds), wherein the methods involve a cyclical administration regimen comprising a first period of (in some embodiments, 1 to 2 weeks, 2 to 3 weeks, 1 week, 12 days, 2 weeks, or 3 weeks)of subcutaneous administration of an IL-15/IL-15Ra complex to a subject followed by a second period of 1 week to 2 months (in some embodiments, 1 to 2 weeks, 2 to 3 weeks, 2 to 4 weeks, 3 to 4 weeks, 4 to 6 weeks, 4 to 8 weeks, 6 to 8 weeks, 1 week, 12 days, 2 weeks, 3 weeks, 4 weeks, 5 weeks, 6 weeks, 7 weeks or 8 weeks) in which no IL-15/IL-15Ra complex is administered to the subject followed by a third period of 1 week to 3 weeks (in some embodiments, 1 to 2 weeks, 2 to 3 weeks, 1 week, 12 days, 2 weeks, or 3 weeks) of subcutaneous administration of the IL-15/IL-15Ra complex to the subject. Whether this cyclical administration regimen is repeated and how many times it is repeated may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three times, four times, five times, six times, seven times, eight times, nine times, ten times or more. In some embodiments, this cyclical administration regimen is repeated 2 to 5 times, 5 to 8 times, 5 to 10 times, 8 to 10 times, 10 to 15 times, 10 to 20 times, 15 to 20 times, 20 to 30 times, or 25 to 30 times. In some embodiments, this cyclical administration regimen is repeated at least 2 times, at least 3 times, at least 4 times, at least 5 times, at least 6 times, at least 7 times, at least 8 times, at least 9 times, at least 10 times or more. In some embodiments, this cyclical administration regimen is repeated for a duration of time of at least one month, at least two months, at least three months, at least four months, at least five months, at least six months, at least seven months, at least eight months, at least nine months, at least ten months, at least eleven months, at least twelve months, at least 1.5 years, at least 2 years, at least 3 years, at least 4 years, at least 5 years, at least 6 years, at least 7 years, at least 8 years, at least 9 years, at least 10 years or more. In other embodiments, this cyclical administration regimen is repeated for a duration of time of about one month, about two months, about three months, about four months, about five months, about six months, about seven months, about eight months, about nine months, about ten months, about eleven months, about twelve months, about 1.5 years, about 2 years, about 3 years, about 4 years, about 5 years, about 6 years, about 7 years, about 8 years, about 9 years, about 10 years or more. In some embodiments, this cyclical administration regimen is repeated for a duration of time of 1 to 3 months, 1 to 5 months, 2 to 5 months, 2 to 6 months, 3 to 6 months, 5 to 10 months, 6 to 10 months 6 to 12 months, 10 to 12 months, 1 year to 1.5 years, 1 to 2 years, 1 to 3 years, 2 to 4 years, 2 to 5 years, or 5 to 10 years. In some embodiments, the plasma levels of IL-15 and/or lymphocyte counts in the subject are monitored after each dose, after every other dose, or prior to administration of the IL-15/IL-15Ra complex in the third period. During the periods of administration of the IL-15/IL-15Ra complex to the subject, the complex can be administered every 1, 2, 3, 4, 5, 6, or 7 days. In some embodiments, the amount of the IL-15/IL-15Ra complex administered per dose during the first period and/or the third period is in the range between 0.1 to 10 µg/kg, 0.1 to 5 µg/kg, 0.1 to 2.5 µg/kg, 0.1 to 2 µg/kg, 0.1 to 1 µg/kg, or 0.1 to 0.5 µg/kg. In some embodiments, the amount of the IL-15/IL-15Ra complex administered per dose during the first period and/or the third period is in the range approximately 0.1 µg/kg, approximately 0.25 µg/kg, approximately 0.5 µg/kg, approximately 0.75 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg, approximately 9 µg/kg, or approximately 10 µg/kg. In some embodiments, the plasma levels of IL-15 and/or lymphocyte counts in the subject are monitored after each dose, after every other dose, or prior to administration of the IL-15/IL-15Ra complex in the third period.

In one embodiment, provided herein are methods for preventing, treating and/or managing a disorder in which enhancing IL-15-mediated immune function is beneficial (*e.g*., cancer, lymphopenia, immunodeficiencies, infectious diseases, or wounds), wherein the methods involve a cyclical administration regimen comprising a first two week period of subcutaneous administration of an IL-15/IL-15Ra complex to a subject followed by a second two week period in which no IL-15/IL-15Ra complex is administered to the subject followed by a third two week period of subcutaneous administration of the IL-15/IL-15Ra complex to the subject. Whether this cyclical administration regimen is repeated and how many times it is repeated may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three times, four times, five times, six times, seven times, eight times, nine times, ten times or more. In some embodiments, this cyclical administration regimen is repeated 2 to 5 times, 5 to 8 times, 5 to 10 times, 8 to 10 times, 10 to 15 times, 10 to 20 times, 15 to 20 times, 20 to 30 times, or 25 to 30 times. In some embodiments, this cyclical administration regimen is repeated at least 2 times, at least 3 times, at least 4 times, at least 5 times, at least 6 times, at least 7 times, at least 8 times, at least 9 times, at least 10 times or more. In some embodiments, this cyclical administration regimen is repeated for a duration of time of at least one month, at least two months, at least three months, at least four months, at least five months, at least six months, at least seven months, at least eight months, at least nine months, at least ten months, at least eleven months, at least twelve months, at least 1.5 years, at least 2 years, at least 3 years, at least 4 years, at least 5 years, at least 6 years, at least 7 years, at least 8 years, at least 9 years, at least 10 years or more. In other embodiments, this cyclical administration regimen is repeated for a duration of time of about one month, about two months, about three months, about four months, about five months, about six months, about seven months, about eight months, about nine months, about ten months, about eleven months, about twelve months, about 1.5 years, about 2 years, about 3 years, about 4 years, about 5 years, about 6 years, about 7 years, about 8 years, about 9 years, about 10 years or more. In some embodiments, this cyclical administration regimen is repeated for a duration of time of 1 to 3 months, 1 to 5 months, 2 to 5 months, 2 to 6 months, 3 to 6 months, 5 to 10 months, 6 to 10 months 6 to 12 months, 10 to 12 months, 1 year to 1.5 years, 1 to 2 years, 1 to 3 years, 2 to 4 years, 2 to 5 years, or 5 to 10 years. In some embodiments, the plasma levels of IL-15 and/or lymphocyte counts in the subject are monitored after each dose, after every other dose, or prior to administration of the IL-15/IL-15Ra complex in the third period. During the periods of administration of the IL-15/IL-15Ra complex to the subject, the complex can be administered every 1, 2, 3, 4, 5, 6, or 7 days. In some embodiments, the amount of the IL-15/IL-15Ra complex administered per dose during the first period and/or the third period is in the range between 0.1 to 10 µg/kg, 0.1 to 5 µg/kg, 0.1 to 2.5 µg/kg , 0.1 to 2 µg/kg, 0.1 to 1 µg/kg, or 0.1 to 0.5 µg/kg. In some embodiments, the amount of the IL-15/IL-15Ra complex administered per dose during the first period and/or the third period is in the range approximately 0.1 µg/kg, approximately 0.25 µg/kg, approximately 0.5 µg/kg, approximately 0.75 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg, approximately 9 µg/kg, or approximately 10 µg/kg. In some embodiments, the plasma levels of IL-15 and/or lymphocyte counts in the subject are monitored after each dose, after every other dose, or prior to administration of the IL-15/IL-15Ra complex in the third period.

In some embodiments, provided herein is a method for preventing, treating and/or managing a disorder in which enhancing IL-15-mediated immune function is beneficial (*e.g*., cancer, lymphopenia, immunodeficiencies, infectious diseases, or wounds), wherein the method comprises: (a) administering subcutaneously to a subject a dose of approximately 0.1 µg/kg to approximately 10 µg/kg (in some embodiments, approximately 0.1 µg/kg to approximately 5 µg/kg, e.g., approximately 0.1 µg/kg, approximately 0.25 µg/kg, approximately 0.5 µg/kg, approximately 1 µg/kg, approximately 2 µg/kg, or approximately 5 µg/kg) of an IL-15/IL-15Ra complex every 1, 2 or 3 days over a first period of 1 week to 3 weeks; and (b) after a second period of 1 week to 2 months (or 8 weeks) in which no IL-15/IL-15Ra complex is administered to the subject, administering subcutaneously to the subject a dose of approximately 0.1 µg/kg to approximately 10 µg/kg (in some embodiments, approximately 0.1 µg/kg to approximately 5 µg/kg, e.g., approximately 0.1 µg/kg, approximately 0.25 µg/kg, approximately 0.5 µg/kg, approximately 1 µg/kg, approximately 2 µg/kg, or approximately 5 µg/kg) of an IL-15/IL-15Ra complex every 1, 2 or 3 days over a third period of 1 week to 3 weeks. In some embodiments, steps (a) through (b) are repeated two, three, four, five, six, seven, eight, nine, ten or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In a specific embodiment, the subject is a human.

In one embodiment, provided herein is a method for preventing, treating and/or managing a disorder in which enhancing IL-15-mediated immune function is beneficial (*e.g*., cancer, lymphopenia, immunodeficiencies, infectious diseases, or wounds), wherein the method comprises: (a) administering subcutaneously to a subject a dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a third period of 12 to 14 days. In some embodiments, steps (a) through (c) are repeated two, three, four or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In a specific embodiment, the subject is a human.

In another embodiment, provided herein is a method for preventing, treating and/or managing a disorder in which enhancing IL-15-mediated immune function is beneficial (*e.g*., cancer, lymphopenia, immunodeficiencies, infectious diseases, or wounds), wherein the method comprises: (a) administering subcutaneously to a subject a dose of approximately 0.1 µg/kg, approximately 0.5 µg/kg, approximately 1 µg/kg, approximately 2 µg/kg, approximately 3 µg/kg, approximately 4 µg/kg approximately 5 µg/kg, approximately 6 µg/kg, approximately 7 µg/kg, approximately 8 µg/kg or approximately 10 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg approximately 5 µg/kg, approximately 6 µg/kg, approximately 7 µg/kg, approximately 8 µg/kg or approximately 10 µg/kg of an IL-15/IL-15Ra complex every 1, 2 or 3 days over a third period of 12 to 14 days. In some embodiments, steps (a) through (c) are repeated two, three, four or more times. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose, or prior to administration of the IL-15/IL-15Ra complex in the third period. In a specific embodiment, the subject is a human.

In another embodiment, provided herein is a method for treating or managing cancer in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 1 week to 3 weeks; and (b) after a second period of 1 week to 2 months (or 8 weeks) in which no IL-15/IL-15Ra complex is administered to the subject, administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 1 week to 3 weeks. In a specific embodiment, the cancer is melanoma, renal cell carcinoma, lung cancer (*e.g*., non-small cell lung cancer) or colon cancer. In certain embodiments, the cancer is metastatic. In a specific embodiment, the cancer is metastatic melanoma, metastatic renal cell carcinoma, metastatic lung cancer (*e.g.,* metastatic non-small cell lung cancer) or metastatic colon cancer.

In a specific embodiment, provided herein is a method for treating or managing cancer in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In a specific embodiment, the cancer is melanoma, renal cell carcinoma, lung cancer (*e.g.,* non-small cell lung cancer) or colon cancer. In certain embodiments, the cancer is metastatic. In a specific embodiment, the cancer is metastatic melanoma, metastatic renal cell carcinoma, metastatic lung cancer (*e.g*., metastatic non-small cell lung cancer) or metastatic colon cancer.

In another embodiment, provided herein is a method for preventing, treating and/or managing an infectious disease in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 1 week to 3 weeks; and (b) after a second period of 1 week to 2 months (or 8 weeks) in which no IL-15/IL-15Ra complex is administered to the subject, administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 1 week to 3 weeks. In some embodiments, the infectious disease is caused by a viral, bacterial, fungal or parasite infection.

In an embodiment, provided herein is a method for preventing, treating and/or managing an infectious disease in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In some embodiments, the infectious disease is caused by a viral, bacterial, fungal or parasite infection.

In another embodiment, provided herein is a method for preventing, treating and/or managing an immunodeficiency in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 1 week to 3 weeks; and (b) after a second period of 1 week to 2 months (or 8 weeks) in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 1 week to 3 weeks. In some embodiments, the immunodeficiency is caused by AIDS or a disorder (e.g., a genetic disorder).

In an embodiment, provided herein is a method for preventing, treating and/or managing an immunodeficiency in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In some embodiments, the immunodeficiency is caused by AIDS or a disorder (e.g., a genetic disorder).

In another embodiment, provided herein is a method for preventing, treating and/or managing lymphopenia in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 1 week to 3 weeks; and (b) after a second period of 1 week to 2 months (or 8 weeks) in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 1 week to 3 weeks. In some embodiments, the lymphopenia is caused by a therapy (*e.g*., chemotherapy, an antiviral agent, or an immunosuppressive agent), or a disease that causes depletion of peripheral circulating lymphocytes).

In an embodiment, provided herein is a method for preventing, treating and/or managing lymphopenia in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; and (b) after a second period of 12 to 14 days in which no IL-15/IL-15Ra complex is administered to the subject, administering subcutaneously to the subject a dose of approximately 0.1 µg/kg, approximately 0.5 µg/kg, approximately 1 µg/kg, approximately 2 µg/kg, approximately 3 µg/kg, approximately 4 µg/kg, or approximately 5 µg/kg of the IL-15/IL-15Ra complex every 1, 2 or 3 days over a third period of 12 to 14 days. In some embodiments, the lymphopenia is caused by a therapy (e.g., chemotherapy, an antiviral agent, or an immunosuppressive agent), or a disease that causes depletion of peripheral circulating lymphocytes.

In another aspect, provided herein are methods for enhancing IL-15-mediated immune function, comprising: (a) administering subcutaneously to a subject a dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a first period of 1 week to 3 weeks; (b) assessing the plasma levels of IL-15 and/or lymphocyte count in the subject following a second period of 1 week to 2 months (or 8 weeks) in which no IL-15/IL-15Ra complex is administered to the subject; and (c) administering subcutaneously to the subject a dose of the IL-15/IL-15Ra complex every 1, 2, or 3 days over a third period of 1 week to 3 weeks. In some embodiments, the dose of the IL-15/IL-15Ra complex recited in step (c) is greater than the dose administered of the IL-15/IL-15Ra complex recited in step (c). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In a specific embodiment, the subject is a human.

In one embodiment, provided herein are methods for enhancing IL-15-mediated immune function, comprising: (a) administering subcutaneously to a subject a dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (c). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In a specific embodiment, the subject is a human.

In one embodiment, provided herein is a method for enhancing IL-15-mediated immune function, comprising: (a) administering subcutaneously to a subject a first dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a second dose of the IL-15/IL-15Ra complex every 1, 2, or 3 days over a third period of 12 to 14 days, wherein the second dose maintains plasma levels of IL-15 consistent with that achieved by the first dose administered during the first period. In some embodiments, the second dose of the IL-15/IL-15Ra complex recited in step (c) is greater than the first dose administered of the IL-15/IL-15Ra complex recited in step (a). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In a specific embodiment, the subject is a human.

In another embodiment, provided herein is a method for enhancing IL-15-mediated immune function, comprising: (a) administering subcutaneously to a subject a first dose of approximately 0.1 µg/kg, approximately 0.5 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg or approximately 10 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a second dose of the IL-15/IL-15Ra complex every 1, 2, or 3 days over a third period of 12 to 14 days, wherein the second dose maintains plasma levels of IL-15 consistent with that achieved by the first dose administered during the first period. In some embodiments, the second dose of the IL-15/IL-15Ra complex recited in step (c) is greater than the first dose administered of the IL-15/IL-15Ra complex recited in step (a). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In a specific embodiment, the subject is a human.

In another aspect, provided herein are methods for preventing, treating and/or managing a disorder in which enhancing IL-15-mediated immune function is beneficial (*e.g*., cancer, lymphopenia, immunodeficiencies, infectious diseases, or wounds), comprising: (a) administering subcutaneously to a subject a dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a first period of 1 week to 3 weeks; (b) assessing the plasma levels of IL-15 and/or lymphocyte count in the subject following a second period of 1 week to 2 months (or 8 weeks) in which no IL-15/IL-15Ra complex is administered to the subject; and (c) administering subcutaneously to the subject a dose of the IL-15/IL-15Ra complex every 1, 2, or 3 days over a third period of 1 week to 3 weeks. In some embodiments, the dose of the IL-15/IL-15Ra complex recited in step (c) is greater than the dose administered of the IL-15/IL-15Ra complex recited in step (a). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, or after every other dose. In some embodiments, the subject is monitored for side effects such as a decrease in blood pressure and/or an increase in body temperature and/or an increase in cytokines in plasma. In a specific embodiment, the subject is a human. In particular embodiment, the method for preventing, treating and/or managing a disorder in which enhancing IL-15-mediated immune function is beneficial is a method for treating or managing cancer. In a specific embodiment, the cancer is melanoma, renal cell carcinoma, lung cancer (e.g., non-small cell lung cancer) or colon cancer. In another particular embodiment, the method for preventing, treating and/or managing a disorder in which enhancing IL-15-mediated immune function is beneficial is a method for preventing, treating and/or managing an infectious disease. In another particular embodiment, the method for preventing, treating and/or managing a disorder in which enhancing IL-15-mediated immune function is beneficial is a method for preventing, treating and/or managing an immunodeficiency. In another particular embodiment, the method for preventing, treating and/or managing a disorder in which enhancing IL-15-mediated immune function is beneficial is a method for preventing, treating and/or managing lymphopenia.

In some embodiments, provided herein are methods for preventing, treating and/or managing a disorder in which enhancing IL-15-mediated immune function is beneficial (*e.g*., cancer, lymphopenia, immunodeficiencies, infectious diseases, or wounds), wherein the methods comprise: (a) administering subcutaneously to a subject a dose of approximately 0.1 µg/kg to approximately 10 µg/kg (in some embodiment, approximately 0.1 µg/kg to approximately 5 µg/kg) of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (c). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose, after every other dose. In a specific embodiment, the subject is a human.

In one embodiment, provided herein is a method for preventing, treating and/or managing a disorder in which enhancing IL-15-mediated immune function is beneficial (*e.g*., cancer, lymphopenia, immunodeficiencies, infectious diseases, or wounds), wherein the method comprises: (a) administering subcutaneously to a subject a first dose of approximately 0.1 µg/kg to approximately 10 µg/kg (in some embodiments, approximately 0.1 µg/kg to approximately 5 µg/kg) of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a second dose of the IL-15/IL-15Ra complex every 1, 2, or 3 days over a third period of 12 to 14 days, wherein the second dose maintains plasma levels of IL-15 .consistent with that achieved by the first dose administered during the first period. In some embodiments, the second dose of the IL-15/IL-15Ra complex recited in step (c) is greater than the first dose administered of the IL-15/IL-15Ra complex recited in step (a). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In a specific embodiment, the subject is a human.

In another embodiment, provided herein is a method for preventing, treating and/or managing a disorder in which enhancing IL-15-mediated immune function is beneficial (*e.g*., cancer, lymphopenia, immunodeficiencies, infectious diseases, or wounds), wherein the method comprises: (a) administering subcutaneously to a subject a first dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, approximately 5 µg/kg, approximately 6 µg/kg, approximately 7 µg/kg, approximately 8 µg/kg or approximately 10 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a second dose of the IL-15/IL-15Ra complex every 1, 2, or 3 days over a third period of 12 to 14 days, wherein the second dose maintains plasma levels of IL-15 .consistent with that achieved by the first dose administered during the first period. In some embodiments, the second dose of the IL-15/IL-15Ra complex recited in step (c) is greater than the first dose administered of the IL-15/IL-15Ra complex recited in step (a). In some embodiments, the second dose is 0.01 µg/kg, 0.02 µg/kg, 0.03 µg/kg, 0.05 µg/kg, 0.075 µg/kg, 0.1 µg/kg, 0.25 µg/kg, 0.5 µg/kg, 0.75 µg/kg, 1 µg/kg, 1.25 µg/kg, 1.5 µg/kg, or 1.75 µg/kg higher than the first dose. In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In a specific embodiment, the subject is a human.

In another embodiment, provided herein is a method for treating or managing cancer in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In a specific embodiment, the cancer is melanoma, renal cell carcinoma, lung cancer (*e.g*., non-small cell lung cancer) or colon cancer. In certain embodiments, the cancer is metastatic. In specific embodiments, the cancer is metastatic melanoma, metastatic renal cell carcinoma, metastatic lung cancer (*e.g.*, metastatic non-small cell lung cancer), or metastatic colon cancer.

In another embodiment, provided herein is a method for preventing, treating and/or managing an infectious disease in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In some embodiments, the infectious disease is caused by a viral, bacterial, fungal or parasite infection.

In another embodiment, provided herein is a method for preventing, treating and/or managing an immunodeficiency in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In some embodiments, the immunodeficiency is caused by AIDS or a disorder (e.g., a genetic disorder).

In another embodiment, provided herein is a method for preventing, treating and/or managing lymphopenia in a human subject comprising: (a) administering subcutaneously to the subject a dose of approximately 0.1 µg/kg, approximately 0.25 µg/kg, approximately 0.5 µg/kg, approximately 1 µg/kg, approximately 2 µg/kg, approximately 3 µg/kg, approximately 4 µg/kg, or approximately 5 µg/kg of an IL-15/IL-15Ra complex every 1, 2 or 3 days over a first period of 12 to 14 days; (b) assessing the plasma levels of IL-15 and/or lymphocyte count in the subject following a second period of 12 to 14 days in which no IL-15/IL-15Ra complex is administered to the subject; and (c) administering subcutaneously to the subject a dose of the IL-15/IL-15Ra complex every 1, 2, or 3 days over a third period of 12 to 14 days. In some embodiments, the dose of the IL-15/IL-15Ra complex recited in step (c) is greater than the dose administered of the IL-15/IL-15Ra complex recited in step (a). In some embodiments, the plasma levels of IL-15 and/or lymphocyte counts in the subject are monitored prior to the first dose of an IL-15/IL-15Ra complex. In some embodiments, the plasma levels of IL-15 and/or lymphocyte counts in the subject are monitored after each dose or after every other dose. In some embodiments, the subject is monitored for side effects such as a decrease in blood pressure and/or an increase in body temperature and/or an increase in cytokines in plasma. In some embodiments, the lymphopenia is caused by a therapy (*e.g*., chemotherapy, an antiviral agent, an immunosuppressive agent), or a disease that causes depletion of peripheral circulating lymphocytes.

In some embodiments of the methods described herein, an IL-15/IL-15Ra complex is administered subcutaneously every 1, 2 or 3 days in the amount of approximately 0.1 µg/kg, approximately 0.2 µg/kg, approximately 0.25 µg/kg, approximately 0.3 µg/kg, approximately 0.4 µg/kg, approximately 0.5 µg/kg, approximately 0.6 µg/kg, approximately 0.7 µg/kg, approximately 0.8 µg/kg, approximately 0.9 µg/kg, approximately 1 µg/kg, approximately 1.25 µg/kg, approximately 1.5 µg/kg, approximately 1.75 µg/kg, approximately 2 µg/kg, approximately 2.25 µg/kg, approximately 2.5 µg/kg, approximately 2.75 µg/kg, approximately 3 µg/kg, approximately 3.25 µg/kg, approximately 3.5 µg/kg, approximately 3.75 µg/kg, approximately 4 µg/kg, approximately 4.25µg/kg, approximately 4.5 µg/kg, approximately 4.75 µg/kg, approximately 5 µg/kg, approximately 5.5 µg/kg, approximately 6 µg/kg, approximately 6.5 µg/kg, approximately 7 µg/kg, approximately 7.5 µg/kg, approximately 8 µg/kg, approximately 8.5 µg/kg, approximately 9 µg/kg, approximately 9.5 µg/kg, or approximately 10 µg/kg. In one embodiment, described herein are methods comprising subcutaneous administration of an IL-15/IL-15Ra complex every 1, 2 or 3 days in the amount of approximately 0.1 µg/kg to 5 µg/kg, 0.1 µg/kg to 4 µg/kg, 0.1 µg/kg to 3 µg/kg, 0.1 µg/kg to 2.5 µg/kg, 0.1 µg/kg to 2 µg/kg, 0.1 µg/kg to 1 µg/kg, 0.1 µg/kg to 0.5 µg/kg, 0.5 µg/kg to 5 µg/kg, 0.5 µg/kg to 4 µg/kg, 0.5 µg/kg to 3 µg/kg, 0.5 µg/kg to 2.5 µg/kg, 0.1 µg/kg to 2 µg/kg, 0.5 µg/kg to 1 µg/kg, 1 µg/kg to 5 µg/kg, 1 µg/kg to 4 µg/kg, 1 µg/kg to 3 µg/kg, 1 µg/kg to 2.5 µg/kg, 1 µg/kg to 2 µg/kg, 2 µg/kg to 5 µg/kg, 2 µg/kg to 4 µg/kg, 2 µg/kg to 3 µg/kg, 2.5 µg/kg to 5 µg/kg, or 3 µg/kg to 5 µg/kg,

In some embodiments of the methods described herein, an IL-15/IL-15Ra complex is administered in a cyclical regimen, wherein the cyclical regimen comprises (a) a first period comprising subcutaneous administration of the IL-15/IL-15Ra complex to a subject for a time period of 7 days, 8, days, 9 days, 10 days, 11 days, 12 days, 13 days, 14 days, 15 days, 16 days, 17 days, 18 days, 19 days, 20 days or 21 days (*e.g*., every 1, 2 or 3 days during this time period),(b) a second period of 7 days, 8, days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 51 days, 52 days, 53 days, 54 days, 55 days, 56 days, 57 days, 58 days, 59 days, 60 days, 61 days or 62 days during which the IL-15/IL-15Ra complex is not administered to the subject, and (c) a third period comprising subcutaneous administration of the IL-15/IL-15Ra complex to a subject for a time period of 7 days, 8, days, 9 days, 10 days, 11 days, 12 days, 13 days, 14 days, 15 days, 16 days, 17 days, 18 days, 19 days, 20 days or 21 days (e.g., every 1, 2 or 3 days during this time period). In some embodiments of the methods described herein, an IL-15/IL-15Ra complex is administered in a cyclical regimen, wherein the cyclical regimen comprises: (a) a first period comprising subcutaneous administration of the IL-15/IL-15Ra complex to a subject for a time period of 7 days to 21 days, 7 days to 14 days, 7 days to 10 days, 7 days to 18 days, 10 days to 21 days, 10 days to 18 days, 10 days to 14 days, 12 days to 21 days, 12 days to 18 days, 12 days to 16 days, or 12 days to 14 days (e.g., every 1, 2 or 3 days during this time period), (b) a second period of 1 week to 2 weeks, 1 week to 3 weeks, 1 week to 4 weeks, 1 week to 5 weeks, 1 week to 6 weeks, 1 week to 7 weeks, 1 week to 8 weeks, 2 weeks to 3 weeks, 2 weeks to 4 weeks, 2 weeks to 5 weeks, 2 weeks to 6 weeks, 2 weeks to 7 weeks, 2 weeks to 8 weeks, 3 weeks to 4 weeks 3 weeks to 5 weeks, 3 weeks to 6 weeks, 3 weeks to 7 weeks, 3 weeks to 8 weeks, 4 weeks to 5 weeks, 4 weeks to 6 weeks, 4 weeks to 7 weeks, 4 weeks to 8 weeks, 5 weeks to 6 weeks, 5 weeks to 7 weeks, 5 weeks to 8 weeks, 6 weeks to 7 weeks, 6 weeks to 8 weeks, 7 weeeks to 8 weeks during which the IL-15/IL-15Ra complex is not administered to the subject, and (c) a third period comprising subcutaneous administration of the IL-15/IL-15Ra complex to a subject for a time period of 7 days to 21 days, 7 days to 14 days, 7 days to 10 days, 7 days to 18 days, 10 days to 21 days, 10 days to 18 days, 10 days to 14 days, 12 days to 21 days, 12 days to 18 days, 12 days to 16 days, or 12 days to 14 days (*e.g*., every 1, 2 or 3 days during this time period).

In another aspect, provided herein is a method for enhancing IL-15-mediated immune function, comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated a certain number of times and the dose is sequentially escalated as the regimen is repeated. In some embodiments, the plasma levels of IL-15 and/or lymphocyte counts in a subject are monitored at a certain frequency, *e.g*., after the each dose and/or after every other dose. In some embodiments, the subject is monitored for side effects such as a decrease in blood pressure and/or an increase in body temperature and/or an increase in cytokines in plasma. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 days. In some embodiments, the IL-15/IL-15Ra complex is administerd 1,2, 3, 4, 5, 6 or 7 days per week for the first period of time. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In an embodiment, the dose of the first cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In another embodiment, the dose of the second cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg higher than the dose used in the first cycle of the cyclical regimen. In another embodiment, the dose of the first cycle of the cylical regimen is 0.1 µg/kg, 0.25 µg/kg, 0.5 µg/kg, 0.75 µg/kg, or 1 µg/kg, and the dose of each subsequent cycle is 0.1 µg/kg, 0.25 µg/kg, 0.5 µg/kg, 0.75 µg/kg, 1 µg/kg, 1.5 µg/kg, 2 µg/kg, 2.5 µg/kg, 3 µg/kg, 4 µg/kg, 5 µg/kg, 6 µg/kg, 7 µg/kg, 8 µg/kg, 9 µg/kg or 10b µg/kg higher than the first and second doses used in the previous cycle of the cyclical regimen. In some embodiments, the dose of the first cycle of the cyclical regimen is 0.1 µg/kg, the dose of the second cycle of the cylical regimen is 0.25 µg/kg, the dose of the third cycle of the cylical regimen is 0.5 µg/kg, the dose of the fourth cycle of the cylical regimen is 1 µg/kg, and the dose of the fifth cycle of the cylical regimen is 2 µg/kg. In other embodiments, the dose of the first cycle of the cyclical regimen is 0.1 µg/kg to 0.25 µg/kg, the dose of the second cycle of the cylical regimen is 0.5 µg/kg to 1 µg/kg, the dose of the third cycle of the cylical regimen is 1 µg/kg to 3 µg/kg, the dose of the fourth cycle of the cylical regimen is 4 µg/kg to 6 µg/kg, and the dose of the fifth cycle of the cylical regimen is 7 µg/kg to 10 µg/kg.

In one embodiment, provided herein is a method for enhancing IL-15-mediated immune function, comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated at least 5 times, and wherein the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 10 µg/kg, the dose during the fourth cycle of the cylical regimen is 10 µg/kg to 20 µg/kg, and the dose during the fifth cycle of the cylical regimen is 20 µg/kg to 30 µg/kg. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In some embodiments, the plasma levels of IL-15 and/or lymphocyte counts in a subject are monitored at a certain frequency, *e.g*., after the each dose and/or after every other dose. In some embodiments, the subject is monitored for side effects such as a decrease in blood pressure and/or an increase in body temperature and/or an increase in cytokines in plasma.

In another aspect, provided herein is a a method for preventing, treating and/or managing a disorder in which enhancing IL-15-mediated immune function is beneficial (e.g., cancer, an infectious disease, an immunodeficiency or lymphopenia), comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated a certain number of times and the dose is sequentially escalated as the regimen is repeated. In some embodiments, the plasma levels of IL-15 and/or lymphocyte counts in a subject are monitored at a certain frequency, *e.g.*, after the each dose and/or after every other dose. In some embodiments, the subject is monitored for side effects such as a decrease in blood pressure and/or an increase in body temperature and/or an increase in cytokines in plasma. In some embodiments, the cyclical regimen is repeated 2, 3, 4, 5, 6, 7, 8, 9, 10 or more times. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In an embodiment, the dose of the first cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg. In another embodiment, the dose of the second cycle of the cyclical regimen is 0.1 µg/kg, 0.25 µg/kg, 0.5 µg/kg, 0.75 µg/kg, 1 µg/kg, 1.5 µg/kg, 2 µg/kg, 2.5 µg/kg, or 3 µg/kg higher than the dose used in the first cycle of the cyclical regimen. In another embodiment, the dose of the first cycle of the cylical regimen is 0.1 µg/kg, 0.25 µg/kg, 0.5 µg/kg, 0.75 µg/kg, or 1 µg/kg, and the dose of each subsequent cycle is 0.1 µg/kg, 0.25 µg/kg, 0.5 µg/kg, 0.75 µg/kg, 1 µg/kg, 1.5 µg/kg, 2 µg/kg, 2.5 µg/kg, 3 µg/kg, 4 µg/kg, 5 µg/kg, 6 µg/kg, 7 µg/kg, 8 µg/kg, 9 µg/kg or 10 µg/kg higher than the dose used in the previous cycle of the cyclical regimen. In some embodiments, the dose of the first cycle of the cyclical regimen is 0.1 µg/kg, the dose of the second cycle of the cylical regimen is 0.25 µg/kg, the dose of the third cycle of the cylical regimen is 0.5 µg/kg, the dose of the fourth cycle of the cylical regimen is 1 µg/kg, and the dose of the fifth cycle of the cylical regimen is 2 µg/kg. In other embodiments, the dose of the first cycle of the cyclical regimen is 0.1 µg/kg to 0.25 µg/kg, the dose of the second cycle of the cylical regimen is 0.5 µg/kg to 1 µg/kg, the dose of the third cycle of the cylical regimen is 1 µg/kg to 3 µg/kg, the dose of the fourth cycle of the cylical regimen is 4 µg/kg to 6 µg/kg, and the dose of the fifth cycle of the cylical regimen is 7 µg/kg to 10 µg/kg.

In one embodiment, provided herein is a a method for preventing, treating and/or managing a disorder in which enhancing IL-15-mediated immune function is beneficial (e.g., cancer, an infectious disease, an immunodeficiency or lymphopenia), comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated at least 5 times, and wherein the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 10 µg/kg, the dose during the fourth cycle of the cylical regimen is 10 µg/kg to 20 µg/kg, and the dose during the fifth cycle of the cylical regimen is 20 µg/kg to 30 µg/kg. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In some embodiments, the plasma levels of IL-15 and/or lymphocyte counts in a subject are monitored after the each dose and/or after every other dose. In some embodiments, the subject is monitored for side effects such as a decrease in blood pressure and/or an increase in body temperature and/or an increase in cytokines in plasma. In some embodiments, the disorder is an infectious disease. In some embodiments, the disorder is AIDS and the regimen is used to eradicate HIV.

In another embodiment, provided herein is a method for preventing, treating and/or managing a disorder in which enhancing IL-15-mediated immune function is beneficial, comprising subcutaneously administering to subject an IL-15/IL-15Ra complex in a cyclical regimen, wherein each cycle of the cyclical regimen comprises: (a) subcutaneously administering a dose of the IL-15/IL-15Ra complex to the subject at a certain frequency for a first period of time; and (b) no administration of IL-15/IL-15Ra complex for a second period of time, wherein the cylical regimen is repeated, and wherein the dose during each cycle is sequentially escalated until the maximum tolerated dose is achieved or until the subject exhibits one or more adverse events. In some embodiments, the plasma levels of IL-15 and/or lymphocyte counts in a subject are monitored at a certain frequency, e.g., after the each dose and/or after every other dose. In some embodiments, the subject is monitored for side effects such as a decrease in blood pressure and/or an increase in body temperature and/or an increase in cytokines in plasma, and adverse events, such as grade 3 or 4 lymphopenia, grade 3 granulocytopenia, grade 3 leukocytosis (WBC > 100,000/mm³), or organ dysfunction. In some embodiments, the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 10 µg/kg, the dose during the fourth cycle of the cylical regimen is 10 µg/kg to 20 µg/kg, and the dose during the fifth cycle of the cylical regimen is 20 µg/kg to 30 µg/kg. In other embodiments, the dose during the first cycle of the cyclical regimen is 0.1 µg/kg to 1 µg/kg, the dose during the second cyle of the cyclical regimen is 2 µg/kg to 5 µg/kg, the dose during the third cycle of the cyclical regimen is 5 µg/kg to 15 µg/kg, the dose during the fourth cycle of the cylical regimen is 15 µg/kg to 30 µg/kg, and the dose during the fifth cycle of the cylical regimen is 30 µg/kg to 50 µg/kg. In some embodiments, the IL-15/IL-15Ra complex is administered at a frequency of every day, every other day, every 3, 4, 5, 6 days. In some embodiments, the first and second periods of time are the same. In other embodiments, the first and second periods of time are different. In some embodiments, the first period of time for administration of the IL-15/IL-15Ra complex is 1 to 4 weeks, 2 to 4 weeks, 2 to 3 weeks,or 1 to 2 weeks long. In some embodiments, the first period of time for administration of the IL-15/IL-15Ra complex is 1 week, 2 weeks, 3 weeks or 4 weeks long. In some embodiments, the second period of time is 1 week to 2 months, 1 to 8 weeks, 2 to 8 weeks, 1 to 6 weeks, 2 to 6 weeks, 1 to 5 weeks, 2 to 5 weeks, 1 to 4 weeks, 2 to 4 weeks, 2 to 3 weeks or 1 to 2 weeks long. In some embodiments, the second period of time is 1 week, 2 weeks, 3 weeks, 4 weeks, 5 weeks or 6 weeks long. In some embodiments, the disorder is an infectious disease. In some embodiments, the disorder is caused by HIV and the regimen is used to eradicate HIV.

In accordance with the methods described herein, the Therapeutic Agent may be administered to a subject in a pharmaceutical composition. In some embodiments, the Therapeutic Agent is sole/single agent administered to the subject. In other embodiments, the Therapeutic Agents is administered in combination with one or more other therapies (e.g., an antibody that immunospecifically binds to Her2, PD-1 or a ligand of PD-1 (*e.g*., PD-L1). Combination therapy includes concurrent and successive administration of a Therapeutic Agent and another therapy. As used herein, the Therapeutic Agent and another therapy are said to be administered concurrently if they are administered to the patient on the same day, for example, simultaneously, or 1, 2, 3, 4, 5, 6, 7, or 8 hours apart. In contrast, the Therapeutic Agent and the therapy are said to be administered successively if they are administered to the patient on the different days, for example, the Therapeutic Agent and the therapy can be administered at a 1-day, 2-day or 3-day intervals. In the methods described herein, administration of the Therapeutic Agent can precede or follow administration of the second therapy. When administered simultaneously, the Therapeutic Agent and the other therapy can be in the same pharmaceutical composition or in a different pharmaceutical composition.

As a non-limiting example, the Therapeutic Agent and another therapy can be administered concurrently for a first period of time, followed by a second period of time in which the administration of the Therapeutic Agent and the other therapy is alternated. In some embodiments, the Therapeutic Agent and the other therapy can be administered using the following cyclical administration regimen: (a) a dose of the Therapeutic Agent is administered subcutaneously to a subject every 1, 2 or 3 days for 1 week to 3 weeks; (b) a dose of the other therapy is administered to the subject for every 1, 2, 3, 5 or 7 days for approximately 1 week to 3 weeeks; and (c) a dose of the Therapeutic Agent is administered subcutaneously to the subject every 1, 2 or 3 days for 1 week to 3 weeks. Whether this cyclical administration regimen is repeated and how many times it is repeat may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three, four, five, six, seven, eight, nine, ten times or more. In some embodiments, the dose of Therapeutic Agent administered to the subject is a dose of approximately 0.1 µg/kg to 10 µg/kg of the subject.

For example, the Therapeutic Agent and the other therapy can be administered using the following cyclical administration regimen: (a) a dose of the Therapeutic Agent is administered subcutaneously to a subject every 1, 2 or 3 days for approximately a two week period; (b) a dose of the other therapy is administered to the subject for every 1, 2, 3, 5 or 7 days for approximately a two week period; and (c) a dose of the Therapeutic Agent is administered subcutaneously to the subject every 1, 2 or 3 days for approximately a two week period. Whether this cyclical administration regimen is repeated and how many times it is repeat may depend, *e.g.,* the type of symptoms, and the seriousness of the symptoms, and should be decided according to the judgment of the practitioner and each patient's or subject's circumstances. In some embodiments, this cyclical administration regimen is repeated one time, two times, three times or more. In some embodiments, the dose of Therapeutic Agent administered to the subject is a dose of approximately 0.1 µg/kg to 10 µg/kg of the subject. In certain embodiments, the other therapy is an antibody that immunospecifically binds to PD-1 or a ligand thereof (*e.g*., PD-L1). In other embodiments, the other therapy is an antibody that immunospecifically binds to Her2.

For example, a monoclonal antibody that targets a cancer cell can be administered prior to or concurrently with the administration of a Therapeutic Agent. In an embodiment, the monoclonal antibody is given prior to administration of the Therapeutic Agent, wherein the Therapeutic Agent is administered according to a cyclical administration regimen described herein. In an embodiment, the monoclonal antibody immunospecifically binds to Her2 (*e.g*., Herceptin®).

In another non-limiting example, the Therapeutic Agent can be administered to a subject after the administration of another therapy. For example, a chemotherapeutic agent or an immunosuppressive agent can be administrated prior to the administration of a Therapeutic Agent. In an embodiment, the chemotherapeutic agent or immunosuppressive agent is administered for a period of time prior to the administration of the Therapeutic Agent, wherein the Therapeutic Agent is administered according to a cyclical immunization regimen described herein.

In some embodiments, examples of immune function enhanced by the methods described herein include the proliferation/expansion of lymphocytes (*e.g*., increase in the number of lymphocytes), inhibition of apoptosis of lymphocytes, activation of dendritic cells (or antigen presenting cells), and antigen presentation. In particular embodiments, an immune function enhanced by the methods described herein is proliferation/expansion in the number of or activation of CD4⁺ T cells (*e.g*., Th1 and Th2 helper T cells), CD8⁺ T cells (*e.g.,* cytotoxic T lymphocytes, alpha/beta T cells, and gamma/delta T cells), B cells (*e.g.,* plasma cells), memory T cells, memory B cells, dendritic cells (immature or mature), antigen presenting cells, macrophages, mast cells, natural killer T cells (NKT cells), tumor-resident T cells, CD122⁺ T cells, or natural killer cells (NK cells). In one embodiment, the methods described herein enhance the proliferation/expansion or number of lymphocyte progenitors. In some embodiments, the methods described herein increases the number of CD4⁺ T cells (*e.g*., Th1 and Th2 helper T cells), CD8⁺ T cells (*e.g*., cytotoxic T lymphocytes, alpha/beta T cells, and gamma/delta T cells), B cells (*e.g*., plasma cells), memory T cells, memory B cells, dendritic cells (immature or mature), antigen presenting cells, macrophages, mast cells, natural killer T cells (NKT cells), tumor-resident T cells, CD122⁺ T cells, or natural killer cells (NK cells) by approximately 1 fold, 2 fold, 3 fold, 4 fold, 5 fold, 6 fold, 7 fold, 8 fold, 9 fold, 10 fold, 20 fold, or more relative a negative control (e.g., number of the respective cells not treated, cultured, or contacted with a Therapeutic Agent).

In an embodiment, the methods described herein enhance or induce immune function in a subject by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the immune function in a subject not administered the Therapeutic Agent using assays well known in the art, *e.g.*, ELISPOT, ELISA, and cell proliferation assays. In an embodiment, the immune function is cytokine release (*e.g*., interferon-gamma, IL-2, IL-5, IL-10, IL-12, or transforming growth factor (TGF) -beta). In one embodiment, the IL-15 mediated immune function is NK cell proliferation, which can be assayed, *e.g*., by flow cytometry to detect the number of cells expressing markers of NK cells (*e.g*., CD56). In another embodiment, the IL-15 mediated immune function is antibody production, which can be assayed, *e.g*., by ELISA. In some embodiments, the IL-15 mediated immune function is effector function, which can be assayed, *e.g*., by a cytotoxicity assay or other assays well known in the art.

The effect of one or more doses of one or more IL-15/IL-15Ra complexes on peripheral blood lymphocyte counts can be monitored/assessed using standard techniques known to one of skill in the art. Peripheral blood lymphocytes counts in a mammal can be determined by, e.g., obtaining a sample of peripheral blood from said mammal, separating the lymphocytes from other components of peripheral blood such as plasma using, *e.g*., Ficoll-Hypaque (Pharmacia) gradient centrifugation, and counting the lymphocytes using trypan blue. Peripheral blood T-cell counts in mammal can be determined by, e.g., separating the lymphocytes from other components of peripheral blood such as plasma using, e.g., a use of Ficoll-Hypaque (Pharmacia) gradient centrifugation, labeling the T-cells with an antibody directed to a T-cell antigen such as CD3, CD4, and CD8 which is conjugated to FITC or phycoerythrin, and measuring the number of T-cells by FACS. Further, the effect on a particular subset of T cells (*e.g*., CD2⁺, CD4⁺, CD8⁺, CD4⁺RO⁺, CD8⁺RO⁺, CD4⁺RA⁺, or CD8⁺RA⁺) or NK cells can be determined using standard techniques known to one of skill in the art such as FACS.

The plasma levels of IL-15 can be assessed using standard techniques known to one of skill in the art. For example, a plasma can be obtained from a blood sample obtained from a subject and the levels of IL-15 in the plasma can be measured by ELISA.

### 5.6 Adoptive Cell Transfer

In one aspect, host cells that recombinantly express an IL-15Ra polypeptide described herein (*e.g.,* Section 3.1, Section 5.1 and/or Section 5.2, *supra*) are administered to a subject to enhance IL-15-mediated immune function and/or to prevent, treat and/or manage a disorder in which enhancing IL-15-mediated immune function is beneficial, such as cancer, an infectious disease, an immunodeficiency, or lymphopenia. In some embodiments, a host cell(s) described herein that recombinantly expresses IL-15 and IL-15Ra is administered to a subject to enhance IL-15-mediated immune function. In some embodiments, a host cell(s) described herein that recombinantly expresses IL-15 and IL-15Ra is administered to a subject to prevent, treat and/or manage a disorder in which enhancing IL-15-mediated immune function is beneficial, such as cancer, an infectious disease, an immunodeficiency, or lymphopenia.

In some embodiments, provided herein is a method for enhancing IL-15-mediated immune function, comprising administering to a subject a host cell(s) that recombinantly expresses an IL-15Ra polypeptide described herein. In some embodiments, provided herein is a method for enhancing IL-15-mediated immune function is beneficial, comprising administering to a subject a composition comprising a host cell(s) that recombinantly expresses an IL-15Ra polypeptide described herein. In some embodiments, the IL-15Ra polypeptide expressesd by the host cell(s) is an IL-15Ra polypeptide described in Section 5.1, *supra.* In some embodiments, the host cell(s) recombinantly expresses an IL-15 polypeptide in addition to the IL-15Ra polypeptide. In some embodiments, the host cell(s) is an immune cell(s), such as an immune cell(s) obtained or derived from the subject. In some embodiments, the host cell(s) is a lymphocyte(s) (*e.g*., a T lymphocyte(s)), monocyte(s), dendritic cell(s), or Natural Killer cell(s), such as a lymphocyte(s) (*e.g*., a T lymphocyte(s)), monocyte(s), dendritic cell(s), or Natural Killer cell(s) obtained or derived from the subject. In some embodiments, the host cell(s) is a peripheral blood mononuclear cell or a tumor infiltrating lymphocyte, such as a peripheral blood mononuclear cell or a tumor infiltrating lymphocyte obtained or derived from the subject. In some embodiments, the host cell(s) is an immune cell(s), which was isolated from a peripheral blood sample from the subject, cultured in cell culture, and engineered to recombinantly express an IL-15Ra polypeptide (and in some embodiments, an IL-15 polypeptide). In some embodiments, the host cell(s) was derived from the subject receiving the host cell(s). In other embodiments, the host cell(s) was derived from a subject different than the subject receiving the host cell(s).

In some embodiments, provided herein is a method for preventing, treating and/or managing a disorder in which enhancing IL-15-mediated immune function is beneficial, comprising administering to a subject a host cell(s) that recombinantly expresses an IL-15Ra polypeptide described herein. In some embodiments, provided herein is a method for preventing, treating and/or managing a disorder in which enhancing IL-15-mediated immune function is beneficial, comprising administering to a subject a composition comprising a host cell(s) that recombinantly expresses an IL-15Ra polypeptide described herein. In some embodiments, the IL-15Ra polypeptide expressesd by the host cell(s) is an IL-15Ra polypeptide described in Section 5.1, *supra.* In some embodiments, the host cell(s) recombinantly expresses an IL-15 polypeptide in addition to the IL-15Ra polypeptide. In some embodiments, the host cell(s) is a peripheral blood mononuclear cell or tumor infiltrating lymphocyte. In some embodiments, the host cell(s) is an immune cell(s). In some embodiments, the host cell(s) is a lymphocyte(s) (*e.g*., a T lymphocyte(s), such as a CD4+ T-lymphocyte or CD8+ lymphocyte), monocyte(s), dendritic cell(s), or Natural Killer cell(s). In some embodiments, the host cell(s) was derived from the subject receiving the host cell(s). In other embodiments, the host cell(s) was derived from a subject different than the subject receiving the host cell(s). In some embodiments, the host cell(s) for administration to a subject is an immune cell(s), which was isolated from a peripheral blood sample of the subject receiving the host cell(s) or a different subject, cultured in cell culture, and engineered to recombinantly express an IL-15Ra polypeptide (and in some embodiments, an IL-15 polypeptide). *See* Sections 5.7 to 5.9, *infra*, for a discussion of disorders in which enhancing IL-15-mediated immune function is beneficial.

Host cells that recombinantly express IL-15Ra (and in some embodiments, IL-15) may be administered locally or systemically to a subject via any route known to one of skill in the art (*e.g*., parenteral administration, such as subcutaneous, intravenous, or intramuscular administration, or intratumoral administration). In some embodiments, host cells that recombinantly express IL-15Ra (and in some embodiments, IL-15) are implanted or infused into a subject. In some embodiments, host cells that recombinantly express IL-15Ra (and in some embodiments, IL-15) are implanted in a subject and the implant includes a porous, nonporous, or gelatinous material, including membranes, such as sialastic membranes, or fibers in addition to the host cells.

In some of the embodiments in which host cells that recombinantly express IL-15Ra (and, in some embodiments, IL-15) are administered locally, the host cells recombinantly express an IL-15Ra polypeptide in which the cleavage site for an endogenous protease that cleaves native IL-15Ra has been mutated. In some embodiments, the host cells that are administered locally recombinantly express an IL-15Ra derivative comprising one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited. In other embodiments, the host cells that are administered locally recombinantly express an IL-15Ra derivative comprising (i) an extracellular domain of IL-15Ra in which the cleavage site for an endogenous protease that cleaves native IL-15Ra has been mutated, and (ii) all or a fragment of a transmembrane domain of a heterologous molecule in place of all or a fragment of the transmembrane domain of native IL-15Ra. In some embodiments, the host cells that are administered locally recombinantly express an IL-15Ra derivative comprising (i) an extracellular domain of IL-15Ra wherein one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited, and (ii) all or a fragment of a transmembrane domain of a heterologous molecule in place of all or a fragment of the transmembrane domain of native IL-15Ra. In yet other embodiments, the host cells that are administered locally are engineered to recombinantly express any of the IL-15Ra polypeptides described in Section 5.3.2., *supra,* in which the cleavage site for an endogenous protease that cleaves native IL-15Ra has been mutated (e.g., deleted) such that cleavage by an endogenous protease that cleaves native human IL-15Ra is inhibited. In yet other embodiments, the host cells that are administered locally are engineered to recombinantly express any of the IL-15Ra polypeptides described in Section 5.1 or Section 5.3.2., *supra.*

In some embodiments, host cells that recombinantly express IL-15Ra (and in some embodiments, IL-15) are administered to a subject as part of composition. In some embodiments, such a composition comprises a polymer in addition to the host cells. In some embodiments, a suitable dose of host cells that recombinantly express IL-15Ra (and in some embodiments, IL-15) administered to subject may be at least 100, 200, 300, 400, 500, 700, 1,000, 5,000, 10,000, 25,000, 50,000, 100,000, 1 x10⁶, 1 x10⁷, or 1 x10⁸ cells. In some embodiments, a suitable dose of host cells that recombinantly express IL-15Ra (and in some embodiments, IL-15) administered to a subject is between 100 to 10,000, 500 to 10,000, 1,000 to 5,000, 5,000 to 10,000, 5,000 to 20,000, 10,000 to 20,000, 25,000 to 50,000, 50,000 to 100,000, 1 x10⁴ to 1 x10⁵, 1 x10⁵ to 1 x10⁶, 1 x10⁵ to 1 x10⁷, 1 x10⁶ to 1 x10⁸ cells. Host cells that recombinantly express IL-15Ra (and in some embodiments, IL-15) may be administered 1, 2, 3, 4, 5, 6, 7, 8 or more times. The frequency and dose of host cells that recombinantly express IL-15Ra (and in some embodiments, IL-15) which are administered to a subject will vary depending on several factors, including, *e.g.,* the condition of the patient.

In some embodiments, examples of immune function enhanced by the methods described herein include the proliferation/expansion of lymphocytes (*e.g*., increase in the number of lymphocytes), inhibition of apoptosis of lymphocytes, activation of dendritic cells (or antigen presenting cells), and antigen presentation. In particular embodiments, an immune function enhanced by the methods described herein is proliferation/expansion in the number of or activation of CD4⁺ T cells (*e.g*., Th1 and Th2 helper T cells), CD8⁺ T cells (*e.g.,* cytotoxic T lymphocytes, alpha/beta T cells, and gamma/delta T cells), B cells (*e.g.,* plasma cells), memory T cells, memory B cells, dendritic cells (immature or mature), antigen presenting cells, macrophages, mast cells, natural killer T cells (NKT cells), tumor-resident T cells, CD122⁺ T cells, or natural killer cells (NK cells). In one embodiment, the methods described herein enhance the proliferation/expansion or number of lymphocyte progenitors. In some embodiments, the methods described herein increases the number of CD4⁺ T cells (*e.g*., Th1 and Th2 helper T cells), CD8⁺ T cells (*e.g*., cytotoxic T lymphocytes, alpha/beta T cells, and gamma/delta T cells), B cells (*e.g*., plasma cells), memory T cells, memory B cells, dendritic cells (immature or mature), antigen presenting cells, macrophages, mast cells, natural killer T cells (NKT cells), tumor-resident T cells, CD122⁺ T cells, or natural killer cells (NK cells) by approximately 1 fold, 2 fold, 3 fold, 4 fold, 5 fold, 6 fold, 7 fold, 8 fold, 9 fold, 10 fold, 20 fold, or more relative a negative control.

In an embodiment, the methods described herein enhance or induce immune function in a subject by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the immune function in a subject not administered an Engineered Cell(s) using assays well known in the art, *e.g.,* ELISPOT, ELISA, and cell proliferation assays. In an embodiment, the immune function is cytokine release (*e.g*., interferon-gamma, IL-2, IL-5, IL-10, IL-12, or transforming growth factor (TGF) -beta). In one embodiment, the IL-15 mediated immune function is NK cell proliferation, which can be assayed, *e.g.,* by flow cytometry to detect the number of cells expressing markers of NK cells (*e.g.,* CD56). In another embodiment, the IL-15 mediated immune function is antibody production, which can be assayed, *e.g.,* by ELISA. In some embodiments, the IL-15 mediated immune function is effector function, which can be assayed, *e.g.,* by a cytotoxicity assay or other assays well known in the art.

The effect of one or more doses of an Engineered Cell(s) on peripheral blood lymphocyte counts can be monitored/assessed using standard techniques known to one of skill in the art. Peripheral blood lymphocytes counts in a mammal can be determined by, e.g., obtaining a sample of peripheral blood from said mammal, separating the lymphocytes from other components of peripheral blood such as plasma using, *e.g.,* Ficoll-Hypaque (Pharmacia) gradient centrifugation, and counting the lymphocytes using trypan blue. Peripheral blood T-cell counts in mammal can be determined by, e.g., separating the lymphocytes from other components of peripheral blood such as plasma using, e.g., a use of Ficoll-Hypaque (Pharmacia) gradient centrifugation, labeling the T-cells with an antibody directed to a T-cell antigen such as CD3, CD4, and CD8 which is conjugated to FITC or phycoerythrin, and measuring the number of T-cells by FACS. Further, the effect on a particular subset of T cells (*e.g*., CD2⁺, CD4⁺, CD8⁺, CD4⁺RO⁺, CD8⁺RO⁺, CD4⁺RA⁺, or CD8⁺RA⁺) or NK cells can be determined using standard techniques known to one of skill in the art such as FACS.

### 5.7 Cancer Treatment

Provided herein are methods for preventing, treating, and/or managing cancer, comprising administering an effective amount of a Therapeutic Agent or a composition comprising a Therapeutic Agent to a subject in need thereof. In a specific embodiment, the Therapeutic Agent is administered subcutaneously. In a specific embodiment, the cylical administration regimens described herein are used to prevent, treat and/or manage cancer.

Also provided herein are methods for preventing, treating, and/or managing cancer, comprising administering an effective amount of an Engineered Cell(s) or a composition comprising an Engineered Cell(s) to a subject in need thereof.

The effect of a Therapeutic Agent or an Engineered Cell(s) on proliferation of cancer cells can be detected by routine assays, such as by assays that measure the uptake of radiolabeled thymidine. Alternatively, cell viability can be measured by assays that measure lactate dehydrogenase (LDH), a stable cytosolic enzyme that is released upon cell lysis, or by the release of [⁵¹Cr] upon cell lysis. In one embodiment, necrosis measured by the ability or inability of a cell to take up a dye such as neutral red, trypan blue, or ALAMARTM blue (Page et al., 1993, Intl. J. of Oncology 3:473 476). In such an assay, the cells are incubated in media containing the dye, the cells are washed, and the remaining dye, reflecting cellular uptake of the dye, is measured spectrophotometrically.

In another embodiment, the dye is sulforhodamine B (SRB), whose binding to proteins can be used as a measure of cytotoxicity (Skehan et al., 1990, J. Nat'l Cancer Inst. 82:1107 12). In yet another embodiment, a tetrazolium salt, such as MTT, is used in a quantitative colorimetric assay for mammalian cell survival and proliferation by detecting living, but not dead, cells (see, *e.g*., Mosmann, 1983, J. Immunol. Methods 65:55 63).

In other embodiments, apoptotic cells are measured in both the attached and "floating" compartments of the cultures. Both compartments are collected by removing the supernatant, trypsinizing the attached cells, and combining both preparations following a centrifugation wash step (10 minutes, 2000 rpm). The protocol for treating tumor cell cultures with sulindac and related compounds to obtain a significant amount of apoptosis has been described in the literature (see, *e.g.,* Piazza et al., 1995, Cancer Research 55:3110 16). Features of this method include collecting both floating and attached cells, identification of the optimal treatment times and dose range for observing apoptosis, and identification of optimal cell culture conditions.

In another embodiment, apoptosis is quantitated by measuring DNA fragmentation. Commercial photometric methods for the quantitative *in vitro* determination of DNA fragmentation are available. Examples of such assays, including TUNEL (which detects incorporation of labeled nucleotides in fragmented DNA) and ELISA-based assays, are described in Biochemica, 1999, no. 2, pp. 34 37 (Roche Molecular Biochemicals).

In yet another embodiment, apoptosis can be observed morphologically.

Cancer cell lines on which such assays can be performed are well known to those of skill in the art. Apoptosis, necrosis and proliferation assays can also be performed on primary cells, *e.g.,* a tissue explant.

In an embodiment, the proliferation or viability of cancer cells contacted with a Therapeutic Agent or a composition comprising a Therapeutic Agent is inhibited or reduced by at least 2 fold, preferably at least 2.5 fold, at least 3 fold, at least 4 fold, at least 5 fold, at least 7 fold, or at least 10 fold relative to the proliferation of the cancer cells when contacted with a negative control as measured using assays well known in the art, *e.g.,* cell proliferation assays using CSFE, BrdU, and ³H-Thymidine incorporation. In another embodiment, the proliferation of cancer cells contacted with a Therapeutic Agent or a composition comprising a Therapeutic Agent is inhibited or reduced by at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% relative to cancer cells contacted with a negative control as measured using assays well known in the art, *e.g.,* cell proliferation assays using CSFE, BrdU, and ³H-Thymidine incorporation, or those assays described above. In one aspect, the composition comprising a Therapeutic Agent further comprises cells (*e.g*., NK cells or cytotoxic T cells) that are responsive to IL-15 signaling and that can target and exert cytotoxic effects on the cancer cells.

In an embodiment, the proliferation or viability of cancer cells following administration of an Engineered Cell(s) or a composition comprising an Engineered Cell(s) is inhibited or reduced by at least 2 fold, preferably at least 2.5 fold, at least 3 fold, at least 4 fold, at least 5 fold, at least 7 fold, or at least 10 fold relative to the proliferation of the cancer cells following administration of a negative control as measured using assays well known in the art, *e.g.,* cell proliferation assays using CSFE, BrdU, and ³H-Thymidine incorporation. In another embodiment, the proliferation of cancer cells following administration of an Engineered Cell(s) or a composition comprising an Engineered Cell(s) is inhibited or reduced by at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% relative to cancer cells following administration of a negative control as measured using assays well known in the art, *e.g.,* cell proliferation assays using CSFE, BrdU, and ³H-Thymidine incorporation, or those assays described above.

In some embodiments, the administration of a Therapeutic Agent or an Engineered Cell(s) to a subject in accordance with the methods described herein achieves one, two, or three or more results: (1) a reduction in the growth of a tumor or neoplasm; (2) a reduction in the formation of a tumor; (3) an eradication, removal, or control of primary, regional and/or metastatic cancer; (4) a reduction in metastatic spread; (5) a reduction in mortality; (6) an increase in survival rate; (7) an increase in length of survival; (8) an increase in the number of patients in remission; (9) a decrease in hospitalization rate; (10) a decrease in hospitalization lengths; and (11) the maintenance in the size of the tumor so that it does not increase by more than 10%, or by more than 8%, or by more than 6%, or by more than 4%; preferably the size of the tumor does not increase by more than 2%.

In an embodiment, the administration of a Therapeutic Agent or an Engineered Cell(s), or a composition comprising a Therapeutic Agent or an Engineered Cell(s) to a subject with cancer (in some embodiments, an animal model for cancer) in accordance with the methods described herein inhibits or reduces the growth of a tumor by at least 2 fold, preferably at least 2.5 fold, at least 3 fold, at least 4 fold, at least 5 fold, at least 7 fold, or at least 10 fold relative to the growth of a tumor in a subject with cancer (in some embodiments, in the same animal model for cancer) administered a negative control as measured using assays well known in the art. In another embodiment, the administration of a Therapeutic Agent or an Engineered Cell(s), or a composition comprising a Therapeutic Agent or an Engineered Cell(s) to a subject with cancer (in some embodiments, an animal model for cancer) in accordance with the methods described herein inhibits or reduces the growth of a tumor by at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% relative to the growth of a tumor in a subject with cancer (in some embodiments, in the same animal model for cancer) administered a negative control as measured using assays well known in the art.

In an embodiment, the administration of a Therapeutic Agent or an Engineered Cell(s),or a composition comprising a Therapeutic Agent or an Engineered Cell(s) to a subject with cancer (in some embodiments, an animal model for cancer) in accordance with the methods described herein reduces the size of a tumor by at least 2 fold, preferably at least 2.5 fold, at least 3 fold, at least 4 fold, at least 5 fold, at least 7 fold, or at least 10 fold relative to the growth of a tumor in a subject with cancer (in some embodiments, the same animal model for cancer) in accordance with the methods described herein administered a negative control as measured using assays well known in the art. In another embodiment, the administration of a Therapeutic Agent or an Engineered Cell(s), or a composition comprising a Therapeutic Agent or an Engineered Cell(s) to a subject with cancer (in some embodiments, an animal model for cancer) reduces the size of a tumor by at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% relative to the growth of a tumor in a subject with cancer (in some embodiments, the same animal model for cancer) administered a negative control (*e.g*., saline or PBS) as measured using assays well known in the art. In a specific embodiment, the cancer is melanoma, renal cancer, colon cancer, or prostate cancer. In another embodiment, the cancer is metastatic.

A Therapeutic Agent or an Engineered Cell(s) can be administered in combination with one or more other therapies, *e.g*., anti-cancer agents, cytokines or anti-hormonal agents, to treat and/or manage cancer. Non-limiting examples anti-cancer agents are described below. *See* Section 5.10, *infra,* and in particular, Section 5.10.1, *infra.* In one embodiment, the combination of Therapeutic Agent or an Engineered Cell(s)and one or more other therapies provides an additive therapeutic effect relative to the therapeutic effects of the Therapeutic Agent or an Engineered Cell(s) alone or the one or more other therapies alone. In one embodiment, the combination of a Therapeutic Agent or an Engineered Cell(s) and one or more other therapies provides more than an additive therapeutic effect relative to the therapeutic effects of the Therapeutic Agent or an Engineered Cell(s) alone or the one or more other therapies alone. In one embodiment, the combination of a Therapeutic Agent or an Engineered Cell(s) and one or more other therapies provides a synergistic therapeutic effect relative to the therapeutic effects of the Therapeutic Agent or an Engineered Cell(s) alone or the one or more other therapies alone.

In one embodiment, the one or more therapies include, but are not limited to cytokines/growth factors, *e.g*., interleukin (IL) 1, IL-2, IL-3, IL-4, IL-5, IL-6, IL-7, IL-8, IL-10, IL-11, IL-12, Il-15, TNF-α, TNF-β, TGF-β, GM-CSF, and interferon-γ. In one embodiment, the one or more therapies include, but are not limited to receptors, antibodies, or other binding agents that bind to cytokines/growth factors, *e.g*., interleukin (IL) 1, IL-2, IL-3, IL-4, IL-5, IL-6, IL-7, IL-8, IL-10, IL-11, IL-12, TNF-α, TNF-β, TGF-β, GM-CSF, interferon-α, interferon-β, and interferon-γ. In some embodiments, the one or more therapies include, but are not limited to, cells recombinantly expressing a therapeutic protein (or polypeptides), *e.g*., a cytokine, a growth factor, a chemokine, or a fragment or derivative thereof. In a particular embodiment, the one or more therapies include, but are not limited to, cells recombinantly expressing IL-12, IL-6, GM-CSF, interferon-α, interferon-β, interferon-γ or TNF-α. In some embodiments, such therapies are administered prior to, concurrently with, or after administration of a Therapeutic Agent or an Engineered Cell(s), wherein the Therapeutic Agent or an Engineered Cell(s) is administered in accordance with the methods described herein. In some embodiments, such therapies are administered prior to, concurrently with, or after administration of an Engineered Cell(s), wherein the Engineered Cell(s) is administered in accordance with the methods described herein.

A Therapeutic Agent or an Engineered Cell(s) can also be administered in combination with radiation therapy comprising, *e.g*., the use of x-rays, gamma rays and other sources of radiation to destroy the cancer cells. In some embodiments, the radiation treatment is administered as external beam radiation or teletherapy wherein the radiation is directed from a remote source. In other embodiments, the radiation treatment is administered as internal therapy or brachytherapy wherein a radioactive source is placed inside the body close to cancer cells or a tumor mass. In an embodiment, a Therapeutic Agent or an Engineered Cell(s) can be administered in accordance with the methods described herein before, during or after radiation therapy. In one aspect, the Therapeutic Agent or the Engineered Cell(s) can enhance the immune function of cancer patient with a compromised immune system due to anti-cancer therapy. A Therapeutic Agent or an Engineered Cell(s) can also be administered in combination with chemotherapy. In one embodiment, a Therapeutic Agent or an Engineered Cell(s) can be administered in accordance with the methods described herein before, during or after radiation therapy or chemotherapy. In one embodiment, a Therapeutic Agent or an Engineered Cell(s) can be used before, during or after surgery. In one embodiment, methods provided herein include the combination of transplant and a Therapeutic Agent or an Engineered Cell(s).

Non-limiting examples of anti-hormonal agents are anti-hormonal agents that act to regulate or inhibit hormone action on tumors, such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN®V exemestane, formestanie, fadrozole, RIVISOR® vorozole, FEMARA® letrozole, and ARIMIDEX®D anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in aberrant cell proliferation, such as, for example, PKC-alpha, Raf and H-Ras; ribozymes such as a VEGF expression inhibitor (*e.g*., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELX® rmRH; Vinorelbine and Esperamicins (see U.S. Pat. No. 4,675,187), and pharmaceutically acceptable salts, acids or derivatives of any of the above.

In a specific embodiment, a Therapeutic Agent is administered to a subject in combination with an antibody that immunospecifically binds to programmed cell death (PD-1) or a ligand thereof (*e.g*., PD-L1). In another specific embodiment, provided herein is a method for preventing, treating and/or managing cancer comprising subcutaneously administering an IL-15/IL-15Ra complex and administering an antibody that immunospecifically binds to PD-1 or a ligand thereof. In certain embodiments, the antibody is administered after the IL-15/IL-15Ra complex is administered. In other embodiments, the antibody is administered before the IL-15/IL-15Ra complex is administered. In specific embodiments, the cancer is melanoma, prostate cancer, or lung cancer.

In another embodiment, a Therapeutic Agent is administered to a subject in combination with an antibody that immunospecifically binds to Her2 (*e.g*., Herceptin®). In another embodiment, provided herein is a method for preventing, treating and/or managing cancer comprising subcutaneously administering an IL-15/IL-15Ra complex and administering an antibody that immunospecifically binds to Her2 (*e.g*., Herceptin®) In certain embodiments, the antibody is administered after the IL-15/IL-15Ra complex is administered. In other embodiments, the antibody is administered before the IL-15/IL-15Ra complex is administered. In some embodiments, the cancer is breast cancer.

Cancers and related disorders that can be prevented, treated, or managed in accordance with the methods described herein include, but are not limited to, the following: Leukemias including, but not limited to, acute leukemia, acute lymphocytic leukemia, acute myelocytic leukemias such as myeloblastic, promyelocytic, myelomonocytic, monocytic, erythroleukemia leukemias and myelodysplastic syndrome, chronic leukemias such as but not limited to, chronic myelocytic (granulocytic) leukemia, and chronic lymphocytic leukemia, hairy cell leukemia; polycythemia vera; lymphomas such as but not limited to Hodgkin's disease, and non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, and synovial sarcoma; brain tumors including but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, and primary brain lymphoma; breast cancer including, but not limited to, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer, including but not limited to, pheochromocytom and adrenocortical carcinoma; thyroid cancer such as but not limited to papillary or follicular thyroid cancer, medullary thyroid cancer and anaplastic thyroid cancer; pancreatic cancer, including but not limited to, insulinoma, gastrinoma, glucagonoma, vipoma, somatostatin-secreting tumor, and carcinoid or islet cell tumor; pituitary cancers including but not limited to, Cushing's disease, prolactin-secreting tumor, acromegaly, and diabetes insipius; eye cancers including but not limited to, ocular melanoma such as iris melanoma, choroidal melanoma, and cilliary body melanoma, and retinoblastoma; vaginal cancers, including but not limited to, squamous cell carcinoma, adenocarcinoma, and melanoma; vulvar cancer, including but not limited to, squamous cell carcinoma, melanoma, adenocarcinoma, basal cell carcinoma, sarcoma, and Paget's disease; cervical cancers including but not limited to, squamous cell carcinoma, and adenocarcinoma; uterine cancers including but not limited to, endometrial carcinoma and uterine sarcoma; ovarian cancers including but not limited to, ovarian epithelial carcinoma, borderline tumor, germ cell tumor, and stromal tumor; esophageal cancers including but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell (small cell) carcinoma; stomach cancers including but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers including but not limited to hepatocellular carcinoma and hepatoblastoma; gallbladder cancers including but not limited to, adenocarcinoma; cholangiocarcinomas including but not limited to, pappillary, nodular, and diffuse; lung cancers including but not limited to, non-small cell lung cancer, squamous cell carcinoma (epidermoid carcinoma), adenocarcinoma, large-cell carcinoma and small-cell lung cancer; testicular cancers including but not limited to, germinal tumor, seminoma, anaplastic, spermatocytic, nonseminoma, embryonal carcinoma, teratoma carcinoma, choriocarcinoma (yolk-sac tumor); prostate cancers including but not limited to, adenocarcinoma, leiomyosarcoma, and rhabdomyosarcoma; penal cancers; oral cancers including but not limited to, squamous cell carcinoma; basal cancers; salivary gland cancers including but not limited to, adenocarcinoma, mucoepidermoid carcinoma, and adenoidcystic carcinoma; pharynx cancers including but not limited to, squamous cell cancer, and verrucous; skin cancers including but not limited to, basal cell carcinoma, squamous cell carcinoma and melanoma, and superficial spreading melanoma, nodular melanoma, lentigo malignant melanoma, acral lentiginous melanoma; kidney cancers including but not limited to, renal cell cancer, renal cancer, adenocarcinoma, hypernephroma, fibrosarcoma, and transitional cell cancer (renal pelvis and/or uterer); Wilms' tumor; bladder cancers including but not limited to, transitional cell carcinoma, squamous cell cancer, adenocarcinoma, and carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

In one embodiment, the cancer is benign, *e.g*., polyps and benign lesions. In other embodiments, the cancer is metastatic. The Therapeutic Agents or Engineered Cell(s)can be used in the treatment of pre-malignant as well as malignant conditions. Pre-malignant conditions include hyperplasia, metaplasia, and dysplasia. Treatment of malignant conditions includes the treatment of primary as well as metastatic tumors. In a specific embodiment, the cancer is melanoma, colon cancer, renal cell carcinoma, or lung cancer (*e.g*., non-small cell lung cancer). In certain embodiments, the cancer is metastatic melanoma, metastaic colon cancer, metastatic renal cell carcinoma, or metastatic lung cancer (*e.g*., metastatic non-small cell lung cancer).

In some embodiments, a Therapeutic Agent, a composition comprising a Therapeutic Agent, an Engineered Cell(s), a composition an Engineered Cell(s) or combination therapies are administered to a subject suffering from or diagnosed with cancer. In other embodiments, a Therapeutic Agent, a composition comprising a Therapeutic Agent, an Engineered Cell(s), a composition an Engineered Cell(s), or combination therapies are administered to a subject predisposed or susceptible to developing cancer. In some embodiments, a Therapeutic Agent, a composition comprising a Therapeutic Agent, an Engineered Cell(s), a composition an Engineered Cell(s), or combination therapies are administered to a subject that lives in a region where there is a high occurrence rate of cancer. In an embodiment, the cancer is characterized by a pre-malignant tumor or a malignant tumor.

In some embodiments, a Therapeutic Agent, a composition comprising a Therapeutic Agent, an Engineered Cell(s), a composition an Engineered Cell(s), or a combination therapy is administered to a mammal which is 0 to 6 months old, 6 to 12 months old, 1 to 5 years old, 5 to 10 years old, 10 to 15 years old, 15 to 20 years old, 20 to 25 years old, 25 to 30 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, a Therapeutic Agent, a composition comprising a Therapeutic Agent, an Engineered Cell(s), a composition an Engineered Cell(s), or a combination therapy is administered to a human at risk developing cancer. In some embodiments, a Therapeutic Agent, a composition comprising a Therapeutic Agent, an Engineered Cell(s), a composition an Engineered Cell(s), or a combination therapy is administered to a human with cancer. In some embodiments, the patient is a human 0 to 6 months old, 6 to 12 months old, 1 to 5 years old, 5 to 10 years old, 5 to 12 years old, 10 to 15 years old, 15 to 20 years old, 13 to 19 years old, 20 to 25 years old, 25 to 30 years old, 20 to 65 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, a Therapeutic Agent, a composition comprising a Therapeutic Agent, an Engineered Cell(s), a composition an Engineered Cell(s), or a combination therapy is administered to a human infant or a premature human infant. In other embodiments, a Therapeutic Agent, a composition comprising a Therapeutic Agent, an Engineered Cell(s), a composition an Engineered Cell(s), or a combination therapy is administered to a human child. In other embodiments, a Therapeutic Agent, a composition comprising a Therapeutic Agent, an Engineered Cell(s), a composition an Engineered Cell(s), or a combination therapy is administered to a human adult. In yet other embodiments, a Therapeutic Agent, a composition comprising a Therapeutic Agent, an Engineered Cell(s), a composition an Engineered Cell(s), or a combination therapy is administered to an elderly human.

In some embodiments, a Therapeutic Agent, a composition comprising a Therapeutic Agent, an Engineered Cell(s), a composition an Engineered Cell(s), or a combination therapy is administered to a pet, *e.g.,* a dog or cat. In some embodiments, a Therapeutic Agent, a composition comprising a Therapeutic Agent, an Engineered Cell(s), a composition an Engineered Cell(s), or a combination therapy is administered to a farm animal or livestock, *e.g.,* pig, cows, horses, chickens, *etc.*

In some embodiments, a Therapeutic Agent, a composition comprising a Therapeutic Agent, an Engineered Cell(s), a composition an Engineered Cell(s), or a combination therapy is administered to a primate, preferably a human, or another mammal, such as a pig, cow, horse, sheep, goat, dog, cat and rodent, in an immunocompromised state or immunosuppressed state or at risk for becoming immunocompromised or immunosuppressed. In some embodiments, a Therapeutic Agent, a composition comprising a Therapeutic Agent, an Engineered Cell(s), a composition an Engineered Cell(s), or a combination therapy is administered to a subject receiving or recovering from immunosuppressive therapy. In some embodiments, a Therapeutic Agent, a composition comprising a Therapeutic Agent, an Engineered Cell(s), a composition an Engineered Cell(s), or a combination therapy is administered to a subject that has or is at risk of getting AIDS, a viral infection, or a bacterial infection. In some embodiments, a subject that is, will or has undergone surgery, chemotherapy and/or radiation therapy. In some embodiments, a Therapeutic Agent, a composition comprising a Therapeutic Agent, an Engineered Cell(s), a composition an Engineered Cell(s), or a combination therapy is administered to a subject that lives in a nursing home, a group home (*i.e*., a home for 10 or more subjects), or a prison.

In some embodiments, a patient is administered a Therapeutic Agent, a composition comprising a Therapeutic Agent, an Engineered Cell(s), a composition an Engineered Cell(s), or a combination therapy is before any adverse effects or intolerance to therapies other than Therapeutic Agents or Engineered Cell(s) develops. In some embodiments, Therapeutic Agents, compositions comprising Therapeutic Agents, Engineered Cells, compositions comprising Engineered Cells or combination therapies are administered to refractory patients. In a some embodiment, refractory patient is a patient refractory to a standard anti-cancer therapy. In certain embodiments, a patient with cancer, is refractory to a therapy when the cancer has not significantly been eradicated and/or the symptoms have not been significantly alleviated. The determination of whether a patient is refractory can be made either *in vivo* or *in vitro* by any method known in the art for assaying the effectiveness of a treatment, using art-accepted meanings of "refractory" in such a context. In various embodiments, a patient with cancer is refractory when a cancerous tumor has not decreased or has increased.

In some embodiments, a Therapeutic Agent, a composition comprising a Therapeutic Agent, an Engineered Cell(s), a composition an Engineered Cell(s), or combination therapy(ies) is administered to a patient to prevent the onset or reoccurrence of cancer in a patient at risk of developing such cancer. In some embodiments, a Therapeutic Agent, a composition comprising a Therapeutic Agent, an Engineered Cell(s), a composition an Engineered Cell(s), or combination therapy(ies) is administered to a patient who are susceptible to adverse reactions to conventional therapies.

In some embodiments, one or more Therapeutic Agents, compositions comprising Therapeutic Agents, Engineered Cells, compositions comprising Engineered Cells, or combination therapies are administered to a patient who has proven refractory to therapies other than Therapeutic Agents or Engineered Cells, but are no longer on these therapies. In certain embodiments, the patients being managed or treated in accordance with the methods described herein are patients already being treated with antibiotics, anti-cancer agents, or other biological therapy/immunotherapy. Among these patients are refractory patients, patients who are too young for conventional therapies, and patients with reoccurring viral infections despite management or treatment with existing therapies.

In some embodiments, the subject being administered one or more Therapeutic Agents, compositions comprising Therapeutic Agents, Engineered Cells, compositions comprising Engineered Cells, or combination therapies has not received a therapy prior to the administration of the Therapeutic Agents, compositions comprising Therapeutic Agents, Engineered Cells, compositions comprising Engineered Cells, or combination therapies. In other embodiments, one or more Therapeutic Agents, compositions comprising Therapeutic Agents, Engineered Cells, compositions comprising Engineered Cells, or combination therapies are administered to a subject who has received a therapy prior to administration of one or more Therapeutic Agents, compositions comprising Therapeutic Agents, Engineered Cells, compositions comprising Engineered Cells, or combination therapies. In some embodiments, the subject administered a Therapeutic Agent, a composition comprising a Therapeutic Agent, an Engineered Cell(s), or a composition comprising an Engineered Cell(s) was refractory to a prior therapy or experienced adverse side effects to the prior therapy or the prior therapy was discontinued due to unacceptable levels of toxicity to the subject.

In certain embodiments, nucleic acids encoding an IL-15Ra polypeptide (and in certain embodiments, an IL-15), such as described in Section 5.3.2, *supra,* are administered to a patient described in this Section 5.7 with respect to Therapeutic Agents and Engineered Cells. In other words, instead of a Therapeutic Agent or an Engineered Cell(s) being administered to a patient described in this Section 5.7, the nucleic acids are administered to the patient. In certain embodiments, nucleic acids encoding an IL-15Ra polypeptide (and in certain embodiments, an IL-15), such as described in Section 5.3.2, *supra,* are administered to a patient with a cancer described in this Section 5.7. In some embodiments, nucleic acids encoding an IL-15Ra polypeptide (and in certain embodiments, an IL-15), such as described in Section 5.3.2, *supra,* are administered in combination with an additional therapy, such as described in this Section 5.7 and in Section 5.10, *infra.*

In some embodiments, an Engineered Cell is administered to a patient locally (e.g., into a tumor in the patient). In some of the embodiments in which an Engineered Cell is administered locally, such Engineered Cell recombinantly expresses an IL-15Ra polypeptide in which the cleavage site for an endogenous protease that cleaves native IL-15Ra has been mutated. In some embodiments, an Engineered Cell that is administered locally recombinantly expresses an IL-15Ra derivative comprising one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited. In other embodiments, an Engineered Cell that is administered locally recombinantly expresses an IL-15Ra derivative comprising (i) an extracellular domain of IL-15Ra in which the cleavage site for an endogenous protease that cleaves native IL-15Ra has been mutated, and (ii) all or a fragment of a transmembrane domain of a heterologous molecule in place of all or a fragment of the transmembrane domain of native IL-15Ra. In some embodiments, an Engineered Cell that is administered locally recombinantly expresses an IL-15Ra derivative comprising (i) an extracellular domain of IL-15Ra wherein one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited, and (ii) all or a fragment of a transmembrane domain of a heterologous molecule in place of all or a fragment of the transmembrane domain of native IL-15Ra. In yet other embodiments, an Engineered Cell that is administered locally is engineered to recombinantly express any of the IL-15Ra polypeptides described in Section 5.3.2., *supra,* in which the cleavage site for an endogenous protease that cleaves native IL-15Ra has been mutated (e.g., deleted) such that cleavage by an endogenous protease that cleaves native human IL-15Ra is inhibited. In yet other embodiments, an Engineered Cell that is administered locally is engineered to recombinantly express any of the IL-15Ra polypeptides described in Section 5.1 or Section 5.3.2., *supra.*

In some embodiments, a nucleic acid encoding an IL-15Ra derivative is administered to a patient locally (e.g., into a cancer cell in the patient). In some of the embodiments in which a nucleic acid encoding an IL-15Ra derivative is administered locally, the nucleic acid encodes an IL-15Ra polypeptide in which the cleavage site for an endogenous protease that cleaves native IL-15Ra has been mutated. In some embodiments, a nucleic acid encoding an IL-15Ra derivative that is administered locally encodes an IL-15Ra derivative comprising one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited. In other embodiments, a nucleic acid encoding an IL-15Ra derivative that is administered locally encodes an IL-15Ra derivative comprising (i) an extracellular domain of IL-15Ra in which the cleavage site for an endogenous protease that cleaves native IL-15Ra has been mutated, and (ii) all or a fragment of a transmembrane domain of a heterologous molecule in place of all or a fragment of the transmembrane domain of native IL-15Ra. In some embodiments, a nucleic acid encoding an IL-15Ra derivative that is administered locally encodes an IL-15Ra derivative comprising (i) an extracellular domain of IL-15Ra wherein one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited, and (ii) all or a fragment of a transmembrane domain of a heterologous molecule in place of all or a fragment of the transmembrane domain of native IL-15Ra. In yet other embodiments, a nucleic acid encoding an IL-15Ra derivative that is administered locally encodes any of the IL-15Ra polypeptides described in Section 5.3.2., *supra,* in which the cleavage site for an endogenous protease that cleaves native IL-15Ra has been mutated (e.g., deleted) such that cleavage by an endogenous protease that cleaves native human IL-15Ra is inhibited. In yet other embodiments, a nucleic acid encoding an IL-15Ra derivative that is administered locally encodes any of the IL-15Ra polypeptides described in Section 5.1 or Section 5.3.2., *supra.*

### 5.8 Infectious Diseases Treatment

Provided herein are methods for treating, preventing and/or managing an infectious disease in a subject, comprising administering an effective amount of a Therapeutic Agent or a composition comprising a Therapeutic Agent to a subject in need thereof. In an embodiment, the Therapeutic Agent is administered subcutaneously. In an embodiment, the cylical administration regimens described herein are used to prevent, treat and/or manage an infectious disease.

Also provided herein are methods for preventing, treating, and/or managing an infectious disease in a subject, comprising administering an effective amount of an Engineered Cell(s) or a composition comprising an Engineered Cell(s) to a subject in need thereof.

In an embodiment, the induced or enhanced immune response by administration of a Therapeutic Agent or an Engineered Cell(s) to a patient in accordance with the methods described herein is increased production in the patient of antibodies to the infected cells or to the antigens of the pathogen. In an embodiment, the induced or enhanced immune response by administration of a Therapeutic Agent or an Engineered Cell(s) to a patient in accordance with the methods described herein is increased production of antibodies to the pathogen. In another embodiment, the induced or enhanced immune response by administration of a Therapeutic Agent or an Engineered Cell(s) to a patient in accordance with the methods described herein is an increase in effector cell function, *e.g*., antibody-dependent cellular cytotoxicity (ADCC) against the pathogen and/or cells infected with a pathogen in the patient. In some embodiments, the induced or enhanced immune response by administration of a Therapeutic Agent or an Engineered Cell(s) to a patient in accordance with the methods described herein is increase in lymphocyte number, lymphocyte proliferation, and/or lymphocyte activity. In another embodiment, the induced or enhanced immune response by administration of a Therapeutic Agent or an Engineered Cell(s) to a patient in accordance with the methods described herein is an increase in effector cell function, *e.g*., cytotoxic cells or antibody-dependent cellular cytotoxicity (ADCC) against the infected cells in the patient.

In other embodiments, a Therapeutic Agent or an Engineered Cell(s) can be administered in combination with one or more other therapies. Non-limiting examples of other therapies that can be used in combination with Therapeutic Agents or Engineered Cells are described herein. *See* Section 5.10, *infra,* and in particular, Sections 5.10.2 and 5.10.3, *infra.* In one embodiment, the combination of a Therapeutic Agent or an Engineered Cell(s) and one or more other therapies provides an additive therapeutic effect relative to the therapeutic effects of the Therapeutic Agent or Engineered Cell(s) alone or the one or more other therapies alone. In one embodiment, the combination of a Therapeutic Agent or an Engineered Cell(s) and one or more other therapies provides more than an additive therapeutic effect relative to the therapeutic effects of the Therapeutic Agent or Engineered Cell(s) alone or the one or more other therapies alone. In one embodiment, the combination of a Therapeutic Agent or an Engineered Cell(s) and one or more other therapies provides a synergistic therapeutic effect relative to the therapeutic effects of the Therapeutic Agent or Engineered Cell(s) alone or the one or more other therapies alone.

Infectious diseases that can be treated, prevented, and/or managed by Therapeutic Agents are caused by infectious agents including but not limited to bacteria, fungi, protozae, and viruses. Infectious diseases that can be treated, prevented, and/or managed by Engineered Cells are caused by infectious agents including but not limited to bacteria, fungi, protozae, and viruses.

Viral diseases that can be prevented, treated and/or managed in accordance with the methods described herein include, but are not limited to, those caused by hepatitis type A, hepatitis type B, hepatitis type C, influenza, varicella, adenovirus, herpes simplex type I (HSV-I), herpes simplex type II (HSV-II), rinderpest, rhinovirus, echovirus, rotavirus, respiratory syncytial virus, papilloma virus, papova virus, cytomegalovirus, echinovirus, arbovirus, huntavirus, coxsackie virus, mumps virus, measles virus, rubella virus, polio virus, small pox, Epstein Barr virus, human immunodeficiency virus type I (HIV-I), human immunodeficiency virus type II (HIV-II), and agents of viral diseases such as viral miningitis, encephalitis, dengue or small pox.

Bacterial diseases caused by bacteria (*e.g., Escherichia coli, Klebsiella pneumoniae, Staphylococcus aureus, Enterococcus faecials, Candida albicans, Proteus vulgaris, Staphylococcus viridans,* and *Pseudomonas aeruginosa*) that can be prevented, treated and/or managed in accordance with the methods described herein include, but are not limited to, *mycobacteria rickettsia, mycoplasma, neisseria, S. pneumonia, Borrelia burgdorferi* (Lyme disease), *Bacillus antracis* (anthrax), *tetanus, streptococcus, staphylococcus, mycobacterium, pertissus, cholera, plague, diptheria, chlamydia, S. aureus* and *legionella.*

Protozoal diseases caused by protozoa that can be prevented, treated and/or managed in accordance with the methods described herein include, but are not limited to, leishmania, kokzidioa, trypanosoma or malaria.

Parasitic diseases caused by parasites that can be prevented, treated and/or managed in accordance with the methods described herein include, but are not limited to, chlamydia and rickettsia.

In some embodiments, administering a Therapeutic Agent, a composition comprising a Therapeutic Agent, an Engineered Cell(s), or a composition comprising an Engineered Cell(s) to a subject (in some embodiments, an animal model) infected with an infectious agent in accordance with the methods described herein inhibits or reduces replication of the infectious agent by at least 20% to 25%, preferably at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, or up to at least 85% relative to a negative control as determined using an assay described herein or others known to one of skill in the art. In some embodiments, administering a Therapeutic Agent, a composition comprising a Therapeutic Agent, an Engineered Cell(s), or a composition comprising an Engineered Cell(s) to a subject (in some embodiments, an animal model) infected with an infectious agent in accordance with the methods described herein inhibits or reduces replication of the infectious agent by at least 1.5 fold, 2 fold, 2.5 fold, 3 fold, 4 fold, 5 fold, 8 fold, 10 fold, 15 fold, 20 fold, or 2 to 5 fold, 2 to 10 fold, 5 to 10 fold, or 5 to 20 fold relative to a negative control as determined using an assay described herein or others known to one of skill in the art. In other embodiments, administering a Therapeutic Agent, a composition comprising a Therapeutic Agent, an Engineered Cell(s), or a composition comprising an Engineered Cell(s) to a subject (in some embodiments, an animal model) infected with an infectious agent in accordance with the methods described herein inhibits or reduces replication of the infectious agent by 1 log, 1.5 logs, 2 logs, 2.5 logs, 3 logs, 3.5 logs, 4 logs, 5 logs or more relative to a negative control as determined using an assay described herein or others known to one of skill in the art.

In some embodiments, administering a Therapeutic Agent, a composition comprising a Therapeutic Agent, an Engineered Cell(s), or a composition comprising an Engineered Cell(s) to a subject (in some embodiments, an animal model) infected with an infectious agent in accordance with the methods described herein reduces the titer of the infectious agent by at least 20% to 25%, preferably at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, or up to at least 85% relative to a negative control as determined using an assay described herein or others known to one of skill in the art. In some embodiments, administering a Therapeutic Agent, a composition comprising a Therapeutic Agent, an Engineered Cell(s), or a composition comprising an Engineered Cell(s) to a subject (in some embodiments, an animal model) infected with an infectious agent in accordance with the methods described herein reduces the titer of the infectious agent by at least 1.5 fold, 2 fold, 2.5 fold, 3 fold, 4 fold, 5 fold, 8 fold, 10 fold, 15 fold, 20 fold, or 2 to 5 fold, 2 to 10 fold, 5 to 10 fold, or 5 to 20 fold relative to a negative control as determined using an assay described herein or others known to one of skill in the art. In other embodiments, administering aTherapeutic Agent, a composition comprising a Therapeutic Agent, an Engineered Cell(s), or a composition comprising an Engineered Cell(s) to a subject (in some embodiments, an animal model) infected with an infectious agent in accordance with the methods described herein reduces the titer of the infectious agent by 1 log, 1.5 logs, 2 logs, 2.5 logs, 3 logs, 3.5 logs, 4 logs, 5 logs or more relative to a negative control as determined using an assay described herein or others known to one of skill in the art.

In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject suffering from an infectious disease caused by infectious agents including, but not limited to bacteria, fungi, protozae, and viruses. In other embodiments, a Therapeutic Agent(s), a composition(s) comprising Therapeutic Agent(s), an Engineered Cell(s), or a composition(s) comprising an Engineered Cell(s),or a combination therapy(ies) is administered to a subject predisposed or susceptible to an infectious disease. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that lives in a region where there has been or might be an outbreak with infections by infectious agents. In some embodiments, the infection is a latent infection. In other embodiments, the infection by the infectious agent is an active infection. In yet other embodiments, the infection by the infectious agent is a chronic viral infection. In an embodiment, the infection is a viral infection. In a specific embodiment, the virus infects humans.

In some embodiments, a Therapeutic Agent(s), a composition(s) comprising Therapeutic Agent(s), an Engineered Cell(s), or a composition(s) comprising an Engineered Cell(s),or a combination therapy(ies) is administered to a mammal which is 0 to 6 months old, 6 to 12 months old, 1 to 5 years old, 5 to 10 years old, 10 to 15 years old, 15 to 20 years old, 20 to 25 years old, 25 to 30 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a human at risk for a virus infection. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a human with a virus infection. In some embodiments, the patient is a human 0 to 6 months old, 6 to 12 months old, 1 to 5 years old, 5 to 10 years old, 5 to 12 years old, 10 to 15 years old, 15 to 20 years old, 13 to 19 years old, 20 to 25 years old, 25 to 30 years old, 20 to 65 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a human infant or premature human infant. In other embodiments, a Therapeutic Agent(s), a composition(s) comprising Therapeutic Agent(s), an Engineered Cell(s), or a composition(s) comprising an Engineered Cell(s),or a combination therapy(ies) is administered to a human child. In other embodiments, a Therapeutic Agent(s), a composition(s) comprising Therapeutic Agent(s), an Engineered Cell(s), or a composition(s) comprising an Engineered Cell(s),or a combination therapy(ies) is administered to a human adult. In yet other embodiments, a Therapeutic Agent(s), a composition(s) comprising Therapeutic Agent(s), an Engineered Cell(s), or a composition(s) comprising an Engineered Cell(s),or a combination therapy(ies) is administered to an elderly human.

In some embodiments, a Therapeutic Agent(s), a composition(s) comprising Therapeutic Agent(s), an Engineered Cell(s), or a composition(s) comprising an Engineered Cell(s),or a combination therapy(ies) is administered to a pet, *e.g.,* a dog or cat. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a farm animal or livestock, *e.g*., pig, cows, horses, chickens, etc. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a bird, *e.g*., ducks or chicken.

In some embodiments, a Therapeutic Agent(s), a composition(s) comprising Therapeutic Agent(s), an Engineered Cell(s), or a composition(s) comprising an Engineered Cell(s),or a combination therapy(ies) is administered to a primate, preferably a human, or another mammal, such as a pig, cow, horse, sheep, goat, dog, cat and rodent, in an immunocompromised state or immunosuppressed state or at risk for becoming immunocompromised or immunosuppressed. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject receiving or recovering from immunosuppressive therapy. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that has or is at risk of getting cancer, AIDS, another infection, or a bacterial infection. In some embodiments, a subject that is, will or has undergone surgery, chemotherapy and/or radiation therapy. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that has cystic fibrosis, pulmonary fibrosis, or another disease which makes the subject susceptible to an infection. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that has, will have or had a tissue transplant. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that lives in a nursing home, a group home (*i.e*., a home for 10 or more subjects), or a prison. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that attends school (*e.g*., elementary school, middle school, junior high school, high school or university) or daycare. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that works in the healthcare area, such as a doctor or a nurse, or in a hospital. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a subject that is pregnant or will become pregnant.

In some embodiments, a patient is administered a Therapeutic Agent(s), a composition(s) comprising Therapeutic Agent(s), an Engineered Cell(s), or a composition(s) comprising an Engineered Cell(s),or a combination therapy(ies) before any adverse effects or intolerance to therapies other than Therapeutic Agents or Engineered Cells develops. In some embodiments, a Therapeutic Agent(s), a composition(s) comprising Therapeutic Agent(s), an Engineered Cell(s), or a composition(s) comprising an Engineered Cell(s),or a combination therapy(ies) is administered to refractory patients. In a some embodiment, refractory patient is a patient refractory to a standard therapy. In some embodiments, a patient with an infection, is refractory to a therapy when the infection has not significantly been eradicated and/or the symptoms have not been significantly alleviated. The determination of whether a patient is refractory can be made either *in vivo* or *in vitro* by any method known in the art for assaying the effectiveness of a treatment of infections, using art-accepted meanings of "refractory" in such a context. In various embodiments, a patient with an infection is refractory when replication of the infectious agent has not decreased or has increased.

In some embodiments, a Therapeutic Agent(s), a composition(s) comprising Therapeutic Agent(s), an Engineered Cell(s), or a composition(s) comprising an Engineered Cell(s),or a combination therapy(ies) is administered to a patient to prevent the onset or reoccurrence of infections (*e.g*., viral infections) in a patient at risk of developing such infections. In some embodiments, a Therapeutic Agent(s), a composition(s) comprising Therapeutic Agent(s), an Engineered Cell(s), or a composition(s) comprising an Engineered Cell(s),or a combination therapy(ies) is administered to a patient who are susceptible to adverse reactions to conventional therapies.

In some embodiments, one or more Therapeutic Agents, compositions comprising Therapeutic Agents, Engineered Cells, compositions comprising Engineered Cells, or combination therapies are administered to a patient who has proven refractory to therapies other than Therapeutic Agents or Engineered Cells, but are no longer on these therapies. In some embodiments, the patients being managed or treated in accordance with the methods of this invention are patients already being treated with antibiotics, anti-virals, anti-fungals, or other biological therapy/immunotherapy. Among these patients are refractory patients, patients who are too young for conventional therapies, and patients with reoccurring viral infections despite management or treatment with existing therapies.

In some embodiments, the subject being administered one or more Therapeutic Agents, compositions comprising Therapeutic Agents, Engineered Cells, compositiosn comprising Engineered Cells, or combination therapies has not received a therapy prior to the administration of the Therapeutic Agents, compositions comprising Therapeutic Agents, Engineered Cells, compositions comprising the Engineered Cells or combination therapies. In other embodiments, one or more Therapeutic Agents, compositions comprising Therapeutic Agents, Engineered Cells, compositions comprising the Engineered Cells, or combination therapies are administered to a subject who has received a therapy prior to administration of one or more Therapeutic Agents or compositions comprising one or more Therapeutic Agents, Engineered Cells, compositions comprising the Engineered Cells, or combination therapies. In some embodiments, the subject administered a Therapeutic Agent, a composition comprising a Therapeutic Agent, an Engineered Cell(s), or a composition comprising an Engineered Cell(s) was refractory to a prior therapy or experienced adverse side effects to the prior therapy or the prior therapy was discontinued due to unacceptable levels of toxicity to the subject.

In some embodiments, nucleic acids encoding an IL-15Ra polypeptide (and in some embodiments, an IL-15), such as described in Section 5.3.2, *supra,* are administered to a patient described in this Section 5.8 with respect to Therapeutic Agents and Engineered Cells. In other words, instead of a Therapeutic Agent or an Engineered Cell(s) being administered to a patient described in this Section 5.8, the nucleic acids are administered to the patient. In some embodiments, nucleic acids encoding an IL-15Ra polypeptide (and in some embodiments, an IL-15), such as described in Section 5.3.2, *supra,* are administered to a patient with an infectious disease described in this Section 5.8. In some embodiments, nucleic acids encoding an IL-15Ra polypeptide (and in some embodiments, an IL-15), such as described in Section 5.3.2, *supra,* are administered in combination with an additional therapy, such as described in this Section 5.8 and in Section 5.10, *infra.*

In some embodiments, an Engineered Cell is administered to a patient locally (e.g., into the site of infection). In some of the embodiments in which an Engineered Cell is administered locally, such Engineered Cell recombinantly expresses an IL-15Ra polypeptide in which the cleavage site for an endogenous protease that cleaves native IL-15Ra has been mutated. In some embodiments, an Engineered Cell that is administered locally recombinantly expresses an IL-15Ra derivative comprising one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited. In other embodiments, an Engineered Cell that is administered locally recombinantly expresses an IL-15Ra derivative comprising (i) an extracellular domain of IL-15Ra in which the cleavage site for an endogenous protease that cleaves native IL-15Ra has been mutated, and (ii) all or a fragment of a transmembrane domain of a heterologous molecule in place of all or a fragment of the transmembrane domain of native IL-15Ra. In some embodiments, an Engineered Cell that is administered locally recombinantly expresses an IL-15Ra derivative comprising (i) an extracellular domain of IL-15Ra wherein one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited, and (ii) all or a fragment of a transmembrane domain of a heterologous molecule in place of all or a fragment of the transmembrane domain of native IL-15Ra. In yet other embodiments, an Engineered Cell that is administered locally is engineered to recombinantly express any of the IL-15Ra polypeptides described in Section 5.3.2., *supra,* in which the cleavage site for an endogenous protease that cleaves native IL-15Ra has been mutated (e.g., deleted) such that cleavage by an endogenous protease that cleaves native human IL-15Ra is inhibited. In yet other embodiments, an Engineered Cell that is administered locally is engineered to recombinantly express any of the IL-15Ra polypeptides described in Section 5.1 or Section 5.3.2., *supra.*

In some embodiments, a nucleic acid encoding an IL-15Ra derivative is administered to a patient locally (e.g., into the site of infection or into an infected cell in the patient). In some of the embodiments in which a nucleic acid encoding an IL-15Ra derivative is administered locally, the nucleic acid encodes an IL-15Ra polypeptide in which the cleavage site for an endogenous protease that cleaves native IL-15Ra has been mutated. In some embodiments, a nucleic acid encoding an IL-15Ra derivative that is administered locally encodes an IL-15Ra derivative comprising one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited. In other embodiments, a nucleic acid encoding an IL-15Ra derivative that is administered locally encodes an IL-15Ra derivative comprising (i) an extracellular domain of IL-15Ra in which the cleavage site for an endogenous protease that cleaves native IL-15Ra has been mutated, and (ii) all or a fragment of a transmembrane domain of a heterologous molecule in place of all or a fragment of the transmembrane domain of native IL-15Ra. In some embodiments, a nucleic acid encoding an IL-15Ra derivative that is administered locally encodes an IL-15Ra derivative comprising (i) an extracellular domain of IL-15Ra wherein one, two, three, four, five, six, seven or eight mutations (e.g., additions, substitutions or deletions, such as substitutions or deletions in one, two, three, four, five, six, seven or eight amino acid residues) in SEQ ID NO:26 such that cleavage by an endogenous protease that cleaves native human IL-15Ra is inhibited, and (ii) all or a fragment of a transmembrane domain of a heterologous molecule in place of all or a fragment of the transmembrane domain of native IL-15Ra. In yet other embodiments, a nucleic acid encoding an IL-15Ra derivative that is administered locally encodes any of the IL-15Ra polypeptides described in Section 5.3.2., *supra,* in which the cleavage site for an endogenous protease that cleaves native IL-15Ra has been mutated (e.g., deleted) such that cleavage by an endogenous protease that cleaves native human IL-15Ra is inhibited. In yet other embodiments, a nucleic acid encoding an IL-15Ra derivative that is administered locally encodes any of the IL-15Ra polypeptides described in Section 5.1 or Section 5.3.2., *supra.*

### 5.9 Immunodeficiencies & Lymhopenia

Provided herein are methods for treating, preventing and/or managing an immunodeficiency or lymphopenia in a subject, comprising administering an effective amount of a Therapeutic Agent or a composition comprising a Therapeutic Agent to a subject in need thereof. In an embodiment, the Therapeutic Agent is administered subcutaneously. In an embodiment, the cylical administration regimens described herein are used to prevent, treat and/or manage an immunodeficiency or lymphopenia.

Also provided herein are methods for preventing, treating, and/or managing an immunodeficiency or lymphopenia in a subject, comprising administering an effective amount of an Engineered Cell(s) or a composition comprising an Engineered Cell(s) to a subject in need thereof.

In other embodiments, a Therapeutic Agent or an Engineered Cell(s) can be administered in combination with one or more other therapies. Non-limiting examples of other therapies that can be used in combination with Therapeutic Agents or Engineered Cells are described herein. *See* Section 5.10, *infra.* In one embodiment, the combination of a Therapeutic Agent or an Engineered Cell(s) and one or more other therapies provides an additive therapeutic effect relative to the therapeutic effects of the Therapeutic Agent or Engineered Cell(s) alone or the one or more other therapies alone. In one embodiment, the combination of a Therapeutic Agent or an Engineered Cell(s) and one or more other therapies provides more than an additive therapeutic effect relative to the therapeutic effects of the Therapeutic Agent or Engineered Cell(s) alone or the one or more other therapies alone. In one embodiment, the combination of a Therapeutic Agent or an Engineered Cell(s) and one or more other therapies provides a synergistic therapeutic effect relative to the therapeutic effects of the Therapeutic Agent or Engineered Cell(s) alone or the one or more other therapies alone.

In some embodiments, a Therapeutic Agent, a composition comprising a Therapeutic Agent, an Engineered Cell(s), a composition comprising an Engineered Cell(s), or a combination therapy is administered to a mammal which is 0 to 6 months old, 6 to 12 months old, 1 to 5 years old, 5 to 10 years old, 10 to 15 years old, 15 to 20 years old, 20 to 25 years old, 25 to 30 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, the patient is a human 0 to 6 months old, 6 to 12 months old, 1 to 5 years old, 5 to 10 years old, 5 to 12 years old, 10 to 15 years old, 15 to 20 years old, 13 to 19 years old, 20 to 25 years old, 25 to 30 years old, 20 to 65 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, a Therapeutic Agent, a composition comprising a Therapeutic Agent, an Engineered Cell(s), a composition comprising an Engineered Cell(s), or a combination therapy is administered to a human infant or premature human infant. In other embodiments, a Therapeutic Agent, composition comprising a Therapeutic Agent, an Engineered Cell(s), a composition comprising an Engineered Cell(s), or a combination therapy is administered to a human child. In other embodiments, a Therapeutic Agent, composition comprising a Therapeutic Agent, an Engineered Cell(s), a composition comprising an Engineered Cell(s), or a combination therapy is administered to a human adult. In yet other embodiments, a Therapeutic Agent, a composition comprising a Therapeutic Agent, an Engineered Cell(s), a composition comprising an Engineered Cell(s), or a combination therapy is administered to an elderly human.

In some embodiments, a Therapeutic Agent, a composition comprising a Therapeutic Agent, an Engineered Cell(s), a composition comprising an Engineered Cell(s), or a combination therapy is administered to a pet, *e.g.,* a dog or cat. In some embodiments, a Therapeutic Agent, a composition comprising a Therapeutic Agent, an Engineered Cell(s), a composition comprising an Engineered Cell(s), or a combination therapy is administered to a farm animal or livestock, *e.g*., pig, cows, horses, chickens, etc. In some embodiments, a Therapeutic Agent, a composition comprising a Therapeutic Agent, an Engineered Cell(s), a composition comprising an Engineered Cell(s), or a combination therapy is administered to a bird, *e.g*., ducks or chicken.

In some embodiments, a Therapeutic Agent, a composition comprising a Therapeutic Agent, an Engineered Cell(s), a composition comprising an Engineered Cell(s), or a combination therapy is administered to a primate, preferably a human, or another mammal, such as a pig, cow, horse, sheep, goat, dog, cat and rodent, in an immunocompromised state or immunosuppressed state or at risk for becoming immunocompromised or immunosuppressed. In some embodiments, a Therapeutic Agent, a composition comprising a Therapeutic Agent, an Engineered Cell(s), a composition comprising an Engineered Cell(s), or a combination therapy is administered to a primate, preferably a human, or another mammal, such as a pig, cow, horse, sheep, goat, dog, cat and rodent, with an immunodeficiency. In some embodiments, a Therapeutic Agent, a composition comprising a Therapeutic Agent, an Engineered Cell(s), a composition comprising an Engineered Cell(s), or a combination therapy is administered to a subject receiving or recovering from immunosuppressive therapy. In some embodiments, a Therapeutic Agent, a composition comprising a Therapeutic Agent, an Engineered Cell(s), a composition comprising an Engineered Cell(s), or a combination therapy is administered to a subject that has or is at risk of getting cancer, AIDS, another infection, or a bacterial infection. In some embodiments, a subject that is, will or has undergone surgery, chemotherapy and/or radiation therapy. In some embodiments, a Therapeutic Agent, a composition comprising a Therapeutic Agent, an Engineered Cell(s), a composition comprising an Engineered Cell(s), or a combination therapy is administered to a subject that has, will have or had a tissue transplant. In some embodiments, a Therapeutic Agent, a composition comprising a Therapeutic Agent, an Engineered Cell(s), a composition comprising an Engineered Cell(s), a combination therapy is administered to a subject that lives in a nursing home, a group home (*i.e*., a home for 10 or more subjects), or a prison. In some embodiments, a Therapeutic Agent, a composition comprising a Therapeutic Agent, an Engineered Cell(s), a composition comprising an Engineered Cell(s), or a combination therapy is administered to a subject that attends school (*e.g*., elementary school, middle school, junior high school, high school or university) or daycare. In some embodiments, a Therapeutic Agent, a composition comprising a Therapeutic Agent, an Engineered Cell(s), a composition comprising an Engineered Cell(s), or a combination therapy is administered to a subject that works in the healthcare area, such as a doctor or a nurse, or in a hospital. In some embodiments, a Therapeutic Agent, a composition comprising a Therapeutic Agent, an Engineered Cell(s), a composition comprising an Engineered Cell(s), or a combination therapy is administered to a subject that is pregnant or will become pregnant.

In some embodiments, a Therapeutic Agent, a composition comprising a Therapeutic Agent, an Engineered Cell(s), a composition comprising an Engineered Cell(s), or a combination therapy is administered to a subject that has been diagnosed as lymphopenic. The terms "lymphopenia" or "lymphocytopenia" or "lymphocytic leucopenia" interchangeably refer to an abnormally small number of lymphocytes in the circulating blood or in peripheral circulation. Quantitatively, lymphopenia can be described by various cutoffs. In some embodiments, a patient is suffering from lymphopenia when their circulating blood total lymphocyte count falls below about 600/mm³. In some embodiments, a patient suffering from lymphopenia has less than about 2000/µL total circulating lymphocytes at birth, less than about 4500/µL total circulating lymphocytes at about age 9 months, or less than about 1000/µL total circulating lymphocytes patients older than about 9 months.

Lymphocytopenia has a wide range of possible causes, including viral (*e.g*., HIV or hepatitis infection), bacterial (*e.g*., active tuberculosis infection), and fungal infections; chronic failure of the right ventricle of the heart, Hodgkin's disease and cancers of the lymphatic system, leukemia, a leak or rupture in the thoracic duct, side effects of prescription medications including anticancer agents, antiviral agents, and glucocorticoids, malnutrition resulting from diets that are low in protein, radiation therapy, uremia, autoimmune disorders, immune deficiency syndromes, high stress levels, and trauma. Lymphopenia may also be of unknown etiology (*i.e*., idiopathic lymphopenia). Peripheral circulation of all types of lymphocytes or subpopulations of lymphocytes (*e.g*., CD4⁺ T cells) may be depleted or abnormally low in a patient suffering from lymphopenia. *See, e.g.,* The Merck Manual, 18th Edition, 2006, Merck & Co.

In some embodiments, a patient is administered a Therapeutic Agent, a composition comprising a Therapeutic Agent, an Engineered Cell(s), a composition comprising an Engineered Cell(s), or a combination therapy before any adverse effects or intolerance to therapies other than Therapeutic Agents or Engineered Cells develops. In some embodiments, Therapeutic Agents, compositions comprising Therapeutic Agents, Engineered Cells, compositions comprising Engineered Cells, or combination therapies are administered to refractory patients. In an embodiment, refractory patient is a patient refractory to a standard therapy. In some embodiments, a patient with an immunodeficiency or lymphopenia, is refractory to a therapy when the infection has not significantly been eradicated and/or the symptoms have not been significantly alleviated. The determination of whether a patient is refractory can be made either *in vivo* or *in vitro* by any method known in the art for assaying the effectiveness of a treatment of infections, using art-accepted meanings of "refractory" in such a context. In various embodiments, a patient with an infection is refractory when replication of the infectious agent has not decreased or has increased.

In some embodiments, Therapeutic Agents, compositions comprising Therapeutic Agents, Engineered Cells, compositions comprising Engineered Cells, or combination therapies are administered to a patient to prevent the onset or reoccurrence of an immunodeficiency or lymphopenia in a patient at risk of developing such infections. In some embodiments, Therapeutic Agents, compositions comprising Therapeutic Agents, Engineered Cells, compositions comprising Engineered Cells, or combination therapies are administered to a patient who are susceptible to adverse reactions to conventional therapies. In some embodiments, one or more Therapeutic Agents, compositions comprising Therapeutic Agents, Engineered Cells, compositions comprising Engineered Cells, or combination therapies are administered to a patient who has proven refractory to therapies other than Therapeutic Agents or Engineered Cells, but are no longer on these therapies.

In some embodiments, the subject being administered one or more Therapeutic Agents, compositions comprising Therapeutic Agents, Engineered Cells, compositions comprising Engineered Cells, or combination therapies has not received a therapy prior to the administration of the Therapeutic Agents, compositions comprising Therapeutic Agents, Engineered Cells, compositions comprising Engineered Cells, or combination therapies. In other embodiments, one or more Therapeutic Agents, compositions comprising Therapeutic Agents, Engineered Cells, compositions comprising Engineered Cells, or combination therapies are administered to a subject who has received a therapy prior to administration of one or more Therapeutic Agents, compositions comprising Therapeutic Agents, Engineered Cells, compositions comprising Engineered Cells, or combination therapies. In some embodiments, the subject administered a Therapeutic Agent, a composition comprising a Therapeutic Agent, an Engineered Cell(s), or a composition comprising an Engineered Cell(s) was refractory to a prior therapy or experienced adverse side effects to the prior therapy or the prior therapy was discontinued due to unacceptable levels of toxicity to the subject.

In some embodiments, nucleic acids encoding an IL-15Ra polypeptide (and in some embodiments, an IL-15), such as described in Section 5.3.2, *supra,* are administered to a patient described in this Section 5.9 with respect to Therapeutic Agents and Engineered Cells. In other words, instead of a Therapeutic Agent or an Engineered Cell(s) being administered to a patient described in this Section 5.9, the nucleic acids are administered to the patient. In some embodiments, nucleic acids encoding an IL-15Ra polypeptide (and in some embodiments, an IL-15), such as described in Section 5.3.2, *supra,* are administered to a patient with an immunodeficiency or lymphopenia described in this Section 5.9. In some embodiments, nucleic acids encoding an IL-15Ra polypeptide (and in some embodiments, an IL-15), such as described in Section 5.3.2, *supra,* are administered in combination with an additional therapy, such as described in this Section 5.9 and in Section 5.10, *infra.*

### 5.10 Additional/Combination Therapy

Other therapies that can be used in combination with a Therapeutic Agent(s), Engineered Cell(s), or nucleic acids encoding an IL-15Ra (and in some embodiments, an IL-15 polypeptide) for the prevention, treatment and/or management of a disease that is affected by IL-15 function/signaling, *e.g*., cancer, infectious disease, lymphopenia, immunodeficiency and wounds, include, but are not limited to, small molecules, synthetic drugs, peptides (including cyclic peptides), polypeptides, proteins, nucleic acids (*e.g*., DNA and RNA nucleotides including, but not limited to, antisense nucleotide sequences, triple helices, RNAi, and nucleotide sequences encoding biologically active proteins, polypeptides or peptides), antibodies, synthetic or natural inorganic molecules, mimetic agents, and synthetic or natural organic molecules. Some examples of such therapies include, but are not limited to, immunomodulatory agents (*e.g*., interferon), anti-inflammatory agents (*e.g.,* adrenocorticoids, corticosteroids (*e.g*., beclomethasone, budesonide, flunisolide, fluticasone, triamcinolone, methylprednisolone, prednisolone, prednisone, hydrocortisone), glucocorticoids, steriods, and non-steriodal anti- inflammatory drugs (*e.g*., aspirin, ibuprofen, diclofenac, and COX-2 inhibitors), pain relievers, leukotreine antagonists (*e.g*., montelukast, methyl xanthines, zafirlukast, and zileuton), beta2-agonists (*e.g*., albuterol, biterol, fenoterol, isoetharie, metaproterenol, pirbuterol, salbutamol, terbutalin formoterol, salmeterol, and salbutamol terbutaline), anticholinergic agents (*e.g*., ipratropium bromide and oxitropium bromide), sulphasalazine, penicillamine, dapsone, antihistamines, anti-malarial agents (*e.g*., hydroxychloroquine), anti-viral agents (*e.g*., nucleoside analogs (*e.g*., zidovudine, acyclovir, gangcyclovir, vidarabine, idoxuridine, trifluridine, and ribavirin), foscarnet, amantadine, rimantadine, saquinavir, indinavir, ritonavir, and AZT) and antibiotics (*e.g*., dactinomycin (formerly actinomycin), bleomycin, erythomycin, penicillin, mithramycin, and anthramycin (AMC)).

Any therapy which is known to be useful, or which has been used or is currently being used for the prevention, management, and/or treatment of a disease that is affected by IL-15 function/signaling can be used in combination with a Therapeutic Agent(s), an Engineered Cell(s), or nucleic acids encoding an IL-15Ra (and in some embodiments, an IL-15 polypeptide). See, *e.g.,* Gilman et al., Goodman and Gilman's: The Pharmacological Basis of Therapeutics, 10th ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; Cecil Textbook of Medicine, 20th Ed., Bennett and Plum (eds.), W.B. Saunders, Philadelphia, 1996, and Physicians' Desk Reference (66th ed. 2012) for information regarding therapies (*e.g*., prophylactic or therapeutic agents) which have been or are currently being used for preventing, treating and/or managing disease or disorder that is affected by IL-15 function/signaling, *e.g*., cancer, infectious disease, lymphopenia, immunodeficiency and wounds.

### 5.10.1 Anti-Cancer Agents

Non-limiting examples of one or more other therapies that can be used in combination with a Therapeutic Agent(s), an Engineered Cell(s), or nucleic acids encoding an IL-15Ra (and in some embodiments, an IL-15 polypeptide) include immunomodulatory agents, such as but not limited to, chemotherapeutic agents and non-chemotherapeutic immunomodulatory agents. Non-limiting examples of chemotherapeutic agents include methotrexate, cyclosporin A, leflunomide, cisplatin, ifosfamide, taxanes such as taxol and paclitaxol, topoisomerase I inhibitors (*e.g*., CPT-11, topotecan, 9-AC, and GG-211), gemcitabine, vinorelbine, oxaliplatin, 5-fluorouracil (5-FU), leucovorin, vinorelbine, temodal, cytochalasin B, gramicidin D, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin homologs, and cytoxan. Examples of non-chemotherapeutic immunomodulatory agents include, but are not limited to, anti-T cell receptor antibodies (*e.g*., anti-CD4 antibodies (*e.g*., cM-T412 (Boeringer), IDEC-CE9.1® (IDEC and SKB), mAB 4162W94, Orthoclone and OKTcdr4a (Janssen-Cilag)), anti-CD3 antibodies (*e.g*., Nuvion (Product Design Labs), OKT3 (Johnson & Johnson), or Rituxan (IDEC)), anti-CD5 antibodies (*e.g*., an anti-CD5 ricin-linked immunoconjugate), anti-CD7 antibodies (e.g., CHH-380 (Novartis)), anti-CD8 antibodies, anti-CD40 ligand monoclonal antibodies (*e.g*., IDEC-131 (IDEC)), anti-CD52 antibodies (*e.g*., CAMPATH 1H (Ilex)), anti-CD2 antibodies (*e.g*., MEDI-507 (Medlmmune, Inc., International Publication Nos. WO 02/098370 and WO 02/069904), anti-CD 11a antibodies (*e.g*., Xanelim (Genentech)), and anti-B7 antibodies (*e.g*., IDEC-114) (IDEC)); anti-cytokine receptor antibodies (*e.g*., anti-IFN receptor antibodies, anti-IL-2 receptor antibodies (*e.g*., Zenapax (Protein Design Labs)), anti-IL-4 receptor antibodies, anti-IL-6 receptor antibodies, anti-IL-10 receptor antibodies, and anti-IL-12 receptor antibodies), anti-cytokine antibodies (*e.g*., anti-IFN antibodies, anti-TNF-α antibodies, anti-IL-1β antibodies, anti-IL-6 antibodies, anti-IL-8 antibodies (*e.g*., ABX-IL-8 (Abgenix)), anti-IL-12 antibodies and anti-IL-23 antibodies)); CTLA4-immunoglobulin; LFA-3TIP (Biogen, International Publication No. WO 93/08656 and U.S. Patent No. 6,162,432); soluble cytokine receptors (*e.g*., the extracellular domain of a TNF-α receptor or a fragment thereof, the extracellular domain of an IL-1β receptor or a fragment thereof, and the extracellular domain of an IL-6 receptor or a fragment thereof); cytokines or fragments thereof (*e.g*., interleukin (IL)-2, IL-3, IL-4, IL-5, IL-6, IL-7, IL-8, IL-9, IL-10, IL-11, IL-12, IL-15, IL-23, TNF-α, TNF-β, interferon (IFN)-α, IFN-β, IFN-γ, and GM-CSF); and anti-cytokine antibodies (*e.g*., anti-IL-2 antibodies, anti-IL-4 antibodies, anti-IL-6 antibodies, anti-IL-10 antibodies, anti-IL-12 antibodies, anti-IL-15 antibodies, anti-TNF-α antibodies, and anti-IFN-γ antibodies), and antibodies that immunospecifically bind to tumor-associated antigens (*e.g*., Herceptin®). In some embodiments, an immunomodulatory agent is an immunomodulatory agent other than a chemotherapeutic agent. In other embodiments an immunomodulatory agent is an immunomodulatory agent other than a cytokine or hemapoietic such as IL-1, IL-2, IL-4, IL-12, IL-15, TNF, IFN-α, IFN-β, IFN-γ, M-CSF, G-CSF, IL-3 or erythropoietin. In yet other embodiments, an immunomodulatory agent is an agent other than a chemotherapeutic agent and a cytokine or hemapoietic factor.

Non-limiting examples of anti-cancer agents that can be used as therapies in combination with aTherapeutic Agent(s), an Engineered Cell(s), or nucleic acids encoding an IL-15Ra (and in some embodiments, an IL-15 polypeptide) include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alpha-2a; interferon alpha-2b; interferon alpha-nl ; interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; HMG-CoA reductase inhibitor (such as but not limited to, Lovastatin, Pravastatin, Fluvastatin, Statin, Simvastatin, and Atorvastatin); loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; Vitaxin®; vorozole; zanoterone; zeniplatin; zilascorb; and zinostatin stimalamer. Additional anti-cancer drugs are 5-fluorouracil and leucovorin. These two agents are particularly useful when used in methods employing thalidomide and a topoisomerase inhibitor. In some embodiments, a anti-cancer agent is not a chemotherapeutic agent.

### 5.10.2 Antiviral Agents

Antiviral agents that can be used in combination with a Therapeutic Agent(s), an Engineered Cell(s), or nucleic acids encoding an IL-15Ra (and in some embodiments, an IL-15 polypeptide) include, but are not limited to, non-nucleoside reverse transcriptase inhibitors, nucleoside reverse transcriptase inhibitors, protease inhibitors, and fusion inhibitors. In one embodiment, the antiviral agent is selected from the group consisting of amantadine, oseltamivir phosphate, rimantadine, and zanamivir. In another embodiment, the antiviral agent is a non-nucleoside reverse transcriptase inhibitor selected from the group consisting of delavirdine, efavirenz, and nevirapine. In another embodiment, the antiviral agent is a nucleoside reverse transcriptase inhibitor selected from the group consisting of abacavir, didanosine, emtricitabine, emtricitabine, lamivudine, stavudine, tenofovir DF, zalcitabine, and zidovudine. In another embodiment, the antiviral agent is a protease inhibitor selected from the group consisting of amprenavir, atazanavir, fosamprenav, indinavir, lopinavir, nelfinavir, ritonavir, and saquinavir. In another embodiment, the antiviral agent is a fusion inhibitor such as enfuvirtide.

Additional, non-limiting examples of antiviral agents for use in combination with a Therapeutic Agent(s), an Engineered Cell(s), or nucleic acids encoding an IL-15Ra (and in some embodiments, an IL-15 polypeptide) include the following: rifampicin, nucleoside reverse transcriptase inhibitors (*e.g*., AZT, ddI, ddC, 3TC, d4T), non-nucleoside reverse transcriptase inhibitors (*e.g*., delavirdine efavirenz, nevirapine), protease inhibitors (*e.g*., aprenavir, indinavir, ritonavir, and saquinavir), idoxuridine, cidofovir, acyclovir, ganciclovir, zanamivir, amantadine, and palivizumab. Other examples of anti-viral agents include but are not limited to acemannan; acyclovir; acyclovir sodium; adefovir; alovudine; alvircept sudotox; amantadine hydrochloride (SYMMETRELTM); aranotin; arildone; atevirdine mesylate; avridine; cidofovir; cipamfylline; cytarabine hydrochloride; delavirdine mesylate; desciclovir; didanosine; disoxaril; edoxudine; enviradene; enviroxime; famciclovir; famotine hydrochloride; fiacitabine; fialuridine; fosarilate; foscamet sodium; fosfonet sodium; ganciclovir; ganciclovir sodium; idoxuridine; kethoxal; lamivudine; lobucavir; memotine hydrochloride; methisazone; nevirapine; oseltamivir phosphate (TAMIFLUTM); penciclovir; pirodavir; ribavirin; rimantadine hydrochloride (FLUMADINETM); saquinavir mesylate; somantadine hydrochloride; sorivudine; statolon; stavudine; tilorone hydrochloride; trifluridine; valacyclovir hydrochloride; vidarabine; vidarabine phosphate; vidarabine sodium phosphate; viroxime; zalcitabine; zanamivir (RELENZATM); zidovudine; and zinviroxime.

### 5.10.3 Antibacterial Agents

Antibacterial agents, including antibiotics, that can be used in combination with a Therapeutic Agent(s), an Engineered Cell(s), or nucleic acids encoding an IL-15Ra (and in some embodiments, an IL-15 polypeptide) include, but are not limited to, aminoglycoside antibiotics, glycopeptides, amphenicol antibiotics, ansamycin antibiotics, cephalosporins, cephamycins oxazolidinones, penicillins, quinolones, streptogamins, tetracyclins, and analogs thereof. In some embodiments, antibiotics are administered in combination with a Therapeutic Agent to prevent and/or treat a bacterial infection.

In an specific, a Therapeutic Agent(s), an Engineered Cell(s), or nucleic acids encoding an IL-15Ra (and in some embodiments, an IL-15 polypeptide) are used in combination with other protein synthesis inhibitors, including but not limited to, streptomycin, neomycin, erythromycin, carbomycin, and spiramycin.

In one embodiment, the antibacterial agent is selected from the group consisting of ampicillin, amoxicillin, ciprofloxacin, gentamycin, kanamycin, neomycin, penicillin G, streptomycin, sulfanilamide, and vancomycin. In another embodiment, the antibacterial agent is selected from the group consisting of azithromycin, cefonicid, cefotetan, cephalothin, cephamycin, chlortetracycline, clarithromycin, clindamycin, cycloserine, dalfopristin, doxycycline, erythromycin, linezolid, mupirocin, oxytetracycline, quinupristin, rifampin, spectinomycin, and trimethoprim.

Additional, non-limiting examples of antibacterial agents for use in combination with a Therapeutic Agent(s), an Engineered Cell(s), or nucleic acids encoding an IL-15Ra (and in some embodiments, an IL-15 polypeptide) include the following: aminoglycoside antibiotics (*e.g*., apramycin, arbekacin, bambermycins, butirosin, dibekacin, neomycin, neomycin, undecylenate, netilmicin, paromomycin, ribostamycin, sisomicin, and spectinomycin), amphenicol antibiotics (*e.g*., azidamfenicol, chloramphenicol, florfenicol, and thiamphenicol), ansamycin antibiotics (*e.g*., rifamide and rifampin), carbacephems (*e.g*., loracarbef), carbapenems (*e.g*., biapenem and imipenem), cephalosporins (*e.g*., cefaclor, cefadroxil, cefamandole, cefatrizine, cefazedone, cefozopran, cefpimizole, cefpiramide, and cefpirome), cephamycins (*e.g*., cefbuperazone, cefmetazole, and cefininox), folic acid analogs (*e.g*., trimethoprim), glycopeptides (*e.g*., vancomycin), lincosamides (*e.g*., clindamycin, and lincomycin), macrolides (*e.g*., azithromycin, carbomycin, clarithomycin, dirithromycin, erythromycin, and erythromycin acistrate), monobactams (*e.g*., aztreonam, carumonam, and tigemonam), nitrofurans (*e.g*., furaltadone, and furazolium chloride), oxacephems *(e.g.,* flomoxef, and moxalactam), oxazolidinones *(e.g.,* linezolid), penicillins (*e.g*., amdinocillin, amdinocillin pivoxil, amoxicillin, bacampicillin, benzylpenicillinic acid, benzylpenicillin sodium, epicillin, fenbenicillin, floxacillin, penamccillin, penethamate hydriodide, penicillin o benethamine, penicillin 0, penicillin V, penicillin V benzathine, penicillin V hydrabamine, penimepicycline, and phencihicillin potassium), quinolones and analogs thereof (*e.g*., cinoxacin, ciprofloxacin, clinafloxacin, flumequine, grepagloxacin, levofloxacin, and moxifloxacin), streptogramins (*e.g*., quinupristin and dalfopristin), sulfonamides (*e.g*., acetyl sulfamethoxypyrazine, benzylsulfamide, noprylsulfamide, phthalylsulfacetamide, sulfachrysoidine, and sulfacytine), sulfones (*e.g*., diathymosulfone, glucosulfone sodium, and solasulfone), and tetracyclines (*e.g*., apicycline, chlortetracycline, clomocycline, and demeclocycline). Additional examples include cycloserine, mupirocin, tuberin amphomycin, bacitracin, capreomycin, colistin, enduracidin, enviomycin, and 2,4 diaminopyrimidines (*e.g*., brodimoprim).

### 5.11 Use of Host Cells Expressing IL-15Ra as Feeder Cells

In one aspect, provided herein are methods for propagating, activating, and/or differentiating cells, comprising contacting an Engineered Cell(s) with another cell(s) that is responsive to IL-15. In some embodiments, provided herein are methods for propagating, activating and/or differentiating cells, comprising contacting an Engineered Cell(s) with another cell(s) that is responsive to IL-15, and isolating the cell(s) responsive to IL-15 from the Engineered Cell(s). In some embodiments, the Engineered Cell(s) is contacted with the IL-15 responsive cell(s) in the presence of an IL-15 polypeptide if the Engineered Cell(s) does not express IL-15 polypeptide. The cell(s) responsive to IL-15 can be isolated from the Engineered Cell(s) using techniques known to one of skill in the art, including, *e.g*., FACS.

In some embodiments, provided herein are methods for propagating, activating and/or differentiating cells, comprising co-culturing an Engineered Cell(s) with another cell(s) that is responsive to IL-15. In some embodiments, provided herein are methods for propagating, activating and/or differentiating cells, comprising co-culturing an Engineered Cell(s) with another cell(s) that is responsive to IL-15, and isolating the cell(s) responsive to IL-15 from the Engineered Cell(s). In some embodiments, provided herein are methods for propagating, activating and/or differentiating cells, comprising co-culturing an irradiated Engineered Cell(s) with another cell(s) that is responsive to IL-15. In some embodiments, provided herein are methods for propagating, activating and/or differentiating cells, comprising co-culturing an irradiated Engineered Cell(s) with another cell(s) that is responsive to IL-15, and isolating the cell(s) responsive to IL-15 from the Engineered Cell(s). In some embodiments, the Engineered Cell(s) and is co-cultured with the IL-15 responsive cell(s) in the presence of an IL-15 polypeptide if the Engineered Cell(s) does not express IL-15 polypeptide. The Engineered Cell(s) and cell(s) responsive to IL-15 are co-cultured *ex vivo* for a period of time sufficient to result in propagation, activation and/or differentiation of the cell responsive to IL-15. In some embodiments, the Engineered Cell(s) and cell(s) responsive to IL-15 are co-cultured *ex vivo* for at least 1 hour, 2 hours, 3 hours, 4 hours, 5 hours, 6 hours, 7 hours, 8 hours, 9 hours, 10 hours, 11 hours, 12 hours, 14 hours, 16 hours, 18 hours, 20 hours, 22 hours, 24 hours, 30 hours, 36 hours, 42 hours, 48 hours or 72 hours. In some embodiments, the Engineered Cell(s) and cell(s) responsive to IL-15 are co-cultured *ex vivo* for 1 to 2 hours, 2 to 4 hours, 4 to 6 hours, 6 to 12 hours, 12 to 18 hours, 18 to 24 hours, 12 to 24 hours, 24 to 36 hours, 36 to 48 hours, 24 to 48 hours, 48 to 72 hours, 2 to 3 days, 3 to 5 days, 5 to 8 days, 1 to 2 weeks, or 2 to 3 weeks. The cell(s) responsive to IL-15 can be isolated from the Engineered Cell(s) using techniques known to one of skill in the art, including, *e.g*., fluorescence activated cell sorting (FACS) or magnetic sepration using antibody-coated magnetic beads.

In some embodiments, the Engineered Cell(s) is an immune cell, such as a monocyte, macrophage, lymphocyte or dendritic cell. In other embodiments, the Engineered Cell(s) is a cell line, such as described in Section 5.3.2, *supra.* In some embodiments, the Engineered Cell(s) is irradiated such that it undergoes only a limited number of rounds of cell division.

In some embodiments, the Engineered Cell(s) is a host cell described in Section 5.3.2 or 5.6, *supra.* In some embodiments, the Engineered Cell(s) is a host cell that recombinantly expresses an IL-15Ra polypeptide and an IL-15 polypeptide. In other embodiments, the Engineered Cell(s) is a host cell that recombinantly expresses an IL-15Ra polypeptide but not an IL-15 polypeptide. In some embodiments, the Engineered Cell(s) is a host cell that recombinantly expresses an IL-15Ra polypeptide described in Section 5.1 and/or Section 5.2, *supra.* In some embodiments, the Engineered Cell(s) is a host cell that recombinantly expresses an IL-15Ra polypeptide in which the cleavage site for an endogenous protease that cleaves native IL-15Ra has been mutated. In one embodiment, the Engineered Cell(s) is a host cell that recombinantly expresses an IL-15Ra derivative comprising one, two, three, four, five, six, seven or eight mutations in the extracellular domain cleavage site of IL-15Ra such that cleavage of the IL-15Ra by an endogenous protease that cleaves native IL-15Ra is inhibited. In some embodiments, the Engineered Cell(s) is a host cell that recombinantly expresses an IL-15Ra derivative in which the amino acid sequence PQGHSDTT (SEQ ID NO:26) is mutated such that cleavage by an endogenous protease that cleaves native human IL-15Ra is inhibited. In some embodiments, one, two, three, four, five, six, seven, or eight amino acid substitutions and/or deletions are introduced into the amino acid sequence PQGHSDTT (SEQ ID NO: 26) of human IL-15Ra such that cleavage by an endogenous proteases that cleaves native human IL-15Ra is inhibited. In some embodiments, the Engineered Cell(s) is a host cell that recombinantly expresses an IL-15Ra derivative in which the amino acid sequence PQGHSDTT (SEQ ID NO:26) is replaced with a cleavage site that is recognized and cleaved by a heterologous protease. Non-limiting examples of such heterologous protease cleavage sites include Arg-X-X-Arg (SEQ ID NO:7), which is recognized and cleaved by furin protease; and A-B-Pro-Arg-X-Y (SEQ ID NO:8) (A and B are hydrophobic amino acids and X and Y are nonacidic amino acids) and Gly-Arg-Gly, which are recognized and cleaved by the thrombin protease. In some embodiments, the Engineered Cell(s) is a host cell that recombinantly expresses IL-15 in addition to IL-15Ra.

In another embodiment, the Engineered Cell(s) is a host cell that recombinantly expresses an IL-15Ra derivative, wherein the IL-15Ra derivative: (i) comprises a mutated extracellular cleavage site that inhibits cleavage by an endogenous protease that cleaves native IL-15Ra, and (ii) lacks all or a fragment of the transmembrane domain of native IL-15Ra. In some embodiments, the Engineered Cell(s) is a host cell that recombinantly expresses an IL-15Ra derivative, wherein the IL-15Ra derivative comprises: (i) one, two, three, four, five, six, seven or eight mutations (e.g., substitutions and/or deletions) in the extracellular cleavage site of IL-15Ra such that cleavage of IL-15Ra by an endogenous protease that cleaves native IL-15Ra is inhibited, and (ii) all or a fragment of a transmembrane domain of a heterologous molecule in place of all or a fragment of the transmembrane domain of native IL-15Ra. In some embodiments, the Engineered Cell(s) is a host cell that recombinantly expresses an IL-15Ra derivative, wherein the IL-15Ra derivative comprises: (i) one, two, three, four, five, six, seven or eight mutations (e.g., substitutions and/or deletions) in the amino acid sequence PQGHSDTT (SEQ ID NO:26), and (ii) all or a fragment of a transmembrane domain of a heterologous molecule in place of all or a fragment of the transmembrane domain of native IL-15Ra. In accordance with these embodiments, the IL-15Ra derivatives may or may not comprise all or a fragment of the cytoplasmic tail of native IL-15Ra. In some embodiments, the heterologous molecule is CD4, CD8, or MHC. In some embodiments, the Engineered Cell(s) is a host cell that recombinantly expresses IL-15 in addition to IL-15Ra.

In some embodiments, the cell responsive to IL-15 is an immune cell, such as an NK cell, a monocyte, a myeloid cell, a dendritic cell, or a lymphocyte (*e.g*., a T-lymphocyte, such as a CD4+ or CD8+ cell). In some embodiments, the cell responsive to IL-15 (i.e., IL-15-responsive cell) is a peripheral blood mononuclear cell or a tumor infiltrating lymphocyte. In some embodiments, the cell responsive to IL-15 is a cell disclosed in the Section 6, *infra,* as proliferating, activating and/or differentiating in response to IL-15. In other embodiments, the cell responsive to IL-15 (i.e., IL-15-responsive cell) is a stroma cell or an endothelial cell. In some embodiments, the cell responsive to IL-15 is engineered to express a peptide, polypeptide, or protein of interest, such as an antibody, a chimeric antigen receptor, a cytokine,or a growth factor.

In some embodiments, the IL-15 responsive cells isolated following *ex vivo* co-culture with an Engineered Cell(s) are administered to a subject to prevent, treat, and/or manage a disorder. In some embodiments, the IL-15 responsive cells which are administered to a subject were derived from the subject (*i.e*., the IL-15 responsive cells are autologous). In other embodiments, the IL-15 responsive cells which are administered to a subject were derived from a different subject.

In some embodiments, IL-15 responsive cells, which are immune cells isolated following ex vivo co-culture with an Engineered Cell(s), are administered to a subject to enhance immune function and/or prevent, treat, and/or manage a disorder in which the administration of immune cells is beneficial. In some embodiments, the IL-15 responsive cells isolated following *ex vivo* co-culture with an Engineered Cell(s) are administered to a subject to enhance an IL-15-mediated immune function. In some embodiments, the IL-15 responsive cells isolated following *ex vivo* co-culture with an Engineered Cell(s) are administered to a subject to prevent, treat or manage a disorder in which enhancing IL-15-mediated immune function is beneficial, such as cancer, an infectious disease (e.g., an infectious disease caused or associated with a viral, bacterial, fungal or parasite infection), an immunodeficiency (*e.g*., AIDS) or lymphopenia.

The IL-15 responsive cells isolated following *ex vivo* co-culture with an Engineered Cell(s) can be used to treat any of the conditions disclosed in Sections 5.7 to 5.9, *supra.* In some embodiments, the IL-15 responsive cells isolated following ex vivo co-culture with an Engineered Cell(s) are utilized to prevent, treat and/or manage cancer, such as melanoma, renal cell carcinoma, non-small cell lung cancer, colon cancer or another cancer disclosed in Section 5.7, *supra.* The IL-15 responsive cells isolated following *ex vivo* co-culture with an Engineered Cell(s) may be administered locally or systemically to a subject via any route known to one of skill in the art (*e.g*., parenteral administration, such as subcutaneous, intravenous,or intramuscular administration, or intratumoral administration). In some embodiments, the IL-15 responsive cells isolated following *ex vivo* co-culture with an Engineered Cell(s)are implanted or infused into a subject. In some embodiments, the IL-15 responsive cells isolated following *ex vivo* co-culture with an Engineered Cell(s) are implanted in a subject and the implant includes a porous, non-porous, or gelatinous material, including membranes, such as sialastic membranes, or fibers in addition to the host cells. In some embodiments, the IL-15 responsive cells isolated following *ex vivo* co-culture with an Engineered Cell(s) are administered to a subject as part of composition, wherein the composition comprises a polymer in addition to the host cells. In some embodiments, a suitable dose of IL-15 responsive cells isolated following *ex vivo* co-culture with an Engineered Cell(s) administered to subject may be at least 100, 200, 300, 400, 500, 700, 1,000, 5,000, 10,000, 25,000, 50,000, 100,000, 1 x10⁶, 1 x10⁷, or 1 x10⁸ cells. In some embodiments, a suitable dose of host cells that recombinantly express IL-15Ra (and in some embodiments, IL-15) administered to a subject is between 100 to 10,000, 500 to 10,000, 1,000 to 5,000, 5,000 to 10,000, 5,000 to 20,000, 10,000 to 20,000, 25,000 to 50,000, 50,000 to 100,000, 1 x10⁴ to 1 x10⁵, 1 x10⁵ to 1 x10⁶, 1 x10⁵ to 1 x10⁷, 1 x10⁶ to 1 x10⁸ cells. The IL-15 responsive cells isolated following *ex vivo* co-culture with an Engineered Cell(s) may be administered 1, 2, 3, 4, 5, 6, 7, 8 or more times. The frequency and dose of IL-15 responsive cells isolated following *ex vivo* co-culture with an Engineered Cell(s) which are administered to a subject will vary depending on several factors, including, *e.g*., the condition of the patient.

### 5.12 Biological Activity

### 5.12.1 Assays for Testing the Function of the Therapeutic Agent or Engineered Cell(s)

Provided herein are methods to identify agents that modulate the activity of IL-15/IL-15Ra complexes. The activity of an agent can be assayed with an IL-15 sensitive cell line, *e.g.,* CTLL-2 cells, a mouse cytotoxic T lymphoma cell line (ATCC Accession No. TIB-214) or TF1-β cells. See, *e.g*., International Publication No. WO 05/085282.

To assess the activity of Therapeutic Agents, proliferation of CTLL-2 or TF1-β cells cultured in the presence or absence of one or more Therapeutic Agents can be assessed by ³H-thymidine incorporation assays well known in the art and described in International Publication No. WO 05/085282.

In one aspect, the Therapeutic Agent or Engineered Cell(s) increases an immune response that can be, *e.g.,* an antibody response (humoral response) or a cellular immune response, *e.g.,* cytokine secretion (*e.g*., interferon-gamma), helper activity or cellular cytotoxicity. In one embodiment, the increased immune response is increased cytokine secretion, antibody production, effector function, T cell proliferation, and/or NK cell proliferation. Various assays to measure such activities are well known in the art, and exemplary descriptions of such assays are provided below.

For example, enzyme-linked immunosorbent assays (ELISA) are well known in the art and are described, *e.g.,* in Section 2.1 of Current Protocols in Immunology, Coligan et al. (eds.), John Wiley and Sons, Inc. 1997. ELISA can be used, *e.g.,* to assay the amount or concentration of IL-15 or IL-15Ra polypeptide.

In another method, the "tetramer staining" assay (Altman et al., 1996, Science 274: 94-96) may be used to identify antigen-specific T-cells and to assess how Therapeutic Agents modulate (*e.g*., enhance or suppress) antigen-specific T cell responses. For example, an MHC molecule containing a specific peptide antigen, such as a tumor-specific antigen, is multimerized to make soluble peptide tetramers and labeled, for example, by complexing to streptavidin. The MHC-peptide antigen complex is then mixed with a population of T cells obtained from a subject administered with an immunogenic composition alone or in combination with a Therapeutic Agent. Biotin is then used to stain T cells which express the tumor-specific antigen of interest.

Furthermore, using the mixed lymphocyte target culture assay, the cytotoxicity of T cells can be tested in a ⁵¹Cr-release assay as described, *e.g.,* in Palladino et al., 1987, Cancer Res. 47:5074-5079. Briefly, the mixed lymphocyte culture is added to a target cell suspension to give different effector:target (E:T) ratios (usually 1:1 to 40:1). The target cells are pre-labeled by incubating 1x10⁶ target cells in culture medium containing 500 µCi of ⁵¹Cr per ml for one hour at 37°C. The cells are washed three times following labeling. Each assay point (E:T ratio) is performed in triplicate and the appropriate controls incorporated to measure spontaneous ⁵¹Cr release (no lymphocytes added to assay) and 100% release (cells lysed with detergent). After incubating the cell mixtures for 4 hours, the cells are pelleted by centrifugation at 200 g for 5 minutes. The amount of ⁵¹Cr released into the supernatant is measured by a gamma counter. The percent cytotoxicity is measured as cpm in the test sample minus spontaneously released cpm divided by the total detergent released cpm minus spontaneously released cpm.

In another embodiment, an ELISPOT assay can be used to measure cytokine release *in vitro* by T cells after administration of an effective amount of a Therapeutic Agent or an Engineered Cell(s) to a subject. Cytokine release is detected by antibodies which are specific for a particular cytokine, *e.g*., interleukin-2, tumor necrosis factor γ or interferon-γ (see, *e.g.,* Scheibenbogen et al., 1997, Int. J. Cancer 71:932-936). The assay is carried out in a microtitre plate which has been pre-coated with an antibody specific for a cytokine of interest which captures the cytokine secreted by T cells. After incubation of T cells for 24-48 hours in the coated wells, the T cells are removed and replaced with a second labeled antibody that recognizes a different epitope on the cytokine. After extensive washing to remove unbound antibody, an enzyme substrate which produces a colored reaction product is added to the plate. The number of cytokine-producing cells is counted under a microscope. This method has the advantages of short assay time, and sensitivity without the need of a large number of cytotoxic T cells.

In some aspects, the immune response induced or enhanced by a Therapeutic Agent or an Engineered Cell(s) is enhanced or increased by at least 2 fold, 3 fold, 4 fold, 5 fold, 6 fold, 7 fold, 8 fold, 9 fold, 10 fold, 11 fold, or 12 fold relative to an immune response elicited by a negative control as determined by any known assay in the art. In some embodiments, the immune response induced by the Therapeutic Agent or the Engineered Cell(s) is enhanced by at least 0.5-2 times, at least 2-5 times, at least 5-10 times, at least 10-50 times, at least 50-100 times, at least 100-200 times, at least 200-300 times, at least 300-400 times or at least 400-500 times relative to the immune response induced by a negative control as assayed by any known method in the art. In some embodiments, the assay used to assess immune response measures the level of antibody production, cytokine production, or cellular cytoxicity, and such assays are well known in the art. In some embodiments, the assay used to measure the immune response is an enzyme-linked immunosorbent assay (ELISA) that determines antibody or cytokine levels, an ELISPOT assay that determines cytokine release, or a ⁵¹Cr release assay that determines cellular cytotoxicity.

In some embodiments, the Therapeutic Agent or Engineered Cell(s) induces or enhances an immune response in a subject that is measured by antibody titer in the serum of the subject, and the antibody titer is at least 0.2 to 5 times, 5 to 20 times, 10 to 30 times, 20 to 50 times, 50 to 200 times, 100 to 500, 200 to 1000 times, or 500 to 2,000 times higher as compared to the antibody titer in the serum of a subject administered a negative control. In some embodiments, the mean serum antibody titer against an antigen in the subject administered the Therapeutic Agent or Engineered Cell(s) is increased by at least 0.5-2 times, at least 2-5 times, at least 5-10 times, at least 10-50 times, at least 50-100 times, at least 100-200 times, at least 200-300 times, at least 300-400 times or at least 400-500 times relative to the mean serum antibody titer in the subject administered a negative control as determined by methods well known in the art.

In another embodiment, provided herein are methods of administering Therapeutic Agents or Engineered Cell(s) to induce or enhance the level of cytokine production or secretion, *e.g*., interferon-γ, (that may be 0.5 to 500 times higher) as compared to the level of cytokine production or secretion in a negative control sample. In some embodiments, the Therapeutic Agent or Engineered Cell(s) iinduces or enhances an immune response that is measured by increased cytokine release, and the cytokine concentration is at least 0.2 to 5 times, 5 to 20 times, 10 to 30 times, 20 to 50 times, 50 to 200 times, 100 to 500, 200 to 1000 times, or 500 to 2,000 times higher as compared to the cytokine concentration of a negative control. In some embodiments, the mean serum cytokine concentration of samples obtained from a subject administered the Therapeutic Agent or Engineered Cell(s) is increased by at least 0.5-2 times, at least 2-5 times, at least 5-10 times, at least 10-50 times, at least 50-100 times, at least 100-200 times, at least 200-300 times, at least 300-400 times or at least 400-500 times relative to the mean serum cytokine concentration of samples obtained from a subject administered a negative control as determined by methods well known in the art. In some embodiments, the negative control can be samples from the subject prior to administration of the Therapeutic Agent or the Engineered Cell(s).

In some embodiments, the Therapeutic Agent or the Engineered Cell(s) induces or enhances NK cell proliferation in a subject that by at least 0.2 to 5 times, 5 to 20 times, 10 to 30 times, 20 to 50 times, 50 to 200 times, 100 to 500, 200 to 1000 times, or 500 to 2,000 times higher relative to NK cell proliferation in a negative control. In some embodiments, the Therapeutic Agent or Engineered Cell(s) induces or enhances T cell proliferation in a subject that by at least 0.2 to 5 times, 5 to 20 times, 10 to 30 times, 20 to 50 times, 50 to 200 times, 100 to 500, 200 to 1000 times, or 500 to 2,000 times higher relative to T cell proliferation in a negative control as determined by methods well known in the art, *e.g.,* flow cytometry, CSFE staining, ³H-thymidine incorporation.

The increase in antibody (humoral) or cellular immune response induced by an effective amount of the Therapeutic Agent or the Engineered Cell(s) can be assessed using various methods well known in the art.

### 5.12.2 Animal Models

Therapeutic Agents, Engineered Cells, purified forms of IL-15Ra (e.g., purified soluble forms of IL-15Ra), or nucleic acids encoding an IL-15Ra (and in some embodiments, an IL-15 polypeptide) are preferably assayed *in vivo* for the desired therapeutic or prophylactic activity prior to use in humans. For example, in one embodiment, a Therapeutic Agent can be administered to the animal at the same time as the onset of a disease or disorder in the animal. In another embodiment, a Therapeutic Agent can be administered to the animal prior to the onset of a disease or disorder in the animal. In another embodiment, a Therapeutic Agent can be administered to the animal subsequent to the onset of a disease or disorder in the animal. In an embodiment, the Therapeutic Agent is administered to the animal more than one time. In another embodiment, the Therapeutic Agent is administered in combination with another therapy.

Therapeutic Agents, Engineered Cells, purified forms of IL-15Ra (e.g., purified soluble forms of IL-15Ra), or nucleic acids encoding an IL-15Ra (and in some embodiments, an IL-15 polypeptide) can be tested in animal models systems including, but are not limited to, rats, mice, chicken, cows, monkeys, pigs, goats, sheep, dogs, rabbits, guinea pigs, etc. In an embodiment, Therapeutic Agents are tested in a mouse model system. Such model systems are widely used and well-known to the skilled artisan.

The anti-cancer activity of a Therapeutic Agent, an Engineered Cell(s), a purified form of IL-15Ra (e.g., a purified soluble form of IL-15Ra), or nucleic acids encoding an IL-15Ra (and in some embodiments, an IL-15 polypeptide) can be determined by using various experimental animal models for the study of cancer well known in the art as described in, *e.g*., Relevance of Tumor Models for Anticancer Drug Development (1999, eds. Fiebig and Burger); Contributions to Oncology (1999, Karger); The Nude Mouse in Oncology Research (1991, eds. Boven and Winograd); and Anticancer Drug Development Guide (1997 ed. Teicher), incorporated herein by reference in their entireties.

Animal models for cancer can be used to assess the efficacy of a Therapeutic Agent or a composition thereof, an Engineered Cell(s) or a composition thereof, a purified form of IL-15Ra (e.g., a purified soluble form of IL-15Ra) or a composition thereof, or nucleic acids encoding an IL-15Ra (and in some embodiments, an IL-15 polypeptide) or a combination therapy comprising a Therapeutic Agent or Engineered Cell(s). Non-limiting examples of animal models for lung cancer include, but are not limited to, lung cancer animal models described by Zhang & Roth (1994, In vivo 8(5):755-69) and a transgenic mouse model with disrupted p53 function (see, *e.g.,* Morris et al., 1998, J La State Med Soc 150(4):179-85). An example of an animal model for breast cancer includes, but is not limited to, a transgenic mouse that overexpresses cyclin D1 (see, *e.g.,* Hosokawa et al., 2001, Transgenic Res 10(5):471-8). An example of an animal model for colon cancer includes, but is not limited to, a TCR-β and p53 double knockout mouse (see, *e.g.,* Kado et al., 2001, Cancer Res 61(6):2395-8). Examples of animal models for pancreatic cancer include, but are not limited to, a metastatic model of Panc02 murine pancreatic adenocarcinoma (see, *e.g*., Wang et al., 2001, Int J Pancreatol 29(1):37-46) and nu-nu mice generated in subcutaneous pancreatic tumours (see, *e.g.,* Ghaneh et al., 2001, Gene Ther 8(3):199-208). Examples of animal models for non-Hodgkin's lymphoma include, but are not limited to, a severe combined immunodeficiency ("SCID") mouse (see, *e.g.,* Bryant et al., 2000, Lab Invest 80(4):553-73) and an IgHmu-HOX11 transgenic mouse (see, *e.g.,* Hough et al., 1998, Proc Natl Acad Sci USA 95(23):13853-8). An example of an animal model for esophageal cancer includes, but is not limited to, a mouse transgenic for the human papillomavirus type 16 E7 oncogene (see, *e.g.,* Herber et al., 1996, J Virol 70(3):1873-81). Examples of animal models for colorectal carcinomas include, but are not limited to, Apc mouse models (see, *e.g.,* Fodde & Smits, 2001, Trends Mol Med 7(8):369-73 and Kuraguchi et al., 2000, Oncogene 19(50):5755-63).

For animal models of infectious diseases, the effectiveness of a Therapeutic Agent, an Engineered Cell(s), a purified form of IL-15Ra (e.g., purified soluble forms of IL-15Ra), or nucleic acids encoding an IL-15Ra polypeptide (and in some embodiments, an IL-15 polypeptide) relative to a negative control can be assessed in animals infected with virus. Samples obtained from these animals (*e.g*., serum, urine, sputum, semen, saliva, plasma, or tissue sample) can be tested for enhancement of immune function, *e.g*., enhancement in cytokine release, enhancement in antibody production, T cell proliferation, NK cell proliferation, with methods well known in the art and described herein. Samples obtained from these animals (*e.g*., serum, urine, sputum, semen, saliva, plasma, or tissue sample) can also be tested for reduction in viral replication via well known methods in the art, *e.g.,* those that measure altered viral replication (as determined, *e.g*., by plaque formation) or the production of viral proteins (as determined, *e.g*., by Western blot, ELISA, or flow cytometry analysis) or viral nucleic acids (as determined, *e.g*., by RT-PCR, northern blot analysis or southern blot). For quantitation of virus in tissue samples, tissue samples are homogenized in phosphate-buffered saline (PBS), and dilutions of clarified homogenates are adsorbed for 1 hour at 37°C onto monolayers of cells (*e.g*., Vero, CEF or MDCK cells). In other assays, histopathologic evaluations are performed after infection, preferably evaluations of the organ(s) the virus is known to target for infection. Virus immunohistochemistry can be performed using a viral-specific monoclonal antibody. Non-limiting exemplary animal models described below can be adapted for other viral systems.

Various animal models for infectious diseases that are well known in the art can be employed to assess the efficacy of Therapeutic Agents, Engineered Cells, purified forms of IL-15Ra (e.g., purified soluble forms of IL-15Ra), or nucleic acids encoding an IL-15Ra polypeptide (and in some embodiments, an IL-15 polypeptide) in preventing, treating, and/or managing infectious diseases, *e.g*.: mouse models of herpes simplex virus (HSV) are described in Crute et al., Nature Medicine, 2002, 8:386-391 and Bolger et al., Antiviral Res., 1997, 35:157-165; guinea pig models of HSV are described in Chen et al., Virol. J, 2004 Nov 23, 1:11; animal models of mouse cytomegalovirus (MCMV) and human cytomegalovirus (HCMV) are described in Kern et al., Antimicrob. Agents Chemother., 2004, 48:4745-4753; Guinea pig models of CMV is described in Bourne et al., Antiviral Res., 2000, 47:103-109, Bravo et al., Antiviral Res., 2003, 60:41-49 and Bravo et al, J. Infectious Diseases, 2006, 193:591-597; animal models of influenza virus are described in Sidwell et al., Antiviral Res., 2000, 48:1-16; and McCauley et al., Antiviral Res., 1995, 27:179-186; mouse models of hepatitis B virus (HBV) are described in Cavanaugh et al., J. Virol., 1997, 71:3236-3243 and Guidotti et al., J. Virol., 1995, 69:6158-6169; mouse models of hepatitis C virus (HCV) are described in Zhu et al., Antimicrobial Agents and Chemother., 2006, 50:3260-3268, Bright et al., Nature, 2005, 436:973-978, Hsu et al., Nat. Biotechnol., 2003, 21:519-525, Ilan et al., J. Infect. Dis. 2002, 185:153-161, Kneteman et al., Hepatology, 2006, 43:1346-1353, Mercer et al., Nat. Med., 2001, 7:927-933, and Wu et al., Gastroenterology, 2005, 128:1416-1423; animal models of HIV are described in Ayash-Rashkovsky et al., FASEB J., 2005, 19:1149-1151, Mosier et al., Semin. Immunol., 1996, 8:255-262, Mosier et al., Hosp. Pract. (Off Ed)., 1996, 31:41-48, 53-55, 59-60, Bonyhadi et al., Mol. Med. Today, 1997, 3:246-253, Jolicoeur et al., Leukemia, 1999, 13:S78-S80, Browning et al., Proc. Natl. Acad. Sci. USA, 1997, 94:14637-14641, and Sawada et al., J. Exp. Med., 1998, 187:1439-1449, and Schito et al., Curr. HIV Res., 2006, 4:379-386.

Other animal models for viral infections can also be used to assess the efficacy of a Therapeutic Agent or a composition thereof, an Engineered Cell(s) or composition thereof, a purified form of IL-15Ra (e.g., a purified soluble form of IL-15Ra) or a composition thereof, nucleic acids encoding an IL-15Ra polypeptide (and in some embodiments, an IL-15 polypeptide), or a combination therapy comprising a Therapeutic Agent or an Engineered Cell(s), *e.g*., animal models for viral infections such as EBV-associated diseases, gammaherpesviruses, infectious mononucleosis, simian immunodeficiency virus ("SIV"), Borna disease virus infection, hepatitis, varicella virus infection, viral pneumonitis, Epstein-Barr virus pathogenesis, feline immunodeficiency virus ("FIV"), HTLV type 1 infection, human rotaviruses, and genital herpes have been developed (see, *e.g.,* Hayashi et al., 2002, Histol Histopathol 17(4):1293-310; Arico et al., 2002, J Interferon Cytokine Res 22(11):1081-8; Flano et al., 2002, Immunol Res 25(3):201-17; Sauermann, 2001, Curr Mol Med 1(4):515-22; Pletnikov et al., 2002, Front Biosci 7:d593-607; Engler et al., 2001, Mol Immunol 38(6):457-65; White et al., 2001, Brain Pathol 11(4):475-9; Davis & Matalon, 2001, News Physiol Sci 16:185-90; Wang, 2001, Curr Top Microbiol Immunol. 258:201-19; Phillips et al., 2000, J Psychopharmacol. 14(3):244-50; Kazanji, 2000, AIDS Res Hum Retroviruses. 16(16):1741-6; Saif et al., 1996, Arch Virol Suppl. 12:153-61; and Hsiung et al., 1984, Rev Infect Dis. 6(1):33-50).

Other animal models for viral respiratory infections include, but not limited to, PIV (see, *e.g.,* Shephard et al., 2003 Res Vet Sci 74(2): 187-190; Ottolini et al., 2002 J Infect Dis 186(12): 1713-1717), and RSV (see, *e.g.,* Culley et al., 2002 J Exp Med 196(10): 1381-1386; and Curtis et al., 2002 Exp Biol Med 227(9): 799-802).

The Therapeutic Agent, composition thereof, or combination therapy comprising the Therapeutic Agent can be tested for their ability to decrease the time course of viral infection. The Engineered Cell(s), composition thereof, or combination therapy comprising the Engineered Cell(s) can be tested for their ability to decrease the time course of viral infection. The purified forms of IL-15Ra (e.g., purified soluble forms of IL-15Ra) or nucleic acids encoding the IL-15Ra polypeptide (and in some embodiments, an IL-15 polypeptide) can be tested for their ability to decrease the time course of viral infection.

Animal models for bacterial infections can also be used to assess the efficacy of a Therapeutic Agent or a composition thereof, an Engineered Cell(s) or composition thereof, a purified form of IL-15Ra (e.g., a purified soluble form of IL-15Ra) or a composition thereof, nucleic acids encoding an IL-15Ra polypeptide (and in some embodiments, an IL-15 polypeptide), or a combination therapy comprising a Therapeutic Agent or an Engineered Cell(s). Animal models for bacterial infections such as *H. pylori-infection, genital mycoplasmosis, primary sclerosing cholangitis, cholera,* chronic lung infection with *Pseudomonas aeruginosa,* Legionnaires' disease, gastroduodenal ulcer disease, bacterial meningitis, gastric *Helicobacter* infection, *pneumococcal otitis* media, experimental allergic neuritis, leprous neuropathy, mycobacterial infection, endocarditis, Aeromonas-associated enteritis, *Bacteroides fragilis* infection, syphilis, *streptococcal* endocarditis, acute hematogenous osteomyelitis, human scrub typhus, toxic shock syndrome, anaerobic infections, *Escherichia coli* infections, and *Mycoplasma pneumoniae* infections have been developed (see, *e.g.,* Sugiyama et al., 2002, J Gastroenterol. 37 Suppl 13:6-9; Brown et al., 2001, Am J Reprod Immunol. 46(3):232-41; Vierling, 2001, Best Pract Res Clin Gastroenterol. 15(4):591-610; Klose, 2000, Trends Microbiol. 8(4):189-91; Stotland et al., 2000, Pediatr Pulmonol. 30(5):413-24; Brieland et al., 2000, Immunopharmacology 48(3):249-52; Lee, 2000, Baillieres Best Pract Res Clin Gastroenterol. 14(1):75-96; Koedel & Pfister, 1999, Infect Dis Clin North Am. 13(3):549-77; Nedrud, 1999, FEMS Immunol Med Microbiol. 24(2):243-50; Prellner et al., 1999, Microb Drug Resist. 5(1):73-82; Vriesendorp, 1997, J Infect Dis. 176 Suppl 2:S164-8; Shetty & Antia, 1996, Indian J Lepr. 68(1):95-104; Balasubramanian et al., 1994, Immunobiology 191(4-5):395-401; Carbon et al., 1994, Int J Biomed Comput. 36(1-2):59-67; Haberberger et al., 1991, Experientia. 47(5):426-9; Onderdonk et al., 1990, Rev Infect Dis. 12 Suppl 2:S169-77; Wicher & Wicher, 1989, Crit Rev Microbiol. 16(3):181-234; Scheld, 1987, J Antimicrob Chemother. 20 Suppl A:71-85; Emslie & Nade, 1986, Rev Infect Dis. 8(6):841-9; Ridgway et al., 1986, Lab Anim Sci. 36(5):481-5; Quimby & Nguyen, 1985, Crit Rev Microbiol. 12(1):1-44; Onderdonk et al., 1979, Rev Infect Dis. 1(2):291-301; Smith, 1976, Ciba Found Symp. (42):45-72, and Taylor-Robinson, 1976, Infection. 4(1 Suppl):4-8).

The Therapeutic Agent or composition thereof, or combination therapy comprising the Therapeutic Agent can be tested for their ability to decrease the time course of bacterial infection, *e.g*., a bacterial respiratory infection by at least 25%, at least 50%, at least 60%, at least 75%, at least 85%, at least 95%, or at least 99% relative to a negative control using methods well known in the art.

The efficacy of Therapeutic Agents, compositions thereof, an Engineered Cell(s) or composition thereof, a purified form of IL-15Ra (e.g., a purified soluble form of IL-15Ra) or a composition thereof, nucleic acids encoding an IL-15Ra polypeptide (and in some embodiments, an IL-15 polypeptide), or a combination therapy comprising a Therapeutic Agent or an Engineered Cell(s) for the prevention, treatment and/or management of a fungal infection can be assessed in animal models for such infections. Animal models for fungal infections such as *Candida* infections, zygomycosis, *Candida mastitis,* progressive disseminated trichosporonosis with latent trichosporonemia, disseminated candidiasis, pulmonary paracoccidioidomycosis, pulmonary aspergillosis, *Pneumocystis carinii* pneumonia, cryptococcal meningitis, coccidioidal meningoencephalitis and cerebrospinal vasculitis, Aspergillus niger infection, Fusarium keratitis, paranasal sinus mycoses, Aspergillus fumigatus endocarditis, tibial dyschondroplasia, Candida glabrata vaginitis, oropharyngeal candidiasis, X-linked chronic granulomatous disease, tinea pedis, cutaneous candidiasis, mycotic placentitis, disseminated trichosporonosis, allergic bronchopulmonary aspergillosis, mycotic keratitis, *Cryptococcus neoformans* infection, fungal peritonitis, Curvularia geniculata infection, *staphylococcal* endophthalmitis, sporotrichosis, and dermatophytosis have been developed (see, *e.g.,* Arendrup et al., 2002, Infection 30(5):286-91; Kamei, 2001, Mycopathologia 152(1):5-13; Guhad et al., 2000, FEMS Microbiol Lett.192(1):27-31; Yamagata et al., 2000, J Clin Microbiol. 38(9):32606; Andrutis et al., 2000, J Clin Microbiol. 38(6):2317-23; Cock et al., 2000, Rev Inst Med Trop Sao Paulo 42(2):59-66; Shibuya et al., 1999, Microb Pathog. 27(3):123-31; Beers et al., 1999, J Lab Clin Med. 133(5):423-33; Najvar et al., 1999, Antimicrob Agents Chemother.43(2):413-4; Williams et al., 1988, J Infect Dis. 178(4):1217-21; Yoshida, 1988, Kansenshogaku Zasshi. 1998 Jun;72(6):621-30; Alexandrakis et al., 1998, Br J Ophthalmol. 82(3):306-11; Chakrabarti et al., 1997, J Med Vet Mycol. 35(4):295-7; Martin et al., 1997, Antimicrob Agents Chemother. 41(1):13-6; Chu et al., 1996, Avian Dis. 40(3):715-9; Fidel et al., 1996, J Infect Dis. 173(2):425-31; Cole et al., 1995, FEMS Microbiol Lett. 15;126(2):177-80; Pollock et al., 1995, Nat Genet. 9(2):202-9; Uchida et al., 1994, Jpn J Antibiot. 47(10):1407-12; : Maebashi et al., 1994, J Med Vet Mycol. 32(5):349-59; Jensen & Schonheyder, 1993, J Exp Anim Sci. 35(4):155-60; Gokaslan & Anaissie, 1992, Infect Immun. 60(8):3339-44; Kurup et al., 1992, J Immunol. 148(12):3783-8; Singh et al., 1990, Mycopathologia. 112(3):127-37; Salkowski & Balish, 1990, Infect Immun. 58(10):3300-6; Ahmad et al., 1986, Am J Kidney Dis. 7(2):153-6; Alture-Werber E, Edberg SC, 1985, Mycopathologia. 89(2):69-73; Kane et al., 1981, Antimicrob Agents Chemother. 20(5):595-9; Barbee et al., 1977, Am J Pathol. 86(1):281-4; and Maestrone et al., 1973, Am J Vet Res. 34(6):833-6). Animal models for fungal respiratory infections such as *Candida albicans, Aspergillus fumigatus,* invasive pulmonary aspergillosis, *Pneumocystis carinii,* pulmonary cryptococcosis, *Pseudomonas aeruginosa, Cunninghamella bertholletia* (see, *e.g.,* Aratani et al., 2002 Med Mycol 40(6):557-563; Bozza et al., 2002 Microbes Infect 4(13): 1281-1290; Kurup et al., 2002 Int Arch Allergy Immunol 129(2):129-137; Hori et al., 2002 Eur J Immuno 32(5): 1282-1291; Rivera et al., 2002 J Immuno 168(7): 3419-3427; Vassallo et al., 2001, Am J Respir Cell Mol Biol 25(2): 203-211; Wilder et al., 2002 Am J Respir Cell Mol Biol 26(3): 304-314; Yonezawa et al., 2000 J Infect Chemother 6(3): 155-161; Cacciapuoti et al., 2000 Antimicrob Agents Chemother 44(8): 2017-2022; and Honda et al., 1998 Mycopathologia 144(3):141-146).

The Therapeutic Agents or compositions thereof, Engineered Cells or compositions thereof, a purified form of IL-15Ra (e.g., a purified soluble form of IL-15Ra) or a composition thereof, nucleic acids encoding an IL-15Ra polypeptide (and in some embodiments, an IL-15 polypeptide), or a combination therapies comprising Therapeutic Agents or Engineered Cells can be tested for their ability to decrease the time course of fungal respiratory infection by at least 25%, at least 50%, at least 60%, at least 75%, at least 85%, at least 95%, or at least 99%. Techniques known to those of skill in the art can be used to analyze the function of the Therapeutic Agents or compositions thereof, Engineered Cells or compositions thereof, nucleic acids encoding an IL-15Ra polypeptide (and in some embodiments, an IL-15 polypeptide), or a combination therapies comprising Therapeutic Agents or Engineered Cells *in vivo.*

### 6. EXAMPLES

In some aspects of the examples 1-3 presented below, the IL-15/IL-15Ra complexes were made as follows: Human HEK 293T cells were transfected with nucleic acid expression construct for IL-15 (*e.g*., SEQ ID NO: 23) in combination with a nucleic acid expression construct for IL-15Ra (*e.g*., SEQ ID NO: 25) or IL-15Ra-Fc. The IL-15/IL-15Ra complexes composed of IL-15 (*e.g*., SEQ ID NO: 24 or SEQ ID NO: 1 without the signal peptide) and IL-15Ra (*e.g*., a soluble form of SEQ ID NO:3, such as SEQ ID NO:32) or IL-15Ra-Fc fusion protein (*e.g*., SEQ ID NO: 21 or 22) were purified. In specific embodiments, the IL-15/soluble IL-15Ra complexes referenced in examples 5-9 are composed of IL-15 (SEQ ID NO:1 without the signal peptide) and soluble IL-15Ra (SEQ ID NO: 32 without the signal peptide).

### 6.1 Example 1. Study of safety and pharmacokinetic properties of IL-15/IL-15Ra heterodimer after a single dose administration in preclinical studies in non-human primates.

To evaluate safety and pharmacokinetic properties IL-15/IL-15Ra heterodimer after a single dose administration, 5 rhesus macaques were treated as outlined in Table 1. *E.coli* derived IL-15 was administered i.v. at 5 µg/Kg (substudy 2); IL-15/IL-15Ra heterodimer was administered i.v. at 5 and 2 µg/Kg (substudy 3 and 4, respectively) and s.c. at 5 µg/Kg (substudy 5). One macaque received saline solution i.v. and served as control (substudy 1).

**Table 1. Study design to evaluate safety and pharmacokinetics of IL-15/IL-15Ra heterodimer after a single injection either i.v. or s.c.**

| Macaque substudy | Treatment | Dose (µg/Kg) | Route of Administration | # Dose | Rhesus Macaque |
|---|---|---|---|---|---|
| 1 | Saline | 0 | i.v. | 1 | 1 (M511-study1) |
| 2 | IL-15 | 5 | i.v. | 1 | 1 (M694- study 1) |
| 3 | IL-15/ sIL-15Ra | 5 | i.v. | 1 | 1 (M710- study 1) |
| 4 | IL-15/ sIL-15Ra | 2 | i.v. | 1 | 1 (M092- study 2) |
| 5 | IL-15/ sIL-15Ra | 5 | s.c. | 1 | 2 (M572, M703-study 5) |

Macaques were anesthetized and injected i.v. or s.c. with the different preparations (1 ml or 0.5 ml in saline solution, respectively). Blood pressure and temperature were followed over time. Macaque M710 (substudy 3) developed low blood pressure 18 minutes post i.v. infusion of IL-15/IL-15Ra (5 µg/Kg) and required i.v. liquids and a bolus of 5 ml Hetastarch. A total of 200 ml fluids were provided. A second animal M092 (substudy 4) was injected with a lower dose of IL-15/IL-15Ra heterodimer i.v. (3 µg/Kg). The lower dose of IL-15/sIL-15Ra heterodimer resulted also in a drop in blood pressure 30 minutes after i.v. injection. The animal was given a rapid infusion of 50 ml or LRS and 5 ml of bolus Hetastarch. A total of 200 ml fluids were administered. Both animals recovered from anesthesia and did not require any further treatment. No hypotension developed in animals M572 and M703 (Group/substudy 5) after s.c. injection of 5 µg/kg of IL-15/IL-15Ra. No significant changes in animal body temperature were observed.

Animals were also monitored for the following serum values: glucose, blood urea nitrogen, creatinine, total protein, albumin, bilirubin, alkaline phosphatase, alkaline transaminase, aminotransferase, cholesterol, calcium, phosphate, sodium, potassium, chloride, globulin, creatine phosphokinase, and for the following hematological parameters: hemoglobin, hematocrit, WBC, RBC, mean corpuscular volume, mean corpuscular hemoglobin, mean corpuscular hemoglobin concentration, platelets, neutrophils, lymphocytes, monocytes, eosinophils and basophils. All the values were in the physiological range, with the exception of CPK. All the animals (including control) showed an acute and transient increase in the level of CPK, which was attributed to the anesthesia. A slight decrease in the total WBC and total lymphocyte counts was observed 24-48 hours after one single dose of any IL-15 formulations. No measurements were done at later time points for these animals.

Blood was drawn at 0, 10, 20, 30, 45, 60 min, 2, 4, 8, 24 and 48 hours after i.v. injection (Group/substudy 1, 2, 3 and 4) and at 0, 1, 2, 4, 6, 8, 24, 48 and 72 hours after s.c. injection (Group/substudy 5) and levels of plasma IL-15 were determined by ELISA (QuantiGlo, R&D Systems, Q1500B), according to manufacturer's instructions. 10 min after i.v. injection at the dose of 5µg/Kg, animal receiving IL-15/IL-15Ra showed a plasma peak level of IL-15 around 50 ng/ml while the animal receiving monomeric IL-15 had a plasma peak level of IL-15 of around 30 ng/ml (Figure 4A). This difference suggests that IL-15/IL-15Ra heterodimer is a more stable molecule than monomeric IL-15, which is rapidly eliminated from the circulation. According to the one-phase exponential decay analysis, heterodimeric IL-15/IL-15Ra has a plasma half-life of 50-60 min while monomeric IL-15 has an half-life of 12 min. Injection of a lower dose of IL-15/sIL-15Ra resulted in lower peak level after 10 min (around 15 ng/ml), but the kinetics of decay of plasma IL-15 concentration overtime was similar (Figure 4A).

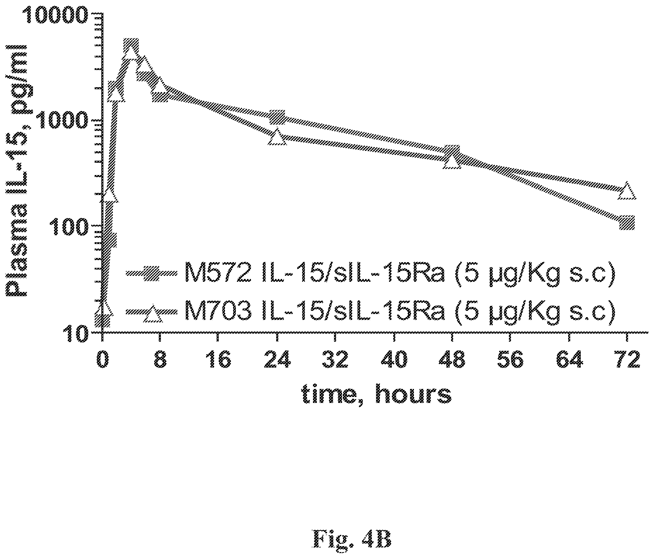

Upon s.c. injection of IL-15/IL-15Ra, a plasma peak level of IL-15 of 4-5 ng/ml was detected at 4 hours after the injection. The decline in plasma concentration of IL-15 overtime was slower in comparison to i.v. injection. The macaques maintained plasma IL-15 levels of ∼1 ng/ml at 24 hours after injection (Figure 4B). Interestingly, IL-15 levels obtained with one single s.c. injection of IL-15/IL-15Ra heterodimer were comparable with the IL-15 levels obtained at day 2 after CIV infusion of monomeric IL-15 given at 20 µg/Kg. The s.c. injection of monomeric IL-15 (at 20 or 40 µg/Kg) was characterized in previous studies as having a more rapid decay with levels around 0.1 ng/ml at 24 hours after injection (see, e.g., Sneller et al., Blood, 2011 Dec 22,118(26):6845-6848).

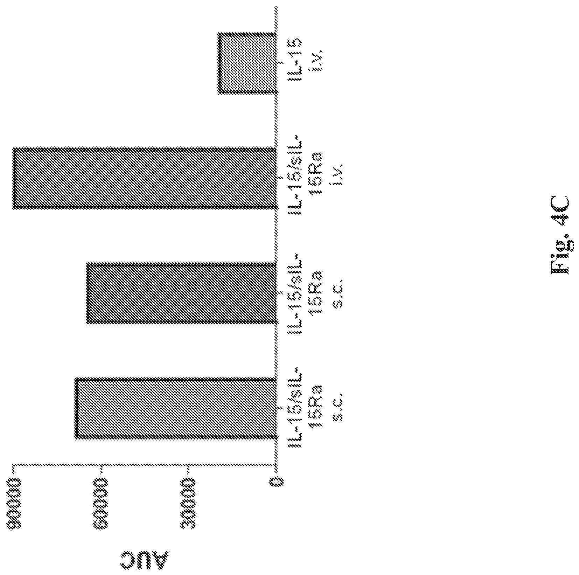

The Area Under the Curve for plasma IL-15 was also measured in the injected macaques. Similar AUCs (for plasma IL-15 levels overtime) were observed upon administration of 5 µg/Kg of IL-15/sIL-15Ra either i.v. or s.c. This was the result of the long half life of the heterodimeric IL-15/sIL-15Ra. The AUC values obtained by the heterodimer were 4-5 fold higher than the AUC of monomeric IL-15 upon i.v. injection at 5 µg/Kg dose (Figure 4C).

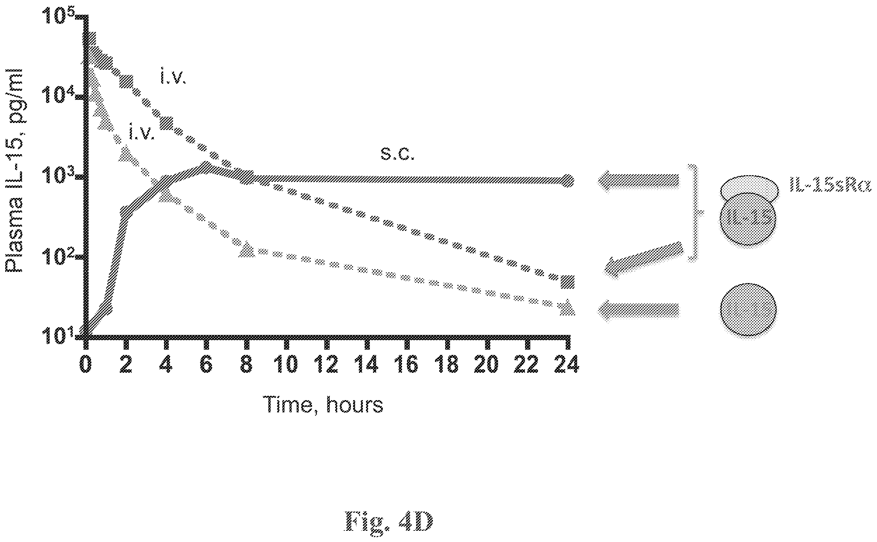

The pharmacokinetic properties of IL-15/sIL-15Ra delivered via s.c. injection were compared to the properties of IL-15/sIL-15Ra delivered via i.v. injection and to the properties of E. *coli*-produced IL-15 delivered via i.v. injection by administering the same concentration (5 µg/Kg) of IL-15/sIL-15Ra (or IL-15) via these delivery methods and assessing plasma levels of IL-15 at various time points. Results are presented in Figure 4D.

### Conclusions

Taken together, these data suggest that IL-15/sIL-15Ra heterodimer has a longer plasma half-life compared to E. coli-derived monomeric IL-15. The bolus i.v. doses of IL-15/IL-15Ra (5 and 2 µg/Kg) caused a transient drop in blood pressure that was treated by i.v. fluid support. Administration of the same dose of IL-15/IL-15Ra heterodimer s.c. results in lower acute peak but more prolonged plasma levels of IL-15 in comparison to i.v. injection. No changes in blood pressure were observed after s.c. injection. Therefore, s.c. injection minimized adverse effects associated with i.v. injection and resulted in more sustained bioactive levels of circulating IL-15.

### 6.2 Example 2. Studies evaluating toxicity, immunogenicity and effects on immune system homeostasis of human IL-15/sIL-15Ra after repeated injections.

Based on the results obtained after single administration of IL-15/IL-15Ra, additional studies were performed in order to evaluate toxicity, immunogenicity and effects on immune system homeostasis of human IL-15/sIL-15Ra after repeated injections. As outlined in Table 2, Group 1 included 2 rhesus macaques that received 12 daily i.v. injections of IL-15/sIL-15Ra at the dose of 2 µg/Kg. Group 2 included 2 rhesus macaques that received 5 s.c. injections (one every 3 days) of IL-15/sIL-15Ra at the dose of 5 µg/Kg. Group 2 underwent a second treatment cycle identical to the first one. This second cycle was conducted 60 days after the conclusion of the first cycle, and after the hematological parameters of these macaques were back to normal levels.

**Table 2. Study design to evaluate toxicity, immunogenicity and immunological effects of IL-15/IL-15Ra heterodimer after repeated injections either i.v. or s.c.**

| Group | Treatment | Dose (µg/Kg/ injection) | Route of Administration | Duration of Treatment | Total Doses/ Cycle | # Cycles | # Rhesus Macaques |
|---|---|---|---|---|---|---|---|
| 1 | IL-15/ IL-15Ra | 2 | i.v. | 12 days (daily injections) | 12 | 1 | 2 (P571, P575 - Study 3) |
| 2 | IL-15/ IL-15Ra | 5 | s.c. | 12 days (injections every 3 days) | 5 | 2 | 2 (P572, M695 - Study 6) |

Figure 5 presents the design of the study which is summarized in Table 2 and also includes testing of 2 additional rhesus macaques that received 5 s.c. injections (one every 3 days) of IL-15/sIL-15RaFc at the dose of 5 µg/Kg (i.e., P570 and P574) (Group 3), in addition to testing of 2 rhesus macaques that received 5 s.c. injections (one every 3 days) of IL-15/sIL-15Ra at the dose of 5 µg/Kg (i.e., P572 and M695) (Group 2).

The animals included in the studies were examined at different time points for clinical toxicity, had chemistry and hematological laboratory analysis, and were evaluated for immunological parameters as consequence of IL-15/IL-15Ra administrations. Development of auto-antibodies specific for human IL-15 and IL-15Ra were also evaluated. These animals were not sacrificed after conclusion of the treatment and no pathology data were obtained. Complete necropsy is to be performed (see below).

### Group 1:

Macaques P571 and P575 were sedated and received IL-15/IL-15Ra (2 µg/Kg) in 1 ml saline solution i.v. Blood pressure and temperature were followed over time. After each injection, both macaques developed low blood pressure 15 minutes post i.v. infusion of IL-15/IL-15Ra and required i.v. liquids for a total of 50-60 ml. No changes in animal temperature were observed. All animals were off anesthesia at 1 hour after injections and had normal recoveries. Blood was drawn at 0, 15, 30 min, 1, 4 and 24 hours after the first and the second i.v. injections and at day 7, 12, 14, 21, 28 and 47 after the initiation of the treatment. The concentration of human IL-15 in macaque plasma was evaluated using a chemiluminescent immunoassay (Quantiglo Q1500B, R&D Systems), according to the manufacturer's recommendations. i.v. injections of 2 µg/Kg of IL-15/IL-15Ra resulted in peak plasma IL-15 levels around 10 ng/ml and an half-life of approximately 1 hour with levels similar to baseline at 24 hours. Repeated i.v. injections did not affect either the peak plasma level of IL-15 achieved after each injection or the kinetics of decay (Figure 6).

Peripheral blood mononuclear cells (PBMCs) were isolated by Ficoll density gradient centrifugation for the samples collected at day 0, 2, 7, 12, 14, 21, 28 and 47 and cells were frozen in fetal bovine serum plus 10% DMSO and stored in liquid nitrogen until analysis. Immunophenotypic analysis was performed using a mix of directly conjugated anti-human antibodies: APC-Cy7 CD3, AmCyan CD4, AF405-CD8, FITC-CD95, PerCpCy5.5-CD28, AF700-CD45RA, APC-CCR7, PECy7-CD16, PE-NKp44, PE-NKp46, PE-gammadelta, PE-CD25, V450-CD20, APC-CD27, AF700-CD40. For the detection of Tregs, surface staining of cells was followed by intracellular staining with FITC-Foxp3 Ab, using the Foxp3 Staining Buffer Set (eBioscience). The data from labeled cell samples were acquired in a LSR II Flow Cytometer (BD) and were analyzed using FlowJo software (Tree Star, San Carlos, CA).

To determine the immunogenicity of human IL-15/IL-15Ra preparations, macaque plasma samples were screened for the presence of antibodies against human IL-15 or human IL-15Ra by Western immunoblot. 20 and 50 ng of IL-15/IL-15Ra were dissolved in a buffer containing SDS, loaded on a polyacrylamide gel and transferred onto nitrocellulose membranes. The presence of antibodies reacting against human IL-15 or human IL-15Ra was evaluated using monkey plasma before the initiation of the treatment cycle and at day 28. Both macaque did not develop either anti-human IL-15 or anti-human IL-15Ra antibodies (dilution 1:2000).

### Groups 2 and 3:

Macaques P572 and M695 were sedated and received IL-15/sIL-15Ra (5 µg/Kg) in 0.5 ml saline solution s.c. (Group 2). Blood pressure and temperature were followed over time. No changes in blood pressure and animal temperature were observed. All animals were off anesthesia at 1 hour after injections and had normal recoveries. Routine clinical chemistry and hematology parameters were measured at day 0, 7 and 14 after each treatment cycle. Blood was drawn at 0, 1, 2, 4, 6, 8, 24 and 48 hours after the first and the second s.c. injections and at 0, 4 and 24 hours after the third and fourth s.c. injections and at 0, 4, 24 and 48 hours after the last s.c. injection of the first treatment cycle. After a rest period of approximately 2 months, a second treatment cycle was conducted. Blood was drawn at 0, 2, 4, 6, 8, 24 and 48 hours after the first s.c. injection and at 0, 4 and 24 hours after the second, third and fourth s.c. injections and at 0, 4, 24 and 72 hours after the last s.c. injection of the second treatment cycle. Blood samples were also collected at day 22, 39 and 60 after initiation of the second treatment cycle. Substantially the same procedure was performed with macaques P570 and P574 that received IL-15/sIL-15RaFc (5 µg/Kg) (Group 3) instead of IL-15/sIL-15Ra.

The concentration of human IL-15 in macaque plasma was evaluated using a chemiluminescent immunoassay (Quantiglo Q1500B, R&D Systems), according to the manufacturer's recommendations. Pharmacokinetics of heterodimeric IL-15 forms in macaques (Groups 2 and 3) administered s.c. at 5 µg/Kg was evaluated by ELISA after the first injection (Figure 7). Further, peak plasma IL-15 levels of 1-2 ng/ml were measured at a time of 2-6 hours after each s.c injection. Systemic IL-15 levels higher than 200 pg/ml were also maintained for up to 24 hours, while the measurement at 72 hours showed concentration back to baseline levels (Figure 8 shows the results for Group 2 and Figure 9 shows the results for Group 3). Importantly, repeated treatment cycles with IL-15/IL-15Ra did not affect the plasma level of IL-15 achieved after each injection, contrary to what was previously reported for *E.coli*-derived IL-15 (see, e.g., Sneller et al., Blood, 2011 Dec 22,118(26):6845-6848).

Peripheral blood mononuclear cells (PBMCs) were isolated by Ficoll density gradient centrifugation for the samples collected at day 0, 2, 7 and 14 after initiation of the first treatment cycle and at 6 hours and day 1, 2, 7, 13, 15, 22, 39 and 60 after initiation of the second treatment cycle. The cells were frozen in fetal bovine serum plus 10% DMSO and stored in liquid nitrogen until analysis. Immunophenotypic analysis was performed using a mix of directly conjugated anti-human antibodies: APC-Cy7 CD3, AmCyan CD4, AF405-CD8, FITC-CD95, PerCpCy5.5-CD28, AF700-CD45RA, PECy7-CD16, PE-NKp44, PE-NKp46, PE-gammadelta, APC-CD20, FITC-annexin. For the detection of proliferating cells, surface staining of cells was followed by intracellular staining with AF700-Ki67 Ab (BD Pharmingen), using the Foxp3 Staining Buffer Set (eBioscience). The data from labeled cell samples were acquired in a LSR II Flow Cytometer (BD) and were analyzed using FlowJo software (Tree Star, San Carlos, CA).

At baseline, the percentage of CD16+NK cells that express Ki67 was below 5% for macaque M695 and around 15% for macaque P572. The percentage of CD4+, CD8+ and gammadelta T cells was below 10% before IL-15s/IL-15Ra administration. IL-15/sIL-15Ra treatment resulted in increased proliferation of CD16+NK cells at day 7 (80% for the two macaques) and at day 14 (40% for macaque M695 and 60% for macaque P572) after the first injection (Figures 10 shows results for Group 2 treated with IL-15/sIL-15Ra, and Figure 11 shows results for Groups 2 and 3 treated with IL-15/sIL-15Ra and IL-15/sIL-15RaFc, respectively). Similarly, IL-15/sIL-15Ra induced robust proliferation of CD8+ T cells (30 to 53% cells were Ki-67+) and gamma delta T cells (45 to 84% of cells were Ki-67+) that peaked at day 7 after the first injection and remained elevated at day 14 (Figures 10 shows results for Group 2, and Figure 11 shows results for Groups 2 and 3). A more modest increase in the proliferating CD4+ T cells proportion was also observed at day 7 after the first injection (Figures 10 shows results for Group 2, and Figure 11 shows results for Groups 2 and 3).

Repeated treatment cycles with IL-15/sIL-15Ra (Group 2) resulted in similar peak expansion of NK cells (Figure 12), and proliferation of NK cells after the 1^{st} and 2^{nd} treatment cycles with IL-15/sIL-15RaFc (Group 3) reflected plasma IL-15 levels (Figure 13). In Group 2, during both cycles the levels of cycling NK cells were increased at day 7 and 13 or 14, and cycling of NK cells was induced earlier during the second IL-15 regimen (day 2).

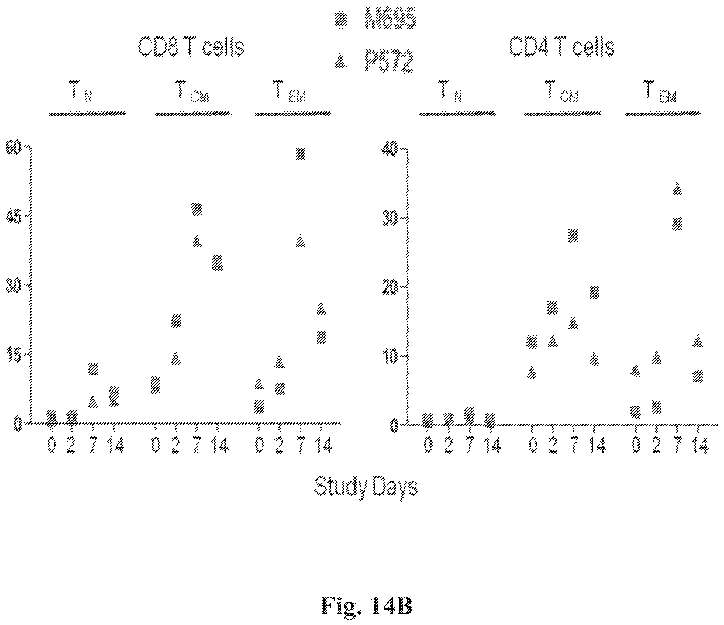

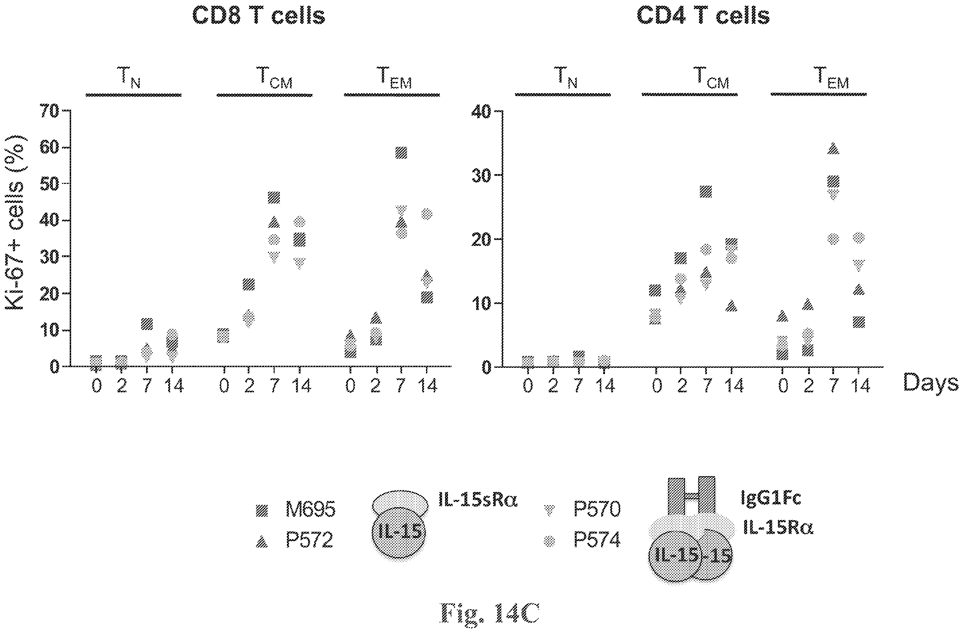

The different subsets within the CD8+ and CD4+ T cells were also examined for their proliferative capability in response to IL-15/IL-15Ra treatment. T naive (T_{N}) were defined as CD95-CD28+, T central memory (T_{CM}) as CD95+CD28+ and T effector memory (T_{EM}) as CD95+CD28-. Before IL-15/sIL-15Ra administration few cells in each compartment were Ki-67+. Both CD4+ and CD8+ T_{EM} rapidly expand upon IL-15/IL-15Ra treatment with an increase in the percentage of Ki-67+ cells between 5 and 15 fold at day 7 after the first injections. Both CD4+ and CD8+ T_{EM} expand upon IL-15/IL-15RaFc treatment as well. The effects of IL-15/sIL-15Ra (Group 2) and IL-15/sIL-15RaFc (Group 3) on effector memory CD4 T cells and central and effector memory CD8 cells T cells are shown in Figures 14A-C. Repeated IL-15/sIL-15Ra s.c. injections induced also a 5-and a 2-fold increase in the proliferation of CD8+ and CD4+ T_{CM}, respectively at day 7. A substantial increase in the frequency of proliferative CD8+ T_{N} cells was also observed at day 7 and 14 while CD4+ T_{N} cells did not respond to IL-15/IL-15Ra (Figure 14). Importantly, the observed frequency of Ki-67+ cells in each subsets is similar to the ones previously reported for 12 daily i.v. injections or CIV of *E*. *coli*-derived IL-15 (see, e.g., Sneller et al., Blood, 2011 Dec 22,118(26):6845-6848; Lugli et al., Blood, 2010 Oct 28, 116(17):3238-3248; Lugli et al., Blood, 2011 Sep 1, 118(9):2520-2529).

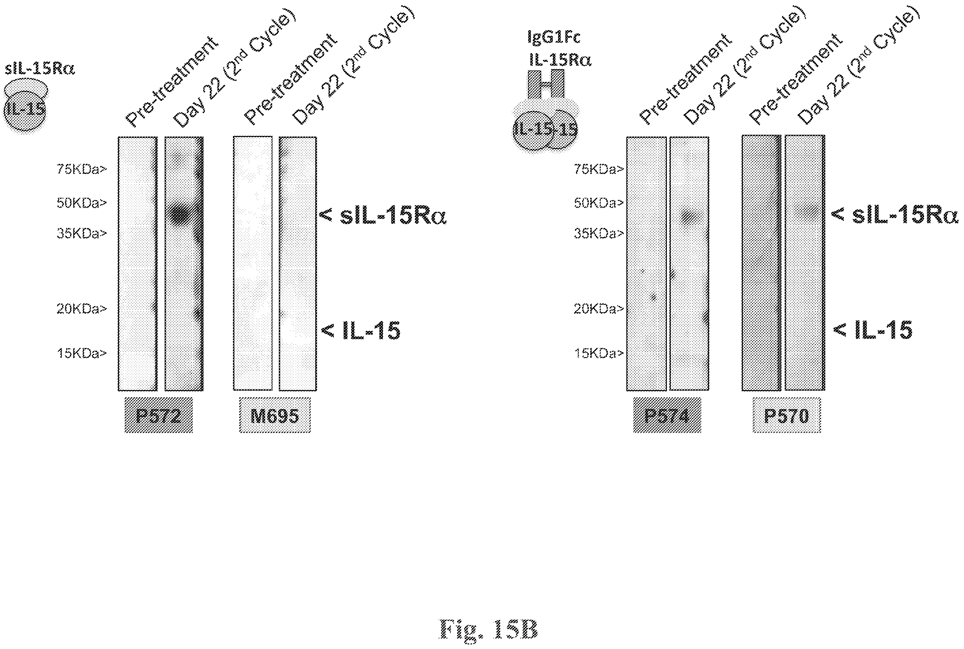

To determine the immunogenicity of human IL-15/IL-15Ra preparations, macaque plasma samples were screened for the presence of antibodies against human IL-15 or human IL-15Ra by Western immunoblot. 20 and 50 ng of IL-15/IL-15Ra were dissolved in a buffer containing SDS, loaded on a polyacrylamide gel and transferred onto nitrocellulose membranes. The presence of antibodies reacting against human IL-15, human IL-15Ra was evaluated using monkey plasma before the initiation of the first treatment cycle and at day 22 after the initiation of the second treatment cycle. No animals developed anti-IL-15 antibodies (Figure 15B). For macaque P572, the pre-treatment sample tested negative at the dilution 1:500, 1:2000 and 1: 8000. However, the sample from day 22 reacted against human IL-15Ra (approximately 42 KDa) but failed to detect human IL-15 (approximately 15 KDa) (Figure 15A). Macaque M695 did not develop either anti-human IL-15 or anti-human IL-15Ra antibodies (dilution 1:2000) (Figure 15B). The development of Abs against human IL-15Ra in one of the treated macaques may be a consequence of the difference between human and rhesus macaque IL-15Ra. Both animals (P574 and P570) receiving IL-15/IL-15RaFc developed anti-IL-15Ra antibodies (Figure 15B). Whereas the percentage of homology between human and rhesus macaques IL-15 is 97.5%, the percentage of homology between the two species mature secreted IL-15Ra is 91%.

### 6.3 Example 3. Study evaluating toxicity, plasma levels of IL-15 and other parameters after repeated subcutaneous injections of rhesus macaques with IL-15/sIL-15Ra.

Based on the results presented in Example 2, an additional study was performed in order to evaluate toxicity, immunogenicity and effects on immune system homeostasis of human IL-15/sIL-15Ra after repeated injections. As outlined in Table 3, Group 1 included 8 rhesus macaques that received 6 s.c. injections of IL-15/sIL-15Ra at the dose of 5 µg/Kg on day 0, 2, 4, 7, 9 and 12. Group 2 included 8 rhesus macaques that served as control (received 6 s.c. injections with saline solution on the same day). Both groups underwent a second treatment cycle identical to the first one. This second cycle was conducted 2 weeks after the conclusion of the first cycle, and after the hematological parameters of these macaques were back to normal levels. 4 macaques/group were sacrificed at day 14 and at day 30 after start of the second treatment cycle.

**Table 3. Study design to evaluate toxicity, immunogenicity and immunological effects of IL-15/IL-15Ra heterodimer after repeated s.c. injections.**

| Group | Treatment | Dose (µg/Kg/ injection) | Route of Administration | Duration of Treatment | Total Doses/ Cycle | # Cycles | # Rhesus Macaques |
|---|---|---|---|---|---|---|---|
| 1 | IL-15/ IL-15Ra | 5 | s.c. | 12 days (s.c. at 0, 2, 4, 7, 9 and 12) | 6 | 2 | 8 |
| 2 | Saline Solution | 5 | s.c. | 12 days (s.c. at 0, 2, 4, 7, 9 and 12) | 6 | 2 | 8 |

Macaques were sedated and received either IL-15/sIL-15Ra (5 µg/Kg) or saline solution in 0.5 ml saline solution s.c. Blood pressure and temperature were followed over time. No changes in blood pressure and animal temperature were observed. All animals were off anesthesia at 1 hour after injections and had normal recoveries. Routine clinical chemistry and hematology parameters were measured at day 0, 7 and 14 after each treatment cycle. Blood was drawn at different time points after the s.c. injections. The concentration of human IL-15 in macaque plasma was evaluated using a chemiluminescent immunoassay (Quantiglo Q1500B, R&D Systems), according to the manufacturer's recommendations. Peak plasma IL-15 levels of 1-2 ng/ml were measured at 6 hours after each s.c injection. The results are presented in Figures 16 and 17. Interestingly, levels of IL-15 at 48 hours after each s.c. injection were lower after the second injection in each treatment cycle, suggesting the concomitant expansion of cell consuming IL-15. Importantly, repeated treatment cycles with IL-15/IL-15Ra did not affect the plasma level of IL-15 achieved after each injection, contrary to what was previously reported for E.coli-derived IL-15 (see, e.g., Sneller et al., Blood, 2011 Dec 22,118(26):6845-6848) (Figure 16). Figure 17 shows peak and trough levels of plasma IL-15 heterodimers. Figure 18 shows mean arterial pressure in 8 macaques injected with IL-15 heterodimer v. controls, and Figure 19 shows mean body temperature in 8 macaques injected with IL-15 heterodimer v. controls. Figures 18 and 19 show that at a dose of 5 µg/Kg there is practically no change in blood pressure or body temperature of macaques injected s.c. relative to controls.

### Conclusions for Examples 2 and 3

The data presented in Examples 2 and 3 show, *inter alia*, that: (1) heterodimeric IL-15 delivered s.c. every other day leads to sustained elevated levels of plasma IL-15; (2) sustained plasma levels of IL-15 lead to sustained IL-15 activity for the entire period of 2-weeks-on, 2-weeks-off s.c. administration; (3) the trough plasma levels of IL-15 decrease over the period of s.c. administration, indicating higher utilization of IL-15 by the expanding lymphocytes throughout the body; and (4) s.c. administration avoids high peak levels and toxicity associated with i.v. administration. Further, at a dose of 5 µg/kg body weight there is no change in blood pressure or body temperature of macaques injected s.c., while the same dose of IL-15 heterodimer administered by intravenous bolus injection leads to blood pressure drop. Therefore, the observed side effects are lower by s.c. administration, whereas the IL-15 activity is maintained. In addition, there is no accumulation of IL-15 heterodimers after s.c. administration every 2 days; on the contrary, the consumption of IL-15 appears to increase due to the expanding numbers of dividing lymphocytes, which bind IL-15 heterodimers.

On the basis of these data, described herein are, *inter alia*, formulations of IL-15 heterodimer for injection where the plasma levels reach peak levels after 4 hours and the plasma levels are maintained above the basal plasma levels for at least 24 hours after a single administration. This facilitates once a day, once per 2 days or once per 3 days administration, depending on the desired levels of lymphocyte stimulation.

A formulation that achieves these specifications in humans is, for example, a sterile solution of IL-15 heterodimer at a concentration of 1-10 mg/ml in physiological saline. The s.c. administration in physiological saline solution achieves the desired range of plasma concentration. In addition, different formulations can be applied to further increase the bioavailability and stability of IL-15. The desired plasma levels in humans are between 10,000 pg/ml plasma and 1 pg/ml plasma using a standard human IL-15 ELISA (Quantiglo Q1500B, R&D Systems, performed according to the manufacturer's recommendations). More preferably the desired plasma levels are between 1,000 pg/ml plasma and 1 pg/ml plasma. Most preferably the desired plasma levels are between 1,000 pg/ml plasma and 10 pg/ml plasma. These levels are anticipated to minimize side effects and maximize lymphocyte growth and activation.

In humans it is anticipated that the plasma levels detected will decrease over time due to the lymphocyte expansion that will increase binding to cells and decrease the plasma levels. One method to maintain consistent plasma levels is the increasing of the IL-15 dose of heterodimer over time.

It can be further concluded that the data presented herein suggest that repeated administration of IL-15/IL-15Ra is non-toxic and immunogenic. IL-15/sIL-15Ra treatment resulted in increased proliferation of CD16+NK cells, induced robust proliferation of CD8+ T cells and gamma delta T cells, and resulted in some increase in CD4+ T cells proportion. Importantly, repeated treatment cycles with IL-15/IL-15Ra did not affect the plasma level of IL-15 achieved after each injection, contrary to what was previously reported for *E.coli-*derived IL-15. The data further suggest that cyclical treatment regimen in which IL-15/IL-15Ra is administered subcutaneously minimized toxicity while achieving plasma levels of IL-15 above the basal levels. In particular, the results presented herein suggest desirability of cyclical treatment regimen in which IL-15/IL-15Ra is administered subcutaneously, in particular, every 2 or 3 days, in the first treatment cycle (e.g., for 2 weeks), followed by "off-treatment" cycle in which IL-15/IL-15Ra is not administered (in particular, for 2 weeks to 2 months, e.g., 2 weeks or 2 months), and followed by the second treatment cycle in which IL-15/IL-15Ra is administered subcutaneously, in particular, every 2 or 3 days (e.g., for 2 weeks).

### 6.4 Example 4. Generation and Characterization of TL-15/TL-15Ra heterodimer stably expressed by a HEK293 cell line.

This example demonstrates efficient production of IL-15/soluble IL-15Ra (sIL-15Rα) non-covalently linked but stable heterodimer in clonal human HEK293 cells and release of the processed IL-15/sIL-15Rα heterodimer in the medium. As demonstrated herein, purification of the IL-15 and sIL-15Rα polypeptides allowed identification of the proteolytic cleavage site of IL-15Rα and characterization of multiple glycosylation sites. Further, administration of purified, reconstituted IL-15/sIL-15Rα heterodimer resulted in sustained plasma levels and in robust expansion of NK and T cells in mice, demonstrating pharmacokinetics and in vivo bioactivity superior to E. coli derived single-chain IL-15. These identified properties of heterodimeric IL-15 provide a strong rationale for use of this molecule for clinical applications.

Previously, the mechanism responsible for the shedding of the heterodimer from the cell surface was poorly understood and the C-terminus sequence of naturally cleaved sIL-15Rα was unknown. For these reasons, the molecular characterization of the bioactive heterodimeric cytokine is important. Isolation of the heterodimer from human tissues is difficult, because of the small quantities of circulating cytokine (Dudley et al., J. Clin. Oncol., 2008, 26:5233-5239). This example describes the development of methods for production and purification of IL-15/IL-15Ra heterodimers synthesized, processed and secreted by human cells after gene transfer. In particular, stable, clonal HEK293-derived human cell lines overproducing naturally processed IL-15/sIL-15Rα heterodimers and an efficient purification procedure to yield biologically active heterodimeric IL-15/sIL-15Rα cytokine were developed. This also allowed the characterization of the IL-15/sIL-15Rα complexes, determination of the amino acid sequence and the proteolytic cleavage site of the sIL-15Rα, analysis of the overall glycosylation, and evaluation of pharmacokinetics and in vivo bioactivity.

### 6.4.1 Experimental Methods

### Generation of mammalian cell lines overproducing heterodimeric IL-15/IL-15Rα.

DNA vectors optimized for the efficient expression of human IL-15 and full-length IL-15Rα (Bergamaschi et al., J. Biol. Chem., 2008, 283:4189-4199; Jalah et al., DNA Cell Biol., 2007, 26:827-840) were used for the generation of stable clonal cell lines. Highly purified, endotoxin-free DNA plasmid (Qiagen EndoFree Giga kit; Hilden, Germany) was linearized by restriction enzyme digestion and purified using Nucleotide Removal Kit (Qiagen) and ethanol precipitated under sterile conditions. HEK293 cells (Life Technologies, #11631017) were stably transfected by the calcium phosphate coprecipitation technique using optimized plasmids. Clones 19.7 and 1.5 were among the highest producers of IL-15/sIL-15Rα. Another HEK293-derived human cell line (clone 2.66) producing IL-15/sIL-15Rα heterodimers was generated using DNA vectors expressing IL-15 and the extracellular region of IL-15Rα (truncated sIL-15Rα, aa 1-175 (Bergamaschi et al., J. Biol. Chem., 2008, 283:4189-4199; Mortier et al., J. Immunol., 2004, 173:1681-1688) of the mature molecule). The cells were expanded and seeded in serum-free media in a hollow fiber system (FiberCell Systems Inc). Glucose consumption was measured daily and serum-free media was replaced when glucose concentration dropped below 100 mg/dL. Cell supernatants (20 ml) were harvested daily for up to 5 months and assayed for IL-15 levels by ELISA (R&D Systems).

### Reverse Phase High Performance Liquid Chromatography (RP-HPLC) separation and analysis of IL-15 Heterodimers.

For analytical RP-HPLC, samples were centrifuged to pellet cellular debris and 100 ml of media containing IL-15/sIL-15Ra complexes were separated by RP-HPLC under non-reducing conditions at a flow rate of 0.3 mL/min on 2.1 x 100 mm Poros® R2/10 column (ABI, USA), using aqueous acetonitrile/ trifluoroacetic acid solvents and a Shimadzu HPLC system equipped with LC-10AD pumps, an SCL-10A system controller, a CTO-10AC oven, an FRC-10A fraction collector, and an SPD-M10AV diode array detector at 55°C. Buffer A was 0.1% trifluoracetic acid (TFA) in water. The column was equilibrated with 10 % Buffer B (0.1% TFA in acetonitrile). The gradient of buffer B was: 10-43%, 9 min; 43-44%, 12 min; 44-85%, 4 min; 85%, 5 min. Peaks were detected by UV absorption at 206 and 280 nm. Quantitation of purified proteins was performed by amino acid analysis using a Hitachi L-8800 Amino Acid Analyzer. Purified IL-15 and sIL-15Rα subunits were mixed at molar ratio 1:1 to allow complex re-association. IL-15/sIL-15Rα complex analysis was performed in both denaturing and non-denaturing conditions on polyacrylamide gels (12% and 4-20% gradient, respectively), and visualized by Coomassie blue staining. Formation of the complexes was confirmed by Western immunoblot analysis, using anti-human IL-15 or anti-human IL-15Rα antibodies (AF315 and AF247, respectively, R&D Systems).

Preparative RP-HPLC was performed using a Dionex HPLC system equipped with Ultimate 3000 Binary pumps Model #HPG-3400A, Ultimate 3000 Photodiode Array Detector Model #PDA-3000, Ultimate 3000 Column Compartment Model #TCC-3000, Ultimate 3000 Solvent Rack and Degasser Model #SRD-3400, Isco Fraction Collector, Model #Foxy 200. Typically, 20 mL per run were loaded directly on the column after centrifugation to remove cell debris.

Initial purification was performed on aWaters RCM 25 x 100 mm uBondapak-C18 column at a flow rate of 5 mL/min at room temperature and flow rate 5 ml/min. Buffer A was 0.1% TFA in water. The gradient of buffer B was: 20% □ -32%, 60 min; 32%□-47%, 40 min; 47%-55%, 1 h 20 min; and 55%-65%, 20 min; 65%-90%, 20 min; and 90%, 10 min. The fractions corresponding to sIL-15Rα and IL-15 were pooled separately and further purified by RP-HPLC under non-reducing conditions. Further purification of sIL-15Ra was done on 16 x 100 mm POROS R2/10 column at 26.0 °C and flow rate 5 mL/min. The gradient of buffer B was: 5%-15%, 10min; 15%-32%, 120 min; 32%-100%, 20 min; 100%, 10 min.

For further purification of IL-15, fractions corresponding to IL-15 were pooled and re-purified first on 16 x 100 mm POROS R2/10 at 26°C at flow rate 5 mL/min. The gradient of buffer B was: 20%-36%, 30 min; 36%-50%, 150 min; 50%-90%, 20 min; 90%, 10 min. Peaks were detected at 206 and 280 nm and analyzed by sequencing using an automated Applied Biosystems Inc. 477 Protein Sequencer; by SDS-polyacrylamide gel electrophoresis (PAGE), and by immunoblot analysis using an Enhanced ChemiLuminescence (ECL) procedure (Amersham Life Science, Arlington Heights, IL). Quantitation of total protein in mixes or purified subunits was done by amino acid analysis using a Hitachi L-8800 Amino Acid Analyzer. The sIL-15Rα and the IL-15 pools were mixed in equimolar quantities and lyophilized.

### Proteolytic digestion to identify C-terminus of sIL-15Rα.

Sixty micrograms of naturally cleaved sIL-15Rα were dissolved in 0.02 M Tris-HCl, pH 8.5, and Lys-C endoproteinase (Boehringer Mannheim Gmbh, Mannheim, Germany) (20:1 w/w protein-protease) was added and incubated for 22 h at 37°C. After digestion, reverse-phase HPLC separation of Lys-C fragments were performed under non-reducing conditions on 2.1 x 100 mm Vydac C18 column at 0.3 mL/min, using a Shimadzu HPLC system. The gradient of buffer B was: 7-30%, 25 min; 30-70%, 5min; 70%, 5 min at 55°C. Peaks were detected at 206 and 280 nm and analyzed by sequencing using an automated Applied Biosystems Inc. 477 A protein sequencer.

*MALDI-TOF* MS-Aliquots of HPLC fractions were mixed with equal volume of matrix solution (alpha-cyano-4-hydroxycinnamic acid), and 1.5 ml of mixture was spotted on target plate and analyzed by MALDI-TOF MS (matrix-assisted laser desorption ionization time-of-flight mass spectrometry) on Ultraflex III TOF/TOF (Bruker Daltonics, Billerica, MA, USA). Spectra were externally calibrated in reflector mode using Bruker Peptide Calibration Standard II. Monoisotopic masses were determined using FlexAnalysis 3.3 (Bruker Daltonics) with the SNAP peak picking algorithm. The spectra were analyzed using BioTools 3.2 software (Bruker Daltonics). Mass spectrometric sequencing of particular peptides was performed by MALDI-TOF MS/MS analysis in the "LIFT" mode with manual selection of precursor ions. Fragment ion spectra were used for SwissProt protein database search by the Mascot MS/MS ion search program (at the website .matrixscience.com) on the NIH Mascot server at the website biospec.nih.gov/. The obtained identifications were confirmed by comparing the BioTools generated fragment ions series with experimental MS/MS data. Confidence in the identification was assessed based on the Mascot Protein or Ion score, which is -10*Log(P), where P is the probability that the observed match is a random event. ISD-MALDI-TOF analysis of intact HPLC purified sIL-15Ra was done using 1,5-diaminonaphthalene (DAN) as matrix as suggested by in Bruker's Technical Note # TN-36. "Automated Acquisition of MALDI-ISD Spectra for the N- and C-terminal Sequence Determination of Intact Proteins." MS/MS tolerance in analysis of ISD spectrum using BioTools was 1.5 Da. Mass spectrometry of the sIL-15Rα preparation was performed on an Applied Biosystems Voyager-DE Pro time-of-flight mass spectrometer operated in linear mode under positive ion conditions. Typical voltages were 25kV accelerating, guide wire 0.15% and grid voltage 91.5%. A nitrogen laser was used at 337 nm with 250 laser shots averaged per spectrum. CovalX HM-1 high mass detector was used with HV-1 set to 2.5 kV and HV-2 at 20kV. Bovine serum albumin and apo-myoglobin were used as standards for external calibration. Sinapic acid was used as matrix. Data analysis was carried out using "Data Explorer" software resident on the Voyager mass spectrometer.

### IL-15 treatment in mice.

Six-week-old female C57BL/6 mice were obtained from Charles River Laboratories, Inc. (Frederick, MD). E. coli-derived monomeric IL-15 and purified IL-15 heterodimers were injected at a dose of 3 µg of IL-15 equivalent/mouse i.p. Mice were bled at different time points after protein injection, and the serum IL-15 levels were measured using human IL-15 chemiluminescent immunoassay (Quanti-Glo, R & D Systems).

### CFSE labeling of cells and cell adoptive transfer in mice.

To make single cell suspensions, spleens from 6-week-old female C57BL/6 mice were gently squeezed through a 100-µm Cell Strainer (Thomas) and washed in RPMI 1640 medium (Invitrogen) to remove any remaining organ stroma. Cells were incubated for 10 min at 37°C with CFSE (2 µM; Molecular Probes) and washed twice. CFSE-labeled cells (20x106) were resuspended in PBS and injected i.v. into congenic mice. IL-15 treatment was performed the day after cell injection. At day 4, mice were sacrificed and splenocytes were analyzed by multiparameter flow cytometry to evaluate the bioactivity of IL-15. Briefly, the cells were washed in FACS buffer containing 0.2% fetal calf serum and stained with the following panel of conjugated rat anti-mouse antibodies: CD3-APCCy7, CD4-PerCP, CD8-Pacific Blue, NK1.1-PeCy7 or -APC (BD Biosciences). The percentage of cells of the original population that had divided in response to IL-15 treatment was calculated based on CFSE intensity. Samples were acquired using a FACSAria flow cytometer (BD Biosciences), and the data were analyzed by FlowJo software (Tree Star, San Carlos, CA). In some experiments, surface staining of cells was followed by intracellular staining with Ki67 antibody (BD Biosciences) for the detection of proliferating cells.

### 6.4.2 Results

### Generation of human cell lines producing high levels of IL-15/sIL-15Rα.

As discussed above, the optimized combination vectors for the co-ordinate expression of the two chains of the human heterodimeric cytokine IL-15/IL-15Rα have been genereated. Yields of secreted bioactive IL-15 achieved by these plasmids were >1,000 fold higher compared to wt IL-15 cDNAs (Bergamaschi et al., J. Biol. Chem., 2008, 283:4189-4199; Jalah et al., DNA Cell Biol., 2007, 26:827-840). These plasmids were used to develop stable clonal IL-15/sIL-15Rα-producing human HEK293 cell lines. Intact genes of IL-15 and IL-15Rα were used to generate these cell lines. Membrane-bound IL-15Rα is composed of 5 domains, the sushi domain responsible for the binding to IL-15, a linker region, the Pro/Thr rich domain, the transmembrane domain and cytoplasmic tail (Anderson et al., J. Biol. Chem., 1995, 270:29862-29869; Dubois et al., J. Biol. Chem., 1999, 274:26978-26984) (Fig. 20). Upon stable introduction of the genes into HEK293 cell lines, the heterodimer was obtained in the culture supernatant in a soluble form, after transport of the IL-15/IL-15Rα to the plasma membrane and proteolytic cleavage of the extracellular part of IL-15Rα by cellular enzymes. The mature secreted IL-15Rα molecule is depicted in Fig. 20. Clones 19.7 and 1.5 were selected as the highest producers of IL-15/sIL-15Rα heterodimers. Both stable clones were grown in continuous culture in serum-free medium, using a hollow fiber culture system, and supernatants were harvested daily. Clone 19.7 produced 70 mg IL-15/Lt (calculated as monomer IL-15 by ELISA) for up to 5 months (Fig. 21A). HPLC analysis under non-reducing conditions of weekly samples of supernatants produced by clone 19.7 collected from day 29 through day 137 in the bioreactor demonstrated stable production of IL-15/sIL-15Rα heterodimer over this interval (Fig. 21B). Similar results were obtained for clone 1.5. These results demonstrated that high levels of IL-15/sIL-15Rα heterodimeric cytokine production were achieved in stable human HEK293-derived cell lines. A DNA vector encoding the extracellular portion of IL-15Rα (truncated sIL-15Rα, encoding the signal peptide and 175 aa of the mature IL-15Rα) has been generated (Bergamaschi et al., J. Biol. Chem., 2008, 283:4189-4199). An additional cell line (clone 2.66) was established producing the engineered IL-15/sIL-15Rα complexes after gene transfer of genes encoding IL-15 and the truncated sIL-15Rα form. The known C-terminus sequence of truncated sIL-15Rα generated from clone 2.66 (Fig. 20) was used as control for the identification of the natural cleavage site on IL-15Rα after expression of the full-length molecule on the cell membrane (see below).

### Purification IL-15/sIL-15Rα by HPLC under non-reducing condition.

Reverse-phase HPLC (RP-HPLC) was used to purified the complex under non-reducing conditions, which keeps intact the disulfide bonds of the non-covalently associated IL-15 and sIL-15Rα subunits but dissociates the subunits from each other. The chains were purified and characterized separately, and they were subsequently re-associated in vitro at 1:1 molar ratio to regenerate the intact heterodimeric cytokine. The first purification step was performed using Waters RCM 25x100 mm mBondapak-C18 column, with the IL-15 and sIL-15Rα subunits dissociating in the acetonitrile containing buffer used for separation. Figure 22A shows a typical RP-HPLC elution profile of heterodimer produced from clone 19.7. The content of sIL-15Rα and IL-15 peaks were analyzed by SDS-PAGE (Fig. 22B) and the identities of both proteins were confirmed by immunoblot analysis (not shown). sIL-15Rα and IL-15 were eluted as broad peaks in several fractions (Fx), Fx 1-3 for IL-15Rα and Fx 4-13 for IL-15. Comparisons of the different fractions revealed small differences in size among eluted proteins (Fig. 22), likely due to differences in posttranslational modifications, (e.g. glycosylation). To achieve >95% purity for sIL-15Rα, a pool of Fx 1-3 was subjected to chromatography on a 16 x 100 mm POROS R2/10 column (Fig. 23A and 23B). IL-15 containing Fx 4-13 fractions were also re-purified on the same column (Supplementary Fig. 23C and 23D). All purifications were performed under non-reducing conditions to maintain natural disulfide bonds in both proteins. The final pools of sIL-15Rα (Fig. 23A-B, Fx1-3) and IL-15 (Fig. 23C-D, Fx 2-5) were analyzed by N-terminal Edman sequencing and the amount of each protein was determined by quantitative amino acid analysis. The molar ratio of the purified proteins recovered from the HPLC separation was approximately 1:1. sIL-15Rα and IL-15 were mixed at equivalent molar amounts in PBS, allowed to re-associate, then analyzed by native PAGE. Fig. 22C shows sIL-15Rα (lane 1), IL-15 (lane 2) and IL-15/sIL-15Rα complex (lane 3) visualized by Coomassie blue staining under non-denaturing conditions. No bands for the single-chain IL-15 or sIL-15Rα were detectable in the lane where IL-15/sIL-15Ra heterodimer was loaded (Fig. 22C, lane 3), indicating essentially quantitative formation of the IL-15/sIL-15Ra complex. Under native conditions, all three molecular species sIL-15Rα, IL-15 and heterodimer were detected as diffuse bands, likely due to heterogeneity of glycosylation in human HEK293 cells.

### Determination of C-terminal sequence of sIL-15Rα.

To determine the C-terminal sequence of naturally cleaved sIL-15Rα, purified sIL-15Rα (from clone 19.7) was digested with Lys-C endoproteinase, generating peptides suitable for N-terminal sequencing and Mass Spectrometry (MS) analyses. Truncated sIL-15Rα produced from clone 2.66, was also purified as described above for clone 19.7 and was used as reference since its C-terminus sequence was known. The expected peptides after Lys-C endoproteinase digestion from truncated sIL-15Rα_2.66 are shown in Table 4. The peptides generated after Lys-C digestion from the naturally cleaved sIL-15Rα_19.7 were separated by RP-HPLC under non-reducing conditions, generating a smaller number of peptides for further analysis. The C-terminal peptide produced a broad peak that eluted early in RP-HPLC and was collected in several fractions (Fig. 24, Fx 21-23). Analysis by N-terminal protein sequencing of fraction 22 obtained after Lys-C proteolysis and RP-HPLC (Fig. 24) revealed 23 amino acid residues XIRDPALVHQRPAPPS(T)VXXAGV corresponding to residues 63-85 of the mature IL-15Rα (residues numbered according to mature AA sequence) along with the 19-mer NWELXAXASHQPPGVYPQG, which corresponds to the sequence after Lys151, the last Lys residue before the IL-15Rα transmembrane domain (Table 5). This suggested that [M+H]+ of the C-terminal peptide should be at least 2038.962 (theoretical [M+H]+ of peptide NWELTASASHQPPGVYPQG which was identified by N-terminal sequencing). Analysis of fraction 22 by MALDI-TOF MS revealed the presence of several peptides with [M+H]+ close to or greater than 2038.962 Da (Fig. 25). Upon protein sequence analysis of fraction 22 (NWELXAXASHQPPGVYPQG), the expected Thr5(156) and Ser7(158) were not detected, while Ser9(160) was detected. This suggested that Thr5 and Ser7 were most likely modified, probably through O-glycosylation. Analysis of m/z 2020.927, 2056.934, 2308.082, 2365.108, 2455.138 and 2770.224 in MS/MS mode showed very similar fragmentation spectra in the low mass region (up to 1225 Da), suggesting that these peptides most likely derived from the same peptide sequence but present different posttranslational modifications. The fragmentation spectra of m/z 2020.927, 2056.943, 2365.108 and 2770.224 in MS/MS mode showed very similar fragmentation spectra in the low mass region (up to 1225 Da), suggesting that these peptides most likely derived from the same peptide sequence but present different posttranslational modifications. The fragmentation spectra of m/z 2020.927, 2056.943, 2365.108 and 2770.224 and their analysis are shown in Fig. 26. Since O-glycosylation was likely to be on Thr5 and Ser7, the formation of unmodified N-terminal b-ions and C-terminal y-ions preceding these residues could be expected, potentially allowing the identification of the corresponding peptide using Mascot search of protein sequences database (Matrix Science). In all cases (m/z 2020.927, 2056.934, 2308.082, 2365.108, 2455.138 and 2770.224), Mascot MS/MS ion search resulted in the identification of the same sequence of IL-15Rα that was obtained by protein sequencing, confirming that the NWELTASASHQPPGVYPQG sequence corresponds to the whole C-terminal peptide of naturally cleaved sIL-15Rα_19.7. A similar analysis was performed on naturally cleaved sIL-15Rα purified from the clone 1.5, leading to the same conclusion. Taken together, these data demonstrate that the proteolytic cleavage of membrane-bound IL-15Rα takes place between Gly170 and His171 in two different cell clones. Truncated engineered sIL-15Rα produced from clone 2.66 includes 5 additional amino acids at the C-terminus (Fig. 20).

**Table 4: Theoretical peptides after Lys-C endoproteinase digestion of truncated sIL-15Rα expressed and purified from clone 2.66.**

| Sequence | AA residues | Calculated mass (Da) |
|---|---|---|
| ITCPPPMSVEHADIWVK | 1-17 | 1923.3 |
| SYSLYSRERYICNSGFK | 18-34 | 2073.3 |
| RK | 35-36 | 302.4 |
| AGTSSLTECVLNK | 37-49 | 1322.5 |
| ATNVAHWTTPSLK | 50-62 | 1425.6 |
| CIRDPALVHQRPAPPSTVTTAGVTPQPESLSPSGK | 63-97 | 3506 |
| EPAASSPSSNNTAATTAAIVPGSQLMPSK | 98-126 | 2786 |
| SPSTGTTEISSHESSHGTPSQTTAK | 127-151 | 2515.6 |
| NWELTASASHQPPGVYPQGHSDTT | 152-175 | 2580.7 |

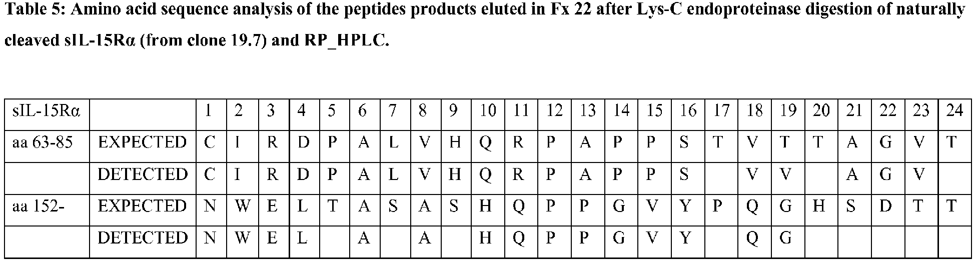

### Identification of post-translational modifications of sIL-15Rα.

Analysis of the naturally cleaved sIL-15Rα expressed from stable human cell lines by MALDI-TOF MS revealed the presence of numerous posttranslational modifications (Fig. 20). The expected molecular mass of the polypeptide chain of sIL-15Rα (170 aa, residues 31-200 of the IL-15Rα protein) is 17839.86 Da (Fig. 20). MALDI-TOF MS analysis of purified sIL-15Rα revealed a broad peak with a center at 34910 Da (Fig. 27), implying that almost half of its molecular mass is due to posttranslational modifications, most likely O- and N-linked glycosylation. Detailed analysis characterized the post-translational modification in the C- and N-terminal region of naturally cleaved sIL-15Rα. The C-terminal peptide with m/z 2020.927 (Fig. 25) was 18 Da less than the predicted NWELTASASHQPPGVYPQG peptide with m/z 2038.962, indicating loss of water. Since O-glycosylations were the likely modifications in the C-terminal region of sIL-15Rα, it is possible that 2020.927 is the product of beta-elimination reaction during the proteolysis of purified sIL-15Rα by Lys-C digestion. A beta-elimination reaction at O-glycosylated Ser and Thr residues would lead to loss of water with formation of dehydroalanine and dehydroaminobutyric acid, correspondingly. Peptides with dehydro amino acid residues containing a reactive double bond are not stable and quickly react with available nucleophilic compounds. The peptide with m/z 2020.927 appeared to be stable and did not react with 2-aminoethanethiol suggesting that its double bond likely reacted intra-molecularly with the hydroxyl group of a neighboring Ser or Thr residue. The ion fragment spectrum of parent ion 2020.927 is shown in Fig. 26A. The peptide with m/z 2056.934 differs from 2020.927 by 36 Da and represented the m/z of the same NWELTASASHQPPGVYPQG peptide, which first lost water due to beta-elimination and then added HCl to the double bond of a dehydro amino acid. The ion fragment spectrum of parent ion 2056.934 is shown on Fig. 26B. Analysis of this spectrum in BioTools with modification (chlorine addition) on Thr5 and Ser7 suggested that O-glycosylation took place at these sites with equal probability. Analysis of ion fragment spectra of parent ions 2308.082, 2365.108, 2455.138, and 2770.224 suggested that they have O-linked oligosaccharides attached to the peptide with m/z 2038.962 (Fig. 26C-D).

A detailed characterization of the N-terminal region of sIL-15Rα. MALDI-TOF MS/MS of m/z 1922.963 was performed (Fig. 24; fraction 40) and identified the N-terminal peptide ITCPPPMSVEHADIWVK of sIL-15Rα. However, m/z 2126.150 of fraction 39 gave a similar fragment ion spectrum, suggesting that both ions correspond to the same peptide. Mascot MS/MS ion search using fragment ions derived from 2126.150 when the parent ion was set as 1922.950 (corresponding to the theoretical m/z of unmodified peptide) confidently identified the same sequence ITCPPPMSVEHADIWVK (Fig. 29A). The mass difference between these m/z is 203.187, which is close to the mass of N-acetylhexosamine (HexNAc; N-acetylgalactosamine or N-acetylglucosamine). Analysis of the spectra with the modification set on Ser8 (Fig. 29B) and Thr2 (Fig. 29C) suggested that the modified residue was most likely Ser8 since no additional expected modified b-ions were detected when modification was set on Thr2. There are several Ser residues in the N-terminal sequence of IL15Ra beyond Ser8. To determine if some of these residues are also modified purified sIL-15Rα was analyzed by In-Source Decay (ISD) MALDI-TOF MS. The spectrum was analyzed using Top-Down Mascot Search of the Sprot human database, which allowed the identification of the mature N-terminal sequence of IL-15Rα (Fig. 30A). Analysis of the spectrum confirmed that Ser8 is partially modified by N-acetylhexosamine (HexNAc) along with Ser18, 20, 23 and 31 (Fig. 30B-F).

In conclusion, the data showed that naturally cleaved sIL-15Rα produced from human cells is heavily glycosylated with N- and O-linked glycosylation both in the N- and C-terminal region of the protein (Fig. 20).

### Determination of in vivo half-life of different forms of IL-15:

IL-15-IL-15/sIL-15Ra heterodimer produced from human cell lines retains all the posttranslational modifications, such as native disulfide bonds and both N- and O-linked glycosylation (Fig. 20). These posttranslational modifications are absent in E. coli-derived monomeric IL-15(Vyas et al., Biotechnol. Prog., 2012, 28:497-507) and along with the lack of the IL-15Rα subunit, could affect the stability, pharmacokinetics and bioactivity IL-15 cytokine.

The purified IL-15 and sIL-15Rα chains were reconstituted as described above (Fig. 22C) and used in experiments *in vivo* to identify their pharmacokinetic and pharmacodynamic properties in comparison to both single-chain non-glycosylated IL-15 produced in E. coli and purified by conventional chromatography(Vyas et al., Biotechnol. Prog., 2012, 28:497-507) and glycosylated single-chain IL-15 produced by human HEK293 cells and purified as described above (Fig. 22). To evaluate the *in vivo* half-life of IL-15/sIL-15Rα heterodimers in comparison to single-chain IL-15, mice (5/group) were injected intraperitoneally (i.p.) with either 3 µg of human E. coli-derived single-chain IL-15, 3 µg of human HEK293 cell-derived single-chain IL-15 or equimolar amount of purified human IL-15/sIL-15Rα (corresponding to 3 µg IL-15 monomer, clone 1.5 lot1). Serum was collected at various times after injection and IL-15 levels were measured by ELISA (Fig. 28). Both single-chain IL-15 reached peak plasma levels at 30 min after protein administration (∼70 ng/ml and ∼25 ng/ml for E.coli-derived and human HEK293 cell-derived IL-15, respectively). The difference in the IL-15 plasma levels between these two preparations was most likely a consequence of the different capability of the ELISA antibodies to detect unglycosylated and glycosylated IL-15 (data not shown). In contrast, administration of IL-15/sIL-15Ra heterodimer resulted in higher peak IL-15 levels 2 h after injection (∼120 ng/ml). The half-lifes of both single-chain IL-15 preparations were similar and less than 30 min, whereas IL-15/sIL-15Rα heterodimer had a significantly extended half-life (approximately 4 hours) (Fig. 28). The area under the curve for the 24 hr period was 20 times higher for the heterodimer, compared to either single-chain IL-15 preparation. Similar results were obtained after i.v. or s.c. administration (data not shown). Human cell produced IL-15/sIL-15Rα heterodimer thus has a favorable pharmacokinetic profile in mice in comparison to single-chain IL-15.

### Bioactivity of heterodimeric IL-15/sIL-15Rα in vivo.

The biological activity of the different forms of IL-15 were compared in mice. CFSE-labeled splenocytes were transferred into C57BL/6 mice. The mice were subsequently treated with either PBS, with 3 µg of E. coli-derived single chain IL-15, or with equimolar amount of IL-15/sIL-15Rα. Proliferation of transferred cells was evaluated 4 days after treatment. IL-15/sIL-15Rα heterodimer induced greater proliferation of donor CD8+ T cells (Fig. 21A, top panels) and NK cells (Fig. 31A, bottom panels) in comparison to single-chain IL-15 with a higher frequency of proliferating cells, and more rounds of cell division. Upon single-chain IL-15 injection, few CD8+ T cells divided once, while IL-15/sIL-15Rα heterodimer induced multiple rounds of division, resulting in a significant increase in the frequency of CD8+ T cells in spleen. Thus, IL-15/sIL-15Rα heterodimer was more stable in vivo, had a prolonged serum half-life and was more bioactive on a molar basis, compared to single chain IL-15. The serum levels of IL-15 correlated with the biological activity, as measured by CFSE dilution of transferred cells (Fig. 31A). Although the overall proliferation of CD4+ T cells did not change upon IL-15 administration, analysis of the different subsets of memory CD4+ T cells showed an expansion of effector memory cells (data not shown), as previously reported (Picker et al., J. Clin. Invest., 2006, 116:1514-1524). The increased proliferation of CD8+ T and NK cells upon IL-15 administration was also confirmed by measuring the frequency of cells expressing the proliferative marker Ki-67. Mice (5/group) were injected i.p. with either 3 µg of human E. coli-derived single-chain IL-15, 3 µg of human HEK293 cell-derived single-chain IL-15 or equimolar amount of purified human IL-15/sIL-15Rα. At day 4 after protein administration, all IL-15 treated mice showed an increased frequency of Ki-67+ CD8+ T and NK cells in comparison to PBS-treated mice. IL-15 heterodimers induced a greater proliferation of these lymphocyte subsets in comparison to both single-chain IL-15 preparations, that were characterized by a similar bioactivity, in agreement with their similar pharmacokinetic profile (Fig. 31B).

The bioactivity of IL-15 heterodimer formulations was comparable between different purification lots. Mice were injected i.p. or i.v. with 3 µg of human IL-15/sIL-15Rα from two separate purification lots and sacrificed at day 3 after injection (Fig. 31C). Administration of human IL-15/sIL-15Rα in mice resulted in an increased frequency of proliferating CD8+ T cells (defined as Ki-67+) in comparison to untreated mice both after i.p. and i.v. delivery. No difference in bioactivity (measured as CD8+ T cell proliferation) was observed comparing IL-15/sIL-15Rα obtained from different production/purification lots (Fig. 31C).

### 6.4.3 Discussion

IL-15 is a important cytokine with potential clinical applications as a lymphocyte growth and activation factor (Waldmann et al., Nat. Rev. Immunol., 2006, 6:595-601). Recombinant human IL-15 generated in *E. coli* has been produced as a non-glycosylated monomer of ∼12 kDa (Vyas et al., Biotechnol. Prog., 2012, 28:497-507). Preclinical studies to evaluate safety, toxicity, pharmacokinetics and pharmacodynamics of monomeric human IL-15 have been conducted in rhesus macaques, and showed increase in the absolute number and proliferation of NK and CD8+ T cells (Berger et al., Blood, 2009, 114:2417-2426; Lugli et al., Blood, 2010, 116:3238-3248; Sneller et al., Blood, 2011, 118:6845-6848; Waldmann et al., Blood, 2011, 117:4787-4795). While monomeric *E*. coli-produced IL-15 is in initial stages of clinical testing, this form of the molecule poses multiple challenges for clinical use due to it instability and rapid plasma clearance (Sneller et al., Blood, 2011, 118:6845-6848; Waldmann et al., Blood, 2011, 117:4787-4795). IL-15 expression is tightly regulated at the transcriptional level, as well as at several posttranscriptional and posttranslational steps such as mRNA stability, generation of alternative spliced isoforms, intracellular trafficking, interaction with IL-15Rα and secretion (Bergamaschi et al., J. Immunol., 2009, 183:3064-3072; Bergamaschi et al., J. Biol. Chem., 2008, 283:4189-4199; Mortier et al., J. Exp. Med., 2008, 205:1213-1225; Bamford et al., J. Immunol., 1998, 160:4418-4426; Onu et al., J. Immunol., 1997, 158:255-262; Tagaya et al., Immunity, 1996, 4:329-336; Tagaya et al., Proc. Natl. Acad. Sci. USA, 1997, 94:14444-14449; Waldmann et al., Annu. Rev. Immunol., 1999, 17:19-49; Duitman et al., Mol. Cell. Biol., 2008, 28:4851-4861).

In the study presented in this example, a systematic approach was employed to reproduce in engineered human cells the natural steps of production and processing of IL-15/sIL-15Rα heterodimers, resulting in efficient production and purification of the bioactive IL-15 heterodimeric cytokine, which appears to have important potential advantages over the prokaryotic produced monomeric form of the molecule. Taking advantage of the stabilization of IL-15 by co-expression with IL-15Rα (Bergamaschi et al., J. Biol. Chem., 2008, 283:4189-4199), combination vectors were produced in the study presented in this example expressing the heterodimeric cytokine IL-15/IL-15Rα, providing strong improvements in yield (Bergamaschi et al., J. Immunol., 2009, 183:3064-3072; Bergamaschi et al., J. Biol. Chem., 2008, 283:4189-4199). This example further shows that the produced vectors were used to develop stable clonal human HEK293 cells that grow in serum free medium and express and secrete high levels of human IL-15/sIL-15Rα complexes (up to 70 mg IL-15/Lt). Thus, an efficient procedure was developed for the purification of biologically active IL-15/sIL-15Rα heterodimers based on non-reducing RP-HPLC. The IL-15 and sIL-15Rα chains of the IL-15 heterodimeric cytokine are non-covalently linked and they can be separated under certain conditions, such as pH<3.5 (Dubois et al., Immunity, 2002, 17:537-547). This allowed the inventors to produce pure preparations of single-chain IL-15 and sIL-15Rα as shown in this example. Importantly, RP-HPLC was performed under non-reducing condition, avoiding the need for protein refolding after purification. Since the Kd of the two chains is ∼10-11 M (Anderson et al., J. Biol. Chem., 1995, 270:29862-29869; Giri et al., Embo. J., 1995, 14:3654-3663), the purified IL-15 and sIL-15Rα subunits can be recombined in vitro in a molar ratio of 1:1 in PBS allowing spontaneous association to form the bioactive heterodimeric cytokine. IL-15 is stabilized in the presence of IL-15Rα, and the generation of IL-15 as heterodimeric cytokine has the additional benefit to be a more stable structure, avoiding denaturation and inactivation and decreasing the possibility of creating immunogenic forms.

HEK293 human cells produce correctly folded, processed and glycosylated human IL-15/sIL-15Rα heterodimeric cytokine. Both the IL-15 and sIL-15Rα subunits of the heterodimeric cytokine contain intramolecular disulfide bonds and are heavily glycosylated (Fig. 20). IL-15 has three potential N-linked glycosylation sites (Kurys et al., J. Biol. Chem., 2000, 275:30653-30659). In agreement with previous publications (Dubois et al., J. Biol. Chem., 1999, 274:26978-26984), the data presented in this example show that sIL-15Rα contains both N- and O-linked carbohydrates both in the N- and C-part of the molecule. Several studies have demonstrated the contribution of glycosylation to the effect of other cytokines and growth factors (for reviews (Sola et al., J. Pharm. Sci., 2009, 98:1223-1245; Chamorey et al., Eur. Cytokine Netw., 2002, 13:154-160)). Glycosylated interferon-β (Karpusas et al., Cell. Mol. Life Sci., 1998, 54:1203-1216), erythropoietin (Takeuchi et al., Glycobiology, 1991, 1:337-346; Gribben et al., Lancet, 1990, 335:434-437), granulocyte colony stimulating factor (Querol et al., Haematologica, 1999, 84:493-498) and IL-7 (Beq et al., Blood, 2009, 114:816-825) are more stable and bioactive in comparison to the non-glycosylated forms. Glycosylation was also reported to affect the interaction with specific receptors, as IL-7 was able to bind glycosylated IL-7Rα 300-fold more tightly than unglycosylated IL-7Rα (McElroy et al., Structure, 2009, 17:54-65). Additionally, production of factors for clinical use in human cells may reduce the risk of immunogenicity. Administration of recombinant human granulocyte macrophage colony stimulating factor was associated with the development of antibodies against the recombinant protein. These antibodies were found to react against sites on the protein that are normally protected by O-linked carbohydrates (Gribben et al., Lancet, 1990, 335:434-437). Similarly, administration of E. coli derived-human IL-7 in humans induced antibodies against the recombinant protein (Rosenberg et al., J. Immunother., 2006, 29:313-319), whereas no anti-IL-7 antibodies were found using the glycosylated cytokine (CYT107) (Perales et al., Blood, 2012, 120:4882-4891). Immunogenicity of *E*. coli-derived single-chain human IL-15 has been reported in macaques where the development of anti-IL-15 antibodies was observed upon s.c. administration (Sneller et al., Blood, 2011, 118:6845-6848).

In this example, pharmacokinetic and pharmacodynamic properties of the purified glycosylated human IL-15 heterodimers were investigated in mice. In comparison to single-chain IL-15 produced both in *E. coli* and in human HEK-293 cells, IL-15/sIL-15Rα complexes showed a more prolonged serum half-life and were more bioactive on a molar basis (see data above). The superior bioactivity of IL-15 in the heterodimeric formulation is mainly the result of the presence of IL-15Rα contributing to increased stability of the protein in vivo. These properties offer the potential to allow lower and less frequent dosing and simpler delivery methods, with increased convenience for both patients and caregivers.

The crystal structures of the heterodimer IL-15/IL-15Ra and of the quaternary IL-15/IL-15Rα/IL-2Rβ-γc complex have been reported (Chirifu et al., 2007, Nat Immunol. 8:1001-1007; Ring et al., 2012, Nat Immunol 13:1187-1195). In these papers, the authors described in detail the amino acids and domains involved in the binding between subunits. Importantly, IL-15 has two distinct binding sites, site I for the binding to IL-2Rβ and site II for the binding to yc. In contrast, IL-15Rα does not contact IL-2Rβ, with a distance of > 15Å separating the subunits at their closest point. It has alsobeen reported that IL-15/IL-15Ra heterodimer binds to IL-2Rβ with an affinity approximately 150-fold greater than that of single-chain IL-15, suggesting that IL-15 stabilization is a major function of IL-15Rα (Ring et al., 2012, Nat Immunol 13:1187-1195).

The production and purification of IL-15 associated with the sushi domain of IL-15Rα linked to the Fc region of IgG1 has been previously reported (Han et al., Cytokine, 2011, 56:804-810). However, the authentically processed and glycosylated IL-15/sIL-15Rα, as produced and purified by the inventors and shown in this example, has the advantage to be the closest to the IL-15 produced in human body and circulating in plasma (Bergamaschi et al., Blood, 2012, 120:e1-8) and may be the least immunogenic form.

The availability of naturally cleaved purified sIL-15Rα has allowed an investigation into the processing and shedding of IL-15Rα from the cell surface. The data provided in this example demonstates the results of MALDI-TOF MS analysis, wherein protein sequencing and Mascot searches of protein sequence databases confidently identified the proteolytic cleavage site of membrane-bound IL-15Rα between Gly170 and His171 of the mature membrane-associated form of IL-15Rα (Fig. 20). The determination of the sequence corresponding to the cleavage site in IL-15Rα and the amino acid sequence of the mature sIL-15Rα by the inventors represent an important finding to determine regulation of the process. Dysregulated shedding of IL-15/IL-15Rα heterodimers from the cell surface may be one mechanism leading to the altered levels of circulating IL-15 upon certain conditions, i.e. lymphodepleting treatments (Bergamaschi et al., Blood, 2012, 120:e1-8; Dudley et al., J. Clin. Oncol., 2008, 26:5233-5239) and autoimmune diseases, such as celiac disease (DePaolo et al., Nature, 2011, 471:220-224), rheumatoid arthritis (Gonzalez-Alvaro et al., Clin. Exp. Rheumatol., 2003, 21:639-642) and multiple sclerosis (Rentzos et al., J. Neurol. Sci., 2006, 241:25-29).

In summary, the study presented in this example demonstrates that high-level production of authentically processed and glycosylated human IL-15/sIL-15Rα heterodimers is achievable in human cells and that RP-HPLC under non-reducing conditions allows the purification of biologically active heterodimers, avoiding protein refolding. Purified glycosylated IL-15 heterodimers have the advantage of increased stability and bioactivity in vivo and, thus, can be advantageously used for therapeutic applications.

### 6.5 Example 5. Study evaluating toxicity, plasma levels of IL-15 and immunological parameters after repeated subcutaneous injections of rhesus macaques with IL-15/sIL-15Ra.

This study was performed in order to evaluate toxicity, immunogenicity and effects on immune system homeostasis of human IL-15/sIL-15Ra after repeated injections. As outlined in Table 6, Group 1 included 8 rhesus macaques that received 6 s.c. injections of IL-15/sIL-15Ra at the dose of 5 µg/kg on day 0, 2, 4, 7, 9 and 11. Group 2 included 8 rhesus macaques that served as control (received 6 s.c. injections with saline solution on the same day). Both groups underwent a second treatment cycle identical to the first one. This second cycle was conducted 4 weeks after start of the first cycle. Eight macaques (4/group) were sacrificed at day 41/42 (immediately after the conclusion of the second treatment); the remaining macaques were sacrificed at day 73/74 (5 weeks after the last IL-15 heterodimer injection).

**Table 6. Study design to evaluate toxicity, immunogenicity and immunological effects of IL-15/IL-15Ra heterodimer after repeated s.c. injections.**

| Group | Treatment | Dose (µg/Kg/ injection) | Route of Administration | Duration of Treatment | Total Doses/ Cycle | # Cycles | # Rhesus Macaques |
|---|---|---|---|---|---|---|---|
| 1 | IL-15/ IL-15Ra | 5 | s.c. | 12 days (s.c. at 0, 2, 4, 7, 9 and 11) | 6 | 2 | 8 |
| 2 | Saline Solution | 5 | s.c. | 12 days (s.c. at 0, 2, 4, 7, 9 and 11) | 6 | 2 | 8 |

The animals included in the studies were examined at different time points for clinical toxicity, had chemistry and hematological laboratory analysis, and were evaluated for immunological parameters as consequence of IL-15/IL-15Ra administrations. Animals were also monitored for the following serum values: glucose, blood urea nitrogen, creatinine, total protein, albumin, bilirubin, alkaline phosphatase, alkaline transaminase, aminotransferase, cholesterol, calcium, phosphate, sodium, potassium, chloride, globulin, creatine phosphokinase, and for the following hematological parameters: hemoglobin, hematocrit, WBC, RBC, mean corpuscular volume, mean corpuscular hemoglobin, mean corpuscular hemoglobin concentration, platelets, neutrophils, lymphocytes, monocytes, eosinophils and basophils.

Macaques were sedated and received either IL-15/sIL-15Ra (5 µg/Kg) or saline solution in 0.5 ml saline solution s.c. Blood pressure and temperature were followed over time. No changes in blood pressure and animal temperature were observed. All animals were off anesthesia at 1 hour after injections and had normal recoveries. Blood was drawn at 0, 6, 8, 12 and 24 after the first and the last s.c. injections for each treatment and at 0, 6 and 24 hours after all other injections. The concentration of human IL-15 in macaque plasma was evaluated using a chemiluminescent immunoassay (Quantiglo Q1500B, R&D Systems), according to the manufacturer's recommendations. The results are presented in Figures 32 and 33. Figure 32 shows body temperature and mean arterial pressure in 8 macaques injected with IL-15 heterodimer v. controls. Figure 33 shows plasma IL-15 levels during the two treatments in 8 macaques injected with IL-15 heterodimer. As shown in Figure 32, at a dose of 5 µg/Kg there is practically no change in blood pressure or body temperature of macaques injected s.c. relative to controls. Analysis of the blood samples of the 8 macaques receiving IL-15/sIL-15Ra heterodimer showed that IL-15 plasma levels during the two treatments were similar, with the exception of the nadir value after the first injection, which was the only point statistically different between treatments one and two (Figure 33). During both treatments, the plasma IL-15 nadir value after the first injection was also higher than the nadir values observed after the subsequent injections, which were similar to normal levels. The decreasing nadir values can be attributed to the increased consumption of circulating IL-15 after repeated injections, which in turn reflects the dynamics of expansion of IL-15 target populations such as CD8+ T and NK cells. Interestingly, NK cells started to proliferate earlier during the second treatment (as early as day 2), suggesting that the NK cells may be in a different activation status regarding their ability to respond to IL-15.

The blood counts and lymphocytes subsets of all the animals were monitored and evaluated before, during and after IL-15 heterodimer administration. Samples of PBMC were taken from the macaques prior, during and after IL-15 heterodimer administration and were stained with antibodies binding to CD3, CD4, CD8 and CD16, and examined by flow cytometry. The results are presented in Figure 34. The data for CD3+CD8+ T cells (Figure 34A) and CD3-CD16+CD8+ NK cells (Figure 34B) are shown as the absolute cell number of each subset per µl of blood at the indicated time points. During the first treatment, IL-15 heterodimer administration resulted in a transient decrease in the absolute count of CD8+ T and CD8+ NK cells at day 2, followed by an IL-15-driven expansion of these subsets. The absolute counts of CD8+ T and CD8+ NK cells peaked at day 7, and declined to baseline levels by day 14. No significant changes were observed in the control animals, with the exception of a mild decrease in the CD8+ T cells count at day 2. During the second treatment, no changes in the absolute count of CD8+ T cells were observed, suggesting a possible migration to periphery in response to IL-15. The absolute counts of CD8+ NK cells increased at day 7 after initiation of the second treatment to levels similar to those obtained during the first treatment, but no lymphopenia was observed at day 2 during the second treatment. Figure 34 shows that repeated administration of IL-15 heterodimers expands NK and CD8 T cells.

The ratio of CD8/CD4 T cells in blood as well as in different tissues at the days of necropsies (day 41/42 and 73/74) were also measured. The results are presented in Figure 35. The CD8/CD4 ratio in blood of the IL-15 heterodimer treated macaques was similar to the one observed in the control animals suggesting that blood may be not the ideal place to look for biological effects induced by IL-15. Immediately after conclusion of the second set of six injections, IL-15 heterodimer treated macaques showed an inverted CD8/CD4 ratio in comparison to controls in both spleen and bone marrow, most likely due to the proliferation of CD8 cells. However, one month after the end of treatment, the ratio normalized in both tissues. Interestingly, inversion in the CD8/CD4 ratio was observed in inguinal lymph nodes and was maintained at both time points, in agreement with published data (Lugli et al. Blood 2010;116:3238).

The bioactivity of IL-15 was also evaluated through the analysis of proliferation of lymphocytes in blood and in tissues. Treatment with heterodimeric IL-15 induced a great expansion of CD8+, gammadelta TCR T cells and NK cells.

It can be concluded that a 12 s.c. injections of IL-15/sIL-15Ra at the dose of 5 µg/Kg is well tolerated in Macaques. The data indicate no accumulation of IL-15 and no evidence of additional toxicity upon repetition of a second 2 week administration.

### 6.6 Example 6. Study Evaluating toxicity, plasma levels of IL-15 and immunological parameters after subcutaneous injections of rhesus macaques with IL-15/sIL-15Ra at Escalated Doses.

This example describes a dose escalation study performed in order to evaluate toxicity, immunogenicity and effects on immune system homeostasis of human IL-15/sIL-15Ra after subcutaneous injections. As outlined in Table 7, Group 1 included 2 rhesus macaques that received 6 s.c. injections of IL-15/soluble IL-15Ra at the dose of 1 µg/kg on day 0, 2, 4, 7, 9 and 11. Group 2 included 2 rhesus macaques that received 6 s.c. injections of IL-15/sIL-15Ra at the dose of 20 µg/kg on day 0, 2, 4, 7, 9 and 11. Group 3 included 2 rhesus macaques that received 6 s.c. injections of IL-15/soluble IL-15Ra at the dose of 50 µg/Kg on day 0, 2, 4, 7, 9 and 11.

**Table 7. Study design to evaluate toxicity, immunogenicity and immunological effects of IL-15/IL-15Ra heterodimer after repeated s.c. injections.**

| Group | Treatment | Dose (µg/Kg/ injection) | Route of Administration | Duration of Treatment | Total Doses/ Cycle | # Cycles | # Rhesus Macaques |
|---|---|---|---|---|---|---|---|
| 1 | IL-15/ IL-15Ra | 1 | s.c. | 12 days (s.c. at 0, 2, 4, 7, 9 and 11) | 6 | 1 | 2 |
| 2 | IL-15/ IL-15Ra | 20 | s.c. | 12 days (s.c. at 0, 2, 4, 7, 9 and 11) | 6 | 1 | 2 |
| 3 | IL-15/ IL-15Ra | 50 | s.c. | 12 days (s.c. at 0, 2, 4, 7, 9 and 11) | 6 | 1 | 2 |

Six macaques were sedated and received IL-15/sIL-15Ra (2 animals/dose) in 0.5 ml saline solution (PBS) s.c. Blood pressure and temperature were followed over time. No changes in blood pressure and animal temperature were observed, with the following exception: one animal (P995) receiving 50 µg/kg had elevated temperature after the third injection, which reached 105 °F at 6 hours post-treatment and was still elevated at 104 °F after 24 hours. All animals were off anesthesia at 1 hour after injections and had normal recoveries.

Blood was drawn at 0, 6, 8, 12 and 24 hours after the first and the last s.c. injections and at 0 and 6 hours after the second, third and fourth s.c. injections. The concentration of human IL-15 in macaque plasma was evaluated using a colorimetric immunoassay (Quantikine human IL-15, R&D Systems), according to the manufacturer's recommendations. The results are presented in Table 8 and Figure 36. Figure 36 shows plasma IL-15 levels in 6 macaques injected with IL-15 heterodimer at escalated doses.

**Table 8. Levels of plasma IL-15 in 6 macaques injected with IL-15 heterodimer at escalated doses of 1 µg/kg, 20 µg/kg, and 50 µg/kg.**

| Plasma IL-15 (pg/ml) | | | | | | |
|---|---|---|---|---|---|---|
| Animal:hours | P942 50ug | P995 50ug | P990 20µg | P999 20µg | P950 1 µg | P994 1 µg |
| 0 | 9.1 | 8.6 | 8 | 9.8 | 8.2 | 8.2 |
| 6 | 6978 | 15320.6 | 3180.3 | 7427 | 53.2 | 58.2 |
| 8 | 8520.5 | 18173.6 | 2122.9 | 4742.5 | 39.1 | 40.1 |
| 12 | 7320.1 | 10657.4 | 1872.4 | 4395.8 | 25.4 | 36.7 |
| 24 | 4350 | 10828.5 | 1947.5 | 2266.5 | 27.1 | 44.3 |
| 48 | 1114.5 | 887.8 | 370.9 | 283.4 | 9.9 | 13.8 |
| 54 | 4530.4 | 14741.8 | 2872.1 | 12031.1 | 82.3 | 114.6 |
| 96 | 39 | 105.9 | 30.4 | 13.6 | 6.9 | 11.8 |
| 120 | 18.9 | 14.1 | 7.5 | 309.1 | 7.6 | 9.8 |
| 126 | 630 | 2285 | 619.6 | 4715.7 | 71.4 | 164 |
| 168 | 49.8 | 97.9 | 5.9 | 11.5 | 6.3 | 7.4 |
| 174 | 895.5 | 1597.5 | 376.9 | 1099.7 | 49.6 | 65.1 |
| 216 | 57.3 | 293.7 | 8.5 | 6.9 | 6.5 | 9.9 |
| 222 | 1746.2 | 2303 | 624.7 | 384.6 | 37.6 | 93.4 |
| 264 | 24.5 | 8.5 | 4.3 | 7.6 | 5 | 20.5 |
| 270 | 86.6 | 1400.6 | 1444.6 | 607.6 | 39.2 | 233 |
| 272 | 39.9 | 867.8 | 540.6 | 612.7 | 31.6 | 191 |
| 276 | 45.9 | 833.6 | 560.2 | 855.1 | 15.3 | 111.3 |
| 288 | 34.3 | 381.7 | 202.6 | 594.6 | 7.4 | 26.1 |
| 312 | 17.1 | 9.5 | | | 5.7 | |
| 336 | | | 3.7 | 6.2 | | 7.6 |

The blood counts and lymphocytes subsets of all the animals were monitored and evaluated before, during and after IL-15 heterodimer administration. Samples of PBMC were taken from the macaques prior, during and after IL-15 heterodimer administration and were stained with antibodies binding to CD3, CD4, CD8 and CD16, and examined by flow cytometry. The results are presented in Figure 37. All doses of IL-15 heterodimer resulted in a 4 to 8-fold increase in the absolute count of NK cells that peaked between day 7 and 14 after start of the treatment. These results suggest that NK cells respond to lower level of IL-15, and even a dose of 1 µg/kg is sufficient to induce a marked expansion. The absolute count of CD8+ T cell in peripheral blood also increased, but in a dose-dependent manner. The highest dose of IL-15 that resulted at peak plasma levels of more than 10 ng/ml showed a 10 fold increase in the absolute counts of CD8 T cells. Figure 37 shows the fold over baseline increase of NK cells and CD8 T cells in peripheral blood in 6 macaques injected with IL-15 heterodimer at escalated doses of 1 µg/kg, 20 µg/kg, and 50 µg/kg.

Animals were sacrificed at day 14 after the first injection (3 days after the last injection) and immunophenotypical analysis was performed in different tissues. The results are presented in Figure 38. Figure 38 shows dose-dependent proliferation of lymphocytes in different tissues upon IL-15 heterodimer s.c. administration. IL-15 heterodimer treatment resulted in great expansion of CD8, NK and gammadelta TCR T cells in lymph nodes, peripheral blood, liver and spleen. In all the tissue analyzed, all the lymphocyte subsets responded to IL-15 in a dose-dependent manner (Figure 38).

Dose escalation using 1, 5, 20 and 50 µg/kg s.c. of heterodimeric IL-15 showed that heterodimeric IL-15 is well tolerated in macaques and that no major side effects were observed. One of the two macaques that received the dose of 50 µg/kg of IL-15 heterodimer showed enlarged lymph nodes (axillary and mesenteric) at necropsy.

### 6.7 Example 7: Study Evaluating Efficacy of IL-15/IL-15Ra Complexes to Treat Melanoma

This example demonstrates the efficacy of IL-15/IL-15Ra complexes for the prevention and treatment of melanoma.

Methods: Mice (8-12 weeks old, female) were randomly distributed into three groups of ten animals. 10⁵ melanoma B16 cells in 0.2 ml PBS were injected IV. Treatments were performed at days 1, 6 and 11 by IP injections of PBS, 9 mcg purified human IL-15/soluble IL-15Ra (molar ratio 1:1), or 9 mcg of human IL-15 (*E.* Coli-derived). The mice were sacrificed at day 21 after tumor cell injection and scored for the presence of tumor nodules in the lungs.

Results: The PBS-treated mice developed numerous and large pulmonary melanoma masses, whereas IL-15-treated mice were characterized by a significantly reduced number of lung nodules (Figure 39, p=0.003 Anova). Different IL-15 formulations, as indicated in the graph, were administered IP at days 1, 6 and 11 after IV injection of 10⁵ melanoma B16 cells. The mice were sacrificed at day 21 after cell injection and scored for the presence of tumor nodules in the lungs. Data are shown in Figure 39, below. The results shown in Figure 39, above indicate a potential therapeutic value of IL-15/soluble IL-15Ra heterodimers to prevent tumor engraftment.

### 6.8 Example 8: Study Evaluating Efficacy of IL-15/IL-15Ra Complexes to Treat Colon Cancer

This example demonstrates the efficacy of IL-15/IL-15Ra complexes for the prevention and treatment of colon cancer.

An established mouse colon cancer model was used to study effects of IL-15/IL-15Ra on tumor therapy. Figure 40, below, shows that wild-type and IL-15 deficient (IL-15⁻/⁻) mice (C57BL/6) injected with MC38 colon carcinoma cells develop tumors in 20 days, with the IL-15 deficient mice developing tumors somewhat faster than the wild type. These results support a mechanism of tumor control at least partially dependent on IL-15.

The efficacy of two different IL-15 formulations, single chain *E. coli* derived IL-15 vs heterodimeric HEK293 cell derived IL-15/soluble IL-15Ra, in delaying tumor development using the above s.c. MC38 colon carcinoma mouse model was assessed.

Methods: Wild type C57BL/6 mice were injected with MC38 colon carcinoma cells SC, and treated with the different IL-15 forms one week later. Mice were given 10 daily IP injections of 3 mcg IL-15/dose.

Results: The data demonstrate the efficacy of single-chain IL-15 in delaying MC38 tumor growth. The data also demonstrates that IL-15/IL-15Ra heterodimers significantly reduce tumor burden in MC38 tumor-bearing mice (Figure 41). Similar results were obtained if the treatment was reduced to 5 daily IP injections at 3 mcg IL-15/dose. Possibly due to their different stability in mice, the IL-15 forms were associated with different toxicity. In particular, weight loss and cachexia were associated with more than 5 daily repeated administration of IL-15/soluble IL-15Rα. The weight loss was reversible, and mice recovered after interruption of IL-15 treatment.

The results shown in Figure 41 indicate a potential therapeutic benefit of IL-15/soluble IL-15Ra heterodimers to retard tumor growth. The route of administration (10 daily IP injections) and dose 3 mcg per mouse (equivalent to 150 mcg/kg) were significant factors in the weight loss.

### 6.9 Example 9. A Phase 1 Study of Subcutaneous Recombinant Human IL-15/sIL15Ra in Adults with Metastatic Cancers

This example describes a study to determine the safety, toxicity profile, dose-limiting toxicity (DLT) and maximum tolerated dose (MTD) of s.c. heterodimeric IL-15 (recombinant heterodimeric IL-15/sIL-15Ra produced in human HEK293 cells) administered to human patients with metastatic unresectable cancers for which curative or palliative measures either do not exist or are not associated with a survival advantage. The study may also: (i) determine the pharmacokinetics of the heterodimer IL-15; (ii) characterize the biological effects of the heterodimeric IL-15 on the percentages and absolute numbers of circulating lymphocyte sets and T-cell subsets (including naive, central and/or effector memory subsets) based on the expression of markers, such as CD56, CD4, CD8, CD45RO, CD45RA, CD28, CD95, CCR7 and CD62L, by flow cytometry; (iii) characterize the plasma levels of pro-inflammatory cytokines; (iv) evaluate the potential antitumor activity of the s.c. heterodimer IL15 by assessing, e.g., the clinical response rate and the time to progression; and/or (v) assess the nature of the T-cell infiltration and immunologic gene expression by, e.g., analysis of pre- and post-treatment fine needle biopsies obtained from patients with easily accessible tumor deposits.

The human patients selected for inclusion in the study meet all of the following criteria:
- Age ≥ 18 years.
- Patients have histologically confirmed (by the NCI Pathology Department) solid tumor malignancy that is metastatic or unresectable and for which standard curative or palliative measures do not exist or are associated with minimal patient survival benefit (as defined by the patient and/or the study physicians). Inclusion of patients having tumors that can be safely biopsied is encouraged.

In addition, the human patients selected for inclusion in the study may also meet one or more, or all of the following criteria:
- Patients have evaluable or measurable disease, defined as at least one lesion that can be accurately measured in at least one dimension (longest diameter to be recorded for non-nodal lesions and short axis for nodal lesions) as ≥20 mm with conventional techniques or as ≥10 mm with spiral CT scan.
- Patients have recovered to < grade 1 CTCAEv4 from toxicity of prior chemotherapy or biologic therapy and must not have had prior chemotherapy or biologic therapy within 4 weeks (6 weeks for nitrosoureas or mitomycin C, 8 weeks for UCN-01).
- It is at least 1 month since the patient has received any prior radiation or major surgery.
- DLCO/VA and FEV-1.0 > 50% of predicted on pulmonary function tests.
- Serum creatinine of ≤ 1.5 X the upper limit of normal.
- AST and ALT < 2.5 x the upper limit of normal.
- Absolute neutrophil count ≥ 1,500/mm³ and platelets ≥ 100,000/mm³.
- Karnofsky performance status ≥ 70% or ECOG ≤ 1
- CNS metastases: Patients who remain asymptomatic after successful definitive treatment of brain metastases (i.e., surgical resection, curative whole brain irradiation, stereotactic radiation therapy, or a combination of these) demonstrating stable or improved radiographic appearance on MRI scan at least 3 months after completion of treatment with no signs of cerebral edema are eligible.

Patients that meet one or more, or all of the following criteria may not be selected as patients for the study:
- Patients who have received any systemic corticosteroid therapy within 3 weeks prior to the start of the study.
- Patients who have received any cytotoxic therapy, immunotherapy, antitumor vaccines or monoclonal antibodies in the 4 weeks prior to the start of the study.
- Patients with a life expectancy of less than 3 months.
- Patients with documented HIV, active bacterial infections, active or chronic hepatitis B, hepatitis C or HTLV-I infection.
- Patients with a positive hepatitis B serology indicative of previous immunization (i.e., HBsAb positive and HBc Ab negative), or a fully resolved acute hepatitis B infection is not an exclusion criterion.
- Patients with a positive hepatitis C serology.
- Patients receiving concurrent anticancer therapy (including, e.g., immunotherapy, immunosuppressive therapy, radiation therapy, chemotherapy, systemic corticosteroids, and other investigational agents) with the exception of hormone therapy for prostate cancer.
- Patients with a history of severe asthma or presently on chronic inhaled corticosteroid medications.
- Patients with a history of autoimmune disease, with the exception of an autoimmune event associated with prior ipilimumab (anti-CTLA-4) therapy that has been completely resolved for more than 4 weeks.
- Patients inability or refusal to practice effective contraception during therapy or the presence of pregnancy or active breastfeeding.

Table 9 shows the planed dose levels of heterodimeric IL-15 for this study. Patients are assigned to a dose level sequentially based on their order of entry into the study. Groups of 3 to 6 patients receive 12 s.c. injections of heterodimeric IL-15 over a six week period (Mon/Wed/Fri for weeks 1,2, 5 and 6). The starting dose is 0.1 µg/kg/day, and in the absence of significant toxicities, dose escalation proceeds to evaluate dose levels of 0.25, 0.5, 1 and 2 µg/kg/day. The treatment extends for 72 days (6 weeks of heterodimeric IL-15 therapy, plus 30 day observation), with an additional 7 days allowed for reasons other than recovery for treatment-related toxicities.

Patients without evidence of an ongoing response after the first six week cycle of treatment discontinue heterodimeric IL-15 injection and are followed until disease progression is documented or they go off study for another reason. (Ongoing response is defined as: >15% decrease in sum of marker lesions and/or improvement or disappearance of some non measurable lesions and/or >10% decrease in tumor markers). Patients who demonstrate a complete response (CR) to treatment receive 2 additional cycles after the cycle where the CR is first documented, at the same dose of heterodimeric IL-15. Depending on the safety and toxicity results, doses higher than 2 µg/kg/day may be considered in subsequent trials after evaluation of pharmacokinetics and pharmacodynamic data.

**Table 9. Planned Dose Levels of Heterodimeric IL-15**

| **Patient Cohort** | **Number of Patients*** | **Dose of SC heterodimeric IL-15 (µg/kg/day x 12 over 6 weeks)** |
|---|---|---|
| 1 | 3 to 6 | 0.1 |
| 2 | 3 to 6 | 0.25 |
| 3 | 3 to 6 | 0.5 |
| 4 | 3 to 6 | 1 |
| 5 | 3 to 6 | 2 |

| | | |
|---|---|---|
| *Nine total patients are treated at the MTD (or highest dose, if an MTD is not reached). | | |

Samples for correlative studies are obtained prior to treatment and at specific times points during and after treatment to assess pharmacokinetics of heterodimeric IL-15, the effect of heterodimeric IL-15 on immune cell subset populations and pro-inflammatory cytokine levels in the peripheral blood and for the development of neutralizing anti-IL-15 or anti-IL-15 Receptor alpha antibodies.

## Claims

1. An IL-15/IL-15 receptor alpha (IL-15Ra) complex for use in treating cancer in a subject in need thereof, comprising administering the IL-15/IL-15Ra complex to the subject using cyclical administration regimen, wherein the cyclical administration regimen comprises: (a) administering subcutaneously to the subject a dose of 0.1 to 10 µg/kg of the IL-15/IL-15Ra complex every 1, 2 or 3 days over a first period of 1 week to 3 weeks; and (b) after a second period of 1 week to 2 months in which no IL-15/IL-15Ra complex is administered to the subject, administering subcutaneously to the subject a dose of 0.1 to 10 µg/kg of the IL-15/IL-15Ra complex every 1, 2 or 3 days over a third period of 1 week to 3 weeks.

2. The IL-15/IL-15Ra complex for use according to claim 1, wherein the cancer is melanoma, renal cell carcinoma, non-small cell lung cancer or colon cancer and/or is a metastatic cancer.

3. The IL-15/IL-15Ra complex for use according to claim 1 or claim 2, wherein the dose of the IL-15/IL-15Ra complex administered over the first period and over the third period is 0.1 µg/kg, 0.25 µg/kg, 0.5 µg/kg, 1 µg/kg, 2 µg/kg or 5 µg/kg.

4. The IL-15/IL-15Ra complex for use according to any one of claims 1-3, wherein the first period, the second period, and/or the third period is 12 to 14 days.

5. The IL-15/IL-15Ra complex for use according to any one of claims 1-4, wherein the cyclical administration regimen is repeated at least 5 or at least 10 times.

6. The IL-15/IL-15Ra complex for use according to any one of claims 1-4, wherein the cyclical administration regimen is repeated for at least 6 months or at least 1 year.

7. The IL-15/IL-15Ra complex for use according to any one of claims 1-6, wherein the IL-15/IL-15Ra complex is a heterodimeric complex of native IL-15 and native soluble IL-15Ra.

8. The IL-15/IL-15Ra complex for use according to any one of claims 1-6, wherein the IL-15 is human IL-15, and wherein IL-15Ra is a soluble form of human IL-15Ra.

9. The IL-15/IL-15Ra complex for use according to any one of claims 1-6, wherein IL-15 comprises amino acid residues 49 to 162 of the amino acid sequence of SEQ ID NO: 1; and IL-15Ra comprises the amino acid sequence of SEQ ID NO: 33, 35, 37, 39, 41 or 45.

10. The IL-15/IL-15Ra complex for use according to any one of claims 1-6, wherein IL-15 comprises amino acid residues 49 to 162 of the amino acid sequence of SEQ ID NO: 1; and IL-15Ra comprises the amino acid sequence of SEQ ID NO: 33; or the IL-15Ra is glycosylated such that glycosylation accounts for at least or more than 20%, 30%, 40% or 50% of the mass of the IL-15Ra.

11. The IL-15/IL-15Ra complex for use according to any one of claims 1-10 which further comprises administering another therapy, wherein the other therapy is an antibody that immunospecifically binds to PD-1 or an antibody that immunospecifically binds to PD-L1.

## Patentansprüche

1. IL-15/IL-15-Rezeptor-alpha(IL-15Ra)-Komplex zur Verwendung bei der Behandlung von Krebs bei einem diese benötigenden Individuum, umfassend Verabreichen des IL-15/IL-15Ra-Komplexes an das Individuum unter Verwendung eines zyklischen Verabreichungsplans, wobei der zyklische Verabreichungsplan Folgendes umfasst: (a) subkutanes Verabreichen einer Dosis von 0,1 bis 10 µg/kg IL-15/IL-15Ra-Komplex an das Individuum alle 1, 2 oder 3 Tage über einen ersten Zeitraum von 1 Woche bis 3 Wochen; und (b) nach einem zweiten Zeitraum von 1 Woche bis 2 Monaten, in dem dem Individuum kein IL-15/IL-15Ra-Komplex verabreicht wird, subkutanes Verabreichen einer Dosis von 0,1 bis 10 µg/kg IL-15/IL-15Ra-Komplex an das Individuum alle 1, 2 oder 3 Tage über einen dritten Zeitraum von 1 Woche bis 3 Wochen.

2. IL-15/IL-15Ra-Komplex zur Verwendung nach Anspruch 1, wobei es sich bei dem Krebs um Melanom, Nierenzellkarzinom, nichtkleinzelligen Lungenkrebs oder Kolonkarzinom und/oder um einen metastasierten Krebs handelt.

3. IL-15/IL-15Ra-Komplex zur Verwendung nach Anspruch 1 oder Anspruch 2, wobei die über den ersten Zeitraum und über den dritten Zeitraum verabreichte Dosis des IL-15/IL-15Ra-Komplexes 0,1 µg/kg, 0,25 µg/kg, 0,5 µg/kg, 1 µg/kg, 2 µg/kg oder 5 µg/kg beträgt.

4. IL-15/IL-15Ra-Komplex zur Verwendung nach einem der Ansprüche 1-3, wobei der erste Zeitraum, der zweite Zeitraum und/oder der dritte Zeitraum 12 bis 14 Tage lang ist.

5. IL-15/IL-15Ra-Komplex zur Verwendung nach einem der Ansprüche 1-4, wobei der zyklische Verabreichungsplan wenigstens 5- oder wenigstens 10-mal wiederholt wird.

6. IL-15/IL-15Ra-Komplex zur Verwendung nach einem der Ansprüche 1-4, wobei der zyklische Verabreichungsplan über wenigstens 6 Monate oder wenigstens 1 Jahr wiederholt wird.

7. IL-15/IL-15Ra-Komplex zur Verwendung nach einem der Ansprüche 1-6, wobei es sich bei dem IL-15/IL-15Ra-Komplex um einen heterodimeren Komplex von nativem IL-15 und nativem löslichen IL-15Ra handelt.

8. IL-15/IL-15Ra-Komplex zur Verwendung nach einem der Ansprüche 1-6, wobei es sich bei dem IL-15 um menschliches IL-15 handelt und wobei es sich bei IL-15Ra um eine lösliche Form von menschlichem IL-15Ra handelt.

9. IL-15/IL-15Ra-Komplex zur Verwendung nach einem der Ansprüche 1-6, wobei IL-15 Aminosäurereste 49 bis 162 der Aminosäuresequenz unter SEQ ID NO: 1 umfasst; und IL-15Ra die Aminosäuresequenz unter SEQ ID NO: 33, 35, 37, 39, 41 oder 45 umfasst.

10. IL-15/IL-15Ra-Komplex zur Verwendung nach einem der Ansprüche 1-6, wobei IL-15 Aminosäurereste 49 bis 162 der Aminosäuresequenz unter SEQ ID NO: 1 umfasst; und IL-15Ra die Aminosäuresequenz unter SEQ ID NO: 33 umfasst; oder der IL-15Ra glykosyliert ist, so dass die Glykosylierung wenigstens oder mehr als 20%, 30%, 40% oder 50% der Masse des IL-15Ra ausmacht.

11. IL-15/IL-15Ra-Komplex zur Verwendung nach einem der Ansprüche 1-10, die ferner das Verabreichen einer anderen Therapie umfasst, wobei es sich bei der anderen Therapie um einen Antikörper, der an PD-1 immunspezifisch bindet, oder einen Antikörper, der an PD-L1 immunspezifisch bindet, handelt.

## Revendications

1. Complexe IL-15 (interleukine 15)/récepteur alpha de l'IL-15 (IL-15Ra) pour une utilisation dans le traitement d'un cancer chez un sujet en ayant besoin, comprenant l'administration du complexe IL-15/IL-15Ra au sujet selon un régime d'administration cyclique, le régime d'administration cyclique comprenant : (a) l'administration sous-cutanée au sujet d'une dose de 0,1 jusqu'à 10 µg/kg du complexe IL-15/IL-15Ra tous les jours, tous les 2 jours ou tous les 3 jours pendant une première période d'1 semaine jusqu'à 3 semaines ; et (b) après une deuxième période d'1 semaine jusqu'à 2 mois sans administration de complexe IL-15/IL-15Ra au sujet, l'administration sous-cutanée au sujet d'une dose de 0,1 jusqu'à 10 µg/kg du complexe IL-15/IL-15Ra tous les jours, tous les 2 jours ou tous les 3 jours pendant une troisième période d'1 semaine jusqu'à 3 semaines.

2. Complexe IL-15/IL-15Ra pour une utilisation selon la revendication 1, le cancer étant un mélanome, un carcinome des cellules rénales, un cancer du poumon non à petites cellules ou un cancer du côlon et/ou un cancer métastasique.

3. Complexe IL-15/IL-15Ra pour une utilisation selon la revendication 1 ou la revendication 2, la dose de complexe IL-15/IL-15Ra administrée pendant la première période et pendant la troisième période étant de 0,1 µg/kg, 0,25 µg/kg, 0,5 µg/kg, 1 µg/kg, 2 µg/kg ou 5 µg/kg.

4. Complexe IL-15/IL-15Ra pour une utilisation selon l'une quelconque des revendications 1-3, la première période, la deuxième période et/ou la troisième période étant de 12 jusqu'à 14 jours.

5. Complexe IL-15/IL-15Ra pour une utilisation selon l'une quelconque des revendications 1-4, le régime d'administration cyclique étant répété au moins 5 ou au moins 10 fois.

6. Complexe IL-15/IL-15Ra pour une utilisation selon l'une quelconque des revendications 1-4, le régime d'administration cyclique étant répété pendant au moins 6 mois ou pendant au moins 1 an.

7. Complexe IL-15/IL-15Ra pour une utilisation selon l'une quelconque des revendications 1-6, le complexe IL-15/IL-15Ra étant un complexe hétérodimérique d'IL-15 native et de IL-15Ra soluble natif.

8. Complexe IL-15/IL-15Ra pour une utilisation selon l'une quelconque des revendications 1-6, l'IL-15 étant une IL-15 humaine et IL-15Ra étant une forme soluble de l'IL-15Ra humain.

9. Complexe IL-15/IL-15Ra pour une utilisation selon l'une quelconque des revendications 1-6, l'IL-15 comprenant les résidus d'acides aminés 49 jusqu'à 162 de la séquence d'acides aminés de SEQ ID NO: 1 ; et le IL-15Ra comprenant la séquence d'acides aminés de SEQ ID NO: 33, 35, 37, 39, 41 ou 45.

10. Complexe IL-15/IL-15Ra pour une utilisation selon l'une quelconque des revendications 1-6, l'IL-15 comprenant les résidus d'acides aminés 49 jusqu'à 162 de la séquence d'acides aminés de SEQ ID NO: 1 ; et le IL-15Ra comprenant la séquence d'acides aminés de SEQ ID NO: 33 ; ou le IL-15Ra étant glycosylé de sorte que la glycosylation représente au moins ou représente plus de 20%, de 30%, de 40% ou de 50% du poids du IL-15Ra.

11. Complexe IL-15/IL-15Ra pour une utilisation selon l'une quelconque des revendications 1-10, comprenant en outre l'administration d'une autre thérapie, l'autre thérapie étant un anticorps qui se lie de manière immunospécifique à la protéine PD-1 ou étant un anticorps qui se lie de manière immunospécifique au ligand PD-L1.
